# EUROPEAN PATENT APPLICATION

(11) **EP 2 631 303 A1**
(43) Date of publication of application: **28.08.2013**
(21) Application number: 13168332.8
(22) Date of filing: 25.09.2009
(51) Int. Cl.: C12Q 1/68, C12N 15/11

(54) **Genetic markers for assessing risk of developing Schizophrenia**

(30) Priority: 25.09.2008 US 100176 P
(62) Divisional of application: 09816956.8
(71) Applicant: SureGene LLC, Louisville, KY 40299 (US)
(72) Inventor: Brennan, Mark David, Jeffersonville, IN Indiana 47130 (US); Ramsey, Timothy Lynn, Shelbyville, KY Kentucky 40065 (US)
(74) Representative: Coehn, Markus

(57) **Abstract**

This document provides methods and materials related to genetic markers of schizophrenia (SZ). For example, this document provides methods for using such genetic markers to assess risk of developing schizophrenia.

## Description

### CLAIM OF PRIORITY

This application claims the benefit of U.S. Provisional Application Serial No. 61/100,176, filed on September 25, 2008, which is incorporated by reference in its entirety herein.

### FEDERALLY SPONSORED RESEARCH OR DEVELOPMENT

The U.S. Government has certain rights in this invention pursuant to Grant Nos. R43 MH078437, N01 MH900001, and MH074027, awarded by the National Institutes of Health.

### TECHNICAL FIELD

This invention relates to genetic markers of schizophrenia (SZ). For example, methods of using such genetic markers for assessing risk of developing SZ are provided.

### BACKGROUND

The schizophrenia spectrum disorders include schizophrenia (SZ), schizotypal personality disorder (SPD), and/or schizoaffective disorder (SD). Schizophrenia (SZ) is considered a clinical syndrome, and is probably a constellation of several pathologies. Substantial heterogeneity is seen between cases, which is thought to reflect multiple overlapping etiologic factors, including both genetic and environmental contributions. SD is characterized by the presence of affective (depressive or manic) symptoms and schizophrenic symptoms within the same, uninterrupted episode of illness. SPD is characterized by a pervasive pattern of social and interpersonal deficits marked by acute discomfort with, and reduced capacity for, close relationships as well as by cognitive or perceptual distortions and eccentricities of behavior, beginning by early adulthood and present in a variety of contexts.

Various genes and chromosomes have been implicated in etiology of SZ. Many studies have suggested the presence of one or more important genes relating to SZ on most or all of the autosomes (Williams et al., Hum. Mol. Genet. 8:1729-1739 (1999); Fallin et al., Am. J. Hum. Genet. 77:918-936 (2005); ; Badner et al., Mol. Psychiatry 7:405-411 (2002); Cooper-Casey et al., Mol. Psychiatry 10:651-656 (2005); Devlin et al., Mol. Psychiatry 7:689-694 (2002); Fallin et al., Am. J. Hum. Genet. 73:601-611 (2003); Jablensky, Mol. Psychiatry 11: 815-836 (2006);

Kirov et al., J. Clin. Invest. 115:1440-1448 (2005); Norton et al., Curr. Opin. Psychiatry 19:158-164 (2006); Owen et al., Mol. Psychiatry 9:14-27 (2004)). However, none of these prior studies have used high resolution genetic association methods to systematically compare genes involved in SZ. Neither have any of these studies demonstrated that genetic polymorphisms in the genes defined herein are important, in particular in the genetic etiology of SZ.

Due to the severity of these disorders, especially the negative impact of a psychotic episode on a patient, and the diminishing recovery after each psychotic episode, there is a need to more conclusively identify individuals who have or are at risk of developing schizophrenia (SZ), schizotypal personality disorder (SPD), or schizoaffective disorder (SD), for example, to confirm clinical diagnoses, to allow for prophylactic therapies, to determine appropriate therapies based on their genotypic subtype, and to provide genetic counseling for prospective parents with a history of the disorder.

### SUMMARY

This disclosure provides methods for determining a subject's risk of developing schizophrenia based on detecting variations in genes involved in a number of pathways including: glutamate signaling and metabolism, cell adhesion, cytoskeletal architecture, vesicle formation, and trafficking, G-protein coupled receptors, carrier proteins and transporters, cell cycle modulators, neuronal development, calcium/calmodulin signaling, neuropeptide signaling, and several additional genes identified by virtue of their interaction with genes in high impact pathways and their expression in the central nervous system. This disclosure also provides methods and materials relating to determining the genetic risk of developing an SSD (e.g., SZ). For example, the allelic and genotypic variants identified as described herein can be used for assessing genetic risk. Specifically, the invention includes methods for assessing genetic risk based on evaluation of 1025 exemplary SNPs relating to SZ risk. Additionally, the specific SNPs can be used to capture copy number variation related to SZ risk. The allelic and genotypic variants thus identified can be used for assessing genetic risk.

In one aspect, this document features methods for determining risk of developing schizophrenia (SZ) in a human subject. Methods can include determining the identity of at least one allele of a single nucleotide polymorphism (SNP) listed in Table 1 (e.g., at nucleotide 31 of SEQ ID NO:656, 665, 248, 639, 25, 494, 700, or 691); and comparing the identity of the allele in the subject with a reference allele, wherein the reference allele is associated with a known risk of developing SZ; and wherein the presence of an allele in the subject that is the same as the reference allele that is associated with the known risk of developing SZ indicates the risk that the subject will develop SZ.

In another aspect, this document features methods for determining risk of developing SZ in a human subject. Methods can include: determining the copy number of at least one single nucleotide polymorphism (SNP) listed in Table 2 (e.g, at nucleotide 31 of SEQ ID NO:665, 314, 679, 597, 523, 619, 402) or Table 3 (e.g., at nucleotide 31 of SEQ ID NO:677, 586, 558, 664, 87, 851, or 500); and comparing the copy number of the SNP in the subject with a reference copy number, wherein the reference copy number is associated with a known risk of developing SZ; and wherein the presence of a copy number of the SNP in the subject that is the same as a reference copy number that is associated with SZ indicates that the risk that the subject will develop SZ.

Determining the identity or copy number of an allele can include obtaining a sample comprising DNA from the subject, and determining identity or copy number of the nucleotide at the polymorphic site. Determining the identity of the nucleotide can include contacting the sample with a probe specific for a selected allele of the polymorphism, and detecting the formation of complexes between the probe and the selected allele of the polymorphism, wherein the formation of complexes between the probe and the test marker indicates the presence of the selected allele in the sample. Determining the identity of an allele can include determining the identity of the nucleotide at position 31 of one of SEQ ID NOs: 1-1025. Determining the copy number of an allele can include contacting the sample with a probe specific for a selected allele, detecting the formation of complexes between the probe and the selected allele of the polymorphism, and quantifying the complexes, wherein the quantification of the complexes indicates the copy number of the selected allele in the sample. A probe can include a fluorescent label, and quantifying the complexes comprises detecting intensity of emission from the label. Determining the copy number of an allele can include detecting the absence or duplication of the allele in the subject. A subject can be a patient having or suspected of having SZ. A subject can have one or more risk factors associated with SZ. Risk factors associated with SZ can include one or more of: a relative afflicted with SZ; and a genetically based phenotypic trait associated with risk for a SZ. A subject can have exhibited or exhibits symptoms of psychosis. Methods can further include selecting or excluding a subject for enrollment in a clinical trial based on the identity of the allele. Methods can further include stratifying a subject population for analysis of a clinical trial based on the identity of the allele in the subjects. Methods can further include confirming a diagnosis of a SZ using psychometric instruments. Methods can further include selecting a treatment for SZ if an allele in the subject is the same as a reference allele associated with SZ. Methods can further include selecting a treatment for SZ if the copy number of the allele in the subject is the same as a reference copy number associated with SZ. Methods can further include administering the selected treatment to the subject. A treatment can include one or more of an anti-psychotic drug, an anti-depressant drug, anti-anxiety drug, mood stabilizer, selective serotonin reuptake inhibitor (SSRI), psychotherapy, or a stimulant. Methods can further include recording the identity of the allele in a tangible medium. A tangible medium can include a computer-readable disk, a solid state memory device, or an optical storage device.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DETAILED DESCRIPTION

This document provides methods for assessing genetic risk for developing SSDs based on evaluation of single nucleotide polymorphisms (SNPs) for genes relating to SSDs including schizophrenia (SZ), schizotypal personality disorder (SPD), and schizoaffective disorder (SD). As described herein, bioinformatic and genetic analyses provided evidence of association of the disclosed SNPs and haplotypes SZ. Specific allelic and copy number variants identified herein can be used to assess genetic risk.

### Definitions

As used herein, an "allele" is one of a pair or series of genetic variants of a polymorphism at a specific genomic location. An "SZ risk allele" is an allele that is associated with increased risk of developing SZ. An "SZ allele" is an allele that is statistically associated with a diagnosis of SZ.

As used herein, "genotype" refers to the diploid combination of alleles for a given genetic polymorphism. A homozygous subject carries two copies of the same allele and a heterozygous subject carries two different alleles.

As used herein, "genetic model" refers to the manner in which an allele influences risk or differential diagnosis. In a "dominant model" the allele impacts the clinical state to the same extent whether present in one copy or two copies, i.e., whether homozygous or heterozygous. In a "recessive model" the allele impacts the clinical state only when homozygous. In an "additive model" the allele impacts the clinical state in proportion to the number of copies present, i.e., the homozygous state has twice the impact of the heterozygous state.

As used herein, a "haplotype" is one or a set of signature genetic changes (polymorphisms) that are normally grouped closely together on the DNA strand, and are usually inherited as a group; the polymorphisms are also referred to herein as "markers." A "haplotype" as used herein is information regarding the presence or absence of one or more genetic markers in a given chromosomal region in a subject. A haplotype can consist of a variety of genetic markers, including indels (insertions or deletions of the DNA at particular locations on the chromosome); single nucleotide polymorphisms (SNPs) in which a particular nucleotide is changed; microsatellites; and minisatellites.

Microsatellites (sometimes referred to as a variable number of tandem repeats or VNTRs) are short segments of DNA that have a repeated sequence, usually about 2 to 5 nucleotides long (e.g., CACACA), that tend to occur in non-coding DNA. Changes in the microsatellites sometimes occur during the genetic recombination of sexual reproduction, increasing or decreasing the number of repeats found at an allele, changing the length of the allele. Microsatellite markers are stable, polymorphic, easily analyzed and occur regularly throughout the genome, making them especially suitable for genetic analysis.

"Copy number variation" (CNV), as used herein, refers to variation from the normal diploid condition for a gene or polymorphism. Individual segments of human chromosomes can be deleted or duplicated such that the subject's two chromosome carry fewer than two copies of the gene or polymorphism (a deletion or deficiency) or two or more copies (a duplication).

"Linkage disequilibrium" refers to when the observed frequencies of haplotypes in a population does not agree with haplotype frequencies predicted by multiplying together the frequency of individual genetic markers in each haplotype.

The term "chromosome" as used herein refers to a gene carrier of a cell that is derived from chromatin and comprises DNA and protein components (e.g., histones). The conventional internationally recognized individual human genome chromosome numbering identification system is employed herein. The size of an individual chromosome can vary from one type to another with a given multi-chromosomal genome and from one genome to another. In the case of the human genome, the entire DNA mass of a given chromosome is usually greater than about 100,000,000 base pairs. For example, the size of the entire human genome is about 3 X 10⁹ base pairs.

The term "gene" refers to a DNA sequence in a chromosome that codes for a product (either RNA or its translation product, a polypeptide). A gene contains a coding region and includes regions preceding and following the coding region (termed respectively "leader" and "trailer"). The coding region is comprised of a plurality of coding segments ("exons") and intervening sequences ("introns") between individual coding segments.

The term "probe" refers to an oligonucleotide. A probe can be single stranded at the time of hybridization to a target. As used herein, probes include primers, i.e., oligonucleotides that can be used to prime a reaction, e.g., a PCR reaction.

The term "label" or "label containing moiety" refers in a moiety capable of detection, such as a radioactive isotope or group containing same, and nonisotopic labels, such as enzymes, biotin, avidin, streptavidin, digoxygenin, luminescent agents, dyes, haptens, and the like. Luminescent agents, depending upon the source of exciting energy, can be classified as radioluminescent, chemiluminescent, bioluminescent, and photoluminescent (including fluorescent and phosphorescent). A probe described herein can be bound, e.g., chemically bound to label-containing moieties or can be suitable to be so bound. The probe can be directly or indirectly labeled.

The term "direct label probe" (or "directly labeled probe") refers to a nucleic acid probe whose label after hybrid formation with a target is detectable without further reactive processing of hybrid. The term "indirect label probe" (or "indirectly labeled probe") refers to a nucleic acid probe whose label after hybrid formation with a target is further reacted in subsequent processing with one or more reagents to associate therewith one or more moieties that finally result in a detectable entity.

The terms "target," "DNA target," or "DNA target region" refers to a nucleotide sequence that occurs at a specific chromosomal location. Each such sequence or portion is preferably at least partially, single stranded (e.g., denatured) at the time of hybridization. When the target nucleotide sequences are located only in a single region or fraction of a given chromosome, the term "target region" is sometimes used. Targets for hybridization can be derived from specimens which include, but are not limited to, chromosomes or regions of chromosomes in normal, diseased or malignant human cells, either interphase or at any state of meiosis or mitosis, and either extracted or derived from living or postmortem tissues, organs or fluids; germinal cells including sperm and egg cells, or cells from zygotes, fetuses, or embryos, or chorionic or amniotic cells, or cells from any other germinating body; cells grown in vitro, from either long-term or short-term culture, and either normal, immortalized or transformed; inter- or intraspecific hybrids of different types of cells or differentiation states of these cells; individual chromosomes or portions of chromosomes, or translocated, deleted or other damaged chromosomes, isolated by any of a number of means known to those with skill in the art, including libraries of such chromosomes cloned and propagated in prokaryotic or other cloning vectors, or amplified in vitro by means well known to those with skill; or any forensic material, including but not limited to blood, or other samples.

The term "hybrid" refers to the product of a hybridization procedure between a probe and a target.

The term "hybridizing conditions" has general reference to the combinations of conditions that are employable in a given hybridization procedure to produce hybrids, such conditions typically involving controlled temperature, liquid phase, and contact between a probe (or probe composition) and a target. Conveniently and preferably, at least one denaturation step precedes a step wherein a probe or probe composition is contacted with a target. Guidance for performing hybridization reactions can be found in Ausubel et al., Current Protocols in Molecular Biology, John Wiley & Sons, N.Y. (2003), 6.3.1-6.3.6. Aqueous and nonaqueous methods are described in that reference and either can be used. Hybridization conditions referred to herein are a 50% formamide, 2X SSC wash for 10 minutes at 45°C followed by a 2X SSC wash for 10 minutes at 37°C.

Calculations of "identity" between two sequences can be performed as follows. The sequences are aligned for optimal comparison purposes (e.g., gaps can be introduced in one or both of a first and a second nucleic acid sequence for optimal alignment and non-identical sequences can be disregarded for comparison purposes). The length of a sequence aligned for comparison purposes is at least 30% (e.g., at least 40%, 50%, 60%, 70%, 80%, 90% or 100%) of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In some embodiments, the percent identity between two nucleotide sequences is determined using the GAP program in the GCG software package, using a Blossum 62 scoring matrix with a gap penalty of 12, a gap extend penalty of 4, and a frameshift gap penalty of 5.

As used herein, the term "substantially identical" is used to refer to a first nucleotide sequence that contains a sufficient number of identical nucleotides to a second nucleotide sequence such that the first and second nucleotide sequences have similar activities. Nucleotide sequences that are substantially identical are at least 80% (e.g., 85%, 90%, 95%, 97% or more) identical.

The term "nonspecific binding DNA" refers to DNA which is complementary to DNA segments of a probe, which DNA occurs in at least one other position in a genome, outside of a selected chromosomal target region within that genome. An example of nonspecific binding DNA comprises a class of DNA repeated segments whose members commonly occur in more than one chromosome or chromosome region. Such common repetitive segments tend to hybridize to a greater extent than other DNA segments that are present in probe composition.

As used herein, the term "stratification" refers to the creation of a distinction between subjects on the basis of a characteristic or characteristics of the subjects. Generally, in the context of clinical trials, the distinction is used to distinguish responses or effects in different sets of patients distinguished according to the stratification parameters. In some embodiments, stratification includes distinction of subject groups based on the presence or absence of particular markers or haplotypes described herein. The stratification can be performed, e.g., in the course of analysis, or can be used in creation of distinct groups or in other ways.

### Methods of Diagnoses and Evaluation of Risk

Described herein are a variety of methods for determining susceptibility to an SSD (e.g., SZ). "Susceptibility" does not necessarily mean that the subject will develop an SSD (e.g., SZ), but rather that the subject is, in a statistical sense, more likely to develop SZ than an average member of the population, i.e., has an increased risk of developing an SSD (e.g., SZ). As used herein, susceptibility to an SSD exists if the subject has a genetic variation, e.g., an allele or CNV, associated with an increased risk of an SSD (e.g., SZ) as described herein. Ascertaining whether the subject has such an allele or CNV is included in the concept of diagnosing susceptibility to an SSD (e.g., SZ) as used herein. Such determination is useful, for example, for purposes of diagnosis, treatment selection, and genetic counseling. Thus, the methods described herein can include obtaining a haplotype associated with an increased risk of an SSD (e.g., SZ) as described herein for the subject.

As used herein, "determining the identity of an allele" or "determining copy number" includes obtaining information regarding the identity, number, presence or absence of one or more specific alleles or SNPs in a subject. Determining the identity of an allele or determining copy number can, but need not, include obtaining a sample comprising DNA from a subject, and/or assessing the identity, copy number, presence or absence of one or more genetic markers in the sample. The individual or organization who performs the determination need not actually carry out the physical analysis of a sample from a subject; the methods can include using information obtained by analysis of the sample by a third party. Thus the methods can include steps that occur at more than one site. For example, a sample can be obtained from a subject at a first site, such as at a health care provider, or at the subject's home in the case of a self-testing kit. The sample can be analyzed at the same or a second site, e.g., at a laboratory or other testing facility.

Determining the identity of an allele or copy number of a SNP can also include or consist of reviewing a subject's medical history, where the medical history includes information regarding the identity, copy number, presence or absence of one or more alleles or SNPs in the subject, e.g., results of a genetic test.

In some embodiments, to determine the identity of an allele or copy number/presence/absence of an allele or genotype described herein, a biological sample that includes nucleated cells (such as blood, a cheek swab or mouthwash) is prepared and analyzed for the copy number, presence or absence of preselected markers. Such diagnoses may be performed by diagnostic laboratories, or, alternatively, diagnostic kits can be manufactured and sold to health care providers or to private individuals for self-diagnosis. Diagnostic or prognostic tests can be performed as described herein or using well known techniques, such as described in U.S. Pat. No. 5,800,998.

Results of these tests, and optionally interpretive information, can be returned to the subject, the health care provider or to a third party payor. The results can be used in a number of ways. The information can be, e.g., communicated to the tested subject, e.g., with a prognosis and optionally interpretive materials that help the subject understand the test results and prognosis. The information can be used, e.g., by a health care provider, to determine whether to administer a specific drug, or whether a subject should be assigned to a specific category, e.g., a category associated with a specific disease endophenotype, or with drug response or non-response. The information can be used, e.g., by a third party payor such as a healthcare payer (e.g., insurance company or HMO) or other agency, to determine whether or not to reimburse a health care provider for services to the subject, or whether to approve the provision of services to the subject. For example, the healthcare payer may decide to reimburse a health care provider for treatments for SZ if the subject has SZ or has an increased risk of developing SZ. The presence or absence of the allele or genotype in a patient may be ascertained by using any of the methods described herein.

### Alleles and Genotypes Associated with SSDs

This document provides methods for diagnosing and assessing genetic risk based on evaluation of single nucleotide polymorphisms (SNPs) for genes relating to SZ-spectrum disorders including schizophrenia (SZ), schizotypal personality disorder (SPD) and schizoaffective disorder (SD). Tables 1-3 and Table A list specific exemplary SNPs that can be used to capture significant haplotype variation in these genes. One of skill in the art will appreciate that additional variants can be identified via TDT using families with multiple affected individuals (such as those collected CCGS) and verified by Case/Control comparisons, e.g., using the methods and markers presented herein. Using the SNP markers described herein, one can determine the alleles, copy number, genotypes or haplotypes in these genes relating to diagnosis or genetic risk of developing SZ. This information can then be used to determine risk of developing SZ, or for making a diagnosis of SZ. The allelic and genotypic variants thus identified can be used for diagnosis and for assessing genetic risk.

In some embodiments, the methods can include determining the presence of a haplotype that includes one or more polymorphisms near D22S526 and/or the polymorphisms in the *Sult4a1* gene and/or polymorphisms within 1 linkage disequilibrium unit (LDU) of these markers, e.g., as described in U.S. Pat. Pub. No. US2006/0177851, incorporated herein in its entirety.

### Linkage Disequilibrium Analysis

Linkage disequilibrium (LD) is a measure of the degree of association between alleles in a population. One of skill in the art will appreciate that haplotypes involving markers in LD with the polymorphisms described herein can also be used in a similar manner to those described herein. Methods of calculating LD are known in the art (see, e.g., Morton et al., Proc. Natl. Acad. Sci. USA 98(9):5217-21 (2001); Tapper et al., Proc. Natl. Acad Sci. USA 102(33):11835-11839 (2005); Maniatis et al., Proc. Natl. Acad. Sci. USA 99:2228-2233 (2002)). Thus, in some cases, the methods can include analysis of polymorphisms that are in LD with a polymorphism described herein. Methods are known in the art for identifying such polymorphisms; for example, the International HapMap Project provides a public database that can be used, see hapmap.org, as well as The International HapMap Consortium, Nature 426:789-796 (2003), and The International HapMap Consortium, Nature 437:1299-1320 (2005). Generally, it will be desirable to use a HapMap constructed using data from individuals who share ethnicity with the subject. For example, a HapMap for African Americans would ideally be used to identify markers in LD with an exemplary marker described herein for use in genotyping a subject of African American descent.

Alternatively, methods described herein can include analysis of polymorphisms that show a correlation coefficient (r²) of value ≥ 0.5 with the markers described herein. Results can be obtained from on line public resources such as HapMap.org on the World Wide Web. The correlation coefficient is a measure of LD, and reflects the degree to which alleles at two loci (for example, two SNPs) occur together, such that an allele at one SNP position can predict the correlated allele at a second SNP position, in the case where r² is > 0.5.

### Identifying Additional Genetic Markers

In general, genetic markers can be identified using any of a number of methods well known in the art. For example, numerous polymorphisms in the regions described herein are known to exist and are available in public databases, which can be searched using methods and algorithms known in the art. Alternately, polymorphisms can be identified by sequencing either genomic DNA or cDNA in the region in which it is desired to find a polymorphism. According to one approach, primers are designed to amplify such a region, and DNA from a subject is obtained and amplified. The DNA is sequenced, and the sequence (referred to as a "subject sequence" or "test sequence") is compared with a reference sequence, which can represent the "normal" or "wild type" sequence, or the "affected" sequence. In some embodiments, a reference sequence can be from, for example, the human draft genome sequence, publicly available in various databases, or a sequence deposited in a database such as GenBank. In some embodiments, the reference sequence is a composite of ethnically diverse individuals.

In general, if sequencing reveals a difference between the sequenced region and the reference sequence, a polymorphism has been identified. The fact that a difference in nucleotide sequence is identified at a particular site that determines that a polymorphism exists at that site. In most instances, particularly in the case of SNPs, only two polymorphic variants will exist at any location. However, in the case of SNPs, up to four variants may exist since there are four naturally occurring nucleotides in DNA. Other polymorphisms, such as insertions and deletions, may have more than four alleles.

The methods described herein can also include determining the presence or absence of other markers known or suspected to be associated with risk of bipolar disorder (BD) and/or SZ, or differentially inherited in BD versus SZ. In some embodiments, the methods include determining the presence or absence of one or more other markers that are or may be associated with BD or SZ, e.g., in one or more genes, e.g., e.g., as described in WO 2009/092032, WO 2009/089120, WO 2009/082743, US2006/0177851, and US2009/0012371 incorporated herein in their entirety. See also, e.g., OMIM entry no. 181500 (SCZD).

### Methods of Determining the Identity of an Allele or Obtaining a Genotype

The methods described herein include determining the identity, copy number, presence, or absence of alleles or genotypes associated with diagnosis or risk of developing SZ. In some embodiments, an association with SZ is determined by the statistical likelihood of the presence of an allele or genotype in an individual with SZ, e.g., an unrelated individual or a first or second-degree relation of the subject, and optionally the statistical likelihood of the absence of the same allele, copy number, or genotype in an unaffected reference individual, e.g., an unrelated individual or a first or second-degree relation of the subject. Thus the methods can include obtaining and analyzing a sample from one or more suitable reference individuals.

Samples that are suitable for use in the methods described herein contain genetic material, e.g., genomic DNA (gDNA). Genomic DNA is typically extracted from biological samples such as blood or mucosal scrapings of the lining of the mouth, but can be extracted from other biological samples including urine or expectorant. The sample itself will typically consist of nucleated cells (e.g., blood or buccal cells) or tissue removed from the subject. The subject can be an adult, child, fetus, or embryo. In some embodiments, the sample is obtained prenatally, either from a fetus or embryo or from the mother (e.g., from fetal or embryonic cells in the maternal circulation). Methods and reagents are known in the art for obtaining, processing, and analyzing samples. In some embodiments, the sample is obtained with the assistance of a health care provider, e.g., to draw blood. In some embodiments, the sample is obtained without the assistance of a health care provider, e.g., where the sample is obtained non-invasively, such as a sample comprising buccal cells that is obtained using a buccal swab or brush, or a mouthwash sample.

In some cases, a biological sample may be processed for DNA isolation. For example, DNA in a cell or tissue sample can be separated from other components of the sample. Cells can be harvested from a biological sample using standard techniques known in the art. For example, cells can be harvested by centrifuging a cell sample and resuspending the pelleted cells. The cells can be resuspended in a buffered solution such as phosphate-buffered saline (PBS). After centrifuging the cell suspension to obtain a cell pellet, the cells can be lysed to extract DNA, e.g., gDNA. See, e.g., Ausubel et al., 2003, supra. The sample can be concentrated and/or purified to isolate DNA. All samples obtained from a subject, including those subjected to any sort of further processing, are considered to be obtained from the subject. Routine methods can be used to extract genomic DNA from a biological sample, including, for example, phenol extraction. Alternatively, genomic DNA can be extracted with kits such as the QIAamp® Tissue Kit (Qiagen, Chatsworth, CA) and the Wizard® Genomic DNA purification kit (Promega). Nonlimiting examples of sources of samples include urine, blood, and tissue.

The copy number, absence or presence of an allele or genotype associated SZ as described herein can be determined using methods known in the art. For example, gel electrophoresis, capillary electrophoresis, size exclusion chromatography, sequencing, and/or arrays can be used to detect the presence or absence of the allele or genotype. Amplification of nucleic acids, where desirable, can be accomplished using methods known in the art, e.g., PCR. In one example, a sample (e.g., a sample comprising genomic DNA), is obtained from a subject. The DNA in the sample is then examined to identify or detect the presence of an allele or genotype as described herein. The allele or genotype can be identified or determined by any method described herein, e.g., by sequencing or by hybridization of the gene in the genomic DNA, RNA, or cDNA to a nucleic acid probe, e.g., a DNA probe (which includes cDNA and oligonucleotide probes) or an RNA probe. The nucleic acid probe can be designed to specifically or preferentially hybridize with a particular polymorphic variant.

Other methods of nucleic acid analysis can include direct manual sequencing (Church and Gilbert, Proc. Natl. Acad Sci. USA 81:1991-1995 (1988); Sanger et al., Proc. Natl. Acad Sci. USA 74:5463-5467 (1977); Beavis et al., U.S. Pat. No. 5,288,644); automated fluorescent sequencing; single-stranded conformation polymorphism assays (SSCP) (Schafer et al., Nat. Biotechnol. 15:33-39 (1995)); clamped denaturing gel electrophoresis (CDGE); two-dimensional gel electrophoresis (2DGE or TDGE); conformational sensitive gel electrophoresis (CSGE); denaturing gradient gel electrophoresis (DGGE) (Sheffield et al., Proc. Natl. Acad Sci. USA 86:232-236 (1989)); denaturing high performance liquid chromatography (DHPLC, Underhill et al., Genome Res. 7:996-1005 (1997)); infrared matrix-assisted laser desorption/ionization (IR-MALDI) mass spectrometry (WO 99/57318); mobility shift analysis (Orita et al., Proc. Natl. Acad. Sci. USA 86:2766-2770 (1989)); restriction enzyme analysis (Flavell et al., Cell 15:25 (1978); Geever et al., Proc. Natl. Acad. Sci. USA 78:5081 (1981)); quantitative real-time PCR (Raca et al., Genet Test 8(4):387-94 (2004)); heteroduplex analysis; chemical mismatch cleavage (CMC) (Cotton et al., Proc. Natl. Acad Sci. USA 85:4397-4401 (1985)); RNase protection assays (Myers et al., Science 230:1242 (1985)); use of polypeptides that recognize nucleotide mismatches, e.g., *E. coli* mutS protein; allele-specific PCR, and combinations of such methods. See, e.g., Gerber et al., U.S. Patent Publication No. 2004/0014095 which is incorporated herein by reference in its entirety.

Sequence analysis can also be used to detect specific polymorphic variants. For example, polymorphic variants can be detected by sequencing exons, introns, 5' untranslated sequences, or 3' untranslated sequences. A sample comprising DNA or RNA is obtained from the subject. PCR or other appropriate methods can be used to amplify a portion encompassing the polymorphic site, if desired. The sequence is then ascertained, using any standard method, and the presence of a polymorphic variant is determined. Real-time pyrophosphate DNA sequencing is yet another approach to detection of polymorphisms and polymorphic variants (Alderborn et al., Genome Research 10(8):1249-1258 (2000)). Additional methods include, for example, PCR amplification in combination with denaturing high performance liquid chromatography (dHPLC) (Underhill et al., Genome Research 7(10):996-1005 (1997)).

In order to detect polymorphisms and/or polymorphic variants, it will frequently be desirable to amplify a portion of genomic DNA (gDNA) encompassing the polymorphic site. Such regions can be amplified and isolated by PCR using oligonucleotide primers designed based on genomic and/or cDNA sequences that flank the site. PCR refers to procedures in which target nucleic acid (e.g., genomic DNA) is amplified in a manner similar to that described in U.S. Patent No. 4,683,195, and subsequent modifications of the procedure described therein. Generally, sequence information from the ends of the region of interest or beyond are used to design oligonucleotide primers that are identical or similar in sequence to opposite strands of a potential template to be amplified. See e.g., PCR Primer: A Laboratory Manual, Dieffenbach and Dveksler, (Eds.); McPherson et al., PCR Basics: From Background to Bench (Springer Verlag, 2000); Mattila et al., Nucleic Acids Res., 19:4967 (1991); Eckert et al., PCR Methods and Applications, 1:17 (1991); PCR (eds. McPherson et al., IRL Press, Oxford); and U.S. Pat. No. 4,683,202. Other amplification methods that may be employed include the ligase chain reaction (LCR) (Wu and Wallace, Genomics 4:560 (1989), Landegren et al., Science 241:1077 (1988), transcription amplification (Kwoh et al., Proc. Natl. Acad Sci. USA 86:1173 (1989)), self-sustained sequence replication (Guatelli et al., Proc. Nat. Acad. Sci. USA 87:1874 (1990)), and nucleic acid based sequence amplification (NASBA). Guidelines for selecting primers for PCR amplification are well known in the art. See, e.g., McPherson et al., PCR Basics: From Background to Bench, Springer-Verlag, 2000. A variety of computer programs for designing primers are available, e.g., 'Oligo' (National Biosciences, Inc, Plymouth Minn.), MacVector (Kodak/IBI), and the GCG suite of sequence analysis programs (Genetics Computer Group, Madison, Wis. 53711).

In some cases, PCR conditions and primers can be developed that amplify a product only when the variant allele is present or only when the wild type allele is present (MSPCR or allele-specific PCR). For example, patient DNA and a control can be amplified separately using either a wild type primer or a primer specific for the variant allele. Each set of reactions is then examined for the presence of amplification products using standard methods to visualize the DNA. For example, the reactions can be electrophoresed through an agarose gel and the DNA visualized by staining with ethidium bromide or other DNA intercalating dye. In DNA samples from heterozygous patients, reaction products would be detected in each reaction.

Real-time quantitative PCR can also be used to determine copy number. Quantitative PCR permits both detection and quantification of specific DNA sequence in a sample as an absolute number of copies or as a relative amount when normalized to DNA input or other normalizing genes. A key feature of quantitative PCR is that the amplified DNA product is quantified in real-time as it accumulates in the reaction after each amplification cycle. Methods of quantification can include the use of fluorescent dyes that intercalate with double-stranded DNA, and modified DNA oligonucleotide probes that fluoresce when hybridized with a complementary DNA.

The first report of extensive copy number variation (CNV) in the human genome used intensity analysis of microarray data to document numerous examples of genes that vary in copy number (Redon et al., Nature 444(7118):444-54 (2006)). Subsequent studies have shown that certain copy number variants are associated with complex genetic diseases such as SZ (Walsh et al., Science 320(5875):539-43 (2008); and Stone et al., Nature 455(7210):237-41 (2008)).

In some embodiments, a peptide nucleic acid (PNA) probe can be used instead of a nucleic acid probe in the hybridization methods described above. PNA is a DNA mimetic with a peptide-like, inorganic backbone, e.g., N-(2-aminoethyl)glycine units, with an organic base (A, G, C, T or U) attached to the glycine nitrogen via a methylene carbonyl linker (see, e.g., Nielsen et al., Bioconjugate Chemistry, The American Chemical Society, 5:1 (1994)). The PNA probe can be designed to specifically hybridize to a nucleic acid comprising a polymorphic variant conferring susceptibility to or indicative of the presence of, for example, SZ.

In some cases, allele-specific oligonucleotides can also be used to detect the presence of a polymorphic variant. For example, polymorphic variants can be detected by performing allele-specific hybridization or allele-specific restriction digests. Allele specific hybridization is an example of a method that can be used to detect sequence variants, including complete haplotypes of a subject (e.g., a mammal such as a human). See Stoneking et al., Am. J. Hum. Genet. 48:370-382 (1991); and Prince et al., Genome Res. 11:152-162 (2001). An "allele-specific oligonucleotide" (also referred to herein as an "allele-specific oligonucleotide probe") is an oligonucleotide that is specific for particular a polymorphism can be prepared using standard methods (see Ausubel et al., Current Protocols in Molecular Biology, supra). Allele-specific oligonucleotide probes typically can be approximately 10-50 base pairs, preferably approximately 15-30 base pairs, that specifically hybridizes to a nucleic acid region that contains a polymorphism. Hybridization conditions are selected such that a nucleic acid probe can specifically bind to the sequence of interest, e.g., the variant nucleic acid sequence. Such hybridizations typically are performed under high stringency as some sequence variants include only a single nucleotide difference. In some cases, dot-blot hybridization of amplified oligonucleotides with allele-specific oligonucleotide (ASO) probes can be performed. See, for example, Saiki et al., Nature (London) 324:163-166 (1986).

In some embodiments, allele-specific restriction digest analysis can be used to detect the existence of a polymorphic variant of a polymorphism, if alternate polymorphic variants of the polymorphism result in the creation or elimination of a restriction site. Allele-specific restriction digests can be performed in the following manner. A sample containing genomic DNA is obtained from the individual and genomic DNA is isolated for analysis. For nucleotide sequence variants that introduce a restriction site, restriction digest with the particular restriction enzyme can differentiate the alleles. In some cases, polymerase chain reaction (PCR) can be used to amplify a region comprising the polymorphic site, and restriction fragment length polymorphism analysis is conducted (see Ausubel et al., Current Protocols in Molecular Biology, supra). The digestion pattern of the relevant DNA fragment indicates the presence or absence of a particular polymorphic variant of the polymorphism and is therefore indicative of the presence or absence of susceptibility to SZ. For sequence variants that do not alter a common restriction site, mutagenic primers can be designed that introduce a restriction site when the variant allele is present or when the wild type allele is present. For example, a portion of a nucleic acid can be amplified using the mutagenic primer and a wild type primer, followed by digest with the appropriate restriction endonuclease.

In some embodiments, fluorescence polarization template-directed dye-terminator incorporation (FP-TDI) is used to determine which of multiple polymorphic variants of a polymorphism is present in a subject (Chen et al., Genome Research 9(5):492-498 (1999)). Rather than involving use of allele-specific probes or primers, this method employs primers that terminate adjacent to a polymorphic site, so that extension of the primer by a single nucleotide results in incorporation of a nucleotide complementary to the polymorphic variant at the polymorphic site.

In some cases, DNA containing an amplified portion may be dot-blotted, using standard methods (see Ausubel et al., *Current Protocols in Molecular Biology,* supra), and the blot contacted with the oligonucleotide probe. The presence of specific hybridization of the probe to the DNA is then detected. Specific hybridization of an allele-specific oligonucleotide probe (specific for a polymorphic variant indicative of susceptibility to an SSD (e.g., SZ)) to DNA from the subject is indicative of susceptibility to an SSD (e.g., SZ).

The methods can include determining the genotype of a subject with respect to both copies of the polymorphic site present in the genome. For example, the complete genotype may be characterized as -/-, as -/+, or as +/+, where a minus sign indicates the presence of the reference or wild type sequence at the polymorphic site, and the plus sign indicates the presence of a polymorphic variant other than the reference sequence. If multiple polymorphic variants exist at a site, this can be appropriately indicated by specifying which ones are present in the subject. Any of the detection means described herein can be used to determine the genotype of a subject with respect to one or both copies of the polymorphism present in the subject's genome.

Methods of nucleic acid analysis to detect polymorphisms and/or polymorphic variants can include, e.g., microarray analysis. Hybridization methods, such as Southern analysis, Northern analysis, or *in situ* hybridizations, can also be used (see Ausubel et al., Current Protocols in Molecular Biology, eds., John Wiley & Sons (2003)). To detect microdeletions, fluorescence in situ hybridization (FISH) using DNA probes that are directed to a putatively deleted region in a chromosome can be used. For example, probes that detect all or a part of a microsatellite marker can be used to detect microdeletions in the region that contains that marker.

In some embodiments, it is desirable to employ methods that can detect the presence of multiple polymorphisms (e.g., polymorphic variants at a plurality of polymorphic sites) in parallel or substantially simultaneously. Oligonucleotide arrays represent one suitable means for doing so. Other methods, including methods in which reactions (e.g., amplification, hybridization) are performed in individual vessels, e.g., within individual wells of a multi-well plate or other vessel may also be performed so as to detect the presence of multiple polymorphic variants (e.g., polymorphic variants at a plurality of polymorphic sites) in parallel or substantially simultaneously according to certain embodiments.

Nucleic acid probes can be used to detect and/or quantify the presence of a particular target nucleic acid sequence within a sample of nucleic acid sequences, e.g., as hybridization probes, or to amplify a particular target sequence within a sample, e.g., as a primer. Probes have a complimentary nucleic acid sequence that selectively hybridizes to the target nucleic acid sequence. In order for a probe to hybridize to a target sequence, the hybridization probe must have sufficient identity with the target sequence, i.e., at least 70% (e.g., 80%, 90%, 95%, 98% or more) identity to the target sequence. The probe sequence must also be sufficiently long so that the probe exhibits selectivity for the target sequence over non-target sequences. For example, the probe will be at least 20 (e.g., 25, 30, 35, 50, 100, 200, 300, 400, 500, 600, 700, 800, 900 or more) nucleotides in length. In some embodiments, the probes are not more than 30, 50, 100, 200, 300, 500, 750, or 1000 nucleotides in length. Probes are typically about 20 to about 1 x 10⁶ nucleotides in length. Probes include primers, which generally refers to a single-stranded oligonucleotide probe that can act as a point of initiation of template-directed DNA synthesis using methods such as PCR (polymerase chain reaction), LCR (ligase chain reaction), etc., for amplification of a target sequence.

The probe can be a test probe such as a probe that can be used to detect polymorphisms in a region described herein (e.g., polymorphisms as described herein). For example, the probe can hybridize to an allele described herein. In some embodiments, the probe can bind to another marker sequence associated with SZ, SPD, SD or BD as described herein.

Control probes can also be used. For example, a probe that binds a less variable sequence, e.g., repetitive DNA associated with a centromere of a chromosome, can be used as a control. Probes that hybridize with various centromeric DNA and locus-specific DNA are available commercially, for example, from Vysis, Inc. (Downers Grove, Ill.), Molecular Probes, Inc. (Eugene, Oreg.), or from Cytocell (Oxfordshire, UK). Probe sets are available commercially such from Applied Biosystems, e.g., the Assays-on-Demand SNP kits Alternatively, probes can be synthesized, e.g., chemically or in vitro, or made from chromosomal or genomic DNA through standard techniques. For example, sources of DNA that can be used include genomic DNA, cloned DNA sequences, somatic cell hybrids that contain one, or a part of one, human chromosome along with the normal chromosome complement of the host, and chromosomes purified by flow cytometry or microdissection. The region of interest can be isolated through cloning, or by site-specific amplification via the polymerase chain reaction (PCR). See, for example, Nath and Johnson, Biotechnic. Histochem. 73(1):6-22 (1998); Wheeless et al., Cytometry 17:319-326 (1994); and U.S. Pat. No. 5,491,224.

In some embodiments, the probes are labeled, e.g., by direct labeling, with a fluorophore, an organic molecule that fluoresces after absorbing light of lower wavelength/higher energy. A directly labeled fluorophore allows the probe to be visualized without a secondary detection molecule. After covalently attaching a fluorophore to a nucleotide, the nucleotide can be directly incorporated into the probe with standard techniques such as nick translation, random priming, and PCR labeling. Alternatively, deoxycytidine nucleotides within the probe can be transaminated with a linker. The fluorophore then is covalently attached to the transaminated deoxycytidine nucleotides. See, e.g., U.S. Pat. No. 5,491,224.

Fluorophores of different colors can be chosen such that each probe in a set can be distinctly visualized. For example, a combination of the following fluorophores can be used: 7-amino-4-methylcoumarin-3-acetic acid (AMCA), TEXAS RED™ (Molecular Probes, Inc., Eugene, OR), 5-(and-6)-carboxy-X-rhodamine, lissamine rhodamine B, 5-(and-6)-carboxyfluorescein, fluorescein-5-isothiocyanate (FITC), 7-diethylaminocoumarin-3-carboxylic acid, tetramethylrhodamine-5-(and-6)-isothiocyanate, 5-(and-6)-carboxytetramethylrhodamine, 7-hydroxycoumarin-3-carboxylic acid, 6-[fluorescein 5-(and-6)-carboxamido]hexanoic acid, N-(4,4-difluoro-5,7-dimethyl-4-bora-3a,4a diaza-3-indacenepropionic acid, eosin-5-isothiocyanate, erythrosin-5-isothiocyanate, and CASCADE™ blue acetylazide (Molecular Probes, Inc., Eugene, OR). Fluorescently labeled probes can be viewed with a fluorescence microscope and an appropriate filter for each fluorophore, or by using dual or triple band-pass filter sets to observe multiple fluorophores. See, for example, U.S. Pat. No. 5,776,688. Alternatively, techniques such as flow cytometry can be used to examine the hybridization pattern of the probes. Fluorescence-based arrays are also known in the art.

In other embodiments, the probes can be indirectly labeled with, e.g., biotin or digoxygenin, or labeled with radioactive isotopes such as ³²P and ³H. For example, a probe indirectly labeled with biotin can be detected by avidin conjugated to a detectable marker. For example, avidin can be conjugated to an enzymatic marker such as alkaline phosphatase or horseradish peroxidase. Enzymatic markers can be detected in standard colorimetric reactions using a substrate and/or a catalyst for the enzyme. Catalysts for alkaline phosphatase include 5-bromo-4-chloro-3-indolylphosphate and nitro blue tetrazolium. Diaminobenzoate can be used as a catalyst for horseradish peroxidase.

In another aspect, this document features arrays that include a substrate having a plurality of addressable areas, and methods of using them. At least one area of the plurality includes a nucleic acid probe that binds specifically to a sequence comprising a polymorphism listed in Tables 1-3 or Table A, and can be used to detect the absence or presence of said polymorphism, e.g., one or more SNPs, microsatellites, minisatellites, or indels, as described herein, to determine or identify an allele or genotype. For example, the array can include one or more nucleic acid probes that can be used to detect a polymorphism listed in Tables 1-3 or Table A. In some embodiments, the array further includes at least one area that includes a nucleic acid probe that can be used to specifically detect another marker associated with SZ or one associated with BD as described herein or known in the art. In some embodiments, the probes are nucleic acid capture probes.

Generally, microarray hybridization is performed by hybridizing a nucleic acid of interest (e.g., a nucleic acid encompassing a polymorphic site) with the array and detecting hybridization using nucleic acid probes. In some cases, the nucleic acid of interest is amplified prior to hybridization. Hybridization and detecting are generally carried out according to standard methods. See, e.g., Published PCT Application Nos. WO 92/10092 and WO 95/11995, and U.S. Pat. No. 5,424,186. For example, the array can be scanned to determine the position on the array to which the nucleic acid hybridizes. The hybridization data obtained from the scan is typically in the form of fluorescence intensities as a function of location on the array.

Arrays can be formed on substrates fabricated with materials such as paper, glass, plastic (e.g., polypropylene, nylon, or polystyrene), polyacrylamide, nitrocellulose, silicon, optical fiber, or any other suitable solid or semisolid support, and can be configured in a planar (e.g., glass plates, silicon chips) or three dimensional (e.g., pins, fibers, beads, particles, microtiter wells, capillaries) configuration. Methods for generating arrays are known in the art and include, e.g., photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145). The array typically includes oligonucleotide hybridization probes capable of specifically hybridizing to different polymorphic variants. Oligonucleotide probes that exhibit differential or selective binding to polymorphic sites may readily be designed by one of ordinary skill in the art. For example, an oligonucleotide that is perfectly complementary to a sequence that encompasses a polymorphic site (i.e., a sequence that includes the polymorphic site, within it or at one end) will generally hybridize preferentially to a nucleic acid comprising that sequence, as opposed to a nucleic acid comprising an alternate polymorphic variant.

Oligonucleotide probes forming an array may be attached to a substrate by any number of techniques, including, without limitation, (i) *in situ* synthesis (e.g., high-density oligonucleotide arrays) using photolithographic techniques; (ii) spotting/printing at medium to low density on glass, nylon or nitrocellulose; (iii) by masking, and (iv) by dot-blotting on a nylon or nitrocellulose hybridization membrane. Oligonucleotides can be immobilized via a linker, including by covalent, ionic, or physical linkage. Linkers for immobilizing nucleic acids and polypeptides, including reversible or cleavable linkers, are known in the art. See, for example, U.S. Patent No. 5,451,683 and WO98/20019. Alternatively, oligonucleotides can be non-covalently immobilized on a substrate by hybridization to anchors, by means of magnetic beads, or in a fluid phase such as in microtiter wells or capillaries. Immobilized oligonucleotide probes are typically about 20 nucleotides in length, but can vary from about 10 nucleotides to about 1000 nucleotides in length.

Arrays can include multiple detection blocks (i.e., multiple groups of probes designed for detection of particular polymorphisms). Such arrays can be used to analyze multiple different polymorphisms. Detection blocks may be grouped within a single array or in multiple, separate arrays so that varying conditions (e.g., conditions optimized for particular polymorphisms) may be used during the hybridization. For example, it may be desirable to provide for the detection of those polymorphisms that fall within G-C rich stretches of a genomic sequence, separately from those falling in A-T rich segments. General descriptions of using oligonucleotide arrays for detection of polymorphisms can be found, for example, in U.S. Pat. Nos. 5,858,659 and 5,837,832. In addition to oligonucleotide arrays, cDNA arrays may be used similarly in certain embodiments.

The methods described herein can include providing an array as described herein; contacting the array with a sample (e.g., a portion of genomic DNA that includes at least a portion of a human chromosome) and/or optionally, a different portion of genomic DNA (e.g., a portion that includes a different portion of a human chromosome, e.g., including another region associated with SZ), and detecting binding of a nucleic acid from the sample to the array. Optionally, the method includes amplifying nucleic acid from the sample, e.g., genomic DNA that includes a portion of a human chromosome described herein, and, optionally, a region that includes another region associated with SZ, prior to or during contact with the array.

In some aspects, the methods described herein can include using an array that can ascertain differential expression patterns or copy numbers of one or more genes in samples from normal and affected individuals (see, e.g., Redon et al., Nature 444(7118):444-54 (2006)). For example, arrays of probes to a marker described herein can be used to measure polymorphisms between DNA from a subject having an SSD (e.g., SZ), and control DNA, e.g., DNA obtained from an individual that does not have SZ, SPD, or SD, and has no risk factors for an SSD (e.g., SZ). Since the clones on the array contain sequence tags, their positions on the array are accurately known relative to the genomic sequence. Different hybridization patterns between DNA from an individual afflicted with an SSD (e.g., SZ) and DNA from a normal individual at areas in the array corresponding to markers in human chromosome 4p and/or 22q as described herein, and, optionally, one or more other regions associated with an SSD (e.g., SZ), are indicative of a risk of SZ. Methods for array production, hybridization, and analysis are described, e.g., in Snijders et al., Nat. Genetics 29:263-264 (2001); Klein et al., Proc. Natl Acad. Sci. USA 96:4494-4499 (1999); Albertson et al., Breast Cancer Research and Treatment 78:289-298 (2003); and Snijders et al., "BAC microarray based comparative genomic hybridization," in: Zhao et al. (eds), Bacterial Artificial Chromosomes: Methods and Protocols, Methods in Molecular Biology, Humana Press, 2002.

In another aspect, this document provides methods of determining the absence or presence of a haplotype associated with SZ as described herein, using an array described above. The methods can include providing a two dimensional array having a plurality of addresses, each address of the plurality being positionally distinguishable from each other address of the plurality having a unique nucleic acid capture probe, contacting the array with a first sample from a test subject who is suspected of having or being at risk for SZ, and comparing the binding of the first sample with one or more references, e.g., binding of a sample from a subject who is known to have an SSD (e.g., SZ), and/or binding of a sample from a subject who is unaffected, e.g., a control sample from a subject who neither has, nor has any risk factors for an SSD (e.g., SZ). In some embodiments, the methods can include contacting the array with a second sample from a subject who has an SSD (e.g., SZ); and comparing the binding of the first sample with the binding of the second sample. In some embodiments, the methods can include contacting the array with a third sample from a cell or subject that does not have SZ and is not at risk for SZ; and comparing the binding of the first sample with the binding of the third sample. In some embodiments, the second and third samples are from first or second-degree relatives of the test subject. In the case of a nucleic acid hybridization, binding with a capture probe at an address of the plurality, can be detected by any method known in the art, e.g., by detection of a signal generated from a label attached to the nucleic acid.

### Communicating a Diagnosis or Risk Assessment

This document also provides methods and materials to assist medical or research professionals in determining whether or not a subject has or is at risk for developing an SSD (e.g., SZ). Medical professionals can be, for example, doctors, nurses, medical laboratory technologists, and pharmacists. Research professionals can be, for example, principle investigators, research technicians, postdoctoral trainees, and graduate students. A professional can be assisted by (1) determining whether specific polymorphic variants are present in a biological sample from a subject, and (2) communicating information about polymorphic variants to that professional.

After information about specific polymorphic variants is reported, a medical professional can take one or more actions that can affect patient care. For example, a medical professional can record information in the patient's medical record regarding the patient's risk of developing an SSD (e.g., SZ). In some cases, a medical professional can record information regarding risk assessment, or otherwise transform the patient's medical record, to reflect the patient's current medical condition. In some cases, a medical professional can review and evaluate a patient's entire medical record and assess multiple treatment strategies for clinical intervention of a patient's condition.

A medical professional can initiate or modify treatment after receiving information regarding a patient's diagnosis of or risk of developing an SSD (e.g., SZ), for example. In some cases, a medical professional can recommend a change in therapy. In some cases, a medical professional can enroll a patient in a clinical trial for, by way of example, detecting correlations between a haplotype as described herein and any measurable or quantifiable parameter relating to the outcome of the treatment as described above.

A medical professional can communicate information regarding a patient's diagnosis of risk of developing an SSD (e.g., SZ) to a patient or a patient's family. In some cases, a medical professional can provide a patient and/or a patient's family with information regarding an SSD (e.g., SZ) and diagnosis or risk assessment information, including treatment options, prognosis, and referrals to specialists. In some cases, a medical professional can provide a copy of a patient's medical records to a specialist.

A research professional can apply information regarding a subject's diagnosis of or risk of developing an SSD (e.g., SZ) to advance scientific research. For example, a researcher can compile data on wild specific polymorphic variants. In some cases, a research professional can obtain a subject's haplotype as described herein to evaluate a subject's enrollment, or continued participation, in a research study or clinical trial. In some cases, a research professional can communicate information regarding a subject's diagnosis of or risk of developing an SSD (e.g., SZ) to a medical professional. In some cases, a research professional can refer a subject to a medical professional.

Any appropriate method can be used to communicate information to another person (e.g., a professional). For example, information can be given directly or indirectly to a professional. For example, a laboratory technician can input a patient's polymorphic variant haplotype as described herein into a computer-based record. In some cases, information is communicated by making an physical alteration to medical or research records. For example, a medical professional can make a permanent notation or flag a medical record for communicating the risk assessment to other medical professionals reviewing the record. In addition, any type of communication can be used to communicate the risk assessment information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

### Articles of Manufacture

Also provided herein are articles of manufacture comprising a probe that hybridizes with a region of human chromosome as described herein and can be used to detect a polymorphism described herein. For example, any of the probes for detecting polymorphisms described herein can be combined with packaging material to generate articles of manufacture or kits. The kit can include one or more other elements including: instructions for use; and other reagents such as a label or an agent useful for attaching a label to the probe. Instructions for use can include instructions for diagnostic applications of the probe for making a diagnosis of or assessing risk of an SSD (e.g., SZ) in a method described herein. Other instructions can include instructions for attaching a label to the probe, instructions for performing *in situ* analysis with the probe, and/or instructions for obtaining a sample to be analyzed from a subject. In some cases, the kit can include a labeled probe that hybridizes to a region of human chromosome as described herein.

The kit can also include one or more additional reference or control probes that hybridize to the same chromosome or another chromosome or portion thereof that can have an abnormality associated with a particular endophenotype. A kit that includes additional probes can further include labels, e.g., one or more of the same or different labels for the probes. In other embodiments, the additional probe or probes provided with the kit can be a labeled probe or probes. When the kit further includes one or more additional probe or probes, the kit can further provide instructions for the use of the additional probe or probes. Kits for use in self-testing can also be provided. Such test kits can include devices and instructions that a subject can use to obtain a biological sample (e.g., buccal cells, blood) without the aid of a health care provider. For example, buccal cells can be obtained using a buccal swab or brush, or using mouthwash.

Kits as provided herein can also include a mailer (e.g., a postage paid envelope or mailing pack) that can be used to return the sample for analysis, e.g., to a laboratory. The kit can include one or more containers for the sample, or the sample can be in a standard blood collection vial. The kit can also include one or more of an informed consent form, a test requisition form, and instructions on how to use the kit in a method described herein. Methods for using such kits are also included herein. One or more of the forms (e.g., the test requisition form) and the container holding the sample can be coded, for example, with a bar code for identifying the subject who provided the sample.

### Databases and Reports

Also provided herein are databases that include a list of polymorphisms as described herein, and wherein the list is largely or entirely limited to polymorphisms identified as useful for determining a diagnosis or susceptibility to an SSD (e.g., SZ) as described herein. The list is stored, e.g., on a flat file or computer-readable medium. The databases can further include information regarding one or more subjects, e.g., whether a subject is affected or unaffected, clinical information such as endophenotype, age of onset of symptoms, any treatments administered and outcomes (e.g., data relevant to pharmacogenomics, diagnostics or theranostics), and other details, e.g., about the disorder in the subject, or environmental or other genetic factors. The databases can be used to detect correlations between a particular haplotype and the information regarding the subject.

The methods described herein can also include the generation of reports, e.g., for use by a patient, care giver, payor, or researcher, that include information regarding a subject's response allele(s), and optionally further information such as treatments administered, treatment history, medical history, predicted response, and actual response. The reports can be recorded in a tangible medium, e.g., a computer-readable disk, a solid state memory device, or an optical storage device.

### Engineered Cells

Also provided herein are engineered cells that harbor one or more polymorphism described herein, e.g., one or more polymorphisms associated with an SSD (e.g., SZ), e.g., a SNP or CNV. Such cells are useful for studying the effect of a polymorphism on physiological function, and for identifying and/or evaluating potential therapeutic agents such as antipsychotics for the treatment of an SSD (e.g., SZ).

As one example, included herein are cells in which one of the various alleles of the genes described herein has been re-created that is associated with an increased risk of an SSD (e.g., SZ). Methods are known in the art for generating cells, e.g., by homologous recombination between the endogenous gene and an exogenous DNA molecule introduced into a cell, e.g., a cell of an animal. In some cases, the cells can be used to generate transgenic animals using methods known in the art.

The cells are preferably mammalian cells (e.g., neuronal type cells) in which an endogenous gene has been altered to include a polymorphism as described herein. Techniques such as targeted homologous recombinations, can be used to insert the heterologous DNA as described in, e.g., Chappel, US 5,272,071; WO 91/06667, published in May 16, 1991.

### EXAMPLES

The invention is further described in the following examples, which do not limit the scope of the invention described in the claims.

### Example 1. Alleles Associated with Risk of Developing Schizophrenia

The Clinical Antipsychotic Trials of Intervention Effectiveness (CATIE), a large federally funded clinical trial designed to assess the efficacy of antipsychotics in a real world setting, is a valuable resource for determining the role of genes in drug response (Stroup et al., Schizophr. Bull. 29:15-31 (2003); Lieberman et al., N. Engl. J. Med. 353:1209-1223 (2005)). As part of the CATIE trial, SNP genotyping was performed for roughly half of the trial participants (Sullivan et al., Mol. Psychiatry 13:570-584 (2008)). When combined with disease status, PANSS scores, and clinical drug response data, the genotyping data allows the identification of genetic variants (e.g., SNPs) that are statistically associated with specific responses to selected treatments.

The design of the CATIE study has been described in detail by others (see, e.g., Stroup et al., Schizophr. Bull. 29:15-31 (2003); Lieberman et al., N. Engl. J. Med. 353:1209-1223 (2005)). Briefly, 1460 subjects were randomly assigned one of several antipsychotics and those who did not respond or chose to quit their current medication were re-randomized to another drug. Details regarding SNP genotyping and quality control have been recently published (Sullivan et al., Mol. Psychiatry 13:570-584 (2008)).

Genotype and phenotype data for the CATIE trial were made available to qualified researchers through the NIMH Center for Collaborative Genetic Studies on Mental Disorders. Data for 417 patients with schizophrenia and 419 unaffected controls self reported as having exclusively European ancestry were evaluated. This same patient population was described in a recent study by Sullivan and coworkers, which confirmed that there is no hidden stratification in the sample (Sullivan et al., Mol. Psychiatry 13:570-584 (2008)).

For the CATIE study, individual cases were diagnosed as having SZ based on DSM-III/IV criteria.

To determine the influence of specific alleles on likelihood of having schizophrenia, case/control association using the PLINK 1.03 whole genome analysis toolset developed by Purcell and coworkers (Purcell et al., Am. J. Hum. Genet. 81:559-575 (2007)). For dichotomous comparisons of affected vs. unaffected individuals, PLINK calculates P values for the allele-specific chi-squared test and the odds ratio (OR; or relative risk) associated with the minor allele.

Table 1 lists alleles and genotypes influencing a diagnosis of SZ. 1170 SZ cases, and 1378 neurologically normal controls were used to identify these alleles. Test 1 lists gene, SEQ ID, NCBI RS#, allele, frequency of allele in cases, p-value, and Odds ratio for cases versus controls.

### Example 2: Copy Number Variants (CNVs) that Contribute to Risk of SZ

Genotyping and phenotype data were obtained from the Genetic Analysis Information Network (GAIN) Database found at ncbi.nlm.nih.gov through dbGaP, at accession number PHS000017.v1.p1. Genotypes and associated phenotype data for the GAIN Genome-Wide Association Study of Schizophrenia were provided by P. Gejman, and genotyping of these samples was provided through the Genetic Association Information Network (GAIN). Golden Helix's SVS 7.0 was used to determine the Copy Number Variation at SNP for the genes of interest. Briefly, GAIN determine SNP and CNV variation for the samples using the Affymetrix 6.0 SNP and CNV array. Probes were hybridized to the array and the intensity of the signal recorded. These raw intensity values were imported into the SVS using the import function of the program. Then CNV status was determined using the following protocol. For the present example, 1170 SZ patients and 1378 neurologically normal controls were used to identify SNPS that can detect CNV regions influencing SZ risk.

Table 2 lists SNPs and associated genes, deletions of which alter the likelihood of SZ diagnosis. P-values were calculated from Chi-square values at each SNP. Odds Ratios were calculated by comparing the frequency of a CNV in cases versus controls using the formula (Case_{freq}*(1-Control_{freq}))/(Control_{freq}*(1-Casef_{freq})).

Table 3 lists SNPs and associated genes, duplications of which event that alter the likelihood of SZ diagnosis. P-values were calculated from Chi-square values at each SNP. Odds Ratios were calculated by comparing the frequency of a CNV in cases versus controls using the formula (Case_{freq}*(1-Control_{freq}))/(Control_{freq}*(1-Case_{freq})).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

| **Table 1: Alleles influencing likelihood of schizophrenia diagnosis** | | | | | | |
|---|---|---|---|---|---|---|
| **Gene** | **Seq ID** | **NCBI RS#** | **Allele** | **Frequency** | **P** | **Odds Ratio** |
| PIK3CD | 1 | 11121472 | A | 0.1456 | 0.0230000 | 1.40 |
| RP1-21O18.1 | 2 | 10803343 | G | 0.0156 | 0.0324300 | 3.20 |
| AGBL4-C1ORF165 | 3 | 2153324 | G | 0.4000 | 0.0050950 | 1.33 |
| AGBL4-C1ORF165 | 4 | 1934368 | C | 0.2900 | 0.0106600 | 1.33 |
| AGBL4-C1ORF165 | 5 | 6667780 | C | 0.3597 | 0.0203800 | 1.27 |
| AGBL4-C1ORF165 | 6 | 4285747 | C | 0.3893 | 0.0032690 | 1.35 |
| AGBL4-C1ORF165 | 7 | 3121522 | A | 0.3401 | 0.0224000 | 1.27 |
| AGBL4-C1ORF165 | 8 | 12043418 | T | 0.3878 | 0.0058610 | 1.33 |
| AGBL4-C1ORF165 | 9 | 3122291 | T | 0.4234 | 0.0175500 | 1.27 |
| SCP2 | 10 | 7546714 | T | 0.1360 | 0.0231900 | 1.41 |
| LRP8 | 11 | 5174 | A | 0.3663 | 0.0044880 | 0.75 |
| LRP8 | 12 | 2297660 | A | 0.3829 | 0.0112400 | 0.78 |
| LRP8 | 13 | 2297657 | A | 0.3521 | 0.0276000 | 0.80 |
| PRKACB | 14 | 6701486 | A | 0.2476 | 0.0157500 | 1.33 |
| PRKACB | 15 | 2642186 | G | 0.2350 | 0.0400800 | 1.28 |
| CGN | 16 | 1573129 | T | 0.1365 | 0.0079840 | 0.70 |
| OLFML2B | 17 | 12025136 | C | 0.2792 | 0.0002507 | 1.52 |
| DPT | 18 | 1018453 | C | 0.5048 | 0.0053390 | 1.32 |
| SEC16B | 19 | 943762 | A | 0.0859 | 0.0097780 | 0.66 |
| SEC16B | 20 | 4652279 | A | 0.0859 | 0.0141700 | 0.67 |
| MR1 | 21 | 3845422 | A | 0.1933 | 0.0052410 | 0.72 |
| LAMC1 | 22 | 6424888 | A | 0.4253 | 0.0024470 | 0.74 |
| LAMC1 | 23 | 2274984 | C | 0.4234 | 0.0032510 | 0.75 |
| LAMC1 | 24 | 2027085 | A | 0.4246 | 0.0044230 | 0.76 |
| KCNK2 | 25 | 2841598 | G | 0.5188 | 0.0000422 | 1.51 |
| KCNK2 | 26 | 10779634 | T | 0.3484 | 0.0032330 | 1.36 |
| KCNK2 | 27 | 2123331 | G | 0.3814 | 0.0351800 | 1.25 |
| KCNK2 | 28 | 17024179 | C | 0.1859 | 0.0330100 | 1.33 |
| KCNK2 | 29 | 4655272 | A | 0.5203 | 0.0144700 | 1.27 |
| KCNK2 | 30 | 10779647 | T | 0.3804 | 0.0095630 | 1.31 |
| KCNK2 | 31 | 6684084 | G | 0.3123 | 0.0404400 | 1.25 |
| KCNK2 | 32 | 10779651 | C | 0.3140 | 0.0453500 | 1.25 |
| KCNK2 | 33 | 10494994 | A | 0.2530 | 0.0196600 | 1.31 |
| ESRRG | 34 | 1984137 | C | 0.4332 | 0.0188000 | 0.79 |
| ESRRG | 35 | 12037068 | C | 0.1745 | 0.0264100 | 1.36 |
| ESRRG | 36 | 12033378 | A | 0.1711 | 0.0477700 | 1.31 |
| GNG4 | 37 | 508208 | G | 0.3864 | 0.0403200 | 1.23 |
| RYR2 | 38 | 2169902 | A | 0.0735 | 0.0102100 | 0.64 |
| RYR2 | 39 | 515474 | C | 0.2200 | 0.0237200 | 1.32 |
| RYR2 | 40 | 4659819 | T | 0.4569 | 0.0094780 | 0.78 |
| FMN2 | 41 | 1542400 | G | 0.4709 | 0.0387700 | 0.82 |
| RGS7 | 42 | 191735 | A | 0.4294 | 0.0496000 | 1.22 |
| PLD5 | 43 | 2036407 | C | 0.3254 | 0.0340200 | 1.26 |
| C2ORF46 | 44 | 182971 | C | 0.2746 | 0.0213700 | 1.30 |
| C2ORF46 | 45 | 3102945 | C | 0.1723 | 0.0056010 | 1.47 |
| C2ORF46 | 46 | 2562011 | C | 0.1819 | 0.0160100 | 1.38 |
| KCNF1 | 47 | 1317761 | A | 0.3623 | 0.0031120 | 1.36 |
| KCNF1 | 48 | 4669647 | T | 0.2265 | 0.0378100 | 1.29 |
| KCNF1 | 49 | 4669651 | T | 0.1850 | 0.0388100 | 1.32 |
| ASXL2 | 50 | 9309437 | T | 0.2033 | 0.0009717 | 1.54 |
| CRIM1 | 51 | 4670549 | T | 0.4606 | 0.0418900 | 1.22 |
| CRIM1 | 52 | 2049367 | A | 0.3065 | 0.0301000 | 1.27 |
| CRIM1 | 53 | 3770876 | C | 0.4318 | 0.0374900 | 1.23 |
| CRIM1 | 54 | 3755197 | A | 0.3038 | 0.0037720 | 0.74 |
| CRIM1 | 55 | 11681392 | T | 0.2995 | 0.0125200 | 0.77 |
| HAAO | 56 | 3816182 | A | 0.2745 | 0.0054010 | 1.37 |
| PLEKHH2 | 57 | 17414362 | A | 0.2307 | 0.0038410 | 1.43 |
| PRKCE | 58 | 6732900 | T | 0.4522 | 0.0136500 | 1.28 |
| PRKCE | 59 | 4953270 | G | 0.4677 | 0.0307800 | 1.24 |
| FBXO11 | 60 | 874869 | G | 0.5024 | 0.0275500 | 1.24 |
| PSME4 | 61 | 805404 | C | 0.2680 | 0.0059070 | 0.74 |
| PSME4 | 62 | 10183655 | T | 0.3862 | 0.0364200 | 0.81 |
| PSME4 | 63 | 17045488 | G | 0.2339 | 0.0055770 | 0.73 |
| ACYP2 | 64 | 6721970 | A | 0.2138 | 0.0007330 | 0.68 |
| CCDC85A | 65 | 10460558 | A | 0.2709 | 0.0002575 | 0.68 |
| AAK1 | 66 | 2871965 | T | 0.4320 | 0.0214300 | 1.26 |
| AAK1 | 67 | 3821277 | G | 0.4115 | 0.0156200 | 1.28 |
| AAK1 | 68 | 6546534 | T | 0.3067 | 0.0451800 | 1.24 |
| AAK1 | 69 | 6712370 | C | 0.2990 | 0.0170000 | 1.30 |
| AAK1 | 70 | 12713679 | G | 0.3777 | 0.0048500 | 1.34 |
| AAK1 | 71 | 10779953 | G | 0.3062 | 0.0317300 | 1.26 |
| AAK1 | 72 | 12471316 | G | 0.4379 | 0.0068780 | 1.31 |
| CTNNA2 | 77 | 17339556 | C | 0.3166 | 0.0012970 | 1.43 |
| CTNNA2 | 78 | 2861914 | G | 0.3270 | 0.0042960 | 1.36 |
| CTNNA2 | 79 | 2060393 | A | 0.4545 | 0.0048890 | 1.32 |
| CTNNA2 | 80 | 17018905 | C | 0.0914 | 0.0024250 | 1.80 |
| NAP5 | 85 | 10176860 | C | 0.2129 | 0.0247700 | 0.77 |
| LRP1B | 86 | 2171107 | A | 0.3043 | 0.0299400 | 1.27 |
| LRP1B | 87 | 10496906 | A | 0.3629 | 0.0014900 | 0.73 |
| LRP1B | 88 | 10183142 | T | 0.5291 | 0.0016930 | 1.36 |
| LRP1B | 89 | 1369542 | T | 0.4830 | 0.0064530 | 1.31 |
| LRP1B | 90 | 12623563 | A | 0.4149 | 0.0192400 | 0.79 |
| LRP1B | 91 | 13382235 | G | 0.0663 | 0.0081100 | 0.62 |
| KYNU | 92 | 10176234 | A | 0.3642 | 0.0281600 | 0.80 |
| KYNU | 93 | 6429997 | C | 0.3926 | 0.0293700 | 0.81 |
| KYNU | 94 | 3816193 | A | 0.1981 | 0.0485300 | 1.29 |
| KIF5C | 95 | 4667369 | C | 0.3618 | 0.0419700 | 0.81 |
| CACNB4 | 96 | 13421383 | T | 0.1885 | 0.0151300 | 0.75 |
| CACNB4 | 97 | 12693235 | C | 0.2297 | 0.0115900 | 0.75 |
| FMNL2 | 98 | 2164402 | G | 0.2530 | 0.0177800 | 0.77 |
| FMNL2 | 99 | 4664114 | T | 0.2817 | 0.0002634 | 0.68 |
| FMNL2 | 100 | 17327752 | A | 0.2380 | 0.0051350 | 0.73 |
| FMNL2 | 101 | 2346182 | G | 0.3329 | 0.0127600 | 0.78 |
| FMNL2 | 102 | 10497108 | C | 0.1158 | 0.0138200 | 0.70 |
| FMNL2 | 103 | 10497111 | A | 0.1208 | 0.0191800 | 0.72 |
| FMNL2 | 104 | 11682487 | C | 0.3341 | 0.0127800 | 0.78 |
| PKP4 | 105 | 2528593 | A | 0.3162 | 0.0288800 | 0.80 |
| PKP4 | 106 | 2711035 | C | 0.0660 | 0.0444400 | 0.69 |
| SCN7A | 107 | 11900439 | A | 0.3125 | 0.0247800 | 0.79 |
| CERKL | 108 | 10490688 | T | 0.3425 | 0.0315200 | 1.25 |
| CERKL | 109 | 895901 | A | 0.3481 | 0.0366200 | 1.24 |
| PDE1A | 110 | 3769794 | C | 0.1824 | 0.0444500 | 0.78 |
| PARD3B | 111 | 7559302 | T | 0.2990 | 0.0117400 | 0.77 |
| NRP2 | 112 | 13396083 | C | 0.4760 | 0.0440300 | 1.22 |
| PIP5K3 | 113 | 10497899 | T | 0.0323 | 0.0421200 | 1.95 |
| CNTN6 | 115 | 17038701 | G | 0.0649 | 0.0003231 | 2.43 |
| CNTN4 | 116 | 1502582 | T | 0.1993 | 0.0214500 | 1.34 |
| CNTN4 | 117 | 13092590 | G | 0.3007 | 0.0109100 | 0.77 |
| CNTN4 | 118 | 17194455 | T | 0.3659 | 0.0114200 | 0.78 |
| CNTN4 | 119 | 1551997 | C | 0.2876 | 0.0179900 | 0.78 |
| ITPR1 | 121 | 4685803 | A | 0.4639 | 0.0456300 | 1.22 |
| IRAK2 | 122 | 6804954 | A | 0.4294 | 0.0149200 | 1.28 |
| ATP2B2 | 123 | 13100027 | T | 0.0785 | 0.0023180 | 0.60 |
| ATP2B2 | 124 | 9860273 | T | 0.0704 | 0.0152900 | 0.65 |
| SLC6A6 | 125 | 2241780 | C | 0.1901 | 0.0316600 | 0.77 |
| SLC6A6 | 126 | 2304510 | G | 0.1432 | 0.0209700 | 0.74 |
| DAZL | 127 | 890640 | A | 0.1594 | 0.0006643 | 1.65 |
| DAZL | 128 | 4234537 | C | 0.1516 | 0.0071020 | 1.49 |
| ARPP-21 | 129 | 735353 | A | 0.4453 | 0.0280100 | 1.25 |
| ARPP-21 | 130 | 11920715 | T | 0.4471 | 0.0414800 | 1.23 |
| STAC | 131 | 17035045 | T | 0.2041 | 0.0146400 | 0.75 |
| STAC | 132 | 4678878 | C | 0.3556 | 0.0278600 | 0.80 |
| ULK4 | 133 | 7627972 | A | 0.4809 | 0.0397100 | 1.22 |
| ULK4 | 134 | 11129908 | G | 0.5132 | 0.0276800 | 1.24 |
| ULK4 | 135 | 7618902 | G | 0.3037 | 0.0192100 | 1.30 |
| ULK4 | 136 | 9878069 | G | 0.3723 | 0.0496100 | 1.22 |
| ULK4 | 137 | 1495696 | C | 0.4566 | 0.0449000 | 1.22 |
| ZNF167 | 138 | 13081859 | G | 0.2876 | 0.0395700 | 0.80 |
| CACNA2D3 | 139 | 17054677 | A | 0.2005 | 0.0069870 | 1.42 |
| FLNB | 140 | 12632456 | A | 0.2852 | 0.0165800 | 1.31 |
| FHIT | 142 | 13320646 | A | 0.4523 | 0.0019850 | 1.37 |
| FHIT | 143 | 7631246 | T | 0.4487 | 0.0034280 | 1.34 |
| FHIT | 144 | 9875228 | T | 0.1811 | 0.0422500 | 1.31 |
| FHIT | 145 | 1470035 | A | 0.4422 | 0.0075020 | 1.31 |
| FHIT | 146 | 9843007 | T | 0.3971 | 0.0209600 | 1.26 |
| FHIT | 147 | 4688167 | C | 0.2955 | 0.0451000 | 1.25 |
| PRICKLE2 | 148 | 17665101 | G | 0.1938 | 0.0432600 | 0.79 |
| ADAMTS9 | 149 | 9847307 | T | 0.0167 | 0.0073290 | 4.73 |
| ADAMTS9 | 150 | 6787633 | G | 0.0131 | 0.0122100 | 5.56 |
| MAGI1 | 151 | 7612634 | C | 0.5181 | 0.0053860 | 1.32 |
| MAGI1 | 152 | 7612636 | G | 0.5180 | 0.0090200 | 1.29 |
| MAGI1 | 153 | 7612644 | C | 0.5181 | 0.0104800 | 1.29 |
| FOXP1 | 154 | 2597312 | C | 0.3565 | 0.0316900 | 1.25 |
| GBE1 | 157 | 2307058 | T | 0.3108 | 0.0168800 | 1.30 |
| GBE1 | 158 | 7613144 | T | 0.3115 | 0.0203600 | 1.29 |
| GBE1 | 159 | 3772891 | A | 0.2271 | 0.0398500 | 0.79 |
| EPHA6 | 160 | 16838196 | A | 0.0503 | 0.0304900 | 0.64 |
| PLCXD2 | 163 | 13074817 | G | 0.4112 | 0.0131700 | 0.78 |
| PLCXD2 | 164 | 4284954 | C | 0.4157 | 0.0146100 | 0.78 |
| PLCXD2 | 165 | 13079085 | C | 0.4161 | 0.0207800 | 0.80 |
| PLCXD2 | 166 | 6768713 | A | 0.4163 | 0.0312900 | 0.81 |
| PLCXD2 | 167 | 12490166 | A | 0.4233 | 0.0435100 | 0.82 |
| EPHB1 | 168 | 39704 | A | 0.4638 | 0.0343900 | 0.81 |
| SPSB4 | 169 | 7620622 | A | 0.2452 | 0.0054850 | 1.40 |
| SPSB4 | 170 | 6780365 | C | 0.1783 | 0.0007646 | 1.60 |
| SPSB4 | 171 | 2086180 | G | 0.1271 | 0.0018360 | 1.66 |
| SERPINI2 | 172 | 4955673 | C | 0.5099 | 0.0096070 | 1.30 |
| SERPINI2 | 173 | 17246389 | C | 0.2715 | 0.0186500 | 1.31 |
| SERPINI2 | 174 | 13062550 | C | 0.4842 | 0.0225700 | 1.25 |
| TNIK | 175 | 9824475 | G | 0.2649 | 0.0045900 | 0.74 |
| PLD1 | 176 | 187229 | T | 0.4171 | 0.0245500 | 1.25 |
| PLD1 | 177 | 181715 | T | 0.4077 | 0.0343400 | 1.24 |
| NLGN1 | 179 | 10936780 | G | 0.0831 | 0.0015080 | 0.60 |
| NLGN1 | 180 | 12634066 | A | 0.0827 | 0.0017380 | 0.60 |
| NLGN1 | 181 | 9828801 | T | 0.0670 | 0.0140000 | 0.64 |
| LEPREL1 | 182 | 9835230 | A | 0.2226 | 0.0027880 | 0.71 |
| LEPREL1 | 183 | 1447935 | T | 0.1896 | 0.0051530 | 0.72 |
| LEPREL1 | 184 | 6444412 | T | 0.2859 | 0.0040510 | 0.74 |
| LEPREL1 | 185 | 1447936 | C | 0.2026 | 0.0104000 | 0.74 |
| LEPREL1 | 186 | 7632740 | C | 0.3050 | 0.0301500 | 0.80 |
| LEPREL1 | 187 | 1526031 | C | 0.2041 | 0.0406100 | 0.79 |
| UBXD7 | 188 | 6774540 | T | 0.4509 | 0.0324200 | 0.81 |
| UBXD7 | 189 | 9841810 | C | 0.4523 | 0.0450600 | 0.82 |
| UBXD7 | 190 | 9847223 | T | 0.4547 | 0.0450800 | 0.82 |
| SLC2A9 | 191 | 1568318 | C | 0.2339 | 0.0444200 | 1.27 |
| SLC2A9 | 192 | 4697692 | T | 0.2124 | 0.0477700 | 1.28 |
| SLC2A9 | 193 | 3733591 | A | 0.1679 | 0.0431800 | 0.78 |
| LDB2 | 194 | 157631 | G | 0.0728 | 0.0204900 | 0.67 |
| LDB2 | 195 | 284210 | T | 0.0704 | 0.0339900 | 0.69 |
| KIAA1239 | 203 | 17575883 | A | 0.1650 | 0.0236800 | 0.75 |
| KIAA1239 | 204 | 17575897 | T | 0.1699 | 0.0341100 | 0.77 |
| UBE2K | 205 | 192779 | A | 0.1835 | 0.0004961 | 0.66 |
| LIMCH1 | 206 | 6447080 | G | 0.4263 | 0.0048350 | 1.33 |
| LIMCH1 | 207 | 6447081 | G | 0.4258 | 0.0068670 | 1.31 |
| LIMCH1 | 208 | 4610372 | G | 0.4259 | 0.0082180 | 1.31 |
| LIMCH1 | 209 | 4861118 | A | 0.4195 | 0.0115200 | 1.29 |
| LOC389207 | 211 | 13105520 | T | 0.3474 | 0.0006047 | 0.71 |
| LOC389207 | 212 | 9994547 | A | 0.3507 | 0.0019500 | 0.73 |
| LOC389207 | 213 | 1561147 | G | 0.2993 | 0.0038420 | 0.74 |
| SCD5 | 214 | 3897960 | A | 0.1791 | 0.0068990 | 0.72 |
| SCD5 | 215 | 17352017 | G | 0.1325 | 0.0383600 | 0.75 |
| HERC3 | 216 | 2972021 | A | 0.2733 | 0.0220900 | 1.30 |
| HERC3 | 217 | 3017906 | G | 0.2977 | 0.0396900 | 1.26 |
| FAM13A1 | 218 | 7657817 | T | 0.1778 | 0.0187500 | 1.37 |
| FAM13A1 | 219 | 12507401 | C | 0.2175 | 0.0200500 | 1.34 |
| COL25A1 | 220 | 9996734 | A | 0.3973 | 0.0476000 | 1.22 |
| ANK2 | 221 | 11942005 | G | 0.1555 | 0.0380200 | 1.34 |
| CAMK2D | 222 | 11098193 | T | 0.2871 | 0.0442900 | 1.25 |
| NDST3 | 223 | 6534079 | A | 0.2146 | 0.0433700 | 0.79 |
| NDST3 | 224 | 4576085 | G | 0.1108 | 0.0017390 | 0.64 |
| GPR103 | 225 | 13110738 | G | 0.2069 | 0.0025070 | 1.48 |
| MAML3 | 226 | 3796633 | G | 0.0776 | 0.0462600 | 0.71 |
| IL15 | 227 | 2139383 | T | 0.5132 | 0.0274900 | 1.24 |
| IL15 | 228 | 1389099 | T | 0.3194 | 0.0056800 | 0.75 |
| IL15 | 229 | 1389098 | T | 0.5048 | 0.0315000 | 1.23 |
| INPP4B | 234 | 1497389 | T | 0.3471 | 0.0057580 | 0.76 |
| INPP4B | 236 | 17715707 | G | 0.3026 | 0.0251400 | 0.79 |
| POU4F2 | 240 | 1104532 | A | 0.1177 | 0.0004148 | 0.61 |
| POU4F2 | 241 | 1394279 | G | 0.1187 | 0.0004232 | 0.61 |
| DCLK2 | 242 | 6535725 | A | 0.4542 | 0.0234900 | 1.25 |
| FSTL5 | 244 | 2082669 | G | 0.1953 | 0.0016150 | 1.53 |
| FSTL5 | 245 | 6814301 | T | 0.3213 | 0.0236900 | 0.79 |
| TLL1 | 246 | 17633393 | T | 0.0293 | 0.0080040 | 2.73 |
| PALLD | 247 | 924511 | T | 0.4199 | 0.0087490 | 1.30 |
| PALLD | 248 | 10033898 | C | 0.4939 | 0.0455700 | 1.22 |
| CASP3 | 249 | 9685847 | G | 0.4426 | 0.0108700 | 0.78 |
| CASP3 | 250 | 2720376 | C | 0.4439 | 0.0146200 | 0.79 |
| PLEKHG4B | 251 | 6554947 | G | 0.3128 | 0.0069270 | 1.35 |
| PLEKHG4B | 252 | 17559144 | T | 0.4545 | 0.0269000 | 1.25 |
| AHRR | 253 | 2721004 | T | 0.3075 | 0.0004755 | 1.48 |
| AHRR | 254 | 2721012 | G | 0.2555 | 0.0012100 | 1.48 |
| DNAH5 | 260 | 6554811 | G | 0.4010 | 0.0186700 | 0.79 |
| DNAH5 | 261 | 2401810 | G | 0.3532 | 0.0186800 | 0.79 |
| DNAH5 | 265 | 10513155 | A | 0.3329 | 0.0023550 | 1.39 |
| DNAH5 | 266 | 7709692 | C | 0.2094 | 0.0017740 | 0.70 |
| DNAH5 | 268 | 9885366 | C | 0.4390 | 0.0002241 | 1.45 |
| DNAH5 | 269 | 1867679 | T | 0.4010 | 0.0059750 | 1.32 |
| DNAH5 | 270 | 6896894 | C | 0.3969 | 0.0060990 | 1.32 |
| DNAH5 | 271 | 16902953 | G | 0.3178 | 0.0372700 | 1.25 |
| DNAH5 | 272 | 9312854 | C | 0.3122 | 0.0468600 | 1.24 |
| DNAH5 | 273 | 339428 | T | 0.5240 | 0.0020490 | 1.35 |
| DNAH5 | 274 | 339424 | T | 0.2446 | 0.0135500 | 1.34 |
| CDH10 | 275 | 7714498 | A | 0.1843 | 0.0482000 | 0.79 |
| CDH10 | 276 | 1896890 | T | 0.2243 | 0.0499700 | 0.80 |
| SLC45A2 | 277 | 10461928 | G | 0.3950 | 0.0260300 | 0.80 |
| SLC45A2 | 278 | 35389 | G | 0.0465 | 0.0188900 | 1.89 |
| SLC1A3 | 279 | 1645651 | A | 0.3616 | 0.0172100 | 1.28 |
| SLC1A3 | 280 | 3776569 | G | 0.4747 | 0.0082700 | 1.30 |
| EGFLAM | 281 | 4869580 | T | 0.1283 | 0.0038730 | 1.59 |
| EGFLAM | 282 | 2589787 | A | 0.0837 | 0.0368600 | 1.50 |
| EGFLAM | 283 | 2434504 | A | 0.4580 | 0.0105700 | 1.29 |
| EGFLAM | 284 | 1428263 | C | 0.2888 | 0.0126300 | 1.33 |
| EGFLAM | 285 | 9292705 | A | 0.0897 | 0.0015590 | 1.86 |
| EGFLAM | 286 | 2731979 | C | 0.1607 | 0.0156600 | 1.41 |
| ITGA2 | 288 | 7719848 | A | 0.2838 | 0.0385800 | 1.26 |
| ELOVL7 | 290 | 4700394 | T | 0.5240 | 0.0082730 | 1.30 |
| ELOVL7 | 291 | 17332824 | C | 0.3961 | 0.0497500 | 0.82 |
| TNPO1 | 292 | 7709640 | C | 0.3468 | 0.0000269 | 1.58 |
| TNPO1 | 293 | 153320 | T | 0.1815 | 0.0024290 | 1.51 |
| TNPO1 | 294 | 34653 | T | 0.1790 | 0.0032120 | 1.50 |
| TNPO1 | 295 | 266444 | C | 0.1766 | 0.0045020 | 1.48 |
| TNPO1 | 296 | 2548331 | A | 0.4270 | 0.0001619 | 0.69 |
| FCHO2 | 297 | 478575 | C | 0.5024 | 0.0001957 | 1.45 |
| FCHO2 | 298 | 185435 | G | 0.1731 | 0.0116100 | 1.42 |
| FCHO2 | 299 | 2277017 | C | 0.3244 | 0.0417000 | 1.25 |
| CMYA5 | 300 | 6880680 | C | 0.0981 | 0.0160600 | 1.54 |
| CMYA5 | 301 | 3828611 | G | 0.0740 | 0.0182300 | 1.64 |
| CMYA5 | 302 | 1991483 | G | 0.3750 | 0.0384900 | 1.24 |
| THBS4 | 303 | 256444 | C | 0.1842 | 0.0279200 | 1.34 |
| THBS4 | 304 | 7736549 | A | 0.1496 | 0.0185900 | 1.42 |
| VCAN | 305 | 178024 | A | 0.3789 | 0.0423000 | 1.23 |
| MEF2C | 306 | 700592 | G | 0.4224 | 0.0215300 | 1.26 |
| GPR98 | 307 | 16868917 | G | 0.0586 | 0.0309900 | 0.66 |
| GPR98 | 309 | 2222244 | A | 0.3701 | 0.0473700 | 1.23 |
| FBXL17 | 311 | 12519388 | T | 0.1940 | 0.0205700 | 1.35 |
| FBXL17 | 312 | 12189428 | G | 0.1902 | 0.0253400 | 1.34 |
| FBXL17 | 316 | 7731562 | C | 0.1843 | 0.0122300 | 1.40 |
| FBXL17 | 318 | 10515385 | C | 0.1850 | 0.0251400 | 1.35 |
| FBXL17 | 319 | 11242664 | G | 0.1795 | 0.0312100 | 1.34 |
| PJA2 | 320 | 1045706 | A | 0.4221 | 0.0179100 | 1.27 |
| PJA2 | 321 | 2963046 | T | 0.3741 | 0.0295800 | 1.25 |
| SNX2 | 322 | 4343835 | C | 0.3942 | 0.0015170 | 0.73 |
| SNX2 | 323 | 4835894 | C | 0.2983 | 0.0055500 | 0.75 |
| SNX2 | 324 | 6872932 | T | 0.4819 | 0.0009375 | 1.39 |
| SNX2 | 325 | 3822367 | G | 0.4795 | 0.0024510 | 1.35 |
| SNX2 | 326 | 10519715 | A | 0.2998 | 0.0078400 | 0.76 |
| SNX2 | 327 | 2407403 | G | 0.3708 | 0.0228200 | 1.27 |
| SNX24 | 328 | 27740 | T | 0.4832 | 0.0016230 | 1.37 |
| SNX24 | 329 | 6888023 | T | 0.3014 | 0.0051010 | 0.75 |
| SNX24 | 330 | 246286 | T | 0.3925 | 0.0300700 | 1.25 |
| SNX24 | 331 | 246266 | C | 0.3927 | 0.0378300 | 1.24 |
| SNX24 | 332 | 1038078 | T | 0.1295 | 0.0318400 | 1.40 |
| SNX24 | 333 | 30028 | C | 0.3462 | 0.0394200 | 1.24 |
| SNX24 | 334 | 385996 | G | 0.3584 | 0.0406900 | 1.24 |
| ADAMTS19 | 335 | 30651 | G | 0.3628 | 0.0229300 | 1.27 |
| TRPC7 | 336 | 950714 | G | 0.1196 | 0.0005632 | 1.80 |
| TRPC7 | 337 | 2649691 | A | 0.1446 | 0.0067400 | 1.51 |
| CTNNA1 | 338 | 906695 | G | 0.2718 | 0.0003555 | 0.68 |
| CTNNA1 | 339 | 10038162 | A | 0.2757 | 0.0010250 | 0.71 |
| CTNNA1 | 340 | 10045605 | C | 0.2745 | 0.0012850 | 0.71 |
| ODZ2 | 341 | 1560654 | T | 0.2163 | 0.0093960 | 0.74 |
| ODZ2 | 342 | 3733988 | C | 0.5024 | 0.0169100 | 1.27 |
| ODZ2 | 343 | 17632540 | T | 0.2723 | 0.0285300 | 0.79 |
| ODZ2 | 344 | 1422421 | T | 0.4272 | 0.0087630 | 1.30 |
| SLC22A23 | 347 | 17136575 | G | 0.1651 | 0.0137700 | 0.73 |
| RNF144B | 354 | 2038745 | A | 0.1539 | 0.0207700 | 0.74 |
| RNF144B | 355 | 1408267 | G | 0.2017 | 0.0225200 | 0.77 |
| RNF144B | 356 | 1324573 | G | 0.4189 | 0.0106700 | 0.78 |
| RNF144B | 358 | 1924467 | T | 0.4077 | 0.0077010 | 1.31 |
| RNF144B | 359 | 9477749 | C | 0.4270 | 0.0189100 | 1.27 |
| RNF144B | 360 | 9396868 | T | 0.1169 | 0.0444400 | 0.75 |
| SLC17A4 | 361 | 1892248 | T | 0.2422 | 0.0008890 | 0.70 |
| SLC17A4 | 362 | 12662869 | A | 0.2566 | 0.0001516 | 0.67 |
| SLC17A1 | 363 | 1575535 | T | 0.2421 | 0.0001745 | 0.66 |
| SLC17A1 | 364 | 1165196 | C | 0.4759 | 0.0138100 | 1.28 |
| SLC17A1 | 365 | 1165177 | T | 0.5000 | 0.0196400 | 1.26 |
| SLC17A1 | 366 | 2070642 | T | 0.2398 | 0.0006451 | 0.69 |
| SLC17A1 | 367 | 4712976 | T | 0.2272 | 0.0027410 | 0.71 |
| SLC17A3 | 368 | 1165205 | T | 0.5097 | 0.0026860 | 1.35 |
| SLC17A2 | 369 | 9467652 | G | 0.3553 | 0.0061210 | 0.76 |
| BTN3A1 | 371 | 17610161 | A | 0.1310 | 0.0157600 | 1.47 |
| BTN3A1 | 372 | 10946824 | G | 0.1854 | 0.0209400 | 1.36 |
| BTN2A3 | 373 | 7751280 | T | 0.1826 | 0.0290400 | 1.34 |
| BTN2A3 | 374 | 10946829 | A | 0.1826 | 0.0290400 | 1.34 |
| BTN3A3 | 375 | 3846847 | T | 0.1818 | 0.0322200 | 1.33 |
| BTNL2 | 376 | 2076533 | A | 0.4627 | 0.0294300 | 1.24 |
| BTNL2 | 377 | 3763307 | T | 0.3062 | 0.0432400 | 1.25 |
| LRFN2 | 378 | 1347257 | T | 0.4356 | 0.0450700 | 0.82 |
| ELOVL5 | 405 | 9474507 | A | 0.1492 | 0.0003639 | 0.63 |
| RIMS1 | 408 | 2250128 | C | 0.3194 | 0.0375900 | 0.80 |
| KLHL32 | 409 | 1206084 | A | 0.2488 | 0.0006100 | 0.69 |
| KLHL32 | 410 | 1629523 | T | 0.1247 | 0.0062920 | 0.68 |
| KLHL32 | 411 | 1737646 | A | 0.1253 | 0.0115400 | 0.70 |
| KLHL32 | 412 | 1766518 | G | 0.1482 | 0.0207100 | 0.74 |
| KLHL32 | 413 | 2294763 | G | 0.0239 | 0.0161500 | 0.51 |
| KLHL32 | 414 | 1206149 | A | 0.4964 | 0.0414000 | 1.22 |
| SLC22A16 | 415 | 17071719 | A | 0.1043 | 0.0000963 | 2.09 |
| SLC22A16 | 416 | 17071722 | T | 0.1081 | 0.0002709 | 1.96 |
| SLC22A16 | 417 | 7768422 | C | 0.1074 | 0.0004135 | 1.90 |
| HS3ST5 | 418 | 1334900 | T | 0.3952 | 0.0002151 | 1.47 |
| TRDN | 419 | 1570187 | C | 0.3046 | 0.0118000 | 0.77 |
| TRDN | 420 | 6915835 | C | 0.3075 | 0.0384000 | 0.80 |
| TRDN | 421 | 2169092 | T | 0.3911 | 0.0472200 | 0.82 |
| NKAIN2 | 422 | 173067 | A | 0.3726 | 0.0432500 | 1.23 |
| EYA4 | 423 | 9321395 | A | 0.2296 | 0.0126200 | 1.36 |
| EYA4 | 424 | 9321396 | G | 0.2297 | 0.0240800 | 1.31 |
| EYA4 | 425 | 9375953 | A | 0.1942 | 0.0297400 | 1.33 |
| EYA4 | 426 | 17641157 | G | 0.1860 | 0.0135400 | 1.39 |
| PDE7B | 428 | 6906788 | T | 0.0731 | 0.0435000 | 1.52 |
| PDE7B | 429 | 6938424 | T | 0.0501 | 0.0348800 | 1.71 |
| PLAGL1 | 430 | 6916498 | A | 0.2315 | 0.0328400 | 1.29 |
| PLAGL1 | 431 | 9484836 | A | 0.2307 | 0.0390900 | 1.28 |
| UTRN | 432 | 9496982 | T | 0.2589 | 0.0330600 | 1.28 |
| SYNE1 | 433 | 2252755 | G | 0.3732 | 0.0324300 | 1.25 |
| SYNE1 | 434 | 718527 | A | 0.4163 | 0.0067790 | 0.77 |
| SYNE1 | 435 | 2763016 | C | 0.3365 | 0.0444900 | 0.81 |
| TFB1M | 436 | 170843 | T | 0.2826 | 0.0066610 | 0.75 |
| TFB1M | 437 | 7775163 | A | 0.3819 | 0.0013430 | 1.40 |
| PARK2 | 438 | 12055483 | T | 0.3878 | 0.0329400 | 0.81 |
| PARK2 | 439 | 6924602 | C | 0.3974 | 0.0432300 | 0.82 |
| PARK2 | 440 | 9355924 | A | 0.3786 | 0.0159500 | 0.79 |
| PDE10A | 441 | 525643 | A | 0.1355 | 0.0128200 | 0.71 |
| PDE10A | 442 | 484842 | G | 0.1543 | 0.0409300 | 0.77 |
| SDK1 | 443 | 663466 | A | 0.2002 | 0.0153800 | 0.75 |
| SDK1 | 444 | 649186 | C | 0.2404 | 0.0369700 | 0.79 |
| SDK1 | 445 | 10951446 | A | 0.4260 | 0.0087200 | 0.77 |
| SDK1 | 446 | 4723434 | G | 0.5060 | 0.0189100 | 1.26 |
| SCIN | 450 | 702477 | C | 0.3029 | 0.0059970 | 0.75 |
| SCIN | 451 | 849760 | C | 0.3498 | 0.0047510 | 0.75 |
| FERD3L | 458 | 11766540 | C | 0.2488 | 0.0033220 | 0.72 |
| STK31 | 459 | 4722266 | A | 0.1723 | 0.0057610 | 1.47 |
| SKAP2 | 460 | 17372780 | C | 0.3580 | 0.0096670 | 1.31 |
| SKAP2 | 461 | 3801846 | A | 0.2550 | 0.0031060 | 1.43 |
| SKAP2 | 462 | 607099 | G | 0.5120 | 0.0098110 | 1.29 |
| SKAP2 | 463 | 13438514 | A | 0.2488 | 0.0056350 | 1.39 |
| SKAP2 | 464 | 17315929 | T | 0.2458 | 0.0080410 | 1.37 |
| SKAP2 | 465 | 10486483 | A | 0.2452 | 0.0122600 | 1.35 |
| SKAP2 | 466 | 3801844 | C | 0.2518 | 0.0033500 | 1.42 |
| CREB5 | 467 | 6977628 | T | 0.2694 | 0.0174400 | 1.31 |
| CREB5 | 468 | 886750 | A | 0.2691 | 0.0180700 | 1.31 |
| CREB5 | 469 | 10225559 | C | 0.2639 | 0.0414100 | 1.26 |
| CHN2 | 470 | 245927 | A | 0.5323 | 0.0003594 | 1.42 |
| PDE1C | 471 | 30568 | C | 0.1615 | 0.0153700 | 1.41 |
| PDE1C | 472 | 30589 | G | 0.1289 | 0.0451600 | 1.36 |
| PDE1C | 473 | 30586 | A | 0.1103 | 0.0101600 | 1.56 |
| BMPER | 474 | 11768532 | A | 0.1787 | 0.0104600 | 0.73 |
| BMPER | 475 | 1946145 | T | 0.2560 | 0.0081850 | 0.75 |
| BMPER | 476 | 1420335 | C | 0.3042 | 0.0207500 | 0.78 |
| EEPD1 | 477 | 11765336 | G | 0.5237 | 0.0256800 | 1.25 |
| EEPD1 | 478 | 197352 | C | 0.2753 | 0.0306100 | 0.79 |
| VPS41 | 479 | 6462865 | G | 0.4044 | 0.0110700 | 0.78 |
| VPS41 | 480 | 17618395 | G | 0.3923 | 0.0167700 | 0.79 |
| VPS41 | 481 | 4723793 | G | 0.3840 | 0.0086410 | 1.31 |
| VPS41 | 482 | 3801127 | G | 0.5216 | 0.0392500 | 1.22 |
| VPS41 | 483 | 10085898 | C | 0.4510 | 0.0189400 | 0.79 |
| VPS41 | 484 | 12532461 | A | 0.2500 | 0.0129200 | 1.34 |
| VPS41 | 485 | 859522 | G | 0.0962 | 0.0223400 | 1.52 |
| CDC2L5 | 486 | 12536726 | G | 0.0944 | 0.0054230 | 1.69 |
| ABCA13 | 490 | 17132335 | A | 0.0716 | 0.0243200 | 0.67 |
| ABCA13 | 491 | 6958356 | A | 0.3842 | 0.0416300 | 0.82 |
| GRB10 | 492 | 6593182 | G | 0.1551 | 0.0308400 | 0.76 |
| GRB10 | 493 | 4947465 | C | 0.1277 | 0.0182900 | 0.72 |
| MAGI2 | 503 | 12531031 | T | 0.1811 | 0.0121700 | 1.40 |
| MAGI2 | 504 | 1918930 | C | 0.1630 | 0.0406500 | 1.33 |
| MAGI2 | 505 | 7782875 | C | 0.0206 | 0.0309200 | 0.52 |
| MAGI2 | 506 | 2191806 | C | 0.1763 | 0.0234700 | 1.36 |
| MAGI2 | 508 | 1528267 | C | 0.1687 | 0.0355400 | 1.34 |
| SEMA3E | 510 | 3801535 | G | 0.2404 | 0.0092120 | 1.37 |
| ABCB4 | 511 | 31671 | G | 0.2309 | 0.0022770 | 1.46 |
| GTPBP10 | 512 | 11972149 | T | 0.2258 | 0.0487400 | 1.28 |
| PPP1R9A | 521 | 1918482 | A | 0.4723 | 0.0160800 | 1.27 |
| PPP1R9A | 522 | 12690919 | T | 0.4395 | 0.0194500 | 0.79 |
| PPP1R9A | 523 | 854520 | T | 0.4303 | 0.0104200 | 0.78 |
| PPP1R9A | 524 | 854535 | C | 0.4236 | 0.0356900 | 0.81 |
| DYNC1I1 | 526 | 42066 | A | 0.1103 | 0.0002832 | 0.59 |
| ZNF3 | 527 | 4424195 | G | 0.2902 | 0.0113800 | 1.33 |
| ZNF3 | 528 | 941290 | A | 0.1509 | 0.0101000 | 0.71 |
| KCND2 | 539 | 17142782 | G | 0.0324 | 0.0090350 | 2.49 |
| CADPS2 | 543 | 1476898 | T | 0.1878 | 0.0180500 | 1.37 |
| CADPS2 | 544 | 2471194 | A | 0.1897 | 0.0268400 | 1.34 |
| CADPS2 | 545 | 17144752 | T | 0.1933 | 0.0281300 | 1.33 |
| SLC13A1 | 546 | 10500090 | C | 0.2512 | 0.0003513 | 0.68 |
| SLC13A1 | 548 | 2140516 | C | 0.2709 | 0.0005947 | 0.69 |
| EXOC4 | 569 | 12536963 | G | 0.3055 | 0.0186200 | 0.78 |
| TBXAS1 | 578 | 12534299 | G | 0.4199 | 0.0494600 | 0.82 |
| PTPRN2 | 592 | 12674047 | T | 0.2344 | 0.0493700 | 1.26 |
| CSMD1 | 598 | 7845140 | G | 0.0537 | 0.0419000 | 1.64 |
| CSMD1 | 604 | 895695 | G | 0.5167 | 0.0165200 | 1.27 |
| CSMD1 | 605 | 12543159 | T | 0.4255 | 0.0181100 | 0.79 |
| CSMD1 | 606 | 1161518 | T | 0.3046 | 0.0177000 | 0.78 |
| MCPH1 | 608 | 2515514 | A | 0.1993 | 0.0007415 | 1.56 |
| MCPH1 | 609 | 11994063 | T | 0.1957 | 0.0379400 | 0.78 |
| SGCZ | 610 | 7822045 | A | 0.2737 | 0.0261500 | 1.29 |
| SLC7A2 | 611 | 2588233 | C | 0.4658 | 0.0074110 | 1.31 |
| SLC7A2 | 612 | 2237817 | C | 0.3902 | 0.0006021 | 1.42 |
| PSD3 | 613 | 17595783 | C | 0.2086 | 0.0252700 | 1.33 |
| PSD3 | 614 | 13275817 | A | 0.2990 | 0.0031010 | 1.39 |
| PSD3 | 615 | 1873085 | C | 0.3138 | 0.0094100 | 1.33 |
| PSD3 | 616 | 1565231 | T | 0.4725 | 0.0333100 | 1.23 |
| ENTPD4 | 627 | 17089269 | C | 0.0728 | 0.0410100 | 1.53 |
| UNC5D | 628 | 2589344 | T | 0.1778 | 0.0302100 | 0.77 |
| SNTG1 | 629 | 906656 | C | 0.3589 | 0.0433900 | 1.23 |
| SNTG1 | 630 | 11986411 | G | 0.3208 | 0.0364800 | 1.26 |
| LYN | 631 | 868541 | A | 0.4366 | 0.0205200 | 0.79 |
| LYN | 632 | 17812677 | T | 0.2201 | 0.0084810 | 0.74 |
| LYN | 633 | 16922508 | C | 0.2302 | 0.0171600 | 0.76 |
| LYN | 634 | 6988853 | A | 0.2476 | 0.0208000 | 0.77 |
| LYN | 635 | 2668003 | C | 0.2464 | 0.0223500 | 0.78 |
| PLAG1 | 636 | 7830138 | C | 0.1811 | 0.0090210 | 0.73 |
| PLAG1 | 637 | 13273123 | G | 0.1627 | 0.0116500 | 0.73 |
| NKAIN3 | 642 | 1901409 | C | 0.3852 | 0.0063910 | 0.76 |
| NKAIN3 | 643 | 10504354 | A | 0.3210 | 0.0449600 | 0.81 |
| NKAIN3 | 644 | 10504356 | G | 0.0803 | 0.0076550 | 0.64 |
| NKAIN3 | 645 | 10504358 | G | 0.0800 | 0.0252100 | 0.69 |
| DEPDC2 | 646 | 3793379 | A | 0.1379 | 0.0138700 | 1.46 |
| DEPDC2 | 647 | 3793381 | A | 0.1355 | 0.0311500 | 1.39 |
| CRISPLD1 | 648 | 16939013 | A | 0.1615 | 0.0015940 | 0.67 |
| CRISPLD1 | 649 | 1455795 | T | 0.0921 | 0.0321600 | 0.71 |
| CRISPLD1 | 650 | 7826672 | T | 0.2883 | 0.0012110 | 0.71 |
| CRISPLD1 | 651 | 6472888 | T | 0.2739 | 0.0009918 | 0.71 |
| CRISPLD1 | 652 | 16939030 | C | 0.1739 | 0.0344300 | 0.77 |
| MMP16 | 653 | 2139481 | T | 0.4081 | 0.0348300 | 1.24 |
| BAALC | 654 | 13279226 | A | 0.2542 | 0.0107500 | 0.76 |
| ZFPM2 | 655 | 13280496 | C | 0.3012 | 0.0056020 | 0.75 |
| ZFPM2 | 656 | 12679854 | T | 0.3365 | 0.0253900 | 0.80 |
| ZFPM2 | 657 | 6981187 | G | 0.3393 | 0.0296600 | 0.80 |
| ZFPM2 | 658 | 6469014 | T | 0.1775 | 0.0233600 | 1.36 |
| ZFPM2 | 659 | 11986654 | G | 0.0829 | 0.0454900 | 1.47 |
| CSMD3 | 661 | 1492678 | T | 0.5025 | 0.0352900 | 1.23 |
| CSMD3 | 663 | 17640016 | A | 0.2745 | 0.0186700 | 1.31 |
| SAMD12 | 677 | 9642841 | C | 0.3627 | 0.0491000 | 1.23 |
| FFR1L6 | 683 | 6981430 | G | 0.3759 | 0.0047760 | 0.75 |
| FFR1L6 | 684 | 7831422 | G | 0.1154 | 0.0189700 | 0.71 |
| FFR1L6 | 685 | 12375254 | A | 0.1196 | 0.0197900 | 0.72 |
| DDEF1 | 691 | 12675390 | C | 0.2512 | 0.0098780 | 0.75 |
| DDEF1 | 692 | 4733776 | T | 0.3840 | 0.0307800 | 0.80 |
| DDEF1 | 693 | 4733572 | A | 0.3973 | 0.0384400 | 0.81 |
| DDEF1 | 694 | 4733788 | G | 0.2069 | 0.0292300 | 0.78 |
| DDEF1 | 695 | 11786705 | G | 0.2105 | 0.0377300 | 0.78 |
| COL22A1 | 701 | 13271565 | T | 0.2494 | 0.0266400 | 1.30 |
| COL22A1 | 703 | 10104531 | T | 0.3726 | 0.0223300 | 1.27 |
| COL22A1 | 704 | 12674962 | C | 0.4615 | 0.0233700 | 1.25 |
| COL22A1 | 705 | 9324496 | T | 0.3906 | 0.0209500 | 1.27 |
| ZC3H3 | 706 | 4874147 | A | 0.3235 | 0.0017120 | 1.41 |
| PIP5K1B | 709 | 963707 | G | 0.3329 | 0.0294700 | 0.80 |
| PIP5K1B | 710 | 1414949 | C | 0.5195 | 0.0205100 | 1.26 |
| TRPM3 | 711 | 718447 | A | 0.2220 | 0.0377800 | 0.79 |
| TMC1 | 712 | 1417614 | T | 0.3493 | 0.0118900 | 1.30 |
| TMC1 | 713 | 2793153 | G | 0.4663 | 0.0051150 | 1.32 |
| TMC1 | 714 | 2589615 | A | 0.4672 | 0.0052340 | 1.32 |
| TMC1 | 715 | 1796991 | G | 0.1950 | 0.0420100 | 1.30 |
| TMC1 | 716 | 13286194 | C | 0.0517 | 0.0086850 | 0.59 |
| PCSK5 | 717 | 1029034 | G | 0.3489 | 0.0285400 | 0.80 |
| GNAQ | 718 | 964423 | T | 0.2907 | 0.0443700 | 1.25 |
| GNAQ | 719 | 7032206 | A | 0.2476 | 0.0197300 | 1.32 |
| GNAQ | 720 | 6560625 | C | 0.2861 | 0.0025030 | 1.41 |
| NTRK2 | 721 | 7038866 | A | 0.2661 | 0.0426500 | 0.80 |
| DIRAS2 | 722 | 690232 | A | 0.2587 | 0.0229200 | 0.78 |
| DIRAS2 | 723 | 1360471 | C | 0.2232 | 0.0404200 | 0.79 |
| NFIL3 | 724 | 2440589 | T | 0.4654 | 0.0235300 | 1.25 |
| FKTN | 730 | 885954 | C | 0.4666 | 0.0206900 | 1.26 |
| FKTN | 731 | 17251166 | C | 0.3305 | 0.0368900 | 1.25 |
| PALM2 | 732 | 1535457 | A | 0.2804 | 0.0005222 | 1.49 |
| PALM2 | 733 | 7849021 | G | 0.3216 | 0.0000471 | 1.57 |
| PALM2 | 734 | 10980026 | G | 0.3258 | 0.0000760 | 1.54 |
| PALM2 | 735 | 10980027 | A | 0.3266 | 0.0001044 | 1.53 |
| PALM2 | 736 | 17202497 | A | 0.3333 | 0.0003072 | 1.48 |
| ZNF618 | 737 | 4978561 | T | 0.3285 | 0.0213400 | 0.79 |
| ZNF618 | 738 | 10982031 | C | 0.3174 | 0.0250200 | 0.79 |
| ZNF618 | 739 | 4979326 | A | 0.3153 | 0.0300000 | 0.80 |
| PTGS1 | 743 | 1234909 | G | 0.1830 | 0.0481300 | 1.30 |
| RALGPS1 | 744 | 665249 | G | 0.3377 | 0.0015720 | 1.40 |
| RALGPS1 | 745 | 10760473 | G | 0.3373 | 0.0020180 | 1.39 |
| RALGPS1 | 746 | 10819264 | T | 0.4688 | 0.0276700 | 0.81 |
| RALGPS1 | 747 | 13298677 | G | 0.2088 | 0.0018200 | 1.49 |
| RALGPS1 | 748 | 1028866 | G | 0.4714 | 0.0401800 | 0.82 |
| SLC25A25 | 749 | 9695803 | C | 0.1432 | 0.0342600 | 1.37 |
| TSC1 | 750 | 7875558 | A | 0.1849 | 0.0035010 | 1.49 |
| TSC1 | 751 | 7858160 | T | 0.1890 | 0.0313800 | 1.33 |
| TSC1 | 752 | 7035308 | A | 0.2644 | 0.0281600 | 1.29 |
| VAV2 | 753 | 10993826 | T | 0.1675 | 0.0055510 | 1.48 |
| VAV2 | 754 | 2073929 | G | 0.1615 | 0.0137900 | 1.42 |
| VAV2 | 755 | 3819500 | T | 0.1595 | 0.0178000 | 1.40 |
| VAV2 | 756 | 7023551 | G | 0.2206 | 0.0377700 | 1.29 |
| PITRM1 | 757 | 7924157 | C | 0.1914 | 0.0075340 | 1.42 |
| PRKCQ | 758 | 2387902 | G | 0.5012 | 0.0037310 | 1.33 |
| PRKCQ | 759 | 11254972 | A | 0.4353 | 0.0051860 | 0.76 |
| PRKCQ | 760 | 4747393 | A | 0.3819 | 0.0175000 | 0.79 |
| CACNB2 | 761 | 12779641 | G | 0.1427 | 0.0161900 | 0.73 |
| ARMC3 | 762 | 1171136 | A | 0.2645 | 0.0089570 | 1.35 |
| ARMC3 | 763 | 9665603 | G | 0.3464 | 0.0002269 | 1.49 |
| ARMC3 | 764 | 10764367 | C | 0.3477 | 0.0005803 | 1.45 |
| ARMC3 | 765 | 1054052 | T | 0.3218 | 0.0226200 | 1.28 |
| ARMC3 | 766 | 12252340 | T | 0.2215 | 0.0334200 | 1.30 |
| ARMC3 | 767 | 1382580 | G | 0.1396 | 0.0389300 | 1.36 |
| ARMC3 | 768 | 1382579 | G | 0.0470 | 0.0357500 | 1.74 |
| JMJD1C | 774 | 12355784 | A | 0.4467 | 0.0067450 | 0.77 |
| JMJD1C | 775 | 3999089 | C | 0.4450 | 0.0070740 | 0.77 |
| CDH23 | 776 | 17635709 | T | 0.2038 | 0.0493200 | 0.79 |
| KCNMA1 | 778 | 4639876 | T | 0.3353 | 0.0200300 | 1.28 |
| SORCS3 | 779 | 902300 | T | 0.2764 | 0.0218900 | 1.30 |
| VTI1A | 780 | 7088500 | C | 0.3103 | 0.0006779 | 1.45 |
| VTI1A | 781 | 7907361 | C | 0.2962 | 0.0011970 | 1.44 |
| VTI1A | 782 | 7096151 | A | 0.3055 | 0.0021240 | 1.40 |
| VTI1A | 783 | 7070850 | G | 0.3067 | 0.0451800 | 1.24 |
| VTI1A | 784 | 6585184 | A | 0.3390 | 0.0499900 | 1.23 |
| ATRNL1 | 785 | 2068463 | C | 0.4153 | 0.0452900 | 1.22 |
| HSPA12A | 786 | 3010460 | C | 0.3612 | 0.0179500 | 1.28 |
| STIM1 | 798 | 2959068 | T | 0.4258 | 0.0093460 | 0.77 |
| STIM1 | 799 | 12803023 | G | 0.4569 | 0.0028360 | 0.75 |
| TRIM21 | 800 | 1426378 | A | 0.1524 | 0.0099630 | 1.46 |
| GALNTL4 | 801 | 4910288 | A | 0.3890 | 0.0198900 | 0.79 |
| GALNTL4 | 802 | 4910289 | C | 0.2578 | 0.0137900 | 0.77 |
| GALNTL4 | 803 | 10831565 | A | 0.4672 | 0.0100100 | 1.29 |
| SPON1 | 804 | 2174146 | T | 0.2243 | 0.0357900 | 1.29 |
| SPON1 | 805 | 11238 | T | 0.2852 | 0.0035120 | 0.74 |
| IGSF22 | 815 | 4537730 | A | 0.2407 | 0.0041330 | 0.73 |
| IGSF22 | 816 | 7125943 | T | 0.2206 | 0.0059280 | 0.73 |
| IGSF22 | 817 | 10766494 | C | 0.2255 | 0.0102600 | 0.75 |
| C11ORF49 | 818 | 4548577 | T | 0.2410 | 0.0178300 | 1.33 |
| C11ORF49 | 819 | 11039112 | T | 0.3433 | 0.0037250 | 1.36 |
| C11ORF49 | 820 | 747650 | A | 0.3437 | 0.0041950 | 1.36 |
| DLG2 | 821 | 2507850 | A | 0.3353 | 0.0039160 | 0.75 |
| DLG2 | 822 | 7108874 | T | 0.2524 | 0.0106300 | 0.76 |
| DLG2 | 823 | 10898133 | A | 0.3624 | 0.0456200 | 0.82 |
| DLG2 | 827 | 2514147 | G | 0.1064 | 0.0098870 | 1.57 |
| DLG2 | 828 | 1943708 | C | 0.3527 | 0.0046480 | 1.35 |
| DLG2 | 829 | 1943701 | G | 0.5252 | 0.0095020 | 1.29 |
| DLG2 | 830 | 2187359 | A | 0.3398 | 0.0243200 | 1.27 |
| DLG2 | 831 | 11234201 | T | 0.4075 | 0.0341200 | 0.81 |
| ELMOD1 | 832 | 684901 | T | 0.3141 | 0.0002093 | 1.51 |
| OPCML | 833 | 10894536 | A | 0.0823 | 0.0049550 | 0.63 |
| TSPAN9 | 837 | 10774140 | C | 0.3126 | 0.0416600 | 0.81 |
| STYK1 | 838 | 1078437 | G | 0.4050 | 0.0082290 | 1.31 |
| STYK1 | 839 | 7350597 | A | 0.4647 | 0.0009245 | 1.39 |
| STYK1 | 840 | 10845198 | T | 0.4050 | 0.0223300 | 1.26 |
| STYK1 | 841 | 10743918 | C | 0.3533 | 0.0466500 | 0.82 |
| LOC729025 | 842 | 1624759 | G | 0.2975 | 0.0448900 | 1.25 |
| LRP1 | 843 | 1800176 | T | 0.2760 | 0.0040280 | 0.74 |
| LRP1 | 844 | 1800168 | C | 0.2816 | 0.0075630 | 0.75 |
| CNOT2 | 845 | 1595410 | C | 0.4129 | 0.0488400 | 0.82 |
| CNOT2 | 846 | 3817487 | A | 0.3969 | 0.0458300 | 0.82 |
| KCNC2 | 847 | 2136036 | G | 0.3333 | 0.0396900 | 0.81 |
| NAV3 | 848 | 7139116 | C | 0.1319 | 0.0173000 | 0.72 |
| NAV3 | 849 | 10777655 | A | 0.1313 | 0.0243500 | 0.73 |
| GAS2L3 | 850 | 17030333 | T | 0.0431 | 0.0009065 | 2.84 |
| SLC46A3 | 853 | 9579226 | T | 0.3534 | 0.0142500 | 0.78 |
| SLC46A3 | 854 | 7339280 | A | 0.4234 | 0.0027410 | 0.75 |
| SLC46A3 | 855 | 715704 | G | 0.4141 | 0.0451800 | 1.22 |
| UBL3 | 856 | 7328052 | A | 0.1039 | 0.0234600 | 1.49 |
| N4BP2L2 | 857 | 208431 | G | 0.2160 | 0.0064940 | 0.73 |
| NBEA | 858 | 7320580 | G | 0.2337 | 0.0249300 | 1.31 |
| NBEA | 859 | 7988705 | A | 0.2265 | 0.0481900 | 1.27 |
| NBEA | 860 | 17769661 | T | 0.1483 | 0.0490100 | 1.33 |
| SLAIN1 | 861 | 1343911 | A | 0.1775 | 0.0061900 | 1.46 |
| GPC5 | 862 | 10851317 | A | 0.4604 | 0.0068410 | 1.31 |
| GPC5 | 863 | 1330469 | T | 0.4486 | 0.0319200 | 0.81 |
| GPC5 | 864 | 9515990 | C | 0.3796 | 0.0288700 | 1.25 |
| GPC6 | 865 | 9561427 | G | 0.3005 | 0.0154800 | 1.31 |
| GPC6 | 866 | 9589816 | A | 0.4012 | 0.0160400 | 1.28 |
| NALCN | 867 | 16958230 | G | 0.0692 | 0.0074300 | 0.63 |
| NALCN | 868 | 625331 | C | 0.1292 | 0.0061870 | 0.69 |
| ITGBL1 | 869 | 4400915 | T | 0.0653 | 0.0362700 | 1.60 |
| WDR23 | 870 | 3825584 | T | 0.3134 | 0.0020680 | 1.40 |
| WDR23 | 871 | 11574503 | C | 0.3505 | 0.0103900 | 1.31 |
| GNG2 | 874 | 2357247 | C | 0.3589 | 0.0066230 | 0.76 |
| GNG2 | 875 | 1959518 | C | 0.3969 | 0.0066270 | 0.76 |
| SAMD4A | 876 | 709939 | C | 0.4246 | 0.0123600 | 0.78 |
| SAMD4A | 877 | 8021957 | T | 0.4224 | 0.0069810 | 0.77 |
| SAMD4A | 878 | 8021151 | G | 0.4115 | 0.0070300 | 0.77 |
| PPP2R5E | 880 | 1255741 | A | 0.3301 | 0.0386300 | 1.25 |
| RGS6 | 882 | 2239268 | C | 0.2760 | 0.0076130 | 0.75 |
| RGS6 | 883 | 12885938 | C | 0.2754 | 0.0076460 | 0.75 |
| KCNK10 | 886 | 11625905 | T | 0.2098 | 0.0181400 | 0.76 |
| KCNK13 | 887 | 11621045 | G | 0.4200 | 0.0029250 | 1.35 |
| KCNK13 | 888 | 11159957 | G | 0.4209 | 0.0043360 | 1.33 |
| PSMC1 | 889 | 7150093 | A | 0.4179 | 0.0038340 | 1.34 |
| PSMC1 | 890 | 6575120 | G | 0.4123 | 0.0050780 | 1.33 |
| PSMC1 | 891 | 2236403 | T | 0.4118 | 0.0060210 | 1.33 |
| RPS6KA5 | 892 | 1286264 | C | 0.3699 | 0.0126600 | 0.78 |
| RPS6KA5 | 893 | 941847 | T | 0.2524 | 0.0237800 | 1.31 |
| RPS6KA5 | 894 | 1286060 | C | 0.2691 | 0.0119300 | 1.33 |
| RPS6KA5 | 895 | 9944098 | T | 0.5193 | 0.0264300 | 1.25 |
| RPS6KA5 | 896 | 7156252 | T | 0.3184 | 0.0059240 | 1.35 |
| RPS6KA5 | 897 | 11159988 | C | 0.3186 | 0.0065270 | 1.34 |
| RPS6KA5 | 898 | 7492628 | G | 0.3423 | 0.0222200 | 1.28 |
| CCDC88C | 901 | 8015982 | C | 0.2566 | 0.0043250 | 1.40 |
| CCDC88C | 902 | 10131741 | G | 0.2542 | 0.0082870 | 1.36 |
| CCDC88C | 903 | 8007791 | A | 0.2536 | 0.0099850 | 1.35 |
| BCL11B | 904 | 2793321 | T | 0.3254 | 0.0009968 | 1.43 |
| BCL11B | 905 | 1257446 | C | 0.3141 | 0.0005659 | 1.46 |
| BCL11B | 906 | 1257416 | G | 0.3529 | 0.0128600 | 1.30 |
| ATP10A | 907 | 4906747 | G | 0.0719 | 0.0298000 | 0.68 |
| ATP10A | 908 | 17667563 | A | 0.1947 | 0.0350500 | 1.32 |
| C15ORF41 | 909 | 1990659 | A | 0.3301 | 0.0255100 | 0.80 |
| GLDN | 910 | 2470172 | C | 0.4440 | 0.0116100 | 1.29 |
| GLDN | 911 | 2470173 | T | 0.2074 | 0.0137300 | 1.37 |
| GLDN | 912 | 16964319 | T | 0.0385 | 0.0182600 | 2.05 |
| GLDN | 913 | 10163098 | G | 0.0349 | 0.0317900 | 1.97 |
| CLK3 | 914 | 11072496 | T | 0.1695 | 0.0067190 | 1.46 |
| CLK3 | 915 | 7161903 | G | 0.1675 | 0.0088530 | 1.44 |
| TBC1D2B | 916 | 16969397 | T | 0.0933 | 0.0019920 | 1.81 |
| TBC1D2B | 917 | 8030999 | G | 0.1295 | 0.0021610 | 1.64 |
| PCSK6 | 918 | 11247300 | A | 0.1527 | 0.0073280 | 1.48 |
| PCSK6 | 919 | 735504 | C | 0.4748 | 0.0495100 | 0.82 |
| A2BP1 | 920 | 9935875 | G | 0.4207 | 0.0444500 | 0.82 |
| A2BP1 | 922 | 1034971 | G | 0.3708 | 0.0348400 | 0.81 |
| TMC5 | 923 | 8058400 | C | 0.2434 | 0.0489700 | 1.26 |
| TMC5 | 924 | 8063463 | G | 0.1954 | 0.0000370 | 1.76 |
| TMC5 | 925 | 2856608 | G | 0.3055 | 0.0040570 | 1.37 |
| TMC5 | 926 | 11640077 | G | 0.3034 | 0.0038910 | 1.38 |
| GDE1 | 927 | 739639 | C | 0.3926 | 0.0128300 | 1.29 |
| GDE1 | 928 | 3751821 | T | 0.0962 | 0.0098070 | 1.61 |
| HYDIN | 929 | 9939485 | G | 0.4475 | 0.0004328 | 0.71 |
| HYDIN | 930 | 9939194 | T | 0.3664 | 0.0012840 | 1.40 |
| HYDIN | 931 | 184647 | C | 0.3522 | 0.0317600 | 0.80 |
| WWOX | 933 | 11648121 | A | 0.1791 | 0.0043000 | 0.71 |
| WWOX | 934 | 12924899 | C | 0.2494 | 0.0315300 | 0.79 |
| WWOX | 935 | 7204203 | A | 0.0354 | 0.0466200 | 1.86 |
| MPHOSPH6 | 936 | 11150443 | C | 0.1265 | 0.0000856 | 1.97 |
| MPHOSPH6 | 937 | 10514533 | C | 0.1492 | 0.0003630 | 1.72 |
| MPHOSPH6 | 938 | 1862820 | G | 0.3819 | 0.0188200 | 1.27 |
| MPHOSPH6 | 939 | 918763 | C | 0.4904 | 0.0009759 | 1.38 |
| MPHOSPH6 | 940 | 902542 | A | 0.1679 | 0.0000579 | 1.80 |
| MPHOSPH6 | 941 | 2967337 | C | 0.1408 | 0.0000754 | 1.88 |
| MPHOSPH6 | 942 | 2967333 | G | 0.1667 | 0.0000047 | 2.00 |
| MPHOSPH6 | 943 | 2967328 | C | 0.1663 | 0.0001142 | 1.76 |
| CDH13 | 944 | 4598906 | T | 0.3449 | 0.0296400 | 0.80 |
| USP10 | 947 | 964453 | G | 0.2739 | 0.0149200 | 1.32 |
| DNAH9 | 948 | 11650055 | T | 0.4511 | 0.0110200 | 0.78 |
| DNAH9 | 949 | 7213187 | G | 0.4463 | 0.0039930 | 1.33 |
| RAB11FIP4 | 950 | 757378 | T | 0.4111 | 0.0069170 | 1.32 |
| CA10 | 952 | 11652641 | G | 0.2041 | 0.0441000 | 1.29 |
| HRNBP3 | 953 | 7212305 | A | 0.1208 | 0.0238800 | 1.44 |
| ZFP161 | 954 | 990072 | T | 0.4275 | 0.0000163 | 0.65 |
| ZFP161 | 955 | 11665417 | T | 0.4344 | 0.0030090 | 1.35 |
| ZFP161 | 956 | 9945680 | A | 0.4354 | 0.0037980 | 1.34 |
| ZFP161 | 957 | 9965501 | T | 0.0275 | 0.0069490 | 2.91 |
| ZFP161 | 958 | 8082898 | G | 0.0191 | 0.0318600 | 2.70 |
| PTPRM | 959 | 8089695 | C | 0.0801 | 0.0492000 | 1.47 |
| PTPRM | 960 | 1866854 | G | 0.3229 | 0.0031190 | 1.38 |
| PTPRM | 961 | 10513901 | A | 0.4928 | 0.0390600 | 1.23 |
| KIAA0802 | 962 | 17407982 | C | 0.2200 | 0.0023400 | 0.71 |
| KIAA0802 | 963 | 17408213 | T | 0.2159 | 0.0026080 | 0.71 |
| OSBPL1A | 964 | 275875 | G | 0.4257 | 0.0078430 | 0.77 |
| CHST9 | 965 | 559547 | T | 0.3413 | 0.0086020 | 0.77 |
| CHST9 | 966 | 12604227 | G | 0.3828 | 0.0291900 | 0.80 |
| CHST9 | 967 | 8084423 | A | 0.4535 | 0.0261100 | 1.25 |
| CHST9 | 968 | 1426879 | C | 0.3205 | 0.0033150 | 1.38 |
| CHST9 | 969 | 4800797 | G | 0.3216 | 0.0039910 | 1.37 |
| NEDD4L | 970 | 4940385 | C | 0.4057 | 0.0265100 | 0.80 |
| NEDD4L | 971 | 7242486 | G | 0.4115 | 0.0400400 | 0.81 |
| NEDD4L | 972 | 17064977 | C | 0.2542 | 0.0401800 | 0.80 |
| NEDD4L | 973 | 11152073 | A | 0.2542 | 0.0424300 | 0.80 |
| CCBE1 | 974 | 17065761 | C | 0.0979 | 0.0433000 | 0.73 |
| CCBE1 | 975 | 12969965 | A | 0.2017 | 0.0212200 | 1.34 |
| CDH7 | 977 | 7228669 | T | 0.3065 | 0.0427800 | 1.25 |
| CDH7 | 978 | 2628210 | G | 0.2141 | 0.0092920 | 0.74 |
| CDH7 | 979 | 2587427 | A | 0.3317 | 0.0249700 | 0.80 |
| CDH7 | 980 | 2587428 | T | 0.2811 | 0.0063980 | 0.75 |
| TXNDC10 | 981 | 309242 | G | 0.1506 | 0.0000764 | 1.84 |
| FERMT1 | 983 | 6516104 | T | 0.1229 | 0.0002756 | 1.85 |
| FERMT1 | 984 | 16991866 | C | 0.1193 | 0.0005018 | 1.81 |
| PLCB1 | 985 | 6055513 | G | 0.4435 | 0.0060730 | 0.76 |
| PLCB4 | 986 | 6056653 | G | 0.1411 | 0.0384900 | 0.76 |
| MACROD2 | 987 | 6079910 | C | 0.1085 | 0.0008739 | 1.83 |
| MACROD2 | 988 | 6043402 | A | 0.0627 | 0.0474900 | 1.56 |
| MACROD2 | 989 | 463020 | G | 0.4248 | 0.0060440 | 0.76 |
| MACROD2 | 990 | 6135667 | T | 0.3305 | 0.0463400 | 0.82 |
| KIF16B | 991 | 6044051 | T | 0.1803 | 0.0000708 | 1.76 |
| KIF16B | 992 | 720592 | T | 0.3138 | 0.0441900 | 1.24 |
| MAPRE1 | 993 | 410488 | A | 0.3959 | 0.0021780 | 0.74 |
| MAPRE1 | 994 | 242529 | C | 0.3353 | 0.0006392 | 1.44 |
| ZSWIM3 | 996 | 1967656 | A | 0.4640 | 0.0035740 | 1.34 |
| ZSWIM3 | 997 | 6514065 | C | 0.4104 | 0.0033630 | 1.35 |
| ZSWIM3 | 998 | 1045493 | G | 0.4315 | 0.0471300 | 1.22 |
| SNAI1 | 999 | 6020157 | A | 0.1468 | 0.0298400 | 0.75 |
| SNAI1 | 1000 | 16995010 | C | 0.1147 | 0.0150300 | 0.70 |
| SNAI1 | 1001 | 6091080 | G | 0.1727 | 0.0003309 | 0.65 |
| SNAI1 | 1002 | 6020187 | G | 0.2064 | 0.0017860 | 0.70 |
| C21ORF37 | 1005 | 12483129 | C | 0.4519 | 0.0044570 | 0.76 |
| C21ORF37 | 1006 | 2824301 | A | 0.5167 | 0.0004203 | 1.41 |
| NCAM2 | 1007 | 9979231 | G | 0.2644 | 0.0095300 | 1.35 |
| PDE9A | 1011 | 2284976 | A | 0.3846 | 0.0019000 | 1.38 |
| ARVCF | 1012 | 2239395 | G | 0.0322 | 0.0229700 | 0.57 |
| ARVCF | 1013 | 12171109 | A | 0.1993 | 0.0448300 | 0.79 |
| SGSM1 | 1014 | 5760752 | C | 0.3182 | 0.0299600 | 1.26 |
| ASPHD2 | 1015 | 8140344 | A | 0.4951 | 0.0038120 | 1.33 |
| ASPHD2 | 1016 | 6005042 | A | 0.4550 | 0.0041750 | 0.75 |
| ASPHD2 | 1017 | 6519650 | C | 0.3373 | 0.0003330 | 0.70 |
| HPS4 | 1018 | 5761552 | T | 0.4507 | 0.0000837 | 0.68 |
| ARFGAP3 | 1019 | 1018448 | A | 0.4246 | 0.0077450 | 0.77 |
| ARFGAP3 | 1020 | 6002963 | G | 0.4272 | 0.0302100 | 0.81 |
| TTLL1 | 1021 | 1052160 | T | 0.3245 | 0.0088850 | 1.33 |
| TTLL1 | 1022 | 135002 | C | 0.4725 | 0.0079110 | 1.30 |
| EFCAB6 | 1023 | 137794 | A | 0.1067 | 0.0406800 | 0.74 |
| EFCAB6 | 1024 | 5764310 | C | 0.0863 | 0.0318600 | 0.71 |
| RIBC2 | 1025 | 6007043 | C | 0.1683 | 0.0004614 | 0.65 |

| **Table 2: CNV Deletions Influencing Schizophrenia Risk** | | | | |
|---|---|---|---|---|
| **Gene** | **Seq ID** | **NCBI RS#** | **P** | **Odds Ratio** |
| CTNNA2 | 81 | 1930 | 0.020961 | 7.99 |
| CTNNA2 | 82 | 10170020 | 0.039020 | 6.85 |
| CTNNA2 | 83 | 6743980 | 0.032919 | >4.00 |
| CTNNA2 | 84 | 13423141 | 0.043911 | 2.45 |
| DNER | 114 | 6436860 | 0.032919 | >4.00 |
| ITPR1 | 120 | 3792490 | 0.021448 | <0.17 |
| ROBO1 | 155 | 1457659 | 0.031601 | 0.14 |
| ROBO1 | 156 | 3821603 | 0.031601 | 0.14 |
| KCNIP4 | 196 | 7694587 | 0.050947 | 0.58 |
| STIM2 | 197 | 4557250 | 0.033306 | 0.58 |
| STIM2 | 198 | 4532223 | 0.033306 | 0.58 |
| STIM2 | 199 | 4274829 | 0.033306 | 0.58 |
| STIM2 | 200 | 4692128 | 0.013672 | 0.43 |
| STIM2 | 201 | 7671671 | 0.021696 | 0.36 |
| INPP4B | 230 | 3102439 | 0.035939 | <0.20 |
| INPP4B | 231 | 1907119 | 0.035939 | <0.20 |
| INPP4B | 232 | 2636645 | 0.021448 | <0.17 |
| INPP4B | 235 | 881611 | 0.030163 | 2.98 |
| INPP4B | 239 | 17016175 | 0.010488 | 0.26 |
| FSTL5 | 243 | 7690342 | 0.037009 | 0.28 |
| DNAH5 | 257 | 6876673 | 0.032919 | >4.00 |
| DNAH5 | 258 | 3777093 | 0.032919 | >4.00 |
| DNAH5 | 259 | 6554808 | 0.032919 | >4.00 |
| DNAH5 | 262 | 7715811 | 0.008964 | >6.00 |
| DNAH5 | 263 | 10513153 | 0.008964 | >6.00 |
| DNAH5 | 264 | 10039621 | 0.050044 | 3.43 |
| DNAH5 | 267 | 6874349 | 0.019794 | 5.14 |
| DNAH5 | 268 | 9885366 | 0.019794 | 5.14 |
| FBXL17 | 313 | 288180 | 0.002420 | 0.44 |
| FBXL17 | 314 | 288173 | 0.000172 | 0.22 |
| FBXL17 | 317 | 288146 | 0.035939 | <0.20 |
| JARID2 | 348 | 4085876 | 0.021448 | <0.17 |
| JARID2 | 349 | 2282827 | 0.035939 | <0.20 |
| ELOVL5 | 379 | 10948744 | 0.008060 | 0.81 |
| ELOVL5 | 380 | 2562898 | 0.009014 | 0.81 |
| ELOVL5 | 381 | 735860 | 0.007927 | 0.81 |
| ELOVL5 | 382 | 2057024 | 0.007927 | 0.81 |
| ELOVL5 | 383 | 1429146 | 0.006239 | 0.80 |
| ELOVL5 | 384 | 2073040 | 0.006239 | 0.80 |
| ELOVL5 | 385 | 9463895 | 0.006239 | 0.80 |
| ELOVL5 | 386 | 2235723 | 0.007090 | 0.81 |
| ELOVL5 | 387 | 1346603 | 0.007090 | 0.81 |
| ELOVL5 | 388 | 9474476 | 0.007090 | 0.81 |
| ELOVL5 | 389 | 2294867 | 0.007090 | 0.81 |
| ELOVL5 | 390 | 9349660 | 0.007090 | 0.81 |
| ELOVL5 | 391 | 974323 | 0.007090 | 0.81 |
| ELOVL5 | 392 | 6909592 | 0.006239 | 0.80 |
| ELOVL5 | 393 | 9367520 | 0.006239 | 0.80 |
| ELOVL5 | 394 | 9395854 | 0.007090 | 0.81 |
| ELOVL5 | 395 | 209485 | 0.007090 | 0.81 |
| ELOVL5 | 396 | 9395856 | 0.009860 | 0.81 |
| ELOVL5 | 397 | 7747926 | 0.010371 | 0.81 |
| ELOVL5 | 398 | 7738788 | 0.010371 | 0.81 |
| ELOVL5 | 399 | 209500 | 0.009167 | 0.81 |
| ELOVL5 | 400 | 9357760 | 0.009167 | 0.81 |
| ELOVL5 | 401 | 9370196 | 0.005869 | 0.80 |
| ELOVL5 | 402 | 209517 | 0.003217 | 0.79 |
| ELOVL5 | 403 | 9370201 | 0.003217 | 0.79 |
| ELOVL5 | 404 | 1579454 | 0.003217 | 0.79 |
| ELOVL5 | 405 | 9474507 | 0.004235 | 0.80 |
| ELOVL5 | 406 | 9296711 | 0.004235 | 0.80 |
| ELOVL5 | 407 | 1429143 | 0.005342 | 0.80 |
| NXPH1 | 447 | 2107280 | 0.032919 | >4.00 |
| NXPH1 | 448 | 10085720 | 0.028460 | 1.78 |
| NXPH1 | 449 | 4455737 | 0.028460 | 1.78 |
| DGKB | 452 | 17168091 | 0.025056 | 1.73 |
| DGKB | 453 | 6972310 | 0.033501 | 1.69 |
| DGKB | 454 | 12699619 | 0.035939 | 0.32 |
| DGKB | 455 | 10499441 | 0.035939 | 0.32 |
| CALN1 | 498 | 11768892 | 0.034094 | 0.35 |
| CALN1 | 499 | 1914381 | 0.034094 | 0.35 |
| MAGI2 | 502 | 12670134 | 0.022848 | 0.21 |
| PPP1R9A | 513 | 10252856 | 0.008106 | 1.28 |
| PPP1R9A | 514 | 6465448 | 0.028295 | 1.38 |
| PPP1R9A | 515 | 854717 | 0.020137 | 1.29 |
| PPP1R9A | 516 | 854737 | 0.006379 | 0.70 |
| PPP1R9A | 517 | 6961415 | 0.002304 | 0.70 |
| PPP1R9A | 518 | 17305991 | 0.020961 | 1.31 |
| PPP1R9A | 520 | 11762113 | 0.008914 | 0.73 |
| PPP1R9A | 523 | 854520 | 0.001873 | 1.50 |
| NRCAM | 532 | 401573 | 0.039482 | 0.70 |
| NRCAM | 535 | 422011 | 0.014469 | 0.74 |
| NRCAM | 536 | 425013 | 0.022980 | 1.33 |
| KCND2 | 542 | 9886190 | 0.019235 | 1.41 |
| SLC13A1 | 547 | 12706494 | 0.028960 | 0.78 |
| SLC13A1 | 549 | 2222514 | 0.004704 | 1.55 |
| SLC13A1 | 550 | 2402637 | 0.044434 | 0.82 |
| GRM8 | 556 | 2299500 | 0.028131 | 1.39 |
| GRM8 | 560 | 13240418 | 0.031601 | 0.80 |
| GRM8 | 561 | 4731330 | 0.010313 | 0.70 |
| GRM8 | 562 | 2299523 | 0.048865 | 0.79 |
| GRM8 | 563 | 2188187 | 0.026857 | 1.60 |
| GRM8 | 564 | 4141415 | 0.040424 | 1.38 |
| GRM8 | 565 | 10226889 | 0.049157 | 0.72 |
| DGKI | 572 | 4732255 | 0.015055 | 1.27 |
| DGKI | 576 | 7781327 | 0.046318 | 1.27 |
| CNTNAP2 | 580 | 10261766 | 0.016489 | 0.79 |
| CNTNAP2 | 581 | 13228526 | 0.023788 | 1.57 |
| CNTNAP2 | 588 | 17417154 | 0.013828 | 1.49 |
| CNTNAP2 | 589 | 1637845 | 0.026091 | 1.96 |
| PTPRN2 | 591 | 221247 | 0.045231 | 1.91 |
| PTPRN2 | 593 | 11767654 | 0.023113 | 1.34 |
| PTPRN2 | 594 | 10231253 | 0.023113 | 0.33 |
| PTPRN2 | 595 | 7789111 | 0.021448 | 2.30 |
| CSMD1 | 597 | 341724 | 0.001131 | 0.62 |
| CSMD1 | 599 | 7001551 | 0.005054 | 1.79 |
| CSMD1 | 601 | 7819225 | 0.043394 | 0.84 |
| PSD3 | 617 | 4298523 | 0.010548 | 1.83 |
| PSD3 | 618 | 2634435 | 0.025494 | 1.62 |
| PSD3 | 619 | 6987039 | 0.002996 | 1.63 |
| PSD3 | 621 | 10106032 | 0.025791 | 0.61 |
| PSD3 | 625 | 11778310 | 0.016210 | 1.65 |
| TOX | 640 | 12115074 | 0.046871 | 1.27 |
| TOX | 641 | 16924623 | 0.017555 | 1.29 |
| CSMD3 | 662 | 1873746 | 0.042883 | 0.80 |
| CSMD3 | 665 | 12676848 | 0.000006 | 2.32 |
| CSMD3 | 666 | 2123489 | 0.020022 | 1.48 |
| CSMD3 | 667 | 10505174 | 0.026242 | 1.44 |
| CSMD3 | 669 | 17602393 | 0.022717 | 0.70 |
| CSMD3 | 671 | 12545742 | 0.048865 | 0.84 |
| CSMD3 | 673 | 6999348 | 0.045500 | 0.74 |
| CSMD3 | 675 | 1402944 | 0.033501 | 1.39 |
| SAMD12 | 676 | 4631494 | 0.032347 | 1.32 |
| SAMD12 | 678 | 9297587 | 0.002542 | 1.52 |
| SAMD12 | 679 | 10955887 | 0.001095 | 1.65 |
| SAMD12 | 681 | 2514591 | 0.005492 | 1.40 |
| SAMD12 | 682 | 2514602 | 0.044434 | 1.54 |
| MTSS1 | 686 | 3829037 | 0.009696 | 1.29 |
| MTSS1 | 687 | 4871517 | 0.047432 | 0.81 |
| MTSS1 | 688 | 919544 | 0.014970 | 0.79 |
| KCNQ3 | 697 | 2469625 | 0.009805 | 4.58 |
| KCNQ3 | 698 | 2436134 | 0.019794 | 5.14 |
| COL22A1 | 702 | 7842696 | 0.009167 | 0.17 |
| SMARCA2 | 707 | 16937123 | 0.005167 | 2.76 |
| FKTN | 725 | 1858744 | 0.041881 | 0.85 |
| FKTN | 726 | 2768294 | 0.034094 | 0.84 |
| FKTN | 727 | 17309806 | 0.031054 | 0.84 |
| FKTN | 728 | 2518106 | 0.031418 | 0.84 |
| FKTN | 729 | 1858743 | 0.025940 | 0.84 |
| FKTN | 730 | 885954 | 0.018800 | 0.83 |
| FKTN | 731 | 17251166 | 0.015141 | 0.82 |
| ASTN2 | 740 | 7032028 | 0.006102 | 10.30 |
| CDK5RAP2 | 742 | 10760103 | 0.048865 | 2.74 |
| MYO3A | 770 | 10764597 | 0.012993 | <0.14 |
| JMJD1C | 772 | 10761739 | 0.035939 | <0.20 |
| JMJD1C | 773 | 10761741 | 0.021448 | <0.17 |
| ATE1 | 788 | 11200107 | 0.032919 | >4.00 |
| ATE1 | 790 | 11818001 | 0.035106 | 4.57 |
| ATE1 | 791 | 10732824 | 0.035106 | 4.57 |
| ATE1 | 792 | 10510101 | 0.019794 | 5.14 |
| ATE1 | 793 | 10788221 | 0.019794 | 5.14 |
| ATE1 | 794 | 3750835 | 0.019794 | 5.14 |
| ATE1 | 795 | 11200257 | 0.035106 | 4.57 |
| ATE1 | 796 | 11200260 | 0.035106 | 4.57 |
| ATE1 | 797 | 11200267 | 0.035106 | 4.57 |
| USH1C | 806 | 2072230 | 0.045500 | 0.40 |
| USH1C | 808 | 1076311 | 0.039715 | 0.80 |
| USH1C | 809 | 2190453 | 0.039715 | 0.80 |
| USH1C | 810 | 2190454 | 0.037446 | 0.79 |
| USH1C | 812 | 7113935 | 0.009805 | 0.45 |
| USH1C | 813 | 7106302 | 0.009375 | 0.48 |
| USH1C | 814 | 2073582 | 0.005082 | 0.45 |
| DLG2 | 825 | 6592142 | 0.037667 | 0.66 |
| DLG2 | 826 | 1914203 | 0.021448 | <0.17 |
| TSPAN9 | 834 | 10848784 | 0.020961 | 7.99 |
| NPAS3 | 872 | 8022434 | 0.017062 | >5.00 |
| NPAS3 | 873 | 11851667 | 0.019457 | 0.13 |
| PPP2R5E | 879 | 6573522 | 0.011095 | 5.72 |
| RGS6 | 881 | 12586947 | 0.011221 | 2.95 |
| RGS6 | 882 | 2239268 | 0.011221 | 2.95 |
| RGS6 | 885 | 2139594 | 0.012632 | 0.74 |
| CCDC88C | 899 | 4900075 | 0.035106 | 4.57 |
| A2BP1 | 921 | 7404422 | 0.034094 | 0.35 |
| WWOX | 932 | 16947095 | 0.006102 | 10.30 |
| CDH13 | 945 | 2061629 | 0.012280 | 0.43 |
| CCBE1 | 976 | 1864307 | 0.035939 | <0.20 |
| BMP7 | 1003 | 2180782 | 0.004756 | >7.00 |

| **Table 3: CNV Duplications Influencing Schizophrenia Risk** | | | | |
|---|---|---|---|---|
| **Gene** | **Seq ID** | **NCBI RS#** | **P** | **Odds Ratio** |
| CTNNA2 | 73 | 10189345 | 0.032919 | >4.00 |
| CTNNA2 | 74 | 3795994 | 0.017062 | >5.00 |
| CTNNA2 | 75 | 7571658 | 0.020961 | 7.99 |
| CTNNA2 | 76 | 6741563 | 0.048865 | 2.74 |
| FHIT | 141 | 2205353 | 0.050044 | 3.43 |
| CBLB | 161 | 12497428 | 0.029298 | 3.81 |
| CBLB | 162 | 6795961 | 0.029298 | 3.81 |
| NLGN1 | 178 | 1553122 | 0.017062 | >5.00 |
| STIM2 | 202 | 725981 | 0.020961 | 7.99 |
| LIMCH1 | 210 | 13113314 | 0.012993 | <0.14 |
| INPP4B | 233 | 2635429 | 0.035939 | <0.20 |
| INPP4B | 237 | 1391099 | 0.016965 | 0.45 |
| INPP4B | 238 | 9308152 | 0.050947 | 0.52 |
| CTNND2 | 255 | 6885224 | 0.035939 | <0.20 |
| CTNND2 | 256 | 2190989 | 0.039020 | 6.85 |
| DNAH5 | 269 | 1867679 | 0.048865 | 2.74 |
| ITGA1 | 287 | 16876270 | 0.039020 | 6.85 |
| PDE4D | 289 | 35285 | 0.021448 | <0.17 |
| GPR98 | 308 | 10043564 | 0.024912 | 2.42 |
| GPR98 | 310 | 1852731 | 0.006102 | 10.30 |
| FBXL17 | 315 | 288172 | 0.020961 | 7.99 |
| FBXL17 | 317 | 288146 | 0.011095 | 5.72 |
| SERPINB6 | 345 | 7751676 | 0.042630 | 1.17 |
| SERPINB6 | 346 | 1358869 | 0.020722 | 1.20 |
| JARID2 | 350 | 9396591 | 0.032919 | >4.00 |
| ATXN1 | 351 | 2237166 | 0.032919 | >4.00 |
| ATXN1 | 352 | 720006 | 0.032919 | >4.00 |
| ATXN1 | 353 | 2237165 | 0.032919 | >4.00 |
| RNF144B | 357 | 577287 | 0.003455 | 0.37 |
| BTN3A1 | 370 | 4712986 | 0.045500 | 0.40 |
| PDE7B | 427 | 9385740 | 0.002963 | <0.10 |
| DGKB | 456 | 1367780 | 0.020961 | 7.99 |
| DGKB | 457 | 1431538 | 0.017062 | >5.00 |
| ABCA13 | 487 | 11983883 | 0.000125 | 0.28 |
| ABCA13 | 488 | 2117089 | 0.000814 | 0.33 |
| ABCA13 | 489 | 7778020 | 0.032919 | >4.00 |
| WBSCR17 | 494 | 12538186 | 0.039020 | 6.85 |
| WBSCR17 | 495 | 648415 | 0.032919 | >4.00 |
| CALN1 | 496 | 12673109 | 0.023516 | 3.43 |
| CALN1 | 497 | 10260183 | 0.023113 | 0.33 |
| CALN1 | 500 | 17503400 | 0.000270 | 0.06 |
| MAGI2 | 501 | 7810778 | 0.011095 | 5.72 |
| MAGI2 | 507 | 10255710 | 0.032919 | >4.00 |
| MAGI2 | 509 | 7778707 | 0.021448 | <0.17 |
| PPP1R9A | 515 | 854717 | 0.005869 | 1.48 |
| PPP1R9A | 519 | 7806304 | 0.024484 | 0.65 |
| PPP1R9A | 520 | 11762113 | 0.047432 | 0.74 |
| PPP1R9A | 521 | 1918482 | 0.019794 | 1.49 |
| PPP1R9A | 523 | 854520 | 0.001989 | 1.75 |
| PPP1R9A | 525 | 854537 | 0.013672 | 1.82 |
| NRCAM | 529 | 409797 | 0.048286 | 1.87 |
| NRCAM | 530 | 449514 | 0.010488 | 1.26 |
| NRCAM | 531 | 107589 | 0.026394 | 0.74 |
| NRCAM | 533 | 404287 | 0.037227 | 1.22 |
| NRCAM | 534 | 369800 | 0.002931 | 1.28 |
| NRCAM | 535 | 422011 | 0.002775 | 2.38 |
| NRCAM | 537 | 13236767 | 0.016965 | 0.70 |
| NRCAM | 538 | 2284290 | 0.003878 | 1.74 |
| KCND2 | 540 | 12111791 | 0.002656 | 1.50 |
| KCND2 | 541 | 10224891 | 0.006170 | 1.45 |
| SLC13A1 | 550 | 2402637 | 0.019907 | 1.37 |
| GRM8 | 551 | 2299449 | 0.008864 | 1.66 |
| GRM8 | 552 | 2237735 | 0.024064 | 2.20 |
| GRM8 | 553 | 9942681 | 0.024064 | 1.21 |
| GRM8 | 554 | 1074728 | 0.047151 | 1.61 |
| GRM8 | 555 | 886176 | 0.021203 | 1.54 |
| GRM8 | 557 | 10487459 | 0.013291 | 1.42 |
| GRM8 | 558 | 1419508 | 0.000007 | 1.78 |
| GRM8 | 559 | 2023735 | 0.012280 | 1.87 |
| GRM8 | 565 | 10226889 | 0.024064 | 1.75 |
| EXOC4 | 566 | 1149550 | 0.040186 | 1.38 |
| EXOC4 | 567 | 7783217 | 0.048865 | 2.74 |
| EXOC4 | 568 | 10488164 | 0.005492 | 1.40 |
| EXOC4 | 570 | 6966403 | 0.035313 | 2.10 |
| DGKI | 571 | 2278829 | 0.002979 | 2.09 |
| DGKI | 572 | 4732255 | 0.014065 | 1.32 |
| DGKI | 573 | 4728415 | 0.002996 | 1.69 |
| DGKI | 574 | 1731913 | 0.030163 | 1.28 |
| DGKI | 575 | 2113578 | 0.008060 | 2.08 |
| DGKI | 577 | 706561 | 0.018481 | 3.21 |
| CNTNAP2 | 579 | 17480133 | 0.031601 | 1.43 |
| CNTNAP2 | 582 | 1548743 | 0.032919 | 0.74 |
| CNTNAP2 | 583 | 7802708 | 0.000396 | >11.00 |
| CNTNAP2 | 584 | 2022226 | 0.002715 | 1.66 |
| CNTNAP2 | 585 | 2373133 | 0.009696 | 2.11 |
| CNTNAP2 | 586 | 10270551 | 0.000003 | 0.59 |
| CNTNAP2 | 587 | 6953679 | 0.041389 | 1.81 |
| PTPRN2 | 590 | 221242 | 0.004098 | 0.72 |
| PTPRN2 | 596 | 10081235 | 0.025940 | 0.69 |
| CSMD1 | 597 | 341724 | 0.028960 | 0.75 |
| CSMD1 | 600 | 750318 | 0.024203 | 1.86 |
| CSMD1 | 602 | 13254344 | 0.006136 | 2.62 |
| CSMD1 | 603 | 895696 | 0.007372 | 1.65 |
| CSMD1 | 607 | 17066296 | 0.001131 | 0.52 |
| PSD3 | 620 | 13259762 | 0.010141 | 1.27 |
| PSD3 | 622 | 747555 | 0.012006 | 1.55 |
| PSD3 | 623 | 7007413 | 0.013518 | 1.57 |
| PSD3 | 624 | 6993404 | 0.018693 | 1.43 |
| PSD3 | 625 | 11778310 | 0.001389 | 2.01 |
| PSD3 | 626 | 1506894 | 0.009805 | 4.58 |
| TOX | 638 | 7845800 | 0.008151 | 1.85 |
| TOX | 639 | 4738732 | 0.021203 | 1.62 |
| CSMD3 | 660 | 10505182 | 0.044171 | 0.71 |
| CSMD3 | 664 | 6994009 | 0.000010 | 2.13 |
| CSMD3 | 665 | 12676848 | 0.048865 | 2.74 |
| CSMD3 | 668 | 1021876 | 0.047715 | 1.39 |
| CSMD3 | 670 | 964764 | 0.000229 | 1.94 |
| CSMD3 | 672 | 11995656 | 0.024064 | 2.20 |
| CSMD3 | 674 | 12680523 | 0.034094 | 0.72 |
| SAMD12 | 677 | 9642841 | 0.000002 | 1.86 |
| SAMD12 | 680 | 12541916 | 0.008016 | 2.65 |
| SAMD12 | 681 | 2514591 | 0.004053 | 3.90 |
| SAMD12 | 682 | 2514602 | 0.004500 | 1.57 |
| MTSS1 | 686 | 3829037 | 0.033501 | 0.73 |
| MTSS1 | 689 | 10956192 | 0.029298 | 2.05 |
| MTSS1 | 690 | 6470263 | 0.040186 | 0.64 |
| KCNQ3 | 696 | 977939 | 0.017062 | >5.00 |
| KCNQ3 | 697 | 2469625 | 0.002542 | >8.00 |
| KCNQ3 | 699 | 7832589 | 0.035939 | <0.20 |
| KCNQ3 | 700 | 1379582 | 0.035939 | <0.20 |
| SMARCA2 | 708 | 10965113 | 0.035106 | 4.57 |
| CDK5RAP2 | 741 | 914617 | 0.034294 | 1.94 |
| MYO3A | 769 | 12773856 | 0.035939 | <0.20 |
| ANK3 | 771 | 4948254 | 0.011284 | 9.14 |
| KCNMA1 | 777 | 2569360 | 0.035939 | <0.2 |
| GRK5 | 787 | 10787948 | 0.019794 | 5.14 |
| ATE1 | 789 | 753455 | 0.029298 | 2.05 |
| USH1C | 807 | 7104083 | 0.013141 | 1.66 |
| USH1C | 808 | 1076311 | 0.006344 | 1.71 |
| USH1C | 809 | 2190453 | 0.003455 | 1.77 |
| USH1C | 810 | 2190454 | 0.016677 | 1.64 |
| USH1C | 811 | 17714673 | 0.020961 | 7.99 |
| DLG2 | 824 | 6592141 | 0.032919 | >4.00 |
| TSPAN9 | 835 | 10848789 | 0.004756 | >7.00 |
| TSPAN9 | 836 | 7969363 | 0.048865 | 2.74 |
| MTIF3 | 851 | 3759434 | 0.000181 | 6.05 |
| MTIF3 | 852 | 3759433 | 0.001194 | 4.03 |
| RGS6 | 884 | 149641 | 0.021448 | <0.17 |
| CCDC88C | 900 | 747159 | 0.035106 | 4.57 |
| CDH13 | 946 | 451434 | 0.011284 | 9.14 |
| CA10 | 951 | 9303589 | 0.035939 | <0.20 |
| ATRN | 982 | 6076524 | 0.000877 | 0.22 |
| PTPRT | 995 | 6016664 | 0.008964 | >6.00 |
| CDH4 | 1004 | 4812313 | 0.034495 | 0.55 |
| NCAM2 | 1008 | 13052286 | 0.032919 | >4.00 |
| NCAM2 | 1009 | 2826815 | 0.029298 | 3.81 |
| NCAM2 | 1010 | 232509 | 0.050044 | 3.43 |

| **TABLE A: Summary of SNPs (NCBI Human Genome Reference Assembly Build 36.3)** | | | | | | | |
|---|---|---|---|---|---|---|---|
| **Seq ID** | **Gene** | **Chr** | **Position** | **Seq ID** | **Gene** | **Chr** | **Position** |
| 1 | PIK3CD | 1 | 9,655,808 | 2 | RP1-21O18.1 | 1 | 15,265,882 |
| 3 | AGBL4-C1ORF165 | 1 | 49,012,555 | 4 | AGBL4-C1ORF165 | 1 | 49,046,634 |
| 5 | AGBL4-C1ORF165 | 1 | 49,107,917 | 6 | AGBL4-C1ORF165 | 1 | 49,124,764 |
| 7 | AGBL4-C1ORF165 | 1 | 49,132,460 | 8 | AGBL4-C1ORF165 | 1 | 49,133,207 |
| 9 | AGBL4-C1ORF165 | 1 | 49,214,610 | 10 | SCP2 | 1 | 53,261,152 |
| 11 | LRP8 | 1 | 53,485,315 | 12 | LRP8 | 1 | 53,504,903 |
| 13 | LRP8 | 1 | 53,519,183 | 14 | PRKACB | 1 | 84,323,437 |
| 15 | PRKACB | 1 | 84,343,593 | 16 | CGN | 1 | 149,764,000 |
| 17 | OLFML2B | 1 | 160,247,126 | 18 | DPT | 1 | 166,964,218 |
| 19 | SEC16B | 1 | 176,175,475 | 20 | SEC16B | 1 | 176,178,830 |
| 21 | MR1 | 1 | 179,279,594 | 22 | LAMC1 | 1 | 181,352,319 |
| 23 | LAMC1 | 1 | 181,374,069 | 24 | LAMC1 | 1 | 181,375,234 |
| 25 | KCNK2 | 1 | 213,267,433 | 26 | KCNK2 | 1 | 213,269,771 |
| 27 | KCNK2 | 1 | 213,286,968 | 28 | KCNK2 | 1 | 213,304,166 |
| 29 | KCNK2 | 1 | 213,312,765 | 30 | KCNK2 | 1 | 213,393,108 |
| 31 | KCNK2 | 1 | 213,405,685 | 32 | KCNK2 | 1 | 213,412,561 |
| 33 | KCNK2 | 1 | 213,428,830 | 34 | ESRRG | 1 | 214,811,868 |
| 35 | ESRRG | 1 | 214,951,990 | 36 | ESRRG | 1 | 214,952,115 |
| 37 | GNG4 | 1 | 233,794,439 | 38 | RYR2 | 1 | 235,793,994 |
| 39 | RYR2 | 1 | 235,885,637 | 40 | RYR2 | 1 | 236,061,906 |
| 41 | FMN2 | 1 | 238,512,719 | 42 | RGS7 | 1 | 239,145,433 |
| 43 | PLD5 | 1 | 240,426,890 | 44 | C2ORF46 | 2 | 8,321,006 |
| 45 | C2ORF46 | 2 | 8,354,700 | 46 | C2ORF46 | 2 | 8,355,126 |
| 47 | KCNF1 | 2 | 10,948,881 | 48 | KCNF1 | 2 | 10,981,799 |
| 49 | KCNF1 | 2 | 10,999,213 | 50 | ASXL2 | 2 | 25,956,101 |
| 51 | CRIM1 | 2 | 36,467,658 | 52 | CRIM1 | 2 | 36,521,723 |
| 53 | CRIM1 | 2 | 36,532,532 | 54 | CRIM1 | 2 | 36,610,853 |
| 55 | CRIM1 | 2 | 36,611,604 | 56 | HAAO | 2 | 42,869,208 |
| 57 | PLEKHH2 | 2 | 43,822,986 | 58 | PRKCE | 2 | 45,919,740 |
| 59 | PRKCE | 2 | 45,927,156 | 60 | FBXO11 | 2 | 47,947,841 |
| 61 | PSME4 | 2 | 53,970,304 | 62 | PSME4 | 2 | 54,064,285 |
| 63 | PSME4 | 2 | 54,136,333 | 64 | ACYP2 | 2 | 54,201,406 |
| 65 | CCDC85A | 2 | 56,425,867 | 66 | AAK1 | 2 | 69,604,707 |
| 67 | AAK1 | 2 | 69,637,782 | 68 | AAK1 | 2 | 69,650,512 |
| 69 | AAK1 | 2 | 69,708,598 | 70 | AAK1 | 2 | 69,716,927 |
| 71 | AAK1 | 2 | 69,740,261 | 72 | AAK1 | 2 | 69,768,980 |
| 73 | CTNNA2 | 2 | 79,724,263 | 74 | CTNNA2 | 2 | 79,726,512 |
| 75 | CTNNA2 | 2 | 79,728,810 | 76 | CTNNA2 | 2 | 79,734,051 |
| 77 | CTNNA2 | 2 | 79,931,793 | 78 | CTNNA2 | 2 | 79,934,690 |
| 79 | CTNNA2 | 2 | 79,942,001 | 80 | CTNNA2 | 2 | 80,337,745 |
| 81 | CTNNA2 | 2 | 80,368,183 | 82 | CTNNA2 | 2 | 80,373,325 |
| 83 | CTNNA2 | 2 | 80,377,211 | 84 | CTNNA2 | 2 | 80,624,319 |
| 85 | NAP5 | 2 | 133,558,451 | 86 | LRP1B | 2 | 140,986,740 |
| 87 | LRP1B | 2 | 142,371,550 | 88 | LRP1B | 2 | 142,377,358 |
| 89 | LRP1B | 2 | 142,395,682 | 90 | LRP1B | 2 | 142,419,855 |
| 91 | LRP1B | 2 | 142,598,638 | 92 | KYNU | 2 | 143,405,309 |
| 93 | KYNU | 2 | 143,455,211 | 94 | KYNU | 2 | 143,516,377 |
| 95 | KIF5C | 2 | 149,600,091 | 96 | CACNB4 | 2 | 152,587,594 |
| 97 | CACNB4 | 2 | 152,632,727 | 98 | FMNL2 | 2 | 152,981,465 |
| 99 | FMNL2 | 2 | 153,086,705 | 100 | FMNL2 | 2 | 153,096,733 |
| 101 | FMNL2 | 2 | 153,097,194 | 102 | FMNL2 | 2 | 153,146,684 |
| 103 | FMNL2 | 2 | 153,147,664 | 104 | FMNL2 | 2 | 153,159,182 |
| 105 | PKP4 | 2 | 159,240,155 | 106 | PKP4 | 2 | 159,240,805 |
| 107 | SCN7A | 2 | 166,998,337 | 108 | CERKL | 2 | 182,130,703 |
| 109 | CERKL | 2 | 182,145,339 | 110 | PDE1A | 2 | 182,763,240 |
| 111 | PARD3B | 2 | 205,659,848 | 112 | NRP2 | 2 | 206,441,948 |
| 113 | PIP5K3 | 2 | 208,956,492 | 114 | DNER | 2 | 229,932,713 |
| 115 | CNTN6 | 3 | 1,414,974 | 116 | CNTN4 | 3 | 2,326,831 |
| 117 | CNTN4 | 3 | 2,395,095 | 118 | CNTN4 | 3 | 2,476,259 |
| 119 | CNTN4 | 3 | 2,509,772 | 120 | ITPR1 | 3 | 4,688,162 |
| 121 | ITPR1 | 3 | 4,715,592 | 122 | IRAK2 | 3 | 10,234,114 |
| 123 | ATP2B2 | 3 | 10,396,240 | 124 | ATP2B2 | 3 | 10,411,869 |
| 125 | SLC6A6 | 3 | 14,495,168 | 126 | SLC6A6 | 3 | 14,495,564 |
| 127 | DAZL | 3 | 16,602,593 | 128 | DAZL | 3 | 16,611,541 |
| 129 | ARPP-21 | 3 | 35,817,608 | 130 | ARPP-21 | 3 | 35,819,665 |
| 131 | STAC | 3 | 36,433,107 | 132 | STAC | 3 | 36,445,311 |
| 133 | ULK4 | 3 | 41,455,051 | 134 | ULK4 | 3 | 41,476,741 |
| 135 | ULK4 | 3 | 41,482,456 | 136 | ULK4 | 3 | 41,501,971 |
| 137 | ULK4 | 3 | 41,507,368 | 138 | ZNF167 | 3 | 44,582,017 |
| 139 | CACNA2D3 | 3 | 55,011,390 | 140 | FLNB | 3 | 58,093,595 |
| 141 | FHIT | 3 | 60,782,868 | 142 | FHIT | 3 | 61,135,266 |
| 143 | FHIT | 3 | 61,137,444 | 144 | FHIT | 3 | 61,146,142 |
| 145 | FHIT | 3 | 61,159,361 | 146 | FHIT | 3 | 61,182,117 |
| 147 | FHIT | 3 | 61,214,250 | 148 | PRICKLE2 | 3 | 64,140,810 |
| 149 | ADAMTS9 | 3 | 64,500,753 | 150 | ADAMTS9 | 3 | 64,507,516 |
| 151 | MAGI1 | 3 | 65,576,699 | 152 | MAGI1 | 3 | 65,576,717 |
| 153 | MAGI1 | 3 | 65,576,735 | 154 | FOXP1 | 3 | 71,021,299 |
| 155 | ROBO1 | 3 | 78,866,959 | 156 | ROBO1 | 3 | 78,867,259 |
| 157 | GBE1 | 3 | 81,669,129 | 158 | GBE1 | 3 | 81,687,869 |
| 159 | GBE1 | 3 | 81,859,450 | 160 | EPHA6 | 3 | 98,268,377 |
| 161 | CBLB | 3 | 107,007,179 | 162 | CBLB | 3 | 107,007,333 |
| 163 | PLCXD2 | 3 | 112,949,845 | 164 | PLCXD2 | 3 | 112,950,789 |
| 165 | PLCXD2 | 3 | 112,953,907 | 166 | PLCXD2 | 3 | 112,960,464 |
| 167 | PLCXD2 | 3 | 112,964,847 | 168 | EPHB1 | 3 | 136,129,897 |
| 169 | SPSB4 | 3 | 142,287,637 | 170 | SPSB4 | 3 | 142,294,879 |
| 171 | SPSB4 | 3 | 142,328,274 | 172 | SERPINI2 | 3 | 168,658,115 |
| 173 | SERPINI2 | 3 | 168,672,301 | 174 | SERPINI2 | 3 | 168,681,607 |
| 175 | TNIK | 3 | 172,402,113 | 176 | PLD1 | 3 | 172,790,551 |
| 177 | PLD1 | 3 | 172,791,538 | 178 | NLGN1 | 3 | 174,870,074 |
| 179 | NLGN1 | 3 | 175,493,649 | 180 | NLGN1 | 3 | 175,515,979 |
| 181 | NLGN1 | 3 | 175,543,000 | 182 | LEPREL1 | 3 | 191,218,155 |
| 183 | LEPREL1 | 3 | 191,244,878 | 184 | LEPREL1 | 3 | 191,245,227 |
| 185 | LEPREL1 | 3 | 191,250,258 | 186 | LEPREL1 | 3 | 191,253,292 |
| 187 | LEPREL1 | 3 | 191,261,577 | 188 | UBXD7 | 3 | 197,576,308 |
| 189 | UBXD7 | 3 | 197,632,452 | 190 | UBXD7 | 3 | 197,636,526 |
| 191 | SLC2A9 | 4 | 9,480,639 | 192 | SLC2A9 | 4 | 9,502,295 |
| 193 | SLC2A9 | 4 | 9,531,228 | 194 | LDB2 | 4 | 16,363,986 |
| 195 | LDB2 | 4 | 16,365,914 | 196 | KCNIP4 | 4 | 20,459,978 |
| 197 | STIM2 | 4 | 26,458,937 | 198 | STIM2 | 4 | 26,459,024 |
| 199 | STIM2 | 4 | 26,459,062 | 200 | STIM2 | 4 | 26,462,717 |
| 201 | STIM2 | 4 | 26,462,750 | 202 | STIM2 | 4 | 26,570,337 |
| 203 | KIAA1239 | 4 | 37,005,415 | 204 | KIAA1239 | 4 | 37,007,192 |
| 205 | UBE2K | 4 | 39,464,805 | 206 | LIMCH1 | 4 | 41,213,350 |
| 207 | LIMCH1 | 4 | 41,213,408 | 208 | LIMCH1 | 4 | 41,221,506 |
| 209 | LIMCH1 | 4 | 41,223,320 | 210 | LIMCH1 | 4 | 41,317,805 |
| 211 | LOC389207 | 4 | 42,652,288 | 212 | LOC389207 | 4 | 42,691,897 |
| 213 | LOC389207 | 4 | 42,693,025 | 214 | SCD5 | 4 | 83,920,815 |
| 215 | SCD5 | 4 | 83,923,596 | 216 | HERC3 | 4 | 89,803,447 |
| 217 | HERC3 | 4 | 89,843,208 | 218 | FAM13A1 | 4 | 89,887,882 |
| 219 | FAM13A1 | 4 | 89,915,913 | 220 | COL25A1 | 4 | 109,934,307 |
| 221 | ANK2 | 4 | 114,340,609 | 222 | CAMK2D | 4 | 114,648,856 |
| 223 | NDST3 | 4 | 119,186,644 | 224 | NDST3 | 4 | 119,256,090 |
| 225 | GPR103 | 4 | 122,487,863 | 226 | MAML3 | 4 | 140,897,308 |
| 227 | IL15 | 4 | 142,764,901 | 228 | IL15 | 4 | 142,911,691 |
| 229 | IL15 | 4 | 142,924,868 | 230 | INPP4B | 4 | 143,414,201 |
| 231 | INPP4B | 4 | 143,421,104 | 232 | INPP4B | 4 | 143,436,219 |
| 233 | INPP4B | 4 | 143,463,006 | 234 | INPP4B | 4 | 143,524,042 |
| 235 | INPP4B | 4 | 143,547,226 | 236 | INPP4B | 4 | 143,566,886 |
| 237 | INPP4B | 4 | 143,593,360 | 238 | INPP4B | 4 | 143,593,371 |
| 239 | INPP4B | 4 | 143,610,749 | 240 | POU4F2 | 4 | 147,739,219 |
| 241 | POU4F2 | 4 | 147,758,283 | 242 | DCLK2 | 4 | 151,378,402 |
| 243 | FSTL5 | 4 | 162,611,763 | 244 | FSTL5 | 4 | 162,953,212 |
| 245 | FSTL5 | 4 | 163,224,500 | 246 | TLL1 | 4 | 167,149,564 |
| 247 | PALLD | 4 | 169,939,539 | 248 | PALLD | 4 | 169,971,032 |
| 249 | CASP3 | 4 | 185,783,023 | 250 | CASP3 | 4 | 185,791,294 |
| 251 | PLEKHG4B | 5 | 238,160 | 252 | PLEKHG4B | 5 | 241,719 |
| 253 | AHRR | 5 | 401,598 | 254 | AHRR | 5 | 431,413 |
| 255 | CTNND2 | 5 | 11,222,945 | 256 | CTNND2 | 5 | 11,637,342 |
| 257 | DNAH5 | 5 | 13,780,350 | 258 | DNAH5 | 5 | 13,782,714 |
| 259 | DNAH5 | 5 | 13,788,147 | 260 | DNAH5 | 5 | 13,795,858 |
| 261 | DNAH5 | 5 | 13,802,952 | 262 | DNAH5 | 5 | 13,822,974 |
| 263 | DNAH5 | 5 | 13,823,000 | 264 | DNAH5 | 5 | 13,825,457 |
| 265 | DNAH5 | 5 | 13,841,886 | 266 | DNAH5 | 5 | 13,842,420 |
| 267 | DNAH5 | 5 | 13,852,381 | 268 | DNAH5 | 5 | 13,854,786 |
| 269 | DNAH5 | 5 | 13,875,140 | 270 | DNAH5 | 5 | 13,887,824 |
| 271 | DNAH5 | 5 | 13,986,416 | 272 | DNAH5 | 5 | 13,989,500 |
| 273 | DNAH5 | 5 | 14,032,397 | 274 | DNAH5 | 5 | 14,034,968 |
| 275 | CDH10 | 5 | 24,728,325 | 276 | CDH10 | 5 | 24,738,958 |
| 277 | SLC45A2 | 5 | 33,979,974 | 278 | SLC45A2 | 5 | 33,990,637 |
| 279 | SLC1A3 | 5 | 36,670,315 | 280 | SLC1A3 | 5 | 36,675,215 |
| 281 | EGFLAM | 5 | 38,269,319 | 282 | EGFLAM | 5 | 38,296,060 |
| 283 | EGFLAM | 5 | 38,337,535 | 284 | EGFLAM | 5 | 38,357,424 |
| 285 | EGFLAM | 5 | 38,382,422 | 286 | EGFLAM | 5 | 38,485,966 |
| 287 | ITGA1 | 5 | 52,294,108 | 288 | ITGA2 | 5 | 52,425,212 |
| 289 | PDE4D | 5 | 58,692,313 | 290 | ELOVL7 | 5 | 60,152,959 |
| 291 | ELOVL7 | 5 | 60,201,944 | 292 | TNPO1 | 5 | 72,123,236 |
| 293 | TNPO1 | 5 | 72,174,208 | 294 | TNPO1 | 5 | 72,182,236 |
| 295 | TNPO1 | 5 | 72,217,160 | 296 | TNPO1 | 5 | 72,292,936 |
| 297 | FCHO2 | 5 | 72,374,865 | 298 | FCHO2 | 5 | 72,384,028 |
| 299 | FCHO2 | 5 | 72,413,771 | 300 | CMYA5 | 5 | 79,058,467 |
| 301 | CMYA5 | 5 | 79,070,418 | 302 | CMYA5 | 5 | 79,074,951 |
| 303 | THBS4 | 5 | 79,376,918 | 304 | THBS4 | 5 | 79,415,294 |
| 305 | VCAN | 5 | 82,914,275 | 306 | MEF2C | 5 | 88,138,619 |
| 307 | GPR98 | 5 | 89,979,411 | 308 | GPR98 | 5 | 89,986,028 |
| 309 | GPR98 | 5 | 90,071,845 | 310 | GPR98 | 5 | 90,428,377 |
| 311 | FBXL17 | 5 | 107,223,636 | 312 | FBXL17 | 5 | 107,224,897 |
| 313 | FBXL17 | 5 | 107,377,610 | 314 | FBXL17 | 5 | 107,380,193 |
| 315 | FBXL17 | 5 | 107,381,014 | 316 | FBXL17 | 5 | 107,400,737 |
| 317 | FBXL17 | 5 | 107,420,461 | 318 | FBXL17 | 5 | 107,556,166 |
| 319 | FBXL17 | 5 | 107,612,678 | 320 | PJA2 | 5 | 108,742,197 |
| 321 | PJA2 | 5 | 108,776,974 | 322 | SNX2 | 5 | 122,116,630 |
| 323 | SNX2 | 5 | 122,137,822 | 324 | SNX2 | 5 | 122,173,535 |
| 325 | SNX2 | 5 | 122,174,171 | 326 | SNX2 | 5 | 122,194,062 |
| 327 | SNX2 | 5 | 122,201,930 | 328 | SNX24 | 5 | 122,232,671 |
| 329 | SNX24 | 5 | 122,255,495 | 330 | SNX24 | 5 | 122,273,273 |
| 331 | SNX24 | 5 | 122,283,900 | 332 | SNX24 | 5 | 122,309,550 |
| 333 | SNX24 | 5 | 122,311,168 | 334 | SNX24 | 5 | 122,383,127 |
| 335 | ADAMTS19 | 5 | 129,039,887 | 336 | TRPC7 | 5 | 135,708,380 |
| 337 | TRPC7 | 5 | 135,713,994 | 338 | CTNNA1 | 5 | 138,167,176 |
| 339 | CTNNA1 | 5 | 138,264,654 | 340 | CTNNA1 | 5 | 138,300,046 |
| 341 | ODZ2 | 5 | 167,527,141 | 342 | ODZ2 | 5 | 167,606,948 |
| 343 | ODZ2 | 5 | 167,621,874 | 344 | ODZ2 | 5 | 167,634,311 |
| 345 | SERPINB6 | 6 | 2,876,878 | 346 | SERPINB6 | 6 | 2,926,223 |
| 347 | SLC22A23 | 6 | 3,395,265 | 348 | JARID2 | 6 | 15,537,710 |
| 349 | JARID2 | 6 | 15,545,369 | 350 | JARID2 | 6 | 15,624,264 |
| 351 | ATXN1 | 6 | 16,755,137 | 352 | ATXN1 | 6 | 16,755,970 |
| 353 | ATXN1 | 6 | 16,762,002 | 354 | RNF144B | 6 | 18,526,687 |
| 355 | RNF144B | 6 | 18,530,528 | 356 | RNF144B | 6 | 18,553,561 |
| 357 | RNF144B | 6 | 18,553,773 | 358 | RNF144B | 6 | 18,561,748 |
| 359 | RNF144B | 6 | 18,566,740 | 360 | RNF144B | 6 | 18,575,501 |
| 361 | SLC17A4 | 6 | 25,876,893 | 362 | SLC17A4 | 6 | 25,892,460 |
| 363 | SLC17A1 | 6 | 25,917,888 | 364 | SLC17A1 | 6 | 25,921,129 |
| 365 | SLC17A1 | 6 | 25,937,638 | 366 | SLC17A1 | 6 | 25,939,191 |
| 367 | SLC17A1 | 6 | 25,950,182 | 368 | SLC17A3 | 6 | 25,978,521 |
| 369 | SLC17A2 | 6 | 26,067,106 | 370 | BTN3A1 | 6 | 26,504,182 |
| 371 | BTN3A1 | 6 | 26,513,969 | 372 | BTN3A1 | 6 | 26,524,633 |
| 373 | BTN2A3 | 6 | 26,532,708 | 374 | BTN2A3 | 6 | 26,534,466 |
| 375 | BTN3A3 | 6 | 26,553,839 | 376 | BTNL2 | 6 | 32,463,841 |
| 377 | BTNL2 | 6 | 32,482,600 | 378 | LRFN2 | 6 | 40,528,210 |
| 379 | ELOVL5 | 6 | 53,213,187 | 380 | ELOVL5 | 6 | 53,221,300 |
| 381 | ELOVL5 | 6 | 53,231,077 | 382 | ELOVL5 | 6 | 53,238,040 |
| 383 | ELOVL5 | 6 | 53,238,244 | 384 | ELOVL5 | 6 | 53,242,957 |
| 385 | ELOVL5 | 6 | 53,246,219 | 386 | ELOVL5 | 6 | 53,254,362 |
| 387 | ELOVL5 | 6 | 53,257,221 | 388 | ELOVL5 | 6 | 53,257,463 |
| 389 | ELOVL5 | 6 | 53,261,913 | 390 | ELOVL5 | 6 | 53,262,169 |
| 391 | ELOVL5 | 6 | 53,267,506 | 392 | ELOVL5 | 6 | 53,271,049 |
| 393 | ELOVL5 | 6 | 53,271,883 | 394 | ELOVL5 | 6 | 53,280,718 |
| 395 | ELOVL5 | 6 | 53,282,407 | 396 | ELOVL5 | 6 | 53,284,867 |
| 397 | ELOVL5 | 6 | 53,292,467 | 398 | ELOVL5 | 6 | 53,294,567 |
| 399 | ELOVL5 | 6 | 53,296,096 | 400 | ELOVL5 | 6 | 53,298,093 |
| 401 | ELOVL5 | 6 | 53,298,209 | 402 | ELOVL5 | 6 | 53,314,948 |
| 403 | ELOVL5 | 6 | 53,318,131 | 404 | ELOVL5 | 6 | 53,341,053 |
| 405 | ELOVL5 | 6 | 53,350,763 | 406 | ELOVL5 | 6 | 53,362,070 |
| 407 | ELOVL5 | 6 | 53,372,444 | 408 | RIMS1 | 6 | 72,967,122 |
| 409 | KLHL32 | 6 | 97,480,306 | 410 | KLHL32 | 6 | 97,589,639 |
| 411 | KLHL32 | 6 | 97,595,870 | 412 | KLHL32 | 6 | 97,601,993 |
| 413 | KLHL32 | 6 | 97,619,298 | 414 | KLHL32 | 6 | 97,673,659 |
| 415 | SLC22A16 | 6 | 110,900,563 | 416 | SLC22A16 | 6 | 110,900,785 |
| 417 | SLC22A16 | 6 | 110,903,098 | 418 | HS3ST5 | 6 | 114,502,447 |
| 419 | TRDN | 6 | 123,982,387 | 420 | TRDN | 6 | 123,991,308 |
| 421 | TRDN | 6 | 124,017,012 | 422 | NKAIN2 | 6 | 124,462,813 |
| 423 | EYA4 | 6 | 133,642,955 | 424 | EYA4 | 6 | 133,643,088 |
| 425 | EYA4 | 6 | 133,651,991 | 426 | EYA4 | 6 | 133,861,896 |
| 427 | PDE7B | 6 | 136,206,096 | 428 | PDE7B | 6 | 136,218,421 |
| 429 | PDE7B | 6 | 136,346,863 | 430 | PLAGL1 | 6 | 144,354,821 |
| 431 | PLAGL1 | 6 | 144,355,380 | 432 | UTRN | 6 | 144,848,384 |
| 433 | SYNE1 | 6 | 152,510,881 | 434 | SYNE1 | 6 | 152,511,829 |
| 435 | SYNE1 | 6 | 152,553,368 | 436 | TFB1M | 6 | 155,655,424 |
| 437 | TFB1M | 6 | 155,663,514 | 438 | PARK2 | 6 | 161,941,630 |
| 439 | PARK2 | 6 | 161,952,077 | 440 | PARK2 | 6 | 162,026,087 |
| 441 | PDE10A | 6 | 165,674,966 | 442 | PDE10A | 6 | 165,679,905 |
| 443 | SDK1 | 7 | 4,152,228 | 444 | SDK1 | 7 | 4,153,102 |
| 445 | SDK1 | 7 | 4,158,337 | 446 | SDK1 | 7 | 4,162,572 |
| 447 | NXPH1 | 7 | 8,590,239 | 448 | NXPH1 | 7 | 8,633,152 |
| 449 | NXPH1 | 7 | 8,633,615 | 450 | SCIN | 7 | 12,627,051 |
| 451 | SCIN | 7 | 12,634,312 | 452 | DGKB | 7 | 14,357,487 |
| 453 | DGKB | 7 | 14,357,894 | 454 | DGKB | 7 | 14,471,463 |
| 455 | DGKB | 7 | 14,472,219 | 456 | DGKB | 7 | 14,529,482 |
| 457 | DGKB | 7 | 14,539,481 | 458 | FERD3L | 7 | 19,167,340 |
| 459 | STK31 | 7 | 23,760,554 | 460 | SKAP2 | 7 | 26,770,543 |
| 461 | SKAP2 | 7 | 26,795,178 | 462 | SKAP2 | 7 | 26,814,684 |
| 463 | SKAP2 | 7 | 26,827,213 | 464 | SKAP2 | 7 | 26,830,698 |
| 465 | SKAP2 | 7 | 26,858,965 | 466 | SKAP2 | 7 | 26,880,921 |
| 467 | CREB5 | 7 | 28,811,671 | 468 | CREB5 | 7 | 28,906,935 |
| 469 | CREB5 | 7 | 28,953,482 | 470 | CHN2 | 7 | 29,215,509 |
| 471 | PDE1C | 7 | 32,037,670 | 472 | PDE1C | 7 | 32,064,508 |
| 473 | PDE1C | 7 | 32,141,959 | 474 | BMPER | 7 | 34,112,924 |
| 475 | BMPER | 7 | 34,176,289 | 476 | BMPER | 7 | 34,184,882 |
| 477 | EEPD1 | 7 | 36,280,985 | 478 | EEPD1 | 7 | 36,391,476 |
| 479 | VPS41 | 7 | 38,798,875 | 480 | VPS41 | 7 | 38,806,116 |
| 481 | VPS41 | 7 | 38,812,386 | 482 | VPS41 | 7 | 38,859,847 |
| 483 | VPS41 | 7 | 38,908,142 | 484 | VPS41 | 7 | 38,911,555 |
| 485 | VPS41 | 7 | 38,936,691 | 486 | CDC2L5 | 7 | 40,063,896 |
| 487 | ABCA13 | 7 | 48,363,317 | 488 | ABCA13 | 7 | 48,380,185 |
| 489 | ABCA13 | 7 | 48,427,057 | 490 | ABCA13 | 7 | 48,454,811 |
| 491 | ABCA13 | 7 | 48,714,222 | 492 | GRB10 | 7 | 50,823,341 |
| 493 | GRB10 | 7 | 50,848,124 | 494 | WBSCR17 | 7 | 70,313,808 |
| 495 | WBSCR17 | 7 | 70,765,881 | 496 | CALN1 | 7 | 71,180,137 |
| 497 | CALN1 | 7 | 71,235,264 | 498 | CALN1 | 7 | 71,338,388 |
| 499 | CALN1 | 7 | 71,339,231 | 500 | CALN1 | 7 | 71,344,745 |
| 501 | MAGI2 | 7 | 77,856,639 | 502 | MAGI2 | 7 | 77,950,934 |
| 503 | MAGI2 | 7 | 78,091,049 | 504 | MAGI2 | 7 | 78,305,682 |
| 505 | MAGI2 | 7 | 78,554,879 | 506 | MAGI2 | 7 | 78,581,862 |
| 507 | MAGI2 | 7 | 78,589,166 | 508 | MAGI2 | 7 | 78,760,470 |
| 509 | MAGI2 | 7 | 78,817,962 | 510 | SEMA3E | 7 | 83,011,138 |
| 511 | ABCB4 | 7 | 86,897,339 | 512 | GTPBP10 | 7 | 89,357,529 |
| 513 | PPP1R9A | 7 | 94,401,225 | 514 | PPP1R9A | 7 | 94,411,224 |
| 515 | PPP1R9A | 7 | 94,431,076 | 516 | PPP1R9A | 7 | 94,479,997 |
| 517 | PPP1R9A | 7 | 94,534,075 | 518 | PPP1R9A | 7 | 94,589,831 |
| 519 | PPP1R9A | 7 | 94,632,721 | 520 | PPP1R9A | 7 | 94,677,380 |
| 521 | PPP1R9A | 7 | 94,681,040 | 522 | PPP1R9A | 7 | 94,717,161 |
| 523 | PPP1R9A | 7 | 94,733,892 | 524 | PPP1R9A | 7 | 94,747,598 |
| 525 | PPP1R9A | 7 | 94,748,184 | 526 | DYNC1I1 | 7 | 95,556,260 |
| 527 | ZNF3 | 7 | 99,480,681 | 528 | ZNF3 | 7 | 99,516,558 |
| 529 | NRCAM | 7 | 107,585,096 | 530 | NRCAM | 7 | 107,598,816 |
| 531 | NRCAM | 7 | 107,608,956 | 532 | NRCAM | 7 | 107,618,181 |
| 533 | NRCAM | 7 | 107,621,970 | 534 | NRCAM | 7 | 107,626,668 |
| 535 | NRCAM | 7 | 107,627,184 | 536 | NRCAM | 7 | 107,628,207 |
| 537 | NRCAM | 7 | 107,738,458 | 538 | NRCAM | 7 | 107,862,002 |
| 539 | KCND2 | 7 | 119,925,320 | 540 | KCND2 | 7 | 119,999,690 |
| 541 | KCND2 | 7 | 120,001,113 | 542 | KCND2 | 7 | 120,018,452 |
| 543 | CADPS2 | 7 | 122,066,038 | 544 | CADPS2 | 7 | 122,074,175 |
| 545 | CADPS2 | 7 | 122,146,928 | 546 | SLC13A1 | 7 | 122,533,104 |
| 547 | SLC13A1 | 7 | 122,560,268 | 548 | SLC13A1 | 7 | 122,596,470 |
| 549 | SLC13A1 | 7 | 122,633,763 | 550 | SLC13A1 | 7 | 122,659,570 |
| 551 | GRM8 | 7 | 125,938,463 | 552 | GRM8 | 7 | 125,940,799 |
| 553 | GRM8 | 7 | 125,974,975 | 554 | GRM8 | 7 | 126,100,080 |
| 555 | GRM8 | 7 | 126,134,129 | 556 | GRM8 | 7 | 126,159,625 |
| 557 | GRM8 | 7 | 126,165,491 | 558 | GRM8 | 7 | 126,174,101 |
| 559 | GRM8 | 7 | 126,208,986 | 560 | GRM8 | 7 | 126,225,935 |
| 561 | GRM8 | 7 | 126,234,800 | 562 | GRM8 | 7 | 126,436,133 |
| 563 | GRM8 | 7 | 126,483,848 | 564 | GRM8 | 7 | 126,674,369 |
| 565 | GRM8 | 7 | 126,692,493 | 566 | EXOC4 | 7 | 132,728,495 |
| 567 | EXOC4 | 7 | 132,754,657 | 568 | EXOC4 | 7 | 132,756,739 |
| 569 | EXOC4 | 7 | 132,889,141 | 570 | EXOC4 | 7 | 133,264,961 |
| 571 | DGKI | 7 | 136,723,471 | 572 | DGKI | 7 | 136,749,535 |
| 573 | DGKI | 7 | 136,769,973 | 574 | DGKI | 7 | 136,775,455 |
| 575 | DGKI | 7 | 136,780,737 | 576 | DGKI | 7 | 136,838,221 |
| 577 | DGKI | 7 | 136,925,970 | 578 | TBXAS1 | 7 | 139,184,991 |
| 579 | CNTNAP2 | 7 | 145,414,794 | 580 | CNTNAP2 | 7 | 145,900,444 |
| 581 | CNTNAP2 | 7 | 146,180,657 | 582 | CNTNAP2 | 7 | 146,405,829 |
| 583 | CNTNAP2 | 7 | 146,414,565 | 584 | CNTNAP2 | 7 | 146,788,354 |
| 585 | CNTNAP2 | 7 | 146,792,712 | 586 | CNTNAP2 | 7 | 146,795,212 |
| 587 | CNTNAP2 | 7 | 147,069,599 | 588 | CNTNAP2 | 7 | 147,073,223 |
| 589 | CNTNAP2 | 7 | 147,710,739 | 590 | PTPRN2 | 7 | 157,017,352 |
| 591 | PTPRN2 | 7 | 157,020,289 | 592 | PTPRN2 | 7 | 157,499,807 |
| 593 | PTPRN2 | 7 | 157,634,704 | 594 | PTPRN2 | 7 | 157,676,114 |
| 595 | PTPRN2 | 7 | 157,714,280 | 596 | PTPRN2 | 7 | 158,015,477 |
| 597 | CSMD1 | 8 | 2,793,553 | 598 | CSMD1 | 8 | 2,864,416 |
| 599 | CSMD1 | 8 | 2,943,570 | 600 | CSMD1 | 8 | 2,954,259 |
| 601 | CSMD1 | 8 | 3,228,881 | 602 | CSMD1 | 8 | 3,230,805 |
| 603 | CSMD1 | 8 | 3,232,022 | 604 | CSMD1 | 8 | 3,232,222 |
| 605 | CSMD1 | 8 | 3,245,103 | 606 | CSMD1 | 8 | 3,250,935 |
| 607 | CSMD1 | 8 | 3,338,555 | 608 | MCPH1 | 8 | 6,414,463 |
| 609 | MCPH1 | 8 | 6,488,786 | 610 | SGCZ | 8 | 14,619,388 |
| 611 | SLC7A2 | 8 | 17,436,984 | 612 | SLC7A2 | 8 | 17,444,141 |
| 613 | PSD3 | 8 | 18,428,754 | 614 | PSD3 | 8 | 18,431,654 |
| 615 | PSD3 | 8 | 18,440,935 | 616 | PSD3 | 8 | 18,457,737 |
| 617 | PSD3 | 8 | 18,465,235 | 618 | PSD3 | 8 | 18,467,749 |
| 619 | PSD3 | 8 | 18,469,732 | 620 | PSD3 | 8 | 18,514,979 |
| 621 | PSD3 | 8 | 18,558,380 | 622 | PSD3 | 8 | 18,736,236 |
| 623 | PSD3 | 8 | 18,749,712 | 624 | PSD3 | 8 | 18,750,003 |
| 625 | PSD3 | 8 | 18,819,182 | 626 | PSD3 | 8 | 18,953,685 |
| 627 | ENTPD4 | 8 | 23,357,109 | 628 | UNC5D | 8 | 35,516,694 |
| 629 | SNTG1 | 8 | 51,805,005 | 630 | SNTG1 | 8 | 51,816,608 |
| 631 | LYN | 8 | 57,039,314 | 632 | LYN | 8 | 57,053,118 |
| 633 | LYN | 8 | 57,054,784 | 634 | LYN | 8 | 57,108,529 |
| 635 | LYN | 8 | 57,110,114 | 636 | PLAG1 | 8 | 57,246,679 |
| 637 | PLAG1 | 8 | 57,263,345 | 638 | TOX | 8 | 59,966,273 |
| 639 | TOX | 8 | 59,972,497 | 640 | TOX | 8 | 59,980,027 |
| 641 | TOX | 8 | 60,198,077 | 642 | NKAIN3 | 8 | 63,649,765 |
| 643 | NKAIN3 | 8 | 63,900,353 | 644 | NKAIN3 | 8 | 63,935,531 |
| 645 | NKAIN3 | 8 | 63,936,341 | 646 | DEPDC2 | 8 | 69,208,963 |
| 647 | DEPDC2 | 8 | 69,209,296 | 648 | CRISPLD1 | 8 | 76,073,693 |
| 649 | CRISPLD1 | 8 | 76,086,655 | 650 | CRISPLD1 | 8 | 76,127,254 |
| 651 | CRISPLD1 | 8 | 76,156,200 | 652 | CRISPLD1 | 8 | 76,158,048 |
| 653 | MMP16 | 8 | 89,276,696 | 654 | BAALC | 8 | 104,271,601 |
| 655 | ZFPM2 | 8 | 106,614,970 | 656 | ZFPM2 | 8 | 106,687,838 |
| 657 | ZFPM2 | 8 | 106,688,431 | 658 | ZFPM2 | 8 | 106,847,240 |
| 659 | ZFPM2 | 8 | 106,880,874 | 660 | CSMD3 | 8 | 113,337,274 |
| 661 | CSMD3 | 8 | 113,443,580 | 662 | CSMD3 | 8 | 113,452,239 |
| 663 | CSMD3 | 8 | 113,467,267 | 664 | CSMD3 | 8 | 113,469,659 |
| 665 | CSMD3 | 8 | 113,473,493 | 666 | CSMD3 | 8 | 113,510,014 |
| 667 | CSMD3 | 8 | 113,554,821 | 668 | CSMD3 | 8 | 114,069,034 |
| 669 | CSMD3 | 8 | 114,093,751 | 670 | CSMD3 | 8 | 114,098,597 |
| 671 | CSMD3 | 8 | 114,104,594 | 672 | CSMD3 | 8 | 114,105,767 |
| 673 | CSMD3 | 8 | 114,160,908 | 674 | CSMD3 | 8 | 114,208,773 |
| 675 | CSMD3 | 8 | 114,359,208 | 676 | SAMD12 | 8 | 119,514,190 |
| 677 | SAMD12 | 8 | 119,514,541 | 678 | SAMD12 | 8 | 119,518,606 |
| 679 | SAMD12 | 8 | 119,531,636 | 680 | SAMD12 | 8 | 119,587,094 |
| 681 | SAMD12 | 8 | 119,680,657 | 682 | SAMD12 | 8 | 119,719,731 |
| 683 | FER1L6 | 8 | 125,053,616 | 684 | FER1L6 | 8 | 125,122,813 |
| 685 | FER1L6 | 8 | 125,123,546 | 686 | MTSS1 | 8 | 125,634,509 |
| 687 | MTSS1 | 8 | 125,700,325 | 688 | MTSS1 | 8 | 125,708,636 |
| 689 | MTSS1 | 8 | 125,737,780 | 690 | MTSS1 | 8 | 125,802,038 |
| 691 | DDEF1 | 8 | 131,286,874 | 692 | DDEF1 | 8 | 131,298,362 |
| 693 | DDEF1 | 8 | 131,368,653 | 694 | DDEF1 | 8 | 131,406,218 |
| 695 | DDEF1 | 8 | 131,423,273 | 696 | KCNQ3 | 8 | 133,208,937 |
| 697 | KCNQ3 | 8 | 133,211,492 | 698 | KCNQ3 | 8 | 133,212,327 |
| 699 | KCNQ3 | 8 | 133,346,913 | 700 | KCNQ3 | 8 | 133,351,663 |
| 701 | COL22A1 | 8 | 139,744,740 | 702 | COL22A1 | 8 | 139,759,058 |
| 703 | COL22A1 | 8 | 139,771,875 | 704 | COL22A1 | 8 | 139,777,590 |
| 705 | COL22A1 | 8 | 139,833,578 | 706 | ZC3H3 | 8 | 144,691,326 |
| 707 | SMARCA2 | 9 | 2,053,356 | 708 | SMARCA2 | 9 | 2,170,694 |
| 709 | PIP5K1B | 9 | 70,545,037 | 710 | PIP5K1B | 9 | 70,687,329 |
| 711 | TRPM3 | 9 | 72,616,673 | 712 | TMC1 | 9 | 74,485,413 |
| 713 | TMC1 | 9 | 74,492,549 | 714 | TMC1 | 9 | 74,493,473 |
| 715 | TMC1 | 9 | 74,507,694 | 716 | TMC1 | 9 | 74,623,740 |
| 717 | PCSK5 | 9 | 77,897,682 | 718 | GNAQ | 9 | 79,856,574 |
| 719 | GNAQ | 9 | 79,934,142 | 720 | GNAQ | 9 | 79,946,188 |
| 721 | NTRK2 | 9 | 86,577,727 | 722 | DIRAS2 | 9 | 92,422,258 |
| 723 | DIRAS2 | 9 | 92,476,677 | 724 | NFIL3 | 9 | 93,218,808 |
| 725 | FKTN | 9 | 107,389,052 | 726 | FKTN | 9 | 107,409,195 |
| 727 | FKTN | 9 | 107,420,176 | 728 | FKTN | 9 | 107,435,753 |
| 729 | FKTN | 9 | 107,445,165 | 730 | FKTN | 9 | 107,449,221 |
| 731 | FKTN | 9 | 107,449,435 | 732 | PALM2 | 9 | 111,506,598 |
| 733 | PALM2 | 9 | 111,552,543 | 734 | PALM2 | 9 | 111,554,864 |
| 735 | PALM2 | 9 | 111,555,049 | 736 | PALM2 | 9 | 111,556,167 |
| 737 | ZNF618 | 9 | 115,804,104 | 738 | ZNF618 | 9 | 115,816,110 |
| 739 | ZNF618 | 9 | 115,820,286 | 740 | ASTN2 | 9 | 119,198,404 |
| 741 | CDK5RAP2 | 9 | 122,346,776 | 742 | CDK5RAP2 | 9 | 122,377,819 |
| 743 | PTGS1 | 9 | 124,225,832 | 744 | RALGPS1 | 9 | 128,769,224 |
| 745 | RALGPS1 | 9 | 128,809,594 | 746 | RALGPS1 | 9 | 128,850,765 |
| 747 | RALGPS1 | 9 | 128,902,817 | 748 | RALGPS1 | 9 | 128,972,837 |
| 749 | SLC25A25 | 9 | 129,899,153 | 750 | TSC1 | 9 | 134,778,093 |
| 751 | TSC1 | 9 | 134,786,003 | 752 | TSC1 | 9 | 134,812,111 |
| 753 | VAV2 | 9 | 135,708,866 | 754 | VAV2 | 9 | 135,710,406 |
| 755 | VAV2 | 9 | 135,710,620 | 756 | VAV2 | 9 | 135,714,835 |
| 757 | PITRM1 | 10 | 3,179,115 | 758 | PRKCQ | 10 | 7,131,891 |
| 759 | PRKCQ | 10 | 7,136,154 | 760 | PRKCQ | 10 | 7,137,198 |
| 761 | CACNB2 | 10 | 18,861,080 | 762 | ARMC3 | 10 | 23,264,868 |
| 763 | ARMC3 | 10 | 23,270,471 | 764 | ARMC3 | 10 | 23,271,006 |
| 765 | ARMC3 | 10 | 23,343,259 | 766 | ARMC3 | 10 | 23,343,422 |
| 767 | ARMC3 | 10 | 23,372,821 | 768 | ARMC3 | 10 | 23,372,952 |
| 769 | MYO3A | 10 | 26,396,941 | 770 | MYO3A | 10 | 26,412,733 |
| 771 | ANK3 | 10 | 61,569,339 | 772 | JMJD1C | 10 | 64,732,014 |
| 773 | JMJD1C | 10 | 64,736,192 | 774 | JMJD1C | 10 | 64,791,571 |
| 775 | JMJD1C | 10 | 64,873,814 | 776 | CDH23 | 10 | 72,997,429 |
| 777 | KCNMA1 | 10 | 78,258,751 | 778 | KCNMA1 | 10 | 78,320,267 |
| 779 | SORCS3 | 10 | 106,525,169 | 780 | VTI1A | 10 | 114,448,959 |
| 781 | VTI1A | 10 | 114,458,127 | 782 | VTI1A | 10 | 114,496,282 |
| 783 | VTI1A | 10 | 114,497,586 | 784 | VTI1A | 10 | 114,504,041 |
| 785 | ATRNL1 | 10 | 117,429,854 | 786 | HSPA12A | 10 | 118,432,901 |
| 787 | GRK5 | 10 | 121,055,451 | 788 | ATE1 | 10 | 123,478,029 |
| 789 | ATE1 | 10 | 123,484,634 | 790 | ATE1 | 10 | 123,634,335 |
| 791 | ATE1 | 10 | 123,644,015 | 792 | ATE1 | 10 | 123,648,119 |
| 793 | ATE1 | 10 | 123,648,218 | 794 | ATE1 | 10 | 123,648,608 |
| 795 | ATE1 | 10 | 123,666,737 | 796 | ATE1 | 10 | 123,669,674 |
| 797 | ATE1 | 10 | 123,679,356 | 798 | STIM1 | 11 | 4,033,624 |
| 799 | STIM1 | 11 | 4,047,324 | 800 | TRIM21 | 11 | 4,371,449 |
| 801 | GALNTL4 | 11 | 11,222,747 | 802 | GALNTL4 | 11 | 11,228,193 |
| 803 | GALNTL4 | 11 | 11,246,740 | 804 | SPON1 | 11 | 13,883,488 |
| 805 | SPON1 | 11 | 14,244,704 | 806 | USH1C | 11 | 17,475,101 |
| 807 | USH1C | 11 | 17,487,060 | 808 | USH1C | 11 | 17,489,173 |
| 809 | USH1C | 11 | 17,489,973 | 810 | USH1C | 11 | 17,490,211 |
| 811 | USH1C | 11 | 17,491,427 | 812 | USH1C | 11 | 17,519,298 |
| 813 | USH1C | 11 | 17,520,065 | 814 | USH1C | 11 | 17,523,687 |
| 815 | IGSF22 | 11 | 18,689,422 | 816 | IGSF22 | 11 | 18,692,523 |
| 817 | IGSF22 | 11 | 18,694,857 | 818 | C11ORF49 | 11 | 46,955,088 |
| 819 | C11ORF49 | 11 | 47,130,872 | 820 | C11ORF49 | 11 | 47,132,581 |
| 821 | DLG2 | 11 | 82,843,293 | 822 | DLG2 | 11 | 82,856,041 |
| 823 | DLG2 | 11 | 82,910,180 | 824 | DLG2 | 11 | 83,186,640 |
| 825 | DLG2 | 11 | 83,187,421 | 826 | DLG2 | 11 | 83,203,872 |
| 827 | DLG2 | 11 | 83,366,230 | 828 | DLG2 | 11 | 84,153,124 |
| 829 | DLG2 | 11 | 84,175,263 | 830 | DLG2 | 11 | 84,198,828 |
| 831 | DLG2 | 11 | 84,234,326 | 832 | ELMOD1 | 11 | 107,051,899 |
| 833 | OPCML | 11 | 131,718,194 | 834 | TSPAN9 | 12 | 3,058,775 |
| 835 | TSPAN9 | 12 | 3,065,463 | 836 | TSPAN9 | 12 | 3,173,346 |
| 837 | TSPAN9 | 12 | 3,331,863 | 838 | STYK1 | 12 | 10,666,462 |
| 839 | STYK1 | 12 | 10,697,767 | 840 | STYK1 | 12 | 10,708,924 |
| 841 | STYK1 | 12 | 10,711,189 | 842 | LOC729025 | 12 | 16,250,464 |
| 843 | LRP1 | 12 | 55,847,718 | 844 | LRP1 | 12 | 55,878,824 |
| 845 | CNOT2 | 12 | 68,831,198 | 846 | CNOT2 | 12 | 69,026,461 |
| 847 | KCNC2 | 12 | 73,584,602 | 848 | NAV3 | 12 | 76,913,545 |
| 849 | NAV3 | 12 | 76,922,312 | 850 | GAS2L3 | 12 | 99,519,980 |
| 851 | MTIF3 | 13 | 26,895,700 | 852 | MTIF3 | 13 | 26,895,999 |
| 853 | SLC46A3 | 13 | 28,185,843 | 854 | SLC46A3 | 13 | 28,217,711 |
| 855 | SLC46A3 | 13 | 28,267,317 | 856 | UBL3 | 13 | 29,194,621 |
| 857 | N4BP2L2 | 13 | 32,021,516 | 858 | NBEA | 13 | 35,086,809 |
| 859 | NBEA | 13 | 35,087,894 | 860 | NBEA | 13 | 35,130,015 |
| 861 | SLAIN1 | 13 | 77,151,328 | 862 | GPC5 | 13 | 91,268,794 |
| 863 | GPC5 | 13 | 91,346,842 | 864 | GPC5 | 13 | 91,372,652 |
| 865 | GPC6 | 13 | 93,138,384 | 866 | GPC6 | 13 | 93,183,430 |
| 867 | NALCN | 13 | 100,517,778 | 868 | NALCN | 13 | 100,522,814 |
| 869 | ITGBL1 | 13 | 101,013,965 | 870 | WDR23 | 14 | 23,657,479 |
| 871 | WDR23 | 14 | 23,671,158 | 872 | NPAS3 | 14 | 32,590,670 |
| 873 | NPAS3 | 14 | 32,658,581 | 874 | GNG2 | 14 | 51,414,961 |
| 875 | GNG2 | 14 | 51,422,662 | 876 | SAMD4A | 14 | 54,319,095 |
| 877 | SAMD4A | 14 | 54,335,279 | 878 | SAMD4A | 14 | 54,335,321 |
| 879 | PPP2R5E | 14 | 63,040,607 | 880 | PPP2R5E | 14 | 63,099,534 |
| 881 | RGS6 | 14 | 71,538,177 | 882 | RGS6 | 14 | 71,539,344 |
| 883 | RGS6 | 14 | 71,540,089 | 884 | RGS6 | 14 | 71,723,690 |
| 885 | RGS6 | 14 | 71,793,717 | 886 | KCNK10 | 14 | 87,792,199 |
| 887 | KCNK13 | 14 | 89,783,756 | 888 | KCNK13 | 14 | 89,785,725 |
| 889 | PSMC1 | 14 | 89,794,414 | 890 | PSMC1 | 14 | 89,794,847 |
| 891 | PSMC1 | 14 | 89,806,078 | 892 | RPS6KA5 | 14 | 90,412,434 |
| 893 | RPS6KA5 | 14 | 90,501,524 | 894 | RPS6KA5 | 14 | 90,527,909 |
| 895 | RPS6KA5 | 14 | 90,557,351 | 896 | RPS6KA5 | 14 | 90,572,871 |
| 897 | RPS6KA5 | 14 | 90,600,927 | 898 | RPS6KA5 | 14 | 90,616,889 |
| 899 | CCDC88C | 14 | 90,821,027 | 900 | CCDC88C | 14 | 90,910,783 |
| 901 | CCDC88C | 14 | 90,916,433 | 902 | CCDC88C | 14 | 90,918,598 |
| 903 | CCDC88C | 14 | 90,920,049 | 904 | BCL11B | 14 | 98,746,376 |
| 905 | BCL11B | 14 | 98,757,526 | 906 | BCL11B | 14 | 98,765,857 |
| 907 | ATP10A | 15 | 23,493,398 | 908 | ATP10A | 15 | 23,513,966 |
| 909 | C15ORF41 | 15 | 34,833,829 | 910 | GLDN | 15 | 49,457,768 |
| 911 | GLDN | 15 | 49,457,862 | 912 | GLDN | 15 | 49,459,928 |
| 913 | GLDN | 15 | 49,460,922 | 914 | CLK3 | 15 | 72,707,585 |
| 915 | CLK3 | 15 | 72,727,323 | 916 | TBCID2B | 15 | 76,083,890 |
| 917 | TBCID2B | 15 | 76,098,398 | 918 | PCSK6 | 15 | 99,842,741 |
| 919 | PCSK6 | 15 | 99,858,640 | 920 | A2BP1 | 16 | 7,444,597 |
| 921 | A2BP1 | 16 | 7,547,113 | 922 | A2BP1 | 16 | 7,568,164 |
| 923 | TMC5 | 16 | 19,365,820 | 924 | TMC5 | 16 | 19,403,812 |
| 925 | TMC5 | 16 | 19,413,252 | 926 | TMC5 | 16 | 19,432,372 |
| 927 | GDE1 | 16 | 19,435,278 | 928 | GDE1 | 16 | 19,455,004 |
| 929 | HYDIN | 16 | 69,765,380 | 930 | HYDIN | 16 | 69,818,152 |
| 931 | HYDIN | 16 | 69,856,735 | 932 | WWOX | 16 | 76,673,124 |
| 933 | WWOX | 16 | 76,739,487 | 934 | WWOX | 16 | 76,793,572 |
| 935 | WWOX | 16 | 77,041,675 | 936 | MPHOSPH6 | 16 | 80,728,868 |
| 937 | MPHOSPH6 | 16 | 80,759,877 | 938 | MPHOSPH6 | 16 | 80,765,783 |
| 939 | MPHOSPH6 | 16 | 80,777,144 | 940 | MPHOSPH6 | 16 | 80,817,367 |
| 941 | MPHOSPH6 | 16 | 80,820,057 | 942 | MPHOSPH6 | 16 | 80,831,708 |
| 943 | MPHOSPH6 | 16 | 80,833,378 | 944 | CDH13 | 16 | 81,390,450 |
| 945 | CDH13 | 16 | 81,430,291 | 946 | CDH13 | 16 | 82,324,245 |
| 947 | USP10 | 16 | 83,325,736 | 948 | DNAH9 | 17 | 11,643,618 |
| 949 | DNAH9 | 17 | 11,671,648 | 950 | RAB11FIP4 | 17 | 26,746,745 |
| 951 | CA10 | 17 | 47,069,220 | 952 | CA10 | 17 | 47,599,744 |
| 953 | HRNBP3 | 17 | 74,845,144 | 954 | ZFP161 | 18 | 5,280,198 |
| 955 | ZFP161 | 18 | 5,283,943 | 956 | ZFP161 | 18 | 5,284,323 |
| 957 | ZFP161 | 18 | 5,398,814 | 958 | ZFP161 | 18 | 5,406,160 |
| 959 | PTPRM | 18 | 7,585,927 | 960 | PTPRM | 18 | 7,892,147 |
| 961 | PTPRM | 18 | 8,041,285 | 962 | KIAA0802 | 18 | 8,840,062 |
| 963 | KIAA0802 | 18 | 8,844,132 | 964 | OSBPL1A | 18 | 20,105,278 |
| 965 | CHST9 | 18 | 22,787,798 | 966 | CHST9 | 18 | 22,874,298 |
| 967 | CHST9 | 18 | 22,874,628 | 968 | CHST9 | 18 | 23,010,213 |
| 969 | CHST9 | 18 | 23,012,354 | 970 | NEDD4L | 18 | 54,173,333 |
| 971 | NEDD4L | 18 | 54,202,587 | 972 | NEDD4L | 18 | 54,258,677 |
| 973 | NEDD4L | 18 | 54,259,246 | 974 | CCBE1 | 18 | 55,220,303 |
| 975 | CCBE1 | 18 | 55,241,702 | 976 | CCBE1 | 18 | 55,483,138 |
| 977 | CDH7 | 18 | 61,594,009 | 978 | CDH7 | 18 | 61,697,973 |
| 979 | CDH7 | 18 | 61,739,825 | 980 | CDH7 | 18 | 61,759,477 |
| 981 | TXNDC10 | 18 | 64,536,945 | 982 | ATRN | 20 | 3,400,447 |
| 983 | FERMT1 | 20 | 6,037,513 | 984 | FERMT1 | 20 | 6,041,177 |
| 985 | PLCB1 | 20 | 8,000,158 | 986 | PLCB4 | 20 | 9,433,807 |
| 987 | MACROD2 | 20 | 15,551,153 | 988 | MACROD2 | 20 | 15,559,316 |
| 989 | MACROD2 | 20 | 15,586,912 | 990 | MACROD2 | 20 | 16,020,951 |
| 991 | KIF16B | 20 | 16,445,469 | 992 | KIF16B | 20 | 16,454,369 |
| 993 | MAPRE1 | 20 | 30,888,730 | 994 | MAPRE1 | 20 | 30,906,484 |
| 995 | PTPRT | 20 | 40,087,340 | 996 | ZSWIM3 | 20 | 43,921,606 |
| 997 | ZSWIM3 | 20 | 43,930,226 | 998 | ZSWIM3 | 20 | 43,939,825 |
| 999 | SNAI1 | 20 | 48,025,165 | 1000 | SNAI1 | 20 | 48,036,030 |
| 1001 | SNAI1 | 20 | 48,037,294 | 1002 | SNAI1 | 20 | 48,051,852 |
| 1003 | BMP7 | 20 | 55,238,534 | 1004 | CDH4 | 20 | 59,359,672 |
| 1005 | C21ORF37 | 21 | 17,711,037 | 1006 | C21ORF37 | 21 | 17,738,145 |
| 1007 | NCAM2 | 21 | 21,399,144 | 1008 | NCAM2 | 21 | 21,451,606 |
| 1009 | NCAM2 | 21 | 21,658,511 | 1010 | NCAM2 | 21 | 21,664,277 |
| 1011 | PDE9A | 21 | 43,061,181 | 1012 | ARVCF | 22 | 18,342,203 |
| 1013 | ARVCF | 22 | 18,371,950 | 1014 | SGSM1 | 22 | 23,675,537 |
| 1015 | ASPHD2 | 22 | 25,130,199 | 1016 | ASPHD2 | 22 | 25,131,287 |
| 1017 | ASPHD2 | 22 | 25,166,780 | 1018 | HPS4 | 22 | 25,200,502 |
| 1019 | ARFGAP3 | 22 | 41,536,894 | 1020 | ARFGAP3 | 22 | 41,576,090 |
| 1021 | TTLL1 | 22 | 41,789,790 | 1022 | TTLL1 | 22 | 41,822,906 |
| 1023 | EFCAB6 | 22 | 42,411,013 | 1024 | EFCAB6 | 22 | 42,527,300 |
| 1025 | RIBC2 | 22 | 44,217,310 | | | | |

Although the present invention is defined in the attached claims, it is to be understood that the invention can alternatively also be defined in accordance with the following embodiments:

### EMBODIMENTS:

1. A method of determining risk of developing schizophrenia (SZ) in a human subject, the method comprising:
   determining the identity of at least one allele of a single nucleotide polymorphism (SNP) listed in Table 1; and
   comparing the identity of the allele in the subject with a reference allele, wherein the reference allele is associated with a known risk of developing SZ; and
   wherein the presence of an allele in the subject that is the same as the reference allele that is associated with the known risk of developing SZ indicates the risk that the subject will develop SZ.
2. A method of determining risk of developing SZ in a human subject, the method comprising:
   determining the copy number of at least one single nucleotide polymorphism (SNP) listed in Table 2 or 3; and
   comparing the copy number of the SNP in the subject with a reference copy number, wherein the reference copy number is associated with a known risk of developing SZ; and
   wherein the presence of a copy number of the SNP in the subject that is the same as a reference copy number that is associated with SZ indicates that the risk that the subject will develop SZ.
3. The method of embodiments 1 or 2, wherein determining the identity or copy number of an allele comprises obtaining a sample comprising DNA from the subject, and determining identity or copy number of the nucleotide at the polymorphic site.
4. The method of embodiment 3, wherein determining the identity of the nucleotide comprises contacting the sample with a probe specific for a selected allele of the polymorphism, and detecting the formation of complexes between the probe and the selected allele of the polymorphism, wherein the formation of complexes between the probe and the test marker indicates the presence of the selected allele in the sample.
5. The method of embodiment 4, wherein determining the identity of an allele comprises determining the identity of the nucleotide at position 31 of one of SEQ ID NOs: 1-1025.
6. The method of embodiment 3, wherein determining the copy number of an allele comprises contacting the sample with a probe specific for a selected allele, detecting the formation of complexes between the probe and the selected allele of the polymorphism, and quantifying the complexes, wherein the quantification of the complexes indicates the copy number of the selected allele in the sample.
7. The method of embodiment 6, wherein the probe comprises a fluorescent label, and quantifying the complexes comprises detecting intensity of emission from the label.
8. The method of embodiment 2, wherein determining the copy number of an allele comprises detecting the absence or duplication of the allele in the subject.
9. The method of embodiment 1 or 2, wherein the subject is a patient having or suspected of having SZ.
10. The method of embodiment or 2, wherein the subject has one or more risk factors associated with SZ.
11. The method of embodiment 8, wherein the risk factors associated with SZ include one or more of: a relative afflicted with SZ; and a genetically based phenotypic trait associated with risk for a SZ.
12. The method of embodiment 1 or 2, wherein the subject has exhibited or exhibits symptoms of psychosis.
13. The method of embodiments 1 or 2, further comprising selecting or excluding a subject for enrollment in a clinical trial based on the identity of the allele.
14. The method of embodiments 1 or 2, further comprising stratifying a subject population for analysis of a clinical trial based on the identity of the allele in the subjects.
15. The method of embodiments 1 or 2, further comprising confirming a diagnosis of a SZ using psychometric instruments.
16. The method of embodiment 1, further comprising selecting a treatment for SZ if an allele in the subject is the same as a reference allele associated with SZ.
17. The method of embodiment 2, further comprising selecting a treatment for SZ if the copy number of the allele in the subject is the same as a reference copy number associated with SZ.
18. The method of embodiment 14 or 15, further comprising administering the selected treatment to the subject.
19. The method of embodiment 14 or 15, wherein the treatment comprises one or more of an anti-psychotic drug, an anti-depressant drug, anti-anxiety drug, mood stabilizer, selective serotonin reuptake inhibitor (SSRI), psychotherapy, or a stimulant.
20. The method of embodiments 1 or 2, further comprising recording the identity of the allele in a tangible medium.
21. The method of embodiments 1 or 2, wherein the tangible medium comprises a computer-readable disk, a solid state memory device, or an optical storage device.
22. The method of embodiment 1, wherein the single nucleotide polymorphism (SNP) is at position 31 of a sequence selected from the group consisting of SEQ ID NO:656, 665, 248, 639, 25, 494, 700, and 691.
23. The method of embodiment 2, wherein the single nucleotide polymorphism (SNP) is at position 31 of a sequence selected from the group consisting of SEQ ID NO:665, 314, 679, 597, 523, 619, 402, 677, 586, 558, 664, 87, 851, and 500.

A number of implementations have been described. Nevertheless, it will be understood that various modifications may be made without departing from the spirit and scope of the following claims. Accordingly, other implementations are within the scope of the following claims.

## Claims

1. A method of determining the risk of developing schizophrenia (SZ) in a human subject, the method comprising:
determining the copy number of at least one single nucleotide polymorphism (SNP), wherein the at least one SNP is selected from the group consisting of: rs341724, rs7819225, rs750318, rs13254344, rs895696, rs7001551, and rs17066296 in the CUB and Sushi Multiple Domains 1 (CSMD1) gene, and a polymorphism that is in linkage disequilibrium (LD) with rs341724, rs7819225, rs750318, rs13254344, rs895696, rs7001551, or rs17066296, in a sample comprising genomic DNA from a human subject; and
comparing the copy number of the at least one SNP in the subject with a reference copy number, wherein the reference copy number is associated with a known risk of developing SZ;
wherein the presence of a copy number of the at least one SNP in the subject as compared to the reference copy number indicates the risk that the human subject will develop SZ.

2. The method of claim 1, wherein a copy number of the at least one SNP that is fewer than two indicates a deletion of the at least one SNP, or a copy number that is more than two of the at least one SNP indicates a duplication of the at least one SNP.

3. The method of claim 2, wherein a deletion in one or both of rs341724 and rs7819225 indicates that the subject has a decreased risk of developing SZ, and/or a deletion of rs7001551 indicates that the subject has an increased risk of developing SZ.

4. The method of claim 2, wherein a duplication in one or both of rs341724 and rs17066296 indicates that the subject has a decreased risk of developing SZ, and/or a duplication in one or more of rs750318, rs13254344, and rs895696 indicates that the subject has an increased risk of developing SZ.

5. The method of claim 1, wherein determining the copy number of a selected SNP from the at least one SNP comprises:
contacting the sample with a probe specific for the selected SNP;
detecting the formation of complexes between the probe and the selected SNP; and
quantifying the complexes,
wherein the quantification of the complexes indicates the copy number of the selected SNP in the sample.

6. The method of claim 5, wherein the probe comprises a fluorescent label, and quantifying the complexes comprises detecting intensity of emission from the label.

7. The method of claim 1, wherein determining the copy number of the at least one SNP comprises detecting the absence or duplication of the at least one SNP in the sample.

8. A method of determining risk of developing schizophrenia (SZ) in a human subject, the method comprising:
determining the identity of at least one single nucleotide polymorphism (SNP) selected from the group consisting of rs 12543159, rs 1161518, rs7845140, and rs895695 in a sample comprising genomic DNA from a human subject;
comparing the identity of the at least one SNP in the sample with a reference allele associated with a known risk of developing SZ,
wherein the presence of an allele of at least one SNP in the subject that is the same as the reference allele indicates the risk that the subject will develop SZ.

9. The method of claim 8, wherein the presence of an T allele at rs 12543159 and/or the presence of a T allele at rs 1161518 in the sample indicates that the subject has a decreased risk of developing SZ, or the presence of a G allele at rs7845140 and/or a G allele at rs895695 indicates that the subject has an increased risk of developing SZ.

10. The method of claim 8, wherein determining the identity of an allele at a selected SNP of the at least one SNP comprises:
contacting the sample with a probe specific for a specific allele at the selected SNP; and
detecting the formation of complexes between the probe and the specific allele at the selected SNP;
wherein formation of complexes between the probe and the specific allele at the selected SNP in the sample indicates the presence of the specific allele at the selected SNP.

11. The method of claim 1, wherein the at least one SNP is at position 31 of SEQ ID NO: 597, 599, 600, 601, 602, 603, or 607.

12. The method of claim 8, wherein the at least one SNP is at position 31 of SEQ ID NO: 598, 604, 605, or 606.

13. The method of claim 1 or 8, wherein the human subject is a patient having or suspected of having SZ, or wherein the human subject has one or more risk factors associated with SZ, optionally wherein the risk factors associated with SZ include one or more of: a relative afflicted with SZ; and a genetically based phenotypic trait associated with risk for SZ.

14. The method of claim 1 or 8, wherein the human subject has exhibited or exhibits symptoms of psychosis.

15. The method of claim 8, further comprising:
selecting or excluding the human subject for enrollment in a clinical trial based on the identity of the at least one SNP;
stratifying a population of the human subjects for analysis of a clinical trial based on the identity of the at least one SNP; or
confirming a diagnosis of SZ in the human subject using psychometric instruments.


SEQUENCE LISTING

<110>  SureGene LLC

<120>  GENETIC MARKERS FOR ASSESSING RISK OF DEVELOPING SCHIZOPHRENIA

<130>  24689-0006EP2

<140>   EP09816956.8
<141>   2009-09-25

<150>   PCT/US2009/058487
<151>   2009-09-25

<150>   US 61/100,176
<151>   2008-09-25


<160>  1025

<170>  PatentIn version 3.5

<210>  1
<211>  61
<212>  DNA
<213>  Homo Sapiens

<400>  1
ctgtcaccta ggctggaggg cagtggttca mtcacggttt tctgcagcct tgacctcctg      60

g                                                                      61


<210>  2
<211>  61
<212>  DNA
<213>  Homo Sapiens

<400>  2
cattggattt caaagcttgc tttttctgtt sgttcttctc tctccttctc tctctgtctc      60

t                                                                      61


<210>  3
<211>  61
<212>  DNA
<213>  Homo Sapiens

<400>  3
tgccagatga gctctctgaa gtcactgtaa kcaattctca gagccaccaa gtttaagtca      60

g                                                                      61


<210>  4
<211>  61
<212>  DNA
<213>  Homo Sapiens

<400>  4
gcaagataga tattagtact cctgcttttt ytgtcaacta aactgaggca cagagatgtt      60

a                                                                   61

<210>  5
<211>  61
<212>  DNA
<213>  Homo Sapiens

<400>  5
ctcaaacttg gcaactctaa aatgagcaag ygagactgaa atgccatcag tgactgggag      60

c                                                                   61

<210>  6
<211>  61
<212>  DNA
<213>  Homo Sapiens

<400>  6
ctaaaacctg ctgtgggcgt cacacttctc ygggagcatc attgggcagc tcagccggcc      60

a                                                                   61

<210>  7
<211>  61
<212>  DNA
<213>  Homo Sapiens

<400>  7
aatgatgaag atttagctat aaggaagttt wtcaaagcac attaagaaag acaatagtta      60

c                                                                   61

<210>  8
<211>  61
<212>  DNA
<213>  Homo Sapiens

<400>  8
tatgcttcat catattagcc tttacctctc yagacaaagt tcatccacct atttggtaag      60

t                                                                   61

<210>  9
<211>  61
<212>  DNA
<213>  Homo Sapiens

<400>  9
aacttggact tttcctgaat atcgaatctg ycagtagcag agaccaatac tgattctctg      60

a                                                                   61

<210>  10
<211>  61
<212>  DNA
<213>  Homo Sapiens

<400>  10
atcatggagg gaattcatat gctgagtagg wtatttgctg gtttttatct tcaagtttcc      60

t                                                                      61


<210>  11
<211>  61
<212>  DNA
<213>  Homo Sapiens

<400>  11
atgatgaatt ctttggtttg tctccccagc ragtggcatt aagccttgaa gatgatggac      60

t                                                                      61


<210>  12
<211>  61
<212>  DNA
<213>  Homo Sapiens

<400>  12
tctctttcaa cctccctgca tgacagctgg maagagccca tccctaatct tcaccaaccg      60

g                                                                      61


<210>  13
<211>  61
<212>  DNA
<213>  Homo Sapiens

<400>  13
ccagggacca gggggccatg aaagatgtta wgtagagaag ggtcagattt aggatttgaa      60

a                                                                      61


<210>  14
<211>  61
<212>  DNA
<213>  Homo Sapiens

<400>  14
taaaatatta cttttataat ttaagtgtgc rtcctctaag ctattcggtt ttgtagttat      60

a                                                                      61


<210>  15
<211>  61
<212>  DNA
<213>  Homo Sapiens

<400>  15
   gacttttcca tgaaagagga aagtttctct scataacaag ggggtggaaa tgaattggat      60
   g                                                                      61


<210>  16
<211>  61

<212>  DNA
<213>  Homo Sapiens

<400>  16
tccagcagtg ccctcctctc tcagaccctg ytcttgtcac atgcctgggt tgcatcttgc        60

a                                                                         61


<210>  17
<211>  61
<212>  DNA
<213>  Homo Sapiens

<400>  17
gggcagaatt ctggcagttc tctgtggtct ygtttttaca aatagaacca gggacagggg        60

t                                                                         61


<210>  18
<211>  61
<212>  DNA
<213>  Homo Sapiens

<400>  18
tgtccttgtc ccctcttttc cattcacttg ytctaaatca atagttctca aaccctccgt        60

g                                                                         61


<210>  19
<211>  61
<212>  DNA
<213>  Homo Sapiens

<400>  19
catcattttg ggtacctgtt gttgttcctc yggctcccga aatatctgag cgagtaggat        60

g                                                                         61


<210>  20
<211>  61
<212>  DNA
<213>  Homo Sapiens

<400>  20
gaagttaatc atgcctacct cacaggattc raaacaatac acaagaagag taatgccaca        60

a                                                                         61


<210>  21
<211>  61
<212>  DNA
<213>  Homo Sapiens

<400>  21
gggtaccctg gccttggggt ggtcagttct raaagataac ctgcacatta tgagttattt        60

c                                                                         61

<210>  22
<211>  61
<212>  DNA
<213>  Homo Sapiens

<400>  22
agttttctcc cttctctgat tgttttatcc rtttttcatt gaataggcca tgctcttgtg      60

a                                                                     61


<210>  23
<211>  61
<212>  DNA
<213>  Homo Sapiens

<400>  23
aattttgtta caaatagtac cttgcataca mattatcaca aaacgtgaaa aattaaacta      60

g                                                                     61


<210>  24
<211>  61
<212>  DNA
<213>  Homo Sapiens

<400>  24
ccttattgag catccacaag cttgtctgcg mgtgtaaaag gagggagaaa ggaccctgcc      60

a                                                                     61


<210>  25
<211>  61
<212>  DNA
<213>  Homo Sapiens

<400>  25
ctcatatgat cacattaata gatgttggag kgacatttga taatatttaa cagctaatcc      60

c                                                                     61


<210>  26
<211>  61
<212>  DNA
<213>  Homo Sapiens

<400>  26
aaactccaga ctgtatgatt ccacagtgta yttccattca tctcatgaag gacatctttg      60

t                                                                     61


<210>  27
<211>  61
<212>  DNA
<213>  Homo Sapiens

<400>  27
gttctagaaa ctatctaaga tgatcaagtc raagttaatt tcacaacttc ttagagtctg      60

g                                                                    61

<210>  28
<211>  61
<212>  DNA
<213>  Homo Sapiens

<400>  28
agagaaagga gattccgtgt gaaacattta ygctaaagta ctgcttttac ctgtaacatc      60

t                                                                    61

<210>  29
<211>  61
<212>  DNA
<213>  Homo Sapiens

<400>  29
gcatactaca tgtaaaacat tgtgctgagg racaagacaa caaggtccct tgtctattgg      60

a                                                                    61

<210>  30
<211>  61
<212>  DNA
<213>  Homo Sapiens

<400>  30
aaaggctgct attatccaaa agtgttgctt wctacaagtt tagcctgaca tctttcagtg      60

g                                                                    61

<210>  31
<211>  61
<212>  DNA
<213>  Homo Sapiens

<400>  31
gaggaaataa tttgcccagg taactaagga rtgatggttc caggaatggc tataatatac      60

t                                                                    61

<210>  32
<211>  61
<212>  DNA
<213>  Homo Sapiens

<400>  32
ccagtcaaat tatattatag gcccatccta ygcaagtata acataaccac atcttaacta      60

a                                                                    61

<210>  33
<211>  61
<212>  DNA
<213>  Homo Sapiens

<400>  33
tttggaaaag gagggtttgt gtgactgcta rggagatgtt ttatattcat tggtcatttg      60

t                                                                      61


<210>  34
<211>  61
<212>  DNA
<213>  Homo Sapiens

<400>  34
aatcgctatc taaacttgat gagtataata ytgcatgtgg agtccctgtc aatttttact      60

a                                                                      61


<210>  35
<211>  61
<212>  DNA
<213>  Homo Sapiens

<400>  35
caaaaaagtc tgatgtttca tcaaggaatt ytatgatttc aaatttgacc ttcctattcc      60

t                                                                      61


<210>  36
<211>  61
<212>  DNA
<213>  Homo Sapiens

<400>  36
tattttaaga ctggaaccta ttaaagtctt rggtacatag gccctagtgg taaacatttc      60

a                                                                      61


<210>  37
<211>  61
<212>  DNA
<213>  Homo Sapiens

<400>  37
gagagtataa atatggcaac ctgtcagtcc ragagtcttt attacaaata acagaagctt      60

a                                                                      61


<210>  38
<211>  61
<212>  DNA
<213>  Homo Sapiens

<400>  38
aaaatttgga gattattttc agggaagaaa raatgatcag gtagttatcc ttatttttaa      60

t                                                                      61


<210>  39

<211>  61
<212>  DNA
<213>  Homo Sapiens

<400>  39
aggttgcaga agaaaatctg aattaccctg sactaactga actctctgat cattcttttt      60

c                                                                      61


<210>  40
<211>  61
<212>  DNA
<213>  Homo Sapiens

<400>  40
tcctcttcca ctgtgcagat tgttagtagt ytgaatttga aggatacgat attttaagag      60

a                                                                      61


<210>  41
<211>  61
<212>  DNA
<213>  Homo Sapiens

<400>  41
atgaatatgg tagcaagtgt cttccttcct ktgaaatatg agttttcatc agctgttgga      60

t                                                                      61


<210>  42
<211>  61
<212>  DNA
<213>  Homo Sapiens

<400>  42
atgccttatt taagaagaag tttctaggaa macccaaaga caacagcggg gaataaaaac      60

a                                                                      61


<210>  43
<211>  61
<212>  DNA
<213>  Homo Sapiens

<400>  43
gcccaggagt tcgagtccag cctgggaaaa stgtgagtcc ctgtctctaa aaaataataa      60

t                                                                      61


<210>  44
<211>  61
<212>  DNA
<213>  Homo Sapiens

<400>  44
tttcacctgt atgggtccat ctcataagct sttgggaaac cgggccacat ggtgggcaat      60

t                                                                      61

<210>  45
<211>  61
<212>  DNA
<213>  Homo Sapiens

<400>  45
tgaactgtag aggagttcac gaccaaatct stgatgcttt ccccagatct ttcttcaggg      60

c                                                                      61


<210>  46
<211>  61
<212>  DNA
<213>  Homo Sapiens

<400>  46
tgaggatgca cagggccagg gcagaattga ytgttgctca gaggatgcac cggggtgtct      60

c                                                                      61


<210>  47
<211>  61
<212>  DNA
<213>  Homo Sapiens

<400>  47
cctgggtctg ggaatcaagt ccttacctgg mtagccttcc tggtgcctgc ccacctgagc      60

g                                                                      61


<210>  48
<211>  61
<212>  DNA
<213>  Homo Sapiens

<400>  48
gccaggagca ggtctggggc ccaccttctg ygttgcgtag accaggcttt ggtcggctgt      60

g                                                                      61


<210>  49
<211>  61
<212>  DNA
<213>  Homo Sapiens

<400>  49
acccaaaagt gactgggctg actgccaaga katggcttta aaccagaaga aatggagatg      60

t                                                                      61


<210>  50
<211>  61
<212>  DNA
<213>  Homo Sapiens

<400>  50

tatttccgtt cagtcatgga tcaaacagcc yatttcagca attgatatgg atatattttc 60

c 61

<210> 51
<211> 61
<212> DNA
<213> Homo Sapiens

<400> 51
gcatggcaga gatgaggttg aagaagtttg ytgggatcaa gtctaaactg gctttgtgtt 60

a 61

<210> 52
<211> 61
<212> DNA
<213> Homo Sapiens

<400> 52
gaagggaatt tcaggctcat attttggtgt matgggcaga tcttgggtga ggttttcatt 60

g 61

<210> 53
<211> 61
<212> DNA
<213> Homo Sapiens

<400> 53
aatattattt gatggcctaa aaaaacttaa mactgtctcc aagaagaaaa aaagaagtgc 60

t 61

<210> 54
<211> 61
<212> DNA
<213> Homo Sapiens

<400> 54
   gaaaagatgg aactcaactg aagtcttgct maaaacagat gcagggggcc tagacaattt 60
   c 61

<210> 55
<211> 61
<212> DNA
<213> Homo Sapiens

<400> 55
gaggaccaag gccttgaatg ccttgttcac ktctgcatcc ccaacactta ggatgacact 60

c 61

<210> 56
<211> 61
<212> DNA

<213>  Homo Sapiens

<400>  56
caccctcttc gatgtgatag tccttcctgg wgttggggcc tccgatgaac atgactttga      60

g                                                                      61


<210>  57
<211>  61
<212>  DNA
<213>  Homo Sapiens

<400>  57
tttttttttt accttgttta gtggtcaagc rttaaccacc atgttcttca gaagagaatt      60

c                                                                      61


<210>  58
<211>  61
<212>  DNA
<213>  Homo Sapiens

<400>  58
caaggccgct aagtttgctc ttacccacca ygtgcagagt gtctcctggg tgcttagtta      60

a                                                                      61


<210>  59
<211>  61
<212>  DNA
<213>  Homo Sapiens

<400>  59
ccaaactgtt tctccaacaa aatacagagg rtacaaagga ataggaatga tttttgcaac      60

a                                                                      61


<210>  60
<211>  61
<212>  DNA
<213>  Homo Sapiens

<400>  60
aggctagttt aagctgcagt aacaaatccc saaaatctcc agtctagctt aaaacaacaa      60

a                                                                      61


<210>  61
<211>  61
<212>  DNA
<213>  Homo Sapiens

<400>  61
gtgagaaagc tgttgttagc taaatggatt mtcttcaatc aaagtacacg agctatggac      60

a                                                                      61

<210>  62
<211>  61
<212>  DNA
<213>  Homo Sapiens

<400>  62
agtatatacg gctgaagcag agttagatca ygtagtacct tgtaaattgg gttaagaatt      60

t                                                                      61


<210>  63
<211>  61
<212>  DNA
<213>  Homo Sapiens

<400>  63
aaacctgtcc tatgacaccc tccatatact rcataatagc caaagacata ttaagcctat      60

c                                                                      61


<210>  64
<211>  61
<212>  DNA
<213>  Homo Sapiens

<400>  64
tggtcagccc gcagctggtc caggggtgcc rtgaccaact tctaccttgg atgctggtgt      60

c                                                                      61


<210>  65
<211>  61
<212>  DNA
<213>  Homo Sapiens

<400>  65
tttcattttt ttctctctaa gaaatggcta rgaaagatca tgttttattg atgctaagat      60

g                                                                      61


<210>  66
<211>  61
<212>  DNA
<213>  Homo Sapiens

<400>  66
catctgcttt ttggaagaac ctaggttgaa yagactgtga ttagctaaga cccagagagt      60

c                                                                      61


<210>  67
<211>  61
<212>  DNA
<213>  Homo Sapiens

<400>  67
ttgccaccca ctcccacaag taatatctcc ragttctgag aatctaagaa tgtgttctac      60

a                                                                   61

<210>  68
<211>  61
<212>  DNA
<213>  Homo Sapiens

<400>  68
ttgaataaat ctgtttgtcc tctcccttac kgatggacac gtgggttatt tcagattggg      60

g                                                                   61

<210>  69
<211>  61
<212>  DNA
<213>  Homo Sapiens

<400>  69
acaccattat ctacccagtc acttaagcca stagcccaag atccaatcta gctttctgtc      60

a                                                                   61

<210>  70
<211>  61
<212>  DNA
<213>  Homo Sapiens

<400>  70
tgtcatatct attcctggca tactcttcat rcatagtcac actgcaatgt agacacaatc      60

a                                                                   61

<210>  71
<211>  61
<212>  DNA
<213>  Homo Sapiens

<400>  71
ttcagaatag ttaagtaaga atcatgtata raaatcagca atagagcagg gcatggtggc      60

t                                                                   61

<210>  72
<211>  61
<212>  DNA
<213>  Homo Sapiens

<400>  72
gtcctgtatt tctcactagg agaatagtgg rattttgatt ttgttttata gggattgctt      60

t                                                                   61

<210>  73
<211>  61
<212>  DNA
<213>  Homo Sapiens

<400>  73
ttagtatgtg gtaacttacc attgtattac yacttagttt ttgaaggtca gaagttcagg        60

c                                                                        61


<210>  74
<211>  61
<212>  DNA
<213>  Homo Sapiens

<400>  74
cagaggtagg tgaattatct ccacagctct mtgataatga caggcattat cagtcattag        60

c                                                                        61


<210>  75
<211>  61
<212>  DNA
<213>  Homo Sapiens

<400>  75
ttcctaattc ataaatattg aatgtacagg ygggatgaca ttctcagtta ggagtcacct        60

t                                                                        61


<210>  76
<211>  61
<212>  DNA
<213>  Homo Sapiens

<400>  76
agaacattgt gattttacta tgcacatctc yaggattcac tccattagaa aaacaggctc        60

c                                                                        61


<210>  77
<211>  61
<212>  DNA
<213>  Homo Sapiens

<400>  77
aaggagccaa gaggcatgat gtaatgcatc magatcttct gtggtgacct tgtaacttct        60

g                                                                        61


<210>  78
<211>  61
<212>  DNA
<213>  Homo Sapiens

<400>  78
gatcttacct caagctgatg cacaattatg kattcatccc agtgctttca gggtagccta        60

g                                                                        61


<210>  79
<211>  61

<212>  DNA
<213>  Homo Sapiens

<400>  79
cagaggaaca aagagagaat tacctttttc ygcagcaact tatattcatg ttttttcatc      60

a                                                                       61


<210>  80
<211>  61
<212>  DNA
<213>  Homo Sapiens

<400>  80
ctggaaagtt ctgaggatcc agatgcacca ytacaagggc attttatagt cttcagtgca      60

g                                                                       61


<210>  81
<211>  61
<212>  DNA
<213>  Homo Sapiens

<400>  81
cgaggatgac actgatggtg acgatactga ygatagtaat aacacttact gaatactcac      60

t                                                                       61


<210>  82
<211>  61
<212>  DNA
<213>  Homo Sapiens

<400>  82
gcctatgcca gcatagtcct cttgagaatg yctcaccttc gcaggtgcaa tcatttataa      60

g                                                                       61


<210>  83
<211>  61
<212>  DNA
<213>  Homo Sapiens

<400>  83
atttagattt ccagttacct ggcaagagaa mgacttaaaa ggaataagtc taggtaaatc      60

t                                                                       61


<210>  84
<211>  61
<212>  DNA
<213>  Homo Sapiens

<400>  84
taaagatgcc cagtggttga acttcctgat ytccaggcct gtactcttct cactatgtca      60

t                                                                       61

<210>  85
<211>  61
<212>  DNA
<213>  Homo Sapiens

<400>  85
catggatagt taacattcag acaggcttat yttcaaatct ggattctatt tactgtgtga      60

c                                                                      61


<210>  86
<211>  61
<212>  DNA
<213>  Homo Sapiens

<400>  86
tgtaaacaca ttctttattg atatgcacat mgtgattctg atttggaata ttaagggtcc      60

c                                                                      61


<210>  87
<211>  61
<212>  DNA
<213>  Homo Sapiens

<400>  87
attttacaga agcttaatat gagtctaaat rgaggtatac aacctaagga gttccactcc      60

c                                                                      61


<210>  88
<211>  61
<212>  DNA
<213>  Homo Sapiens

<400>  88
ttataagaag gtaaatcaac ttctccatgc wgtgcaacaa gccattgtta aatctggatt      60

g                                                                      61


<210>  89
<211>  61
<212>  DNA
<213>  Homo Sapiens

<400>  89
tgtttaaaaa tttaaagcat acttctaaga yatagtggtg attctctata ctagcatgaa      60

c                                                                      61


<210>  90
<211>  61
<212>  DNA
<213>  Homo Sapiens

<400>  90
tcagaggcct tggcctagag ttgagccata maccatattt tagaagtaaa tacattccta      60

a                                                                   61

<210>  91
<211>  61
<212>  DNA
<213>  Homo Sapiens

<400>  91
ttcagttaaa aagtaaagac aaaaagaaaa rtaataaatt tccactttca atatagtaaa      60

t                                                                   61

<210>  92
<211>  61
<212>  DNA
<213>  Homo Sapiens

<400>  92
cattcccgtc tatttggttg tagataaaag rgataagggg cctctttctc aataactact      60

t                                                                   61

<210>  93
<211>  61
<212>  DNA
<213>  Homo Sapiens

<400>  93
tttttcaacg tggtaccaca aatgtggcca saaggatttt cataatggga tattgtacat      60

a                                                                   61

<210>  94
<211>  61
<212>  DNA
<213>  Homo Sapiens

<400>  94
aaaatctgat ataatttttc agagtctgtg rcactaagga gtccacaggg ctgcctaggt      60

g                                                                   61

<210>  95
<211>  61
<212>  DNA
<213>  Homo Sapiens

<400>  95
gtggtggcct cataccatag tagctgctta sttaattatg actaatataa ggaggtatag      60

g                                                                   61

<210>  96
<211>  61
<212>  DNA
<213>  Homo Sapiens

<400>  96
caactctcct acttgatcat gtacaggact yaaaactgta atcattaaaa cccagatgat      60

c                                                                      61


<210>  97
<211>  61
<212>  DNA
<213>  Homo Sapiens

<400>  97
taaaattata tgtttaggtg cattaactat yaactcaaag aggattgtga aaggcctcct      60

t                                                                      61


<210>  98
<211>  61
<212>  DNA
<213>  Homo Sapiens

<400>  98
aaatatagca gctacaatct aatttcattg raagtctcct ccaaatcaca gaagtgccac      60

t                                                                      61


<210>  99
<211>  61
<212>  DNA
<213>  Homo Sapiens

<400>  99
cttttctctc tttatttttt ttaaacagaa ygctatgaac ctacctcctg acaaagccag      60

g                                                                      61


<210>  100
<211>  61
<212>  DNA
<213>  Homo Sapiens

<400>  100
gctagtgaat taagcaaata tgaaatgaca rtgaagtcgt attaatggtt tgtggtatgg      60

a                                                                      61


<210>  101
<211>  61
<212>  DNA
<213>  Homo Sapiens

<400>  101
aatccaatca acccttggta ttgtcagtcc kttaaaattc tagccatttt tatgggcata      60

t                                                                      61


<210>  102

<211>  61
<212>  DNA
<213>  Homo Sapiens

<400>  102
cccgagctgg attaaaatcc aggtcacaga sggactgttt tctctcctct ttcctgaaag      60

g                                                                     61


<210>  103
<211>  61
<212>  DNA
<213>  Homo Sapiens

<400>  103
ataacaggaa tctgattata ctgcaggtca rattatatcc taaagatata tttgtataga      60

a                                                                     61


<210>  104
<211>  61
<212>  DNA
<213>  Homo Sapiens

<400>  104
gcaggagagc aacaatccta aaagagatca ytatgactgc agacagtatt gtgacttccg      60

g                                                                     61


<210>  105
<211>  61
<212>  DNA
<213>  Homo Sapiens

<400>  105
atgtgtggcc caagataatt attactcttc ratgtggccc aaggacgcca aaagattgca      60

c                                                                     61


<210>  106
<211>  61
<212>  DNA
<213>  Homo Sapiens

<400>  106
aaaagagtcc taaggataga tacggtgtta ytagatgttg gctgaagctg gggtaaaaca      60

g                                                                     61


<210>  107
<211>  61
<212>  DNA
<213>  Homo Sapiens

<400>  107
caaacgatca aaggttacat agattagacc maagtctgct ttattccaaa gcttattttc      60

t                                                                     61

<210>  108
<211>  61
<212>  DNA
<213>  Homo Sapiens

<400>  108
ccatggaggc tcctacatgc cacagtaagg kctctgaatt gtatttgata agcagtaggg      60

a                                                                      61


<210>  109
<211>  61
<212>  DNA
<213>  Homo Sapiens

<400>  109
ctcattgtat gcttgctctg tgttcaacat wgtgcaggat gcgagatgaa taagctatca      60

t                                                                      61


<210>  110
<211>  61
<212>  DNA
<213>  Homo Sapiens

<400>  110
aggtcacaga aattcttatt ccttgttttc yctgagttat gacatagtga aatgttcctg      60

t                                                                      61


<210>  111
<211>  61
<212>  DNA
<213>  Homo Sapiens

<400>  111
aaattgtgca attcagggaa tatggcaatt kaaaaccaga tgcttagacc actaagccat      60

c                                                                      61


<210>  112
<211>  61
<212>  DNA
<213>  Homo Sapiens

<400>  112
cacctcccat gtgacctgat gctggagaaa stgtggttta gcgggaggct gatggggaag      60

t                                                                      61


<210>  113
<211>  61
<212>  DNA
<213>  Homo Sapiens

<400>  113

tgcttaaatg tttaaataag cttcttgaaa wctcatggaa cattcaatca caactccaac 60

a 61

<210> 114
<211> 61
<212> DNA
<213> Homo Sapiens

<400> 114
tcatgtatgg agatgggaag tagaatcaag ygggaaatga gaaaacagtg cgttcttcat 60

g 61

<210> 115
<211> 61
<212> DNA
<213> Homo Sapiens

<400> 115
ttaaggtgat ggccttctaa agcagctttc rgaagtttta tttgtcaata tcagacataa 60

a 61

<210> 116
<211> 61
<212> DNA
<213> Homo Sapiens

<400> 116
cagatggatg gatggatgaa tttgtgttct yatgattgag acattacgat aaggggaaag 60

a 61

<210> 117
<211> 61
<212> DNA
<213> Homo Sapiens

<400> 117
ataccttatt tccactcaca ctggttagaa stcagtccca ttgcgttttt tggccatgca 60

c 61

<210> 118
<211> 61
<212> DNA
<213> Homo Sapiens

<400> 118
ctaggtgcag agcaactctc gtgatatggc yagttagact tttcatcttg gtcttgccct 60

t 61

<210> 119
<211> 61
<212> DNA

<213>  Homo Sapiens

<400>  119
ttagtatctg aaagttctta aattgtaggc rtaaatgttt cattttaatt gtgcatttcc      60

a                                                                     61


<210>  120
<211>  61
<212>  DNA
<213>  Homo Sapiens

<400>  120
ctaatacttc caattccatt ccaacaccat rggatttatt ctagccttca ccttttccat      60

t                                                                     61


<210>  121
<211>  61
<212>  DNA
<213>  Homo Sapiens

<400>  121
accctctcaa tggcaaatct tattctaggc rgcagaatta agcagagtac gggtgaaatc      60

g                                                                     61


<210>  122
<211>  61
<212>  DNA
<213>  Homo Sapiens

<400>  122
caaaggtatc tcatagaact aacttcgttc rtcatggttc tggaggtgtt agccaatgca      60

g                                                                     61


<210>  123
<211>  61
<212>  DNA
<213>  Homo Sapiens

<400>  123
ggtttctgct ggaatacttt tggccttgag ygtgttacag atcaaaggga agtatcagag      60

g                                                                     61


<210>  124
<211>  61
<212>  DNA
<213>  Homo Sapiens

<400>  124
agaggggtca ctgctttagt ggaactctca yggaagttca tgacctagga gggtttaaaa      60

a                                                                     61

<210>  125
<211>  61
<212>  DNA
<213>  Homo Sapiens

<400>  125
gagggcctac tgttcaactc aaattgaatt yctgttagta ttctctgctt ctgaagcttc      60

t                                                                      61


<210>  126
<211>  61
<212>  DNA
<213>  Homo Sapiens

<400>  126
gcaagagaca gagggggaca ttttctgaca sagcgttcaa ggccagcagc agctcagggg      60

c                                                                      61


<210>  127
<211>  61
<212>  DNA
<213>  Homo Sapiens

<400>  127
ctgagcttgt gctataacca aatattctgg rtaatggttg gtatatacgt aaccgtagat      60

a                                                                      61


<210>  128
<211>  61
<212>  DNA
<213>  Homo Sapiens

<400>  128
attgatgaaa gtccgtctgg ctgtttcggc yatttgacag caaagatgaa aaaatacatg      60

c                                                                      61


<210>  129
<211>  61
<212>  DNA
<213>  Homo Sapiens

<400>  129
ttagaaataa ttaatgggac tgaggcaagt raccaagaat gcactatgta catagagttt      60

c                                                                      61


<210>  130
<211>  61
<212>  DNA
<213>  Homo Sapiens

<400>  130
ccacactgcc atattgctat acctacaaca ygggatataa aaagcatcct ttaacttatg      60

t                                                                    61

<210>  131
<211>  61
<212>  DNA
<213>  Homo Sapiens

<400>  131
cttgtatttt ttatgcccat catctccagt yggttccctg tgaaagattg cttattcttg      60

c                                                                    61

<210>  132
<211>  61
<212>  DNA
<213>  Homo Sapiens

<400>  132
gaatgatcag aggtcagatc acggaaagga yattagattt tattgtaact acagagaaac      60

t                                                                    61

<210>  133
<211>  61
<212>  DNA
<213>  Homo Sapiens

<400>  133
acaaacatac acagcagctt gaagttcaaa macagtcaga agtagctacc aagaaatagg      60

a                                                                    61

<210>  134
<211>  61
<212>  DNA
<213>  Homo Sapiens

<400>  134
ggtcctacta tcaggaggat gaggtaacaa rcttccccat accacctcag aaaggataaa      60

c                                                                    61

<210>  135
<211>  61
<212>  DNA
<213>  Homo Sapiens

<400>  135
ataaccaagg tgtgaagtgg gaagggtaga sgaagataga aactaccaaa agttactaat      60

g                                                                    61

<210>  136
<211>  61
<212>  DNA
<213>  Homo Sapiens

<400>  136
agggagagaa aattgttatg gcagcaagaa rttctacctt gaaaataaat ctgctttgct      60

a                                                                      61


<210>  137
<211>  61
<212>  DNA
<213>  Homo Sapiens

<400>  137
acatggacta catcttctaa atcattatac mcagaaacgc tctcagtagc tgacaccaat      60

c                                                                      61


<210>  138
<211>  61
<212>  DNA
<213>  Homo Sapiens

<400>  138
cagccccagc acagaaacag gaaatgcatt ytgaggagac aacagctctg ggtacaacaa      60

a                                                                      61


<210>  139
<211>  61
<212>  DNA
<213>  Homo Sapiens

<400>  139
gctctggcac atgttttagg tgtagagaca rcattgggca gtgggaaaag gataagtcaa      60

t                                                                      61


<210>  140
<211>  61
<212>  DNA
<213>  Homo Sapiens

<400>  140
ctgtcctcag gcttggtgga gccagtgaac rtggtggaca atggagatgg cacacacaca      60

g                                                                      61


<210>  141
<211>  61
<212>  DNA
<213>  Homo Sapiens

<400>  141
tgagcattgc cacacaggta ctgttaaaag sgcagaatga aggtacatat gtgatttttg      60

c                                                                      61


<210>  142
<211>  61

<212>  DNA
<213>  Homo Sapiens

<400>  142
gcggggggga cctcatgatt ccatctttat rgcccaactg ttgattaact tccaaatgaa      60

a                                                                     61


<210>  143
<211>  61
<212>  DNA
<213>  Homo Sapiens

<400>  143
attcagcagc atattcaaag agacagagaa kaactgacat gatgctgatg aagcttaaac      60

t                                                                     61


<210>  144
<211>  61
<212>  DNA
<213>  Homo Sapiens

<400>  144
agcagtggcc aagcagacac aagtgatcat yatcatacga acttaatttc tgaccaaatt      60

c                                                                     61


<210>  145
<211>  61
<212>  DNA
<213>  Homo Sapiens

<400>  145
gactattaac tagtaattga tgacaaccaa rgaaggtagt tttcctaata cctacaaaac      60

t                                                                     61


<210>  146
<211>  61
<212>  DNA
<213>  Homo Sapiens

<400>  146
ttggggagaa actgatgttt tgcagatgca wtattaaaac aatccattta taaggaaggc      60

a                                                                     61


<210>  147
<211>  61
<212>  DNA
<213>  Homo Sapiens

<400>  147
cccagaagca gatctggaga caggtattca mgagcaagta ttttatttag gaggttatcg      60

c                                                                     61

<210>  148
<211>  61
<212>  DNA
<213>  Homo Sapiens

<400>  148
tgaactccaa agaaggtaga aatcaactgc rttattgagt aatgtgcata ctttgaatgt        60

t                                                                         61


<210>  149
<211>  61
<212>  DNA
<213>  Homo Sapiens

<400>  149
cagaggcagg tttcatcttt ctgcctcgtc wttcaggaat ggccttaatc ttcctacagt        60

t                                                                         61


<210>  150
<211>  61
<212>  DNA
<213>  Homo Sapiens

<400>  150
gccaactctc caggtggccg agacagggca stccctcagg taacaggggt gaacactggg        60

g                                                                         61


<210>  151
<211>  61
<212>  DNA
<213>  Homo Sapiens

<400>  151
cacagtttgt gctaaacatt atgaggagta saaaaacaaa tcaaaagcct aagaattcgt        60

t                                                                         61


<210>  152
<211>  61
<212>  DNA
<213>  Homo Sapiens

<400>  152
ttatgaggag tacaaaaaca aatcaaaagc staagaattc gttgctagcc atgaaagtcc        60

t                                                                         61


<210>  153
<211>  61
<212>  DNA
<213>  Homo Sapiens

<400>  153
caaatcaaaa gcctaagaat tcgttgctag mcatgaaagt cctctgttgg aacatcctct        60

t 61

<210> 154
<211> 61
<212> DNA
<213> Homo Sapiens

<400> 154
tcctttgctg tgggtccctg tcctgggcac ygtagaattc ttagcagctt tcctggtccc 60

t 61

<210> 155
<211> 61
<212> DNA
<213> Homo Sapiens

<400> 155
gttttacaag tgtttggttt tttgatccac raaatatctc attcaaaagg tttgctttaa 60

t 61

<210> 156
<211> 61
<212> DNA
<213> Homo Sapiens

<400> 156
ttgttagggc agtttcaaaa atgtgctttg kacataacag acctttccca agatggtcat 60

a 61

<210> 157
<211> 61
<212> DNA
<213> Homo Sapiens

<400> 157
aactgaagct ggaccctggg cttctgagtc yaaaggtggt acttcctgtg ctagaccgtg 60

c 61

<210> 158
<211> 61
<212> DNA
<213> Homo Sapiens

<400> 158
gagctttgat ctggactatg taggaaggac wtttcagttg tgtgccttac tttgtacagt 60

g 61

<210> 159
<211> 61
<212> DNA
<213> Homo Sapiens

<400>  159
tttacattat gcaatgaatg ctaattgaca rctgattcat aattagttaa ttgtaaacac      60

t                                                                      61


<210>  160
<211>  61
<212>  DNA
<213>  Homo Sapiens

<400>  160
tgagcactaa atgttccttg agtatgctca ratgactgta atagcagaca ggacatatcc      60

a                                                                      61


<210>  161
<211>  61
<212>  DNA
<213>  Homo Sapiens

<400>  161
aattcttata ctactatatt ttattccctc ractgccagg ttcattaaaa accctttaca      60

t                                                                      61


<210>  162
<211>  61
<212>  DNA
<213>  Homo Sapiens

<400>  162
ttggaattaa tcactctatt ctctgaagcc rcataccact ttgccaccat cagtagtagg      60

a                                                                      61


<210>  163
<211>  61
<212>  DNA
<213>  Homo Sapiens

<400>  163
atctctaaag gttcactgga aatttgcatt rctcagtgaa gaatcaaatc ctagccagat      60

g                                                                      61


<210>  164
<211>  61
<212>  DNA
<213>  Homo Sapiens

<400>  164
tcatgaattc ttgaaagaga tcttaaacta ygatagcaca gaaatcactg aattgcttaa      60

c                                                                      61


<210>  165

<211>  61
<212>  DNA
<213>  Homo Sapiens

<400>  165
ctctctactt ttttaatgga atttgtgaag ycccttaata acaagttgat ttataaagct      60

t                                                                     61


<210>  166
<211>  61
<212>  DNA
<213>  Homo Sapiens

<400>  166
gatgcggcac agacagagac aacaagctat rttttgctaa tgggtcctat caatatgggg      60

c                                                                     61


<210>  167
<211>  61
<212>  DNA
<213>  Homo Sapiens

<400>  167
tgacaagtta ctctaacata atagcgaact rcaaactgca cagggctttg gagcccaact      60

g                                                                     61


<210>  168
<211>  61
<212>  DNA
<213>  Homo Sapiens

<400>  168
tagaaatgaa tccaattcta acacagagag rtataaactt tgggactgtg tattgtgcta      60

t                                                                     61


<210>  169
<211>  61
<212>  DNA
<213>  Homo Sapiens

<400>  169
atcccctggt gagaggtttt ccaaaatgca rtaatacaga aagaatttaa gggaagttct      60

g                                                                     61


<210>  170
<211>  61
<212>  DNA
<213>  Homo Sapiens

<400>  170
aagcactttt catattgtca atcttcccag saaatctgca gagtagacct tagtttcttt      60

t                                                                     61

<210>  171
<211>  61
<212>  DNA
<213>  Homo Sapiens

<400>  171
ttcaagtcct atcccagcca aggtcctagc raactcagct cgtggacctg tctctgacgg      60

t                                                                      61


<210>  172
<211>  61
<212>  DNA
<213>  Homo Sapiens

<400>  172
attaccacta ctcagtttaa agcaacaaat sctcattgaa tagctactat tttttatata      60

c                                                                      61


<210>  173
<211>  61
<212>  DNA
<213>  Homo Sapiens

<400>  173
ttccaaaaaa cagcaataga agactccaca mgaagattgt gtccattttg acttctctgt      60

a                                                                      61


<210>  174
<211>  61
<212>  DNA
<213>  Homo Sapiens

<400>  174
ctaattaatt aaaagaggaa agttttaaaa ygaacacagc atgaacacaa acttatataa      60

g                                                                      61


<210>  175
<211>  61
<212>  DNA
<213>  Homo Sapiens

<400>  175
acagaaggct gctttatgtg caaaaggtag kttaagtctc aaaatataaa atgtgtgtat      60

a                                                                      61


<210>  176
<211>  61
<212>  DNA
<213>  Homo Sapiens

<400>  176

ggctgttaat ggcctgtcat gtgctttttt yggcagagca gtgaggaact ctttgagagc 60

c 61

<210> 177
<211> 61
<212> DNA
<213> Homo Sapiens

<400> 177
aggaaggtac atctcctggg aagtagagct wtcacacaat aatcaattca tgcaaaaagc 60

a 61

<210> 178
<211> 61
<212> DNA
<213> Homo Sapiens

<400> 178
accctatttc actaagtcct catcttaatt yaactagtta cacccacagc aaccttattt 60

c 61

<210> 179
<211> 61
<212> DNA
<213> Homo Sapiens

<400> 179
aaggacagca agatggggta acctaaaata stgttgatag atcacagagg aaataagctg 60

t 61

<210> 180
<211> 61
<212> DNA
<213> Homo Sapiens

<400> 180
actatgcaca gacacatttt tccacattat rttgctgctt cagtaaagat gttgtacttg 60

a 61

<210> 181
<211> 61
<212> DNA
<213> Homo Sapiens

<400> 181
caactgactg cattcccatg agtgactcca ytgagtaagg accacctagc taagctctgt 60

c 61

<210> 182
<211> 61
<212> DNA

<213>  Homo Sapiens

<400>  182
cacaatgtat tatgcatgtg ccgagttgct ragtatggct gatgctgcag cccttgttag      60

t                                                                     61


<210>  183
<211>  61
<212>  DNA
<213>  Homo Sapiens

<400>  183
ggagtaatac agaaactttt agccaaggca yattctctgg gctctcaatc ctttgcagct      60

g                                                                     61


<210>  184
<211>  61
<212>  DNA
<213>  Homo Sapiens

<400>  184
agtgattgat tgtacttgga aagtgaattg ygatgcctct atttattcca cgagtatgag      60

g                                                                     61


<210>  185
<211>  61
<212>  DNA
<213>  Homo Sapiens

<400>  185
ctgtcaatac atatggagaa aatagtttca yggaatcaaa gttatctggt tcacctcctt      60

t                                                                     61


<210>  186
<211>  61
<212>  DNA
<213>  Homo Sapiens

<400>  186
acatgtaaaa gaatataatt tgatgaattt yggcatatgc attcccccat gaatccacac      60

a                                                                     61


<210>  187
<211>  61
<212>  DNA
<213>  Homo Sapiens

<400>  187
ctttgtatat tcctctatag tcagaaacaa yggaatgtcc tagagccctt tagtgaagaa      60

t                                                                     61

<210>  188
<211>  61
<212>  DNA
<213>  Homo Sapiens

<400>  188
gtggccatgg cagacactgt caggctccta ytgaatttca ttctcctctg atttccttac        60

t                                                                         61


<210>  189
<211>  61
<212>  DNA
<213>  Homo Sapiens

<400>  189
acagagaaat cgaattacta agttagggga yagataaggc aaacattcat tagttcacaa        60

a                                                                         61


<210>  190
<211>  61
<212>  DNA
<213>  Homo Sapiens

<400>  190
attttgactc tgatttcttc ccataccttt yggagtccac ttctccgcca ctaacttttt        60

t                                                                         61


<210>  191
<211>  61
<212>  DNA
<213>  Homo Sapiens

<400>  191
agtgtgcaaa tattactcaa atggcaaaca yatgattagg ttaaggatgt tgagaggagg        60

a                                                                         61


<210>  192
<211>  61
<212>  DNA
<213>  Homo Sapiens

<400>  192
agactgcact caacacacag gttaggtact yggattatgt gaggtatcct cctctcatgc        60

t                                                                         61


<210>  193
<211>  61
<212>  DNA
<213>  Homo Sapiens

<400>  193
aagaggtaga ggaggtcctg gctgagagcc rcgtgcagag gagcatccgc ctggtgtccg        60

t 61

<210> 194
<211> 61
<212> DNA
<213> Homo Sapiens

<400> 194
ttattggttc gaactcaccc agctatattt ktactgtgct tgctatatac atgatttaaa 60

a 61

<210> 195
<211> 61
<212> DNA
<213> Homo Sapiens

<400> 195
agaatagtgt ggggaacggt tctgctacat kcctaaaagg agcaaaggga aattaatatt 60

g 61

<210> 196
<211> 61
<212> DNA
<213> Homo Sapiens

<400> 196
acgaagtcct aggtctttgc agatgatcaa rttttggaga agaaacaaat ttactctgca 60

t 61

<210> 197
<211> 61
<212> DNA
<213> Homo Sapiens

<400> 197
caatctgtcc agggtctgaa tagaatgaaa mggtgaggaa aagaagattc actccctctc 60

t 61

<210> 198
<211> 61
<212> DNA
<213> Homo Sapiens

<400> 198
gttttcttct gccctcagac taagccatac ractggctct cctgggtctc cagcttgtca 60

a 61

<210> 199
<211> 61
<212> DNA
<213> Homo Sapiens

<400>  199
ctcctgggtc tccagcttgt caactacaga ytatgggttt tagctttcat aatcatgtag      60

c                                                                      61


<210>  200
<211>  61
<212>  DNA
<213>  Homo Sapiens

<400>  200
atgttgtcca tatgctggtg actcctaaat mtaaatctcc agcttgggct tttcctctga      60

a                                                                      61


<210>  201
<211>  61
<212>  DNA
<213>  Homo Sapiens

<400>  201
aatctccagc ttgggctttt cctctgaatt yctggcctct atccaattac ttactgatgt      60

t                                                                      61


<210>  202
<211>  61
<212>  DNA
<213>  Homo Sapiens

<400>  202
aaaaggagtt taagaataga gtcctcttta wttaggatac attaataaac aaaacataca      60

a                                                                      61


<210>  203
<211>  61
<212>  DNA
<213>  Homo Sapiens

<400>  203
ggatacaggc aaaggaaaac tgtactaggc raggcccata gaataaattg acaggattat      60

t                                                                      61


<210>  204
<211>  61
<212>  DNA
<213>  Homo Sapiens

<400>  204
ttgcttctct gaaattcaat gcataactcc ygataatcca tccagttcat tcaagaagta      60

t                                                                      61


<210>  205
<211>  61

<212>  DNA
<213>  Homo Sapiens

<400>  205
gagcctgaag agagttttac tgcactgaat rggggacaga aagcagtact ggcaacattt      60

t                                                                     61


<210>  206
<211>  61
<212>  DNA
<213>  Homo Sapiens

<400>  206
cttttctagt ttccaaagtc cataatttcc rgacagcagg aatagttatg caaagaacag      60

a                                                                     61


<210>  207
<211>  61
<212>  DNA
<213>  Homo Sapiens

<400>  207
agactgtaaa agatattgga aatggatcaa rgcactctga acatattcaa ataattgctg      60

t                                                                     61


<210>  208
<211>  61
<212>  DNA
<213>  Homo Sapiens

<400>  208
ttcattctca aagagctaaa tttgttttgg kgtgagaggc tgataattag gttgaccctt      60

g                                                                     61


<210>  209
<211>  61
<212>  DNA
<213>  Homo Sapiens

<400>  209
cgccatcccg caaccccctg ttatgttcat rtacttccac ggttctcgcg tcatgcctgt      60

g                                                                     61


<210>  210
<211>  61
<212>  DNA
<213>  Homo Sapiens

<400>  210
tttctattgt ttgtggcttg ttaattttca ygctactcct aaatggctca tatcttactt      60

t                                                                     61

<210>  211
<211>  61
<212>  DNA
<213>  Homo Sapiens

<400>  211
gaaagcacgt ggactttgga atggtgcaca wctgagttcc aattttctaa ttttgctgaa        60

c                                                                         61


<210>  212
<211>  61
<212>  DNA
<213>  Homo Sapiens

<400>  212
tatacataaa cttgggaatt actttactat rtatcatcac tgtgacactt ttgaatttgt        60

t                                                                         61


<210>  213
<211>  61
<212>  DNA
<213>  Homo Sapiens

<400>  213
cacaaggaca gatgatcgta actttattta kgttcaagat agatttctgt agcactacat        60

a                                                                         61


<210>  214
<211>  61
<212>  DNA
<213>  Homo Sapiens

<400>  214
tcacaacagt gcctggagaa cttcttttgc wccttaaaat acttcatctg tctcataagc        60

c                                                                         61


<210>  215
<211>  61
<212>  DNA
<213>  Homo Sapiens

<400>  215
ggaaaaaaat agctaaagag gacatggcaa rgatgcctga tgacattaga ctttccactg        60

t                                                                         61


<210>  216
<211>  61
<212>  DNA
<213>  Homo Sapiens

<400>  216
agggcgtttc taatgctctc tcactcacaa mgttactagc tcaagtgcct caaagtgtag        60

c                                                                   61

<210>  217
<211>  61
<212>  DNA
<213>  Homo Sapiens

<400>  217
ttgggcccac tgttacagaa gttatcgaga sagatggcaa tcaggctgcc tgcaagtctg      60

a                                                                   61

<210>  218
<211>  61
<212>  DNA
<213>  Homo Sapiens

<400>  218
tcctctgaat agattccaat gtggcttcaa yactgggctt tattgcttta tccaccagct      60

c                                                                   61

<210>  219
<211>  61
<212>  DNA
<213>  Homo Sapiens

<400>  219
aaaaaaaaat agaactgaaa ctaacaagta sagactgttc aacatgtgtg atggtaaaat      60

g                                                                   61

<210>  220
<211>  61
<212>  DNA
<213>  Homo Sapiens

<400>  220
gacatctatt attacctcta tacttgtgct rctcaggatg cgtgtcttac ttgcaaagag      60

c                                                                   61

<210>  221
<211>  61
<212>  DNA
<213>  Homo Sapiens

<400>  221
tagatggcac aagttctgga cctagaaaat rcaaaaggca atggttaaat cagccctatt      60

t                                                                   61

<210>  222
<211>  61
<212>  DNA
<213>  Homo Sapiens

<400>  222
cagatagatc ctcaccatca tctgaacact ygaactggac ttccttttct gaattgacca      60

g                                                                      61


<210>  223
<211>  61
<212>  DNA
<213>  Homo Sapiens

<400>  223
cggagaaaaa ccaagggttt gagtaaatca rcataatttg gacagatctc tagaaagaaa      60

a                                                                      61


<210>  224
<211>  61
<212>  DNA
<213>  Homo Sapiens

<400>  224
taattgaaat gaaatgaggc tgagggaaaa kagcaggtag gagaccataa tgactgtatg      60

a                                                                      61


<210>  225
<211>  61
<212>  DNA
<213>  Homo Sapiens

<400>  225
gaattatgag catgtggttt aaaatcagaa rggctacaat aaaaaagaat aggtgcctgc      60

c                                                                      61


<210>  226
<211>  61
<212>  DNA
<213>  Homo Sapiens

<400>  226
ctgtcagaca ttaactcaat tcagagcaaa ragttgcaga gaatgtaggg ggaaggtgca      60

g                                                                      61


<210>  227
<211>  61
<212>  DNA
<213>  Homo Sapiens

<400>  227
agagttggtc aaacccaatg aaaggagggc ygaaacaaca aaaggcaaca ataaaaacag      60

g                                                                      61


<210>  228

<211>  50
<212>  DNA
<213>  Homo Sapiens

<400>  228
attaggttaa atatgtattw ggcaaaaaaa ccacacttac ttttgcacca          50


<210>  229
<211>  61
<212>  DNA
<213>  Homo Sapiens

<400>  229
ggcatcttta gggtgagggg cattattctg yctaacatgc tacttttgct cttgagatgg          60

c                                                                           61


<210>  230
<211>  61
<212>  DNA
<213>  Homo Sapiens

<400>  230
attgaaaaag tagccaaatg ctttaagaaa sggaatagat agagctatag ttccaaagtt          60

t                                                                           61


<210>  231
<211>  61
<212>  DNA
<213>  Homo Sapiens

<400>  231
atttcattct caaaaaggaa tagctatttt satggacaac agatcagtgg tggccagggg          60

t                                                                           61


<210>  232
<211>  61
<212>  DNA
<213>  Homo Sapiens

<400>  232
ctgttgataa gtattccatg atgaaagctt yttgcattca aattaattta agaaaagctg          60

c                                                                           61


<210>  233
<211>  61
<212>  DNA
<213>  Homo Sapiens

<400>  233
cctgtgggca tgtgagtttg taacactacc rttatgatac tgatttgtga gatagtaagc          60

t                                                                           61

<210> 234
<211> 61
<212> DNA
<213> Homo Sapiens

<400> 234
aaggaagtag aaatttaggc agttaggttt kacccacaac ctcatagcct aggtggtaag 60

c 61


<210> 235
<211> 61
<212> DNA
<213> Homo Sapiens

<400> 235
tgggaaaatt atttaactgc ttaacttctt rccagacttt cagatcctgc tgcttctgta 60

t 61


<210> 236
<211> 61
<212> DNA
<213> Homo Sapiens

<400> 236
aagattagtc tcctttatac attttccaca rttgtttcca taaagaaatg ttcttagttt 60

t 61


<210> 237
<211> 61
<212> DNA
<213> Homo Sapiens

<400> 237
acatgtcatc taaaggatag aaactttcac rggatgttct caaaaagaca aacttcatgg 60

a 61


<210> 238
<211> 61
<212> DNA
<213> Homo Sapiens

<400> 238
aaaggataga aactttcaca ggatgttctc raaaagacaa acttcatgga aactttttct 60

t 61


<210> 239
<211> 61
<212> DNA
<213> Homo Sapiens

<400> 239
aagtgctaga agaaattata aagaaaacgc rtagcagatt gggctacata aaaacttccc 60

t 61

<210> 240
<211> 61
<212> DNA
<213> Homo Sapiens

<400> 240
tgtctttatt agcagtgtga gaacagacta rtgtggtaga atggtagctg gtaggggggt 60

g 61

<210> 241
<211> 61
<212> DNA
<213> Homo Sapiens

<400> 241
attgggttgg ccaggaccag cagtacaatt sagatagggc ttgctgagtt ctagtgttac 60

a 61

<210> 242
<211> 61
<212> DNA
<213> Homo Sapiens

<400> 242
ttaatagtgg ttgcgataaa tatctggttc rgcaaaatat gagacaaagc aaactatgct 60

t 61

<210> 243
<211> 61
<212> DNA
<213> Homo Sapiens

<400> 243
gtgggcatcc ttgtcttttt cctgaactta saggagaagc tcaatctctc attgttgaat 60

t 61

<210> 244
<211> 61
<212> DNA
<213> Homo Sapiens

<400> 244
aaaaatagta ttttgaggct ttaaagtagt rtttggcaca taggaagtcc ccatcaattg 60

t 61

<210> 245
<211> 61
<212> DNA
<213> Homo Sapiens

<400>  245
tgagatgatg cttcttaatg taaaattgac yggcatgact accaatgaat atttgatgtg      60

a                                                                     61


<210>  246
<211>  61
<212>  DNA
<213>  Homo Sapiens

<400>  246
agagaagggt gtgtaaatga aaccttaata yttggacaca tttattaatt acccattttt      60

t                                                                     61


<210>  247
<211>  61
<212>  DNA
<213>  Homo Sapiens

<400>  247
tttcaatata atgatggcat gacttttagc ytgtacttga acttgtttgg tccagtcttg      60

t                                                                     61


<210>  248
<211>  61
<212>  DNA
<213>  Homo Sapiens

<400>  248
taacatatct tctttacttc caagttagtg yttttcactc tacatcatgc ccaacacttt      60

c                                                                     61


<210>  249
<211>  61
<212>  DNA
<213>  Homo Sapiens

<400>  249
attccctatt agtcccttct tttcatttct stgtctatcc agcttggagt ccatggttca      60

t                                                                     61


<210>  250
<211>  61
<212>  DNA
<213>  Homo Sapiens

<400>  250
ttaacgtaca gattgactgc atgatgcact yagatttcaa aatgctaaat cttggtggga      60

a                                                                     61


<210>  251
<211>  61

<212>  DNA
<213>  Homo Sapiens

<400>  251
aggtagattg tggtaagcta aagatgtact kataaaccct tgaaacaccc actaacaaag      60

a                                                                      61


<210>  252
<211>  61
<212>  DNA
<213>  Homo Sapiens

<400>  252
cacatgtagc tgctacgaga cagacgctct ygttccggat cagagcagca gacagaaccg      60

g                                                                      61


<210>  253
<211>  61
<212>  DNA
<213>  Homo Sapiens

<400>  253
gctatcacca aaatcgagag gaagcgtttc yatcactcca cgagcttctt tgtggcccct      60

c                                                                      61


<210>  254
<211>  61
<212>  DNA
<213>  Homo Sapiens

<400>  254
agcactctga agcctgtgcc ggctcaaggc kgtttatgga tttagggtgc cctgccataa      60

a                                                                      61


<210>  255
<211>  61
<212>  DNA
<213>  Homo Sapiens

<400>  255
gggccacttt aaagagcatc ttgggttcta yggctttcaa agaaaagcaa agcaaccttg      60

a                                                                      61


<210>  256
<211>  61
<212>  DNA
<213>  Homo Sapiens

<400>  256
gtgtgactaa tgcgaaagct atgcaataaa yaaccatctt cagagttgct ctccttgtag      60

g                                                                      61

<210>  257
<211>  61
<212>  DNA
<213>  Homo Sapiens

<400>  257
aataacgcat tacctgtatg ttatacctat wtgccaacct tagaatcatg gttttctcat      60

t                                                                      61


<210>  258
<211>  61
<212>  DNA
<213>  Homo Sapiens

<400>  258
aatacaaatt ttactaaata gattgtttta yagtgaagga agggaaatct gataatttaa      60

t                                                                      61


<210>  259
<211>  61
<212>  DNA
<213>  Homo Sapiens

<400>  259
catctaaatg cagtcttaca taatgcaagg maattgttat aaccaaaaaa tgtactgcaa      60

a                                                                      61


<210>  260
<211>  61
<212>  DNA
<213>  Homo Sapiens

<400>  260
ttgcctatat tcccaaagga ttaaaataaa kctaagtaca tttccccaaa gtttgaaacg      60

g                                                                      61


<210>  261
<211>  61
<212>  DNA
<213>  Homo Sapiens

<400>  261
cttagttttt gagtcccgag ttaaatctca rtagaggtgg tgaaggaaat ttagcattgt      60

c                                                                      61


<210>  262
<211>  61
<212>  DNA
<213>  Homo Sapiens

<400>  262
ctgctagaac acacgacatg aaattaggaa ygacagtaag gctagaatct gtatcacgaa      60

g 61

<210> 263
<211> 61
<212> DNA
<213> Homo Sapiens

<400> 263
ggaacgacag taaggctaga atctgtatca ygaaggaaac atacttaatg acattaggct 60

a 61

<210> 264
<211> 61
<212> DNA
<213> Homo Sapiens

<400> 264
agtatcaata atggtgacct tattctttac rtagcaaatt gtccttttgt ctctccaaaa 60

a 61

<210> 265
<211> 61
<212> DNA
<213> Homo Sapiens

<400> 265
cacaaaatat gtgtcaattc cacaatccac maagagtttt ttctcttcac caaactcctc 60

c 61

<210> 266
<211> 61
<212> DNA
<213> Homo Sapiens

<400> 266
gaacaactct catgtataca actgaaaaat yacaaaggtg tattcactta acacaaagtg 60

a 61

<210> 267
<211> 61
<212> DNA
<213> Homo Sapiens

<400> 267
gctaaatttg ctcttctttt caatgaattg raatgcacct ccttatggcc aactaggact 60

t 61

<210> 268
<211> 61
<212> DNA
<213> Homo Sapiens

<400>  268
tgcatcttgt tctccactct acagccagaa ygagctttct aaagggcaat gcaatcctcc      60

t                                                                     61


<210>  269
<211>  61
<212>  DNA
<213>  Homo Sapiens

<400>  269
gccaatagtc ccagtgcttt gataggccaa ygttggggga ttacttgagg ctagcctgag      60

c                                                                     61


<210>  270
<211>  61
<212>  DNA
<213>  Homo Sapiens

<400>  270
aaggaagagg gtgccaggcc aagtgtataa ygagggcaag ggccctgtgg cagggctgcg      60

c                                                                     61


<210>  271
<211>  61
<212>  DNA
<213>  Homo Sapiens

<400>  271
agaacctgca aaagtacaca tcaacagctc kttcaaccag caggtggtat gtgcattcaa      60

t                                                                     61


<210>  272
<211>  61
<212>  DNA
<213>  Homo Sapiens

<400>  272
accccaaaaa gctcagtgac tgtctcaaaa staatctatc catctgatgt ttaaaaaata      60

c                                                                     61


<210>  273
<211>  61
<212>  DNA
<213>  Homo Sapiens

<400>  273
gagaaggtaa ggagcctcaa gcgtgtatgg yggggggaat tgaaattttg aataaggtgg      60

g                                                                     61


<210>  274

<211>  61
<212>  DNA
<213>  Homo Sapiens

<400>  274
aatcaggatg acttcagtgt tggccacatg bttatgggtg gatggccttg ggtacatcta        60

a                                                                        61


<210>  275
<211>  61
<212>  DNA
<213>  Homo Sapiens

<400>  275
tgatatattt gaagaaagta ataaggccaa wgtgtttgaa atacagagaa tgaggcagca        60

c                                                                        61


<210>  276
<211>  61
<212>  DNA
<213>  Homo Sapiens

<400>  276
ttcctccagt gcacctactc attgtcacgg yggatcatgt actggatcag cacagaaaga        60

t                                                                        61


<210>  277
<211>  61
<212>  DNA
<213>  Homo Sapiens

<400>  277
ctgccaccca cacttacccc ttgccaccga kgcatagagg catagtccag gcaaacgttg        60

c                                                                        61


<210>  278
<211>  61
<212>  DNA
<213>  Homo Sapiens

<400>  278
atgagaatgg tgctagcttc aggccaaggt rtaggagaca ttgagcatcc tatgtctgaa        60

t                                                                        61


<210>  279
<211>  61
<212>  DNA
<213>  Homo Sapiens

<400>  279
tcactgtgat ttaatattaa ttaaggtagg rtttccacac caccagaaca cctatcccac        60

t                                                                        61

<210>  280
<211>  61
<212>  DNA
<213>  Homo Sapiens

<400>  280
taaaacatcg gcactattaa tctttcacac rctttgttaa aagcttacac acaaatatac      60

c                                                                      61


<210>  281
<211>  61
<212>  DNA
<213>  Homo Sapiens

<400>  281
agaatgagtt atatcctcaa ttggtgacaa wctttctctt ttttttttct acttctattg      60

t                                                                      61


<210>  282
<211>  61
<212>  DNA
<213>  Homo Sapiens

<400>  282
tacatatacc ttcaaggatt tcagtgaagg rcttctttct aactttcctc cttaagataa      60

c                                                                      61


<210>  283
<211>  61
<212>  DNA
<213>  Homo Sapiens

<400>  283
aagctccatg acccaagcat aattctttgg wtttctatgt taaccctagc acagaaagat      60

g                                                                      61


<210>  284
<211>  61
<212>  DNA
<213>  Homo Sapiens

<400>  284
aagagttgac aacctactag agtcagtcaa ygcagttatg ttagagaagg taaaggcgca      60

a                                                                      61


<210>  285
<211>  61
<212>  DNA
<213>  Homo Sapiens

<400>  285

gccttctttc cctttgttca tgaaacagag raaacaccca tcactggtaa ttaatggtag 60

c 61

<210> 286
<211> 61
<212> DNA
<213> Homo Sapiens

<400> 286
agaattgtat ctcattggac aaaagggata ygattgtgtc tcttctgtgc tgccacatgc 60

c 61

<210> 287
<211> 61
<212> DNA
<213> Homo Sapiens

<400> 287
agaggaagtc actggaagag aaatgaaaac mcagaggtag aggagaacac acatctattt 60

t 61

<210> 288
<211> 61
<212> DNA
<213> Homo Sapiens

<400> 288
agtatttctc aagcactttt aagcaaaggt ragtattcat acaagaaatt tagggggaaa 60

a 61

<210> 289
<211> 61
<212> DNA
<213> Homo Sapiens

<400> 289
cattctaaac cttatatgag tctacttgat ygaaaggtca attattccat gttctgaaat 60

g 61

<210> 290
<211> 61
<212> DNA
<213> Homo Sapiens

<400> 290
tcgctgaacg aatgatctca accccacgtg wctctctagc ttcatctctt gcctaacagc 60

a 61

<210> 291
<211> 61
<212> DNA

<213>  Homo Sapiens

<400>  291
aagataagac aatgaaagag aagtcgaagt saggcccggc acggtggctc acgcctgtaa      60

a                                                                      61


<210>  292
<211>  61
<212>  DNA
<213>  Homo Sapiens

<400>  292
atctatctgt tcaattacct cttcattttt yaaactcatt ccatatcatt tccactcatt      60

t                                                                      61


<210>  293
<211>  61
<212>  DNA
<213>  Homo Sapiens

<400>  293
ataggaagac ctaactgtcc aatctgcata yaaaatggaa ttccagtttg ttaggggaag      60

g                                                                      61


<210>  294
<211>  61
<212>  DNA
<213>  Homo Sapiens

<400>  294
tcttcccctc atccccaaga tgcaacattt ytagtgatac caatttgcag gtatttcatc      60

g                                                                      61


<210>  295
<211>  61
<212>  DNA
<213>  Homo Sapiens

<400>  295
tctgtatgta ctgaggaaga atgaggcata ygatacacag aaagaaagca gcagagtaca      60

t                                                                      61


<210>  296
<211>  61
<212>  DNA
<213>  Homo Sapiens

<400>  296
ggaaatttca agtaatgaaa agcctttatt mtgggtttca aatatggtca cctaaactaa      60

t                                                                      61

<210>  297
<211>  61
<212>  DNA
<213>  Homo Sapiens

<400>  297
catactgttg gtaggaatgt aaattgatac ygtctgtctt gtggatagtt tggcagtatt      60

g                                                                      61


<210>  298
<211>  61
<212>  DNA
<213>  Homo Sapiens

<400>  298
cctatgcatc caaataactc acatcacaca rtggcttctt tggatgaatt aaaagtatct      60

a                                                                      61


<210>  299
<211>  61
<212>  DNA
<213>  Homo Sapiens

<400>  299
atcaaaaata aacaaaacta gttttgctct magtaccacc cagacttcaa tgattccata      60

c                                                                      61


<210>  300
<211>  61
<212>  DNA
<213>  Homo Sapiens

<400>  300
agaaagatat gtgctattta agtgtcagag staggattgt ttaggccagt gatttttttt      60

t                                                                      61


<210>  301
<211>  61
<212>  DNA
<213>  Homo Sapiens

<400>  301
tctggagcgt gcagatgaag caggcagtca sggtaatgaa gtcggaaatg caagtccaga      60

g                                                                      61


<210>  302
<211>  61
<212>  DNA
<213>  Homo Sapiens

<400>  302
gcagtttctc tcagttggta tgctaaattt crggccttgt ggcactctga tcaccttctc      60

a                                                                   61

<210>  303
<211>  61
<212>  DNA
<213>  Homo Sapiens

<400>  303
gaaacccaaa gtgtttgctg taatgaccag mgagtgtcag tgaaactcct tagcctaagg      60

a                                                                   61

<210>  304
<211>  61
<212>  DNA
<213>  Homo Sapiens

<400>  304
ttatttatat atttggctcc ccctagtgga mtgtaagcat attgacaata aaggatcatg      60

t                                                                   61

<210>  305
<211>  61
<212>  DNA
<213>  Homo Sapiens

<400>  305
aaatcatgca gttagtgtgt agcagaagca rgtaatccct acagcccaca cagaatttcc      60

a                                                                   61

<210>  306
<211>  61
<212>  DNA
<213>  Homo Sapiens

<400>  306
tatatgtatt gttaaaacat tttgcatatg rgttgttaaa aaaatttcat gggaaaattt      60

a                                                                   61

<210>  307
<211>  61
<212>  DNA
<213>  Homo Sapiens

<400>  307
ttaataagat gaaattaagc actgcagtcc rataatgagg acaagtaggg cctaactaca      60

t                                                                   61

<210>  308
<211>  61
<212>  DNA
<213>  Homo Sapiens

<400>  308
atcacagtga aaagtgatta tagggttcat rctatcagca ggtaacttgg tgaattctac        60

a                                                                         61


<210>  309
<211>  61
<212>  DNA
<213>  Homo Sapiens

<400>  309
ctgtgaagga gatcaccagg gcttggctag rtcacagctc tgtggctgca tctaggtggg        60

g                                                                         61


<210>  310
<211>  61
<212>  DNA
<213>  Homo Sapiens

<400>  310
gtgatgaaaa agttctggaa acatggatag yggtgacagt tgcacaacat tgtcagtgta        60

c                                                                         61


<210>  311
<211>  61
<212>  DNA
<213>  Homo Sapiens

<400>  311
cccatttagt tatgggaaga atactcatgg ygcttactga ctagattttc ctaaccgatt        60

g                                                                         61


<210>  312
<211>  61
<212>  DNA
<213>  Homo Sapiens

<400>  312
atatgtgaga gagtgtaagt gcctgtacgt rtaacatcac ttctgctgta gtcatcatca        60

g                                                                         61


<210>  313
<211>  61
<212>  DNA
<213>  Homo Sapiens

<400>  313
ctaaaggtat atatatagaa ttattattga ygattatcag tattatatca ctgatggata        60

g                                                                         61


<210>  314
<211>  61

<212>  DNA
<213>  Homo Sapiens

<400>  314
tgggatggat gaataaagcg cttatctcca ytagtgtctc cacagtgaat attaccattg      60

t                                                                      61


<210>  315
<211>  61
<212>  DNA
<213>  Homo Sapiens

<400>  315
ctttccctct gaagaagaat tagccttctc stcttccaaa tgtgactttt ctgtttgtgg      60

a                                                                      61


<210>  316
<211>  61
<212>  DNA
<213>  Homo Sapiens

<400>  316
tctctcagta aacatatctc ctatgctagg yaagacaaaa ggattgagaa gacaccatga      60

t                                                                      61


<210>  317
<211>  61
<212>  DNA
<213>  Homo Sapiens

<400>  317
tggcatttta taatgtcctg taggagttaa rtgtgtatgc agaaatggtc ttaaaattgt      60

a                                                                      61


<210>  318
<211>  61
<212>  DNA
<213>  Homo Sapiens

<400>  318
taggaaacat aagcttggtg gattatcgaa mgacctcaaa tgggtcagga gttcgagacc      60

a                                                                      61


<210>  319
<211>  61
<212>  DNA
<213>  Homo Sapiens

<400>  319
cttcagtttt ctaaattatg tttgacatca rgacagtcaa gtcagtccag gatgagaaaa      60

g                                                                      61

<210>  320
<211>  61
<212>  DNA
<213>  Homo Sapiens

<400>  320
cctgtggtcc agggcatatt tgtagtgaac raaataccaa tgatagggaa aagaaccatg      60

g                                                                      61


<210>  321
<211>  61
<212>  DNA
<213>  Homo Sapiens

<400>  321
ttttggggcc ctcttgccct gcatcagttt ygagtgtttg gttaagctgt atttggcccc      60

a                                                                      61


<210>  322
<211>  61
<212>  DNA
<213>  Homo Sapiens

<400>  322
agttcctaaa catggtcctg ggaagagata yggtgaacta gcaatattat tcattctaac      60

a                                                                      61


<210>  323
<211>  61
<212>  DNA
<213>  Homo Sapiens

<400>  323
ggagctttca taagaggttg aagtttttga yccatgacag actccagact ttccataagg      60

t                                                                      61


<210>  324
<211>  61
<212>  DNA
<213>  Homo Sapiens

<400>  324
agaagtttag gttaggcatt tgaattgatt ytattgtagg agaggtcata gactgaatat      60

t                                                                      61


<210>  325
<211>  61
<212>  DNA
<213>  Homo Sapiens

<400>  325
ggtagaatca aattatatgt ccttggtaca rtaaatcttc attaagttcc ttatcaagca      60

t 61

<210> 326
<211> 61
<212> DNA
<213> Homo Sapiens

<400> 326
tgttcatctt ctcgttatct tcagaatctc rtactttgca tatatttaat ttattcttgt 60

c 61

<210> 327
<211> 61
<212> DNA
<213> Homo Sapiens

<400> 327
aggcatatat tgctatcaat catcaatttt rggcattacc tacttatctt ttttatggta 60

a 61

<210> 328
<211> 61
<212> DNA
<213> Homo Sapiens

<400> 328
aaacttttat cctgcactat attcacattt waaaaatttg gttgcacact ttccacaggt 60

a 61

<210> 329
<211> 61
<212> DNA
<213> Homo Sapiens

<400> 329
atgtgtgtaa gttggagact gttgagtgac yaaaagtttg cttctctcat tgaaatagga 60

a 61

<210> 330
<211> 61
<212> DNA
<213> Homo Sapiens

<400> 330
actgacttaa atgggagcta caaagaaaca yagacagtgc caagaagtca tcttaatagg 60

t 61

<210> 331
<211> 61
<212> DNA
<213> Homo Sapiens

<400>  331
aaaaccctat cctctacctt acttatgcta ycttacttct ctatcctttg tgtaatttct        60

g                                                                         61


<210>  332
<211>  61
<212>  DNA
<213>  Homo Sapiens

<400>  332
ctatttttta ttgcagatag cagtagttca ytttctttta ttggcctttt ttccccaaat        60

a                                                                         61


<210>  333
<211>  61
<212>  DNA
<213>  Homo Sapiens

<400>  333
tgatttgtaa taatccccat ttcctttaag mtctcacccc cagagagaga cagtgaagga        60

t                                                                         61


<210>  334
<211>  61
<212>  DNA
<213>  Homo Sapiens

<400>  334
ataaagtaat tgataattcc aaaagtgtca ktccaactaa ttgacccaaa agtaaatttt        60

a                                                                         61


<210>  335
<211>  61
<212>  DNA
<213>  Homo Sapiens

<400>  335
gacagttaac ttacttgcag atcagcttaa sttcttgagg ctttttaaaa gctttgtcag        60

t                                                                         61


<210>  336
<211>  61
<212>  DNA
<213>  Homo Sapiens

<400>  336
tcttagtgat agacatccag gctgcctctc rcttgttgtt accacaaagg aggctgcaaa        60

g                                                                         61


<210>  337

<211>  61
<212>  DNA
<213>  Homo Sapiens

<400>  337
tatctgcctc caaggtacag ggagactgta wgctctgggt gacagttatt tgagtgtgat        60

a                                                                         61


<210>  338
<211>  61
<212>  DNA
<213>  Homo Sapiens

<400>  338
ccaagtcaaa gatctaaaaa gattatggaa rgccaaggaa gccaatgtgg ctagcacaag        60

a                                                                         61


<210>  339
<211>  61
<212>  DNA
<213>  Homo Sapiens

<400>  339
atccagagat acttctcact gttctacgaa rtcttggctc tgctgggttc tctgtggaag        60

t                                                                         61


<210>  340
<211>  61
<212>  DNA
<213>  Homo Sapiens

<400>  340
gaagccatgg tgttccctta aaacctgata yggagggtga ttccccatca tgtctgtatt        60

c                                                                         61


<210>  341
<211>  61
<212>  DNA
<213>  Homo Sapiens

<400>  341
cgggacaggc tcagcctaaa aaggcttcct yctaccctac ctccaggtga tgtggttatg        60

a                                                                         61


<210>  342
<211>  61
<212>  DNA
<213>  Homo Sapiens

<400>  342
tggagtcatc tattatgaca tcaaccagat yatcaccact gccgtgatga ccctcagcaa        60

a                                                                         61

<210>  343
<211>  61
<212>  DNA
<213>  Homo Sapiens

<400>  343
tagccgcaag gtggcatctg tgctgaacaa ygcctactac ctggacaaga tgcactacag      60

c                                                                      61


<210>  344
<211>  61
<212>  DNA
<213>  Homo Sapiens

<400>  344
tgtcaggttc actagaggtg aatgcaaggg yttagagagg tgaggttgca gagggaaaag      60

g                                                                      61


<210>  345
<211>  61
<212>  DNA
<213>  Homo Sapiens

<400>  345
atatggcatg tcagcaggat gaccatacag yatggcttgt ctgggacagt cccagttata      60

c                                                                      61


<210>  346
<211>  61
<212>  DNA
<213>  Homo Sapiens

<400>  346
ccccgggaaa gcttctggaa caacacttta sgggctactg acaggctgct ctgcttctca      60

c                                                                      61


<210>  347
<211>  61
<212>  DNA
<213>  Homo Sapiens

<400>  347
tccagcataa gtgaaaaaac agtgtttcgg rtacgctagt gtttctgaga caaaatatgt      60

a                                                                      61


<210>  348
<211>  61
<212>  DNA
<213>  Homo Sapiens

<400>  348

tgcctgggct ggttgcgaac tcctggtggc ytcaattgat ctttgcactt cagcctccca 60

a 61

<210> 349
<211> 61
<212> DNA
<213> Homo Sapiens

<400> 349
cctgaaacaa cctccctctg ggacagaaat yggcttggga actcaatcat tcaccaaaag 60

g 61

<210> 350
<211> 61
<212> DNA
<213> Homo Sapiens

<400> 350
cataggcaaa aattggggtt tgacttatac rttgacagtt tgcattgctt ggattactaa 60

t 61

<210> 351
<211> 61
<212> DNA
<213> Homo Sapiens

<400> 351
tttcaaaaaa tggtagctgt tattatcata ycactttcat ggaatttcgt tagttactca 60

g 61

<210> 352
<211> 61
<212> DNA
<213> Homo Sapiens

<400> 352
aaagcatcgt gttgtacaca aattttgtca rtcataactc aataatgctg gaaaaataaa 60

a 61

<210> 353
<211> 61
<212> DNA
<213> Homo Sapiens

<400> 353
catgagggtt gcattccctc ttcagttgcc magctttttc aggatgtgag gtcagcatat 60

t 61

<210> 354
<211> 61
<212> DNA

<213>  Homo Sapiens

<400>  354
ctacatttca tataaatgga atgatacaat rtatggtcct ttgtcaatgg cttcacttag      60

c                                                                     61


<210>  355
<211>  61
<212>  DNA
<213>  Homo Sapiens

<400>  355
agtgtgttct aaataacatc tcacactgag racaacattc tgcttattcc acttcagact      60

a                                                                     61


<210>  356
<211>  61
<212>  DNA
<213>  Homo Sapiens

<400>  356
gggttttaaa ttgagatttt gaaatctgaa rgcaatcatt aaggtcagat gtggtcttta      60

g                                                                     61


<210>  357
<211>  61
<212>  DNA
<213>  Homo Sapiens

<400>  357
ttcagtaatc aattctactt tttgtaagta rattgcagtt taagtctttg aatttgtcta      60

t                                                                     61


<210>  358
<211>  61
<212>  DNA
<213>  Homo Sapiens

<400>  358
agcaaataat atacattcat acagttgaac kccacatagc ttttttaaaa aaaataaatc      60

a                                                                     61


<210>  359
<211>  61
<212>  DNA
<213>  Homo Sapiens

<400>  359
gcatgtgccc tcttagccgg attcaaacca stgttgccta cattttcgtg gccatagcag      60

g                                                                     61

<210> 360
<211> 61
<212> DNA
<213> Homo Sapiens

<400> 360
acctaaaaga ctgtgttatc agaggaagag rtcccaaatt tggagtaaag atgggagaaa 60

a 61

<210> 361
<211> 61
<212> DNA
<213> Homo Sapiens

<400> 361
tgctcccagc caattctttc tctaaatcca yttcttccca ttcctattct aatcttctca 60

t 61

<210> 362
<211> 61
<212> DNA
<213> Homo Sapiens

<400> 362
gaacatatgt attctcttca cataggcaat matgatacat cttatcctag ggagacaata 60

t 61

<210> 363
<211> 61
<212> DNA
<213> Homo Sapiens

<400> 363
gaatctttag attgctttgg atcgtacaga yattttaaca acattaattc ttcctatcca 60

t 61

<210> 364
<211> 61
<212> DNA
<213> Homo Sapiens

<400> 364
ttaagtcgct tccagtctgg gctatttcca ytggtagttt tacgtttttc tggtcacata 60

a 61

<210> 365
<211> 61
<212> DNA
<213> Homo Sapiens

<400> 365
attcacttcc ctatgatact gagtttaggc wgcaaatcaa aggaagggct ggctgatgat 60

t 61

<210> 366
<211> 61
<212> DNA
<213> Homo Sapiens

<400> 366
tctgacattc ttcctttctt ttcatactgc ygagtttctg acctatagca atctccttct 60

g 61

<210> 367
<211> 61
<212> DNA
<213> Homo Sapiens

<400> 367
aattcacact aaaaatgact agatgaaaga ygtggtacta gaggctttgt atatgcctag 60

a 61

<210> 368
<211> 61
<212> DNA
<213> Homo Sapiens

<400> 368
tagcacaaaa caatgcaggt tgacccagct wtgtcatttg gtaaaatatc tcccaggcaa 60

t 61

<210> 369
<211> 61
<212> DNA
<213> Homo Sapiens

<400> 369
cagtggatga ttcctttcct taacacatgc rcagttcagg aaattaatca atatggagta 60

g 61

<210> 370
<211> 61
<212> DNA
<213> Homo Sapiens

<400> 370
acctctcaga ggggagccac aggaaggtta ragcagaaaa aggactggac ttacttacat 60

t 61

<210> 371
<211> 61
<212> DNA
<213> Homo Sapiens

<400>  371
tgaagtccat cccgatctga aggttcaccc rtcactaaga gacaaatggt gcttctgtta      60

a                                                                     61


<210>  372
<211>  61
<212>  DNA
<213>  Homo Sapiens

<400>  372
ttctaagggc tctgcttctg agaatataat saatgccttt caaggtccct ctgccctccc      60

t                                                                     61


<210>  373
<211>  61
<212>  DNA
<213>  Homo Sapiens

<400>  373
gtttcttgat cttgtgtcat ggctgtgttt rtagcagcac tgcagggttg gccagttatg      60

g                                                                     61


<210>  374
<211>  61
<212>  DNA
<213>  Homo Sapiens

<400>  374
tatctctccc caggactgga ctctgagccc rtcattgaaa tgagagacca cgaggacggg      60

g                                                                     61


<210>  375
<211>  61
<212>  DNA
<213>  Homo Sapiens

<400>  375
atcttccttg gattatcaaa tgtgagtctg ycattgattc ccattgagac cctccctgat      60

a                                                                     61


<210>  376
<211>  61
<212>  DNA
<213>  Homo Sapiens

<400>  376
ctgagtatct tactacatat tttacaaaag rctcattgtt tcacagtgtg ttattagaat      60

t                                                                     61


<210>  377
<211>  61

<212>  DNA
<213>  Homo Sapiens

<400>  377
aagatgaaag aactaaggta tgaggtgctc wagtaacttt ccaaaggtga ctcagctagg      60

a                                                                      61


<210>  378
<211>  61
<212>  DNA
<213>  Homo Sapiens

<400>  378
tgcattagtg agtcctgagc actgctggat yattacttca gagcatcctg acatgaagga      60

a                                                                      61


<210>  379
<211>  61
<212>  DNA
<213>  Homo Sapiens

<400>  379
tcttactgtt tgagtcagaa gcccaaggta kcttgctatt tttcacactg gcacactgga      60

g                                                                      61


<210>  380
<211>  61
<212>  DNA
<213>  Homo Sapiens

<400>  380
tgtttttgca aactttcagt catataaaaa mcgtaaattt cacttttccc ccattttctt      60

g                                                                      61


<210>  381
<211>  61
<212>  DNA
<213>  Homo Sapiens

<400>  381
acctcaaggg ggccttggtc ctgctccatc ygaagagctg attcattaga cttttgcagt      60

c                                                                      61


<210>  382
<211>  61
<212>  DNA
<213>  Homo Sapiens

<400>  382
aaactctggc aaaatcagga ataatataga yggtgaacat ttgactccta acaattgctg      60

a                                                                      61

<210> 383
<211> 61
<212> DNA
<213> Homo Sapiens

<400> 383
ttgagattac aatcctgaag ttatcactta yactttactc tgagctagat ggggttccaa 60

a 61

<210> 384
<211> 61
<212> DNA
<213> Homo Sapiens

<400> 384
gaatgtataa gaaagctggg acaaaaaagc rgatctccat atctgaggac tgtgataaaa 60

a 61

<210> 385
<211> 61
<212> DNA
<213> Homo Sapiens

<400> 385
tttgaatcag aaagagagat gacttccaac rgagctgaca aacactgcaa cagcaagcaa 60

t 61

<210> 386
<211> 61
<212> DNA
<213> Homo Sapiens

<400> 386
tgaagaattc tgtggaaaag cagaccttta rctgcatctt tttcttccgc agatgtttgg 60

t 61

<210> 387
<211> 61
<212> DNA
<213> Homo Sapiens

<400> 387
ctgtcgtaag ttaaaaatgc atggtaaaca yctatgggta accactcaac agaatgaata 60

a 61

<210> 388
<211> 61
<212> DNA
<213> Homo Sapiens

<400> 388
actctgcatt aagcaactct attggtacca yttttacaac agcacgtgtt cactatgtgt 60

t 61

<210> 389
<211> 61
<212> DNA
<213> Homo Sapiens

<400> 389
acttgctaaa gcagctgtga atttatgcca magaattaaa taaacaaaaa taccaaagac 60

t 61

<210> 390
<211> 61
<212> DNA
<213> Homo Sapiens

<400> 390
tcttctgtgt cttagcattc aatggagtag waatgctaag tataaaaaga aaaatggccg 60

g 61

<210> 391
<211> 61
<212> DNA
<213> Homo Sapiens

<400> 391
tgggccatac actaatttac agaacctctc ycctactgac aggcatccag atgattcctt 60

g 61

<210> 392
<211> 61
<212> DNA
<213> Homo Sapiens

<400> 392
cataaaggct atgactaatg gattgaataa stgatggaca agctgctcac tggggacaga 60

c 61

<210> 393
<211> 61
<212> DNA
<213> Homo Sapiens

<400> 393
acattcataa tggggaaaag gatccagata yagtaaagca aaaacaaaat ccagtttgca 60

a 61

<210> 394
<211> 61
<212> DNA
<213> Homo Sapiens

<400>  394
agaatttgaa ccacatctcc aactctattg mctatcattc ttaaaatacc ccatgcagtc      60

t                                                                      61


<210>  395
<211>  61
<212>  DNA
<213>  Homo Sapiens

<400>  395
catctgttat cacctaatcc cagaagcgac rtaccatccc ttttgcttta ttctgttggt      60

c                                                                      61


<210>  396
<211>  61
<212>  DNA
<213>  Homo Sapiens

<400>  396
tctagaagta accttctaga tgaaaccttc ygtaacctag aagtaacctt ctgctacaga      60

a                                                                      61


<210>  397
<211>  61
<212>  DNA
<213>  Homo Sapiens

<400>  397
ccttttcttt ccacaacatg cctgttcatt yggttcatct ggagttctca tttagttcac      60

t                                                                      61


<210>  398
<211>  61
<212>  DNA
<213>  Homo Sapiens

<400>  398
ctaaatatat acactgaaaa tttgtgtaaa sagccatttc ggctaacact ggtaagagaa      60

a                                                                      61


<210>  399
<211>  61
<212>  DNA
<213>  Homo Sapiens

<400>  399
aagaaaaacc aaattaagtg tcacataaac kttgtaatta atgtaaccat tgactcactg      60

a                                                                      61


<210>  400

<211>  61
<212>  DNA
<213>  Homo Sapiens

<400>  400
ttctaggctt aagataccca ttactgtgtc rtaagccaaa tatctgatac ttatgtccaa      60

a                                                                      61


<210>  401
<211>  61
<212>  DNA
<213>  Homo Sapiens

<400>  401
aggtgatgtg ataccgcacc ctgtagatat ytatgcctgt caagtattca taagcactct      60

t                                                                      61


<210>  402
<211>  61
<212>  DNA
<213>  Homo Sapiens

<400>  402
aatacagctg atggaggcgt caacgacttc wgtaaatatt gaagtattat ttcttctgcc      60

a                                                                      61


<210>  403
<211>  61
<212>  DNA
<213>  Homo Sapiens

<400>  403
tgcaccaaaa gcatcctgac caattcagga rtgctaggcc cttaacatgg atagctctgc      60

c                                                                      61


<210>  404
<211>  61
<212>  DNA
<213>  Homo Sapiens

<400>  404
gatcacatat tgtgattcca cttacatgaa raacctagat aagcaaattt atagaaccag      60

a                                                                      61


<210>  405
<211>  61
<212>  DNA
<213>  Homo Sapiens

<400>  405
tggaactttg ctcatgtctg cgtcagtgga rcctagacct ggaatctcaa ttgtgtgatt      60

t                                                                      61

<210>  406
<211>  61
<212>  DNA
<213>  Homo Sapiens

<400>  406
tatgaagaaa acctgaaaat tctgccaaag rtatccaaac gcgcaccctc tgtcctttgc        60

a                                                                        61


<210>  407
<211>  61
<212>  DNA
<213>  Homo Sapiens

<400>  407
tctacatcta ggctctattc tgtcctattg ratgtactgt ttatcccttt gccaataata        60

t                                                                        61


<210>  408
<211>  61
<212>  DNA
<213>  Homo Sapiens

<400>  408
gaaggataaa caattctccc tcttaactac ygaaaaggag acatatgact tggggcatta        60

g                                                                        61


<210>  409
<211>  61
<212>  DNA
<213>  Homo Sapiens

<400>  409
tctattgtaa attgaccaga acaggaatac rtttgtgaat tcgtaacgta agaaggtagc        60

a                                                                        61


<210>  410
<211>  61
<212>  DNA
<213>  Homo Sapiens

<400>  410
taggacttcc agtactaagt tgaataaaag yggtctctcg tcttctcttg ccatcttcct        60

t                                                                        61


<210>  411
<211>  61
<212>  DNA
<213>  Homo Sapiens

<400>  411

ctgtgtatta catatagaag tactgtatat rgtgaggaat gccatcctgt gtgtgttgtt 60

a 61

<210> 412
<211> 61
<212> DNA
<213> Homo Sapiens

<400> 412
tgcatggagg ccattattac agcagaacca rtggtggagg gcatagaggc caggctgctc 60

t 61

<210> 413
<211> 61
<212> DNA
<213> Homo Sapiens

<400> 413
gcagtcacct acagctgttg gagcttctca rtttatgctc ccactatctc atccaggtat 60

g 61

<210> 414
<211> 61
<212> DNA
<213> Homo Sapiens

<400> 414
tcctccctgt cctcacactt gaacttcatg rtccaatcac ttcaataatt atcttgtcct 60

c 61

<210> 415
<211> 61
<212> DNA
<213> Homo Sapiens

<400> 415
gaaaaaaaac caagccccat ttccttaggc rtctagcatt ctaacacatc actctctctt 60

t 61

<210> 416
<211> 61
<212> DNA
<213> Homo Sapiens

<400> 416
ccagggaatg gacccatgca atctgagtga yggatgaaga aaactgcttt cagtcctaga 60

t 61

<210> 417
<211> 61
<212> DNA

<213>  Homo Sapiens

<400>  417
tgagatcata cataaagagc ttggcccagc ygaggagccg tcacctaacc gtgctcagga      60

g                                                                      61


<210>  418
<211>  61
<212>  DNA
<213>  Homo Sapiens

<400>  418
agaaagaaaa ggatcacagg ggtctcagaa wtgaaaacag gcctctgtaa aaaggcagaa      60

t                                                                      61


<210>  419
<211>  61
<212>  DNA
<213>  Homo Sapiens

<400>  419
acagcttcaa caaagaacat acagtgaaaa ygacttccaa ggttagatca taaaaatacc      60

a                                                                      61


<210>  420
<211>  61
<212>  DNA
<213>  Homo Sapiens

<400>  420
aacaattaca atattgatat caaagaacaa yggtcacaga tcaccgaaac agacatacca      60

a                                                                      61


<210>  421
<211>  61
<212>  DNA
<213>  Homo Sapiens

<400>  421
cccttacatt ttacaccaga ggtaagtgtc ytgctccttt catactaatc cttgccctat      60

t                                                                      61


<210>  422
<211>  61
<212>  DNA
<213>  Homo Sapiens

<400>  422
ttgttagaag acaaccagta tcctacaagg magtaatgct ggtagcaacc gatctttaaa      60

t                                                                      61

<210>  423
<211>  61
<212>  DNA
<213>  Homo Sapiens

<400>  423
ttctttttca ggatccaatt ctgtatcgaa raacttgacc tttaaactag ttaaaagaat      60

c                                                                     61


<210>  424
<211>  61
<212>  DNA
<213>  Homo Sapiens

<400>  424
aagcccagtt tcagaattac tgttaactcc raattgccaa ttacagcttg actttgcagt      60

t                                                                     61


<210>  425
<211>  61
<212>  DNA
<213>  Homo Sapiens

<400>  425
acttcaagtt gttgcagaag ggatgatgag rttaatggag aacagaaaga aggtgaacta      60

g                                                                     61


<210>  426
<211>  61
<212>  DNA
<213>  Homo Sapiens

<400>  426
ctaatatacc agagagataa tggtaactaa kctcttaaaa acacatagag caaccttctg      60

t                                                                     61


<210>  427
<211>  61
<212>  DNA
<213>  Homo Sapiens

<400>  427
gcagatagct cctgaagcct gcaggctagc rgggagctca gttacacaag atgaaggggg      60

t                                                                     61


<210>  428
<211>  61
<212>  DNA
<213>  Homo Sapiens

<400>  428
ttggaaatca tgtatttgca atggtgttct yaaaaattag aatgttaaag agagccaaaa      60

t                                                                   61

<210>  429
<211>  61
<212>  DNA
<213>  Homo Sapiens

<400>  429
aggaataaaa atgaagcaaa taaatgtggt ycatctttct tcaggacctg atgctcctgc      60

a                                                                   61

<210>  430
<211>  61
<212>  DNA
<213>  Homo Sapiens

<400>  430
agtgccatgt tctagaggta caggctccaa raagtccaca ttcaaacatg gttttccagg      60

a                                                                   61

<210>  431
<211>  61
<212>  DNA
<213>  Homo Sapiens

<400>  431
atgaccttgg tttagcggtc agcaggtagc raggaaagtg atctcaaggg ccagaaagat      60

a                                                                   61

<210>  432
<211>  61
<212>  DNA
<213>  Homo Sapiens

<400>  432
aacccgtgat ctccacggca tttctctttg ytgtgatgag gccacacgtg tattacaatg      60

a                                                                   61

<210>  433
<211>  61
<212>  DNA
<213>  Homo Sapiens

<400>  433
aactggagag gctctttcct acctgataag scaacagctc cccggaggta gaaatctttg      60

t                                                                   61

<210>  434
<211>  61
<212>  DNA
<213>  Homo Sapiens

<400>  434
ggttaggttt aggtgaattt tcattcttcc rttttcctaa ttgctaccat aatcacatct      60

t                                                                      61


<210>  435
<211>  61
<212>  DNA
<213>  Homo Sapiens

<400>  435
atattttaat agatgatagc aaagtaaata yggtattcat cacctgaagc atttatcctt      60

t                                                                      61


<210>  436
<211>  61
<212>  DNA
<213>  Homo Sapiens

<400>  436
caccagaaca ataaaagcct caagcaatct yatctatgtg tcacaacaaa cccaacactc      60

a                                                                      61


<210>  437
<211>  61
<212>  DNA
<213>  Homo Sapiens

<400>  437
aacagaacaa tgtaaatttg gctgtttgac rttaacttaa tttgaagtgt taacttttcc      60

c                                                                      61


<210>  438
<211>  61
<212>  DNA
<213>  Homo Sapiens

<400>  438
tatactctag aaaattacgg ttctgttgaa ytggcatgct tttgttaatg cagattgatg      60

c                                                                      61


<210>  439
<211>  61
<212>  DNA
<213>  Homo Sapiens

<400>  439
ctgaaacagg agcttgaagc cttgttctca ygtctaaagc catccaaata taggggccac      60

t                                                                      61


<210>  440
<211>  61

<212>  DNA
<213>  Homo Sapiens

<400>  440
aaaaactctt ttttttccta ttagaacatc raatacttct acagaatatt tctattctat      60

g                                                                     61


<210>  441
<211>  61
<212>  DNA
<213>  Homo Sapiens

<400>  441
tgaaatatct ttttatctca tactaaaaat rcacgttttg ttcaggtaca ggattgctat      60

g                                                                     61


<210>  442
<211>  61
<212>  DNA
<213>  Homo Sapiens

<400>  442
gggataatta atacatggca gtcggggaga raggagggaa aaggaaacga agacgggaga      60

g                                                                     61


<210>  443
<211>  61
<212>  DNA
<213>  Homo Sapiens

<400>  443
gctgtttgat agtgtgtcca gctccatgtc rggtgcaatg ggtgctttgt aaatgctcac      60

a                                                                     61


<210>  444
<211>  61
<212>  DNA
<213>  Homo Sapiens

<400>  444
gcgcccacta tagactttca tgggaagagt yggcctcgtg gtgtcagagg ggagtccaaa      60

t                                                                     61


<210>  445
<211>  61
<212>  DNA
<213>  Homo Sapiens

<400>  445
tgctgaccgc cacggaactg gcagccaaga mataaggcaa tcggaagagt cagactgtcc      60

g                                                                     61

<210>  446
<211>  61
<212>  DNA
<213>  Homo Sapiens

<400>  446
tttttatggt cggacttcag aagcatctcc rttaaagctg ggaccaaggc cagctgtctc        60

c                                                                         61


<210>  447
<211>  61
<212>  DNA
<213>  Homo Sapiens

<400>  447
attggatgct gttctgtgta ctttacagat rttaactcaa ttaatattca caacacattg        60

g                                                                         61


<210>  448
<211>  61
<212>  DNA
<213>  Homo Sapiens

<400>  448
tctctgggtg ttctttgaat atcctgtgaa ygcctctgac tttatgcccc caccttgttt        60

a                                                                         61


<210>  449
<211>  61
<212>  DNA
<213>  Homo Sapiens

<400>  449
aaacaaagaa aagacttcca agccttcaat rttctaagtt gccatggcag cagcaccatc        60

a                                                                         61


<210>  450
<211>  61
<212>  DNA
<213>  Homo Sapiens

<400>  450
tgcaagatac atcttggcta gaacaactga ygaaattttt atgtaacacc ttcaaatgag        60

g                                                                         61


<210>  451
<211>  61
<212>  DNA
<213>  Homo Sapiens

<400>  451
tacaattctc atctctccaa gttggagggt stttaatctg tgctgaagat gaggaggcag        60

a                                                                   61

<210>  452
<211>  61
<212>  DNA
<213>  Homo Sapiens

<400>  452
gtgggacctg attcaccagt gaatgaggag ygtctgaaag ctgcagttca gggagagaaa      60

a                                                                   61

<210>  453
<211>  61
<212>  DNA
<213>  Homo Sapiens

<400>  453
aaatttaagt gtataaaaag ttaggtattt scttgtagaa acttgaaact acaattttgt      60

g                                                                   61

<210>  454
<211>  61
<212>  DNA
<213>  Homo Sapiens

<400>  454
atagaactat aataaatatt tctcaagcta yacaaaataa cgaaaagaaa acataagagt      60

c                                                                   61

<210>  455
<211>  61
<212>  DNA
<213>  Homo Sapiens

<400>  455
ttatcatata aatcctaaac atttcaagtg yacatctact tccagggatc ataaacataa      60

a                                                                   61

<210>  456
<211>  61
<212>  DNA
<213>  Homo Sapiens

<400>  456
aaaagagact accttttcct tgttcatgaa mgctgtttca aaatgaaatg actacattat      60

t                                                                   61

<210>  457
<211>  61
<212>  DNA
<213>  Homo Sapiens

<400>  457
ggtgaatgaa tgaacttgaa aacaggtcat ytgaaattct ccgatcataa aagcaaaaag      60

a                                                                      61

<210>  458
<211>  61
<212>  DNA
<213>  Homo Sapiens

<400>  458
ttaatttgtt taacgttatg gagaattagg ytcataatgt gacagtgata gtcctgaaga      60

a                                                                      61

<210>  459
<211>  61
<212>  DNA
<213>  Homo Sapiens

<400>  459
ggtgctttac tactccagct tctttgaatg rattagagat aatatgggca gaatacagtc      60

t                                                                      61

<210>  460
<211>  61
<212>  DNA
<213>  Homo Sapiens

<400>  460
gttgaaccgt aaacttcgtg tgcgttccta sacgattcaa tctttctaga cttcacttat      60

a                                                                      61

<210>  461
<211>  61
<212>  DNA
<213>  Homo Sapiens

<400>  461
ttggattttg ctagtgatac ttaactaatg rttcatactg tatttgaagt gtctgctgcc      60

a                                                                      61

<210>  462
<211>  61
<212>  DNA
<213>  Homo Sapiens

<400>  462
cgtttacaat gatgaaaaca ttatgaaaac rgtgattggt ggttactcaa cattgtgaat      60

a                                                                      61

<210>  463

<211>  61
<212>  DNA
<213>  Homo Sapiens

<400>  463
ggtatatagg atacaataat ctgtgcattt racgacaaca gtactattta ctcatgacat        60

g                                                                         61


<210>  464
<211>  61
<212>  DNA
<213>  Homo Sapiens

<400>  464
atttgtaact tggcctatca gtttgagacc yttgttctag acactttgta tactgtctca        60

a                                                                         61


<210>  465
<211>  61
<212>  DNA
<213>  Homo Sapiens

<400>  465
taattaagcc acaaagcaca atgcaagttc rataatcact ggaaataata tgccttttaa        60

g                                                                         61


<210>  466
<211>  61
<212>  DNA
<213>  Homo Sapiens

<400>  466
agggggctaa gaatcctcct ataattcagc ycataaaggt gtttaactat agaatgaaca        60

g                                                                         61


<210>  467
<211>  61
<212>  DNA
<213>  Homo Sapiens

<400>  467
   tgtctcctct gtcatctgcc tctggagacg ygactttata cccggcagct ttttgaactc        60
   t                                                                         61


<210>  468
<211>  61
<212>  DNA
<213>  Homo Sapiens

<400>  468
cctttgtgaa ttaatattaa cagataaagt rgagatccga ggcacttaca tgagtaaata        60

a                                                                         61

<210> 469
<211> 61
<212> DNA
<213> Homo Sapiens

<400> 469
ggactattcc cactgtgaca gaaagctcta ygggacagtg ttgcctgcaa tataaaacca 60

a 61

<210> 470
<211> 61
<212> DNA
<213> Homo Sapiens

<400> 470
tcaaaaagct tattatagtc ttagttgaga rccaagcaat tagtgcaact attcacttag 60

t 61

<210> 471
<211> 61
<212> DNA
<213> Homo Sapiens

<400> 471
cagggtattg tgaaatcaca gaagtgttaa sagccagttc cttttttgat catatgaggg 60

a 61

<210> 472
<211> 61
<212> DNA
<213> Homo Sapiens

<400> 472
gataaaaaca aaatgcacag caaggctcgt rtagttaact acaggttctc atgagtacag 60

a 61

<210> 473
<211> 61
<212> DNA
<213> Homo Sapiens

<400> 473
ctcaaaccag tccagcaagc agggactata ragtgaggta gcaggaaata aggctggcaa 60

g 61

<210> 474
<211> 61
<212> DNA
<213> Homo Sapiens

<400> 474

ctctaccttg cctacctgtt caaataaact mttcctttct caaacctctg gtccagtcag 60

g 61

<210> 475
<211> 61
<212> DNA
<213> Homo Sapiens

<400> 475
acccttctga atctctccat ctacctctgc ytatttataa ttcacaagtc tcttggaccc 60

c 61

<210> 476
<211> 61
<212> DNA
<213> Homo Sapiens

<400> 476
agacatgtcc acactcccca gcacacattc ygtaatgtca ccacactggc atgaggttta 60

g 61

<210> 477
<211> 61
<212> DNA
<213> Homo Sapiens

<400> 477
agctcctggc acacggacac ccttgcttac sgttcacatt tctaaaaatg ttgcctctgt 60

c 61

<210> 478
<211> 61
<212> DNA
<213> Homo Sapiens

<400> 478
tggatcttga gaatctgacc ctaaaattca ygtggaagaa caatgaggca aaataatcca 60

a 61

<210> 479
<211> 61
<212> DNA
<213> Homo Sapiens

<400> 479
ctcaaagccc tgtgctggct tcctacctca stcaaattaa aagccaaaga cttctcaagg 60

g 61

<210> 480
<211> 61
<212> DNA

<213>  Homo Sapiens

<400>  480
tcaaactact gaactgcggt cttggacacc rtctttctgg aggatcattt ggcatcatgg      60

a                                                                      61


<210>  481
<211>  61
<212>  DNA
<213>  Homo Sapiens

<400>  481
attatcataa cctttgattc agtaattcca rtcctaagaa tcaatggtag gaaaacattc      60

a                                                                      61


<210>  482
<211>  61
<212>  DNA
<213>  Homo Sapiens

<400>  482
aagtaatttt aactttgtgg aacatgactc kgaattgggt agtagactgg ggcttagttg      60

t                                                                      61


<210>  483
<211>  61
<212>  DNA
<213>  Homo Sapiens

<400>  483
tcagctagct ccttaaagtt cctagaatgt yagaagagta aaggttcctt gaagatagat      60

a                                                                      61


<210>  484
<211>  61
<212>  DNA
<213>  Homo Sapiens

<400>  484
gttatctttg taatattgaa tcttttaatc ygcaaacatg gaacatttct ccatttattt      60

a                                                                      61


<210>  485
<211>  61
<212>  DNA
<213>  Homo Sapiens

<400>  485
cctgttttca ctcatctctt cctttaaccc rgattccaag aaagcttgac aactgtctac      60

a                                                                      61

<210>  486
<211>  61
<212>  DNA
<213>  Homo Sapiens

<400>  486
taagacagct gatcttagat gacagcatca gktgggaaaa aattggtttt gtttctctat        60

a                                                                         61


<210>  487
<211>  61
<212>  DNA
<213>  Homo Sapiens

<400>  487
cctgagggtg agcttggaca ccttggcagg ktcagacaat gaacagactt caaagctcac        60

c                                                                         61


<210>  488
<211>  61
<212>  DNA
<213>  Homo Sapiens

<400>  488
ccatccgtga gcatggcatg tgtttccttt rtttgcgtca tctgtgattt cttttaacag        60

t                                                                         61


<210>  489
<211>  61
<212>  DNA
<213>  Homo Sapiens

<400>  489
   ttccatgctc agaattctaa accagtcttg raatctcact gcatcactgc atataccttt        60
   t                                                                         61


<210>  490
<211>  61
<212>  DNA
<213>  Homo Sapiens

<400>  490
gaggggtcat tttgtcccta actgaggttt mcttgttccc atactgctgc tcatacatct        60

g                                                                         61


<210>  491
<211>  61
<212>  DNA
<213>  Homo Sapiens

<400>  491
atactccctg agtttttaac ttaagtcaaa rtgacttcgc tacgaaaagg gtctacttca        60

g 61

<210> 492
<211> 61
<212> DNA
<213> Homo Sapiens

<400> 492
aagcagggaa tccaaaaatt attctatgag rtatgtggta ttataaaggt ttttatacta 60

a 61

<210> 493
<211> 61
<212> DNA
<213> Homo Sapiens

<400> 493
aatgaaaatc cagtcccaaa gggccaactt scagcaagct taccagaccc aatttgcctg 60

t 61

<210> 494
<211> 61
<212> DNA
<213> Homo Sapiens

<400> 494
cattgtcaac agacatgagc tcttattgtc rctatttttt aagagtcaaa taaaccccac 60

c 61

<210> 495
<211> 61
<212> DNA
<213> Homo Sapiens

<400> 495
tgttccagca caatttgttg aagatattat yccttagccc actgaattgt cttgacaccc 60

t 61

<210> 496
<211> 61
<212> DNA
<213> Homo Sapiens

<400> 496
tgtatccata aaaaataaaa ttggaattca yagttctgaa atcaaacagg aggacactag 60

c 61

<210> 497
<211> 61
<212> DNA
<213> Homo Sapiens

<400>  497
ataaagtgtt gagtatctca tgtaatttct scaataccgt actgaaagtg aaaaacagaa      60

t                                                                      61


<210>  498
<211>  61
<212>  DNA
<213>  Homo Sapiens

<400>  498
ttgaaggcaa ctcctaaaga aatagctcca yagtgtaaaa atcatttaac gctagcacag      60

a                                                                      61


<210>  499
<211>  61
<212>  DNA
<213>  Homo Sapiens

<400>  499
gaatttcgac tttgagtatt ttatatgttc wctttcagcc ttacaagcca agtcaactct      60

t                                                                      61


<210>  500
<211>  61
<212>  DNA
<213>  Homo Sapiens

<400>  500
agtttgtctc caaacaaaag cagcccatgc yatctaaaaa gtaaaggcca ctaggatgaa      60

a                                                                      61


<210>  501
<211>  61
<212>  DNA
<213>  Homo Sapiens

<400>  501
ctattgagtt ctgaagaata tattatgacc rtctgttttc tgtcttcttc gtggtgagaa      60

a                                                                      61


<210>  502
<211>  61
<212>  DNA
<213>  Homo Sapiens

<400>  502
atattcctaa aatgttggta tttctgagta sttctgaccc gaaaagactg gaaagggcac      60

t                                                                      61


<210>  503
<211>  61

<212>  DNA
<213>  Homo Sapiens

<400>  503
gttatttatt tttggagttc tgttaggtta ygaaaatggg ctaggcatgg tggctcacac      60

t                                                                     61


<210>  504
<211>  61
<212>  DNA
<213>  Homo Sapiens

<400>  504
tttatattac cctgttccct aataactttc mgtggcttct tgttaccctc aaggtacaac      60

g                                                                     61


<210>  505
<211>  61
<212>  DNA
<213>  Homo Sapiens

<400>  505
tcattgtatt ttcttccacc tgcccaatct ycagttctgc caggaattta taagattctt      60

g                                                                     61


<210>  506
<211>  61
<212>  DNA
<213>  Homo Sapiens

<400>  506
agtgaatgtc atagtatcaa tcaaatctac saacattctg taagcatgct tgactgtcac      60

t                                                                     61


<210>  507
<211>  61
<212>  DNA
<213>  Homo Sapiens

<400>  507
aaacttcaat tgttttctaa catctttcat sagtatttca cattcgataa tataaagtca      60

t                                                                     61


<210>  508
<211>  61
<212>  DNA
<213>  Homo Sapiens

<400>  508
gcttaagtaa gatcataact aagaagtgaa ygataaaaag gagtcaggct tacaaagatt      60

t                                                                     61

<210>  509
<211>  61
<212>  DNA
<213>  Homo Sapiens

<400>  509
tgacggtaga attaagtttg aggaagattc raggatatgg attaaacagt cattcttctt      60

a                                                                      61


<210>  510
<211>  61
<212>  DNA
<213>  Homo Sapiens

<400>  510
cctcaatatc aaggtaatgt ttatagcact rtattttcac acttcttgat cagtttacct      60

t                                                                      61


<210>  511
<211>  61
<212>  DNA
<213>  Homo Sapiens

<400>  511
taggtttggt attccaaatg tagaaacgaa sgctcagaag agttacttgt cagaaagcaa      60

g                                                                      61


<210>  512
<211>  61
<212>  DNA
<213>  Homo Sapiens

<400>  512
tttcactaat gaagaaaagt attgcacatt kgaaatacac taactggatt gaatatgtaa      60

t                                                                      61


<210>  513
<211>  61
<212>  DNA
<213>  Homo Sapiens

<400>  513
tgccattgaa taacgtgatc aaagtaaaga ygtatgttct caatttggga caaataaaac      60

t                                                                      61


<210>  514
<211>  61
<212>  DNA
<213>  Homo Sapiens

<400>  514
ccctttgttt gtaaagtact tacctcatgc yatctgtgtg agtgtcctac caaacttcca      60

a                                                                   61

<210>  515
<211>  61
<212>  DNA
<213>  Homo Sapiens

<400>  515
atagtagaga ataagcagaa aaaccacaat ragtgatgca cataggtctt ggcctcatgt      60

g                                                                   61

<210>  516
<211>  61
<212>  DNA
<213>  Homo Sapiens

<400>  516
tatatactca gtgatcctag tcctgccttt yataagaagg tggaataatt ttatgctcat      60

t                                                                   61

<210>  517
<211>  61
<212>  DNA
<213>  Homo Sapiens

<400>  517
taatactgag attgtcctag agctagacgt ragtgaatat tgagatagaa agccttatta      60

a                                                                   61

<210>  518
<211>  61
<212>  DNA
<213>  Homo Sapiens

<400>  518
tttccatgag gttagggata acatcttagt yggtagcctt gtttgtattc atatagcact      60

t                                                                   61

<210>  519
<211>  61
<212>  DNA
<213>  Homo Sapiens

<400>  519
ggaggcacct ttgaagatct tccagcctta staaagcatt cagatacctg cagcttcagc      60

t                                                                   61

<210>  520
<211>  61
<212>  DNA
<213>  Homo Sapiens

<400>  520
catatagaat atatttatca gggacttaaa kacaatagca tttctctctt tataagtagg      60

t                                                                      61


<210>  521
<211>  61
<212>  DNA
<213>  Homo Sapiens

<400>  521
agaattcctt tctgtttaat agtactctca rctgaactct attcttttta cattttacta      60

c                                                                      61


<210>  522
<211>  61
<212>  DNA
<213>  Homo Sapiens

<400>  522
aataatacct gcttttctca tctcaagtgg wttcagtgat tgtttatgat aatagaactt      60

g                                                                      61


<210>  523
<211>  61
<212>  DNA
<213>  Homo Sapiens

<400>  523
tcaggagaga aaaaaaatac ttgattggta yctcatgggg ctgttgtaag gaacaaatga      60

g                                                                      61


<210>  524
<211>  61
<212>  DNA
<213>  Homo Sapiens

<400>  524
gttctggcag tttctgaact cttaatgata ytttgatgac atccagttag atgctgaata      60

c                                                                      61


<210>  525
<211>  61
<212>  DNA
<213>  Homo Sapiens

<400>  525
aaagtgaaat agactataag agtggtcaac rtggaataac tgctgccaaa ccctttctgc      60

t                                                                      61


<210>  526

<211>  61
<212>  DNA
<213>  Homo Sapiens

<400>  526
atttaatctg aattgagaaa ctattggttg rctattagga gaaagagctt agaaatgggc        60

c                                                                         61


<210>  527
<211>  61
<212>  DNA
<213>  Homo Sapiens

<400>  527
cactggaatt attttctttt tgtcaagcca rtaatctccc aggcatggtg gtgtgcatct        60

g                                                                         61


<210>  528
<211>  61
<212>  DNA
<213>  Homo Sapiens

<400>  528
tcactctgct atcttctcct ggagtttaca maagcccttc agagtgtaaa caccgatgtg        60

g                                                                         61


<210>  529
<211>  61
<212>  DNA
<213>  Homo Sapiens

<400>  529
aacaacttta atagaatttt gtgtctggag yagtacgtgg caagtatttg gttactggcc        60

t                                                                         61


<210>  530
<211>  61
<212>  DNA
<213>  Homo Sapiens

<400>  530
gggacactac agctcccttg tatggtacaa mataggtgct ctgtgagtga gaaggtggat        60

g                                                                         61


<210>  531
<211>  61
<212>  DNA
<213>  Homo Sapiens

<400>  531
tttatatttt ttactgatag gcagccaagc rtacagtcac tactttgatt tgttaagcct        60

g                                                                         61

<210>  532
<211>  61
<212>  DNA
<213>  Homo Sapiens

<400>  532
taaaacctgt gctttgtaga catttactgc yttatgactg ataagaaaaa aaagcacata      60

t                                                                      61


<210>  533
<211>  61
<212>  DNA
<213>  Homo Sapiens

<400>  533
acctttgatt tctaagtgaa cttcattctt ygccatccct aatttattcc ttgcaacaca      60

c                                                                      61


<210>  534
<211>  61
<212>  DNA
<213>  Homo Sapiens

<400>  534
gcaatataag ctcaaagcct aaagtgtatc rttcacagaa aggaattcat ctccaatctc      60

a                                                                      61


<210>  535
<211>  61
<212>  DNA
<213>  Homo Sapiens

<400>  535
atggattctc ccctagaacc ttcagaaagc racgcagctc tgctcacgca ttggttttag      60

a                                                                      61


<210>  536
<211>  61
<212>  DNA
<213>  Homo Sapiens

<400>  536
tgatgaggca gtaggtgagc atcactgaga ytgtgtgagt caggccccag tcatacaaga      60

c                                                                      61


<210>  537
<211>  61
<212>  DNA
<213>  Homo Sapiens

<400>  537

atttagtttt gacaagattc aaccattcac raaaccaaaa aatgagacat tttagtggct 60

c 61

<210> 538
<211> 61
<212> DNA
<213> Homo Sapiens

<400> 538
tcacagttta agtttttact ggaaagcaaa yaagaacacc agcagtttag atagactgaa 60

a 61

<210> 539
<211> 61
<212> DNA
<213> Homo Sapiens

<400> 539
acaagatctt aatgattttt gcaatttgta rtaccagatt tatttactgt cacttaaaag 60

a 61

<210> 540
<211> 61
<212> DNA
<213> Homo Sapiens

<400> 540
aatgcagagg tgtatggttt ctacacagat kattttttat ggtttattac ttataaaaat 60

g 61

<210> 541
<211> 61
<212> DNA
<213> Homo Sapiens

<400> 541
agcaagatac cagacattga tcaaagcacc rattctataa tagcccactt taataaatta 60

c 61

<210> 542
<211> 61
<212> DNA
<213> Homo Sapiens

<400> 542
tcaatgtctt tcagtaaagt aatgcttcaa mtttcaacct acataatttg aattagaaat 60

g 61

<210> 543
<211> 61
<212> DNA

<213>  Homo Sapiens

<400>  543
gaagacagga gaagctcctc aacacaggga wagaattagt gagtaagagt cctcagggga      60

t                                                                      61


<210>  544
<211>  61
<212>  DNA
<213>  Homo Sapiens

<400>  544
ttatctgcta ctcttgtctt atttcatagc mattgccata gctggagact tcattatctc      60

a                                                                      61


<210>  545
<211>  61
<212>  DNA
<213>  Homo Sapiens

<400>  545
caatcactga cttaataact tctgtccatg ytacaccatc aaatgtgcac ataaatttat      60

t                                                                      61


<210>  546
<211>  61
<212>  DNA
<213>  Homo Sapiens

<400>  546
ttaatgcaca ggaatatgat gccgtaacaa ygactttcct ttctatggtg ggaagagata      60

g                                                                      61


<210>  547
<211>  61
<212>  DNA
<213>  Homo Sapiens

<400>  547
gctttgatcc cagccaagat aatatgttga rgttgtcttt gaatgcatgt atttgacatt      60

t                                                                      61


<210>  548
<211>  61
<212>  DNA
<213>  Homo Sapiens

<400>  548
tcaatttcta gtccgtggtt ggttgatccg ytgaagtaag tcatctgagt ggcctcgacc      60

t                                                                      61

<210>  549
<211>  61
<212>  DNA
<213>  Homo Sapiens

<400>  549
acctccttgg ttctggacat gattcttcta rcaatgcagg caaaattgca tgagcttttt      60

t                                                                      61


<210>  550
<211>  61
<212>  DNA
<213>  Homo Sapiens

<400>  550
tgtgctttga gcacccactg gccatgcatt sctcattaag gttttgcaaa actattctct      60

c                                                                      61


<210>  551
<211>  61
<212>  DNA
<213>  Homo Sapiens

<400>  551
tgccagtagc agctatccca tggtcttcac rtagttggga gactcactaa gacgaagctc      60

a                                                                      61


<210>  552
<211>  61
<212>  DNA
<213>  Homo Sapiens

<400>  552
gaagttcagt gggcactagc aaatctgcac watgcaactg gaagaaactt gtatacatag      60

c                                                                      61


<210>  553
<211>  61
<212>  DNA
<213>  Homo Sapiens

<400>  553
cagcatgccc aataattata agaagaattc rtggaaaagc aaatagggag ttggaatcat      60

t                                                                      61


<210>  554
<211>  61
<212>  DNA
<213>  Homo Sapiens

<400>  554
tggagtttga tgcttgaaag taagaacaga yacaccacgt tattagaaaa catgtatcaa      60

a                                                                    61

<210>  555
<211>  61
<212>  DNA
<213>  Homo Sapiens

<400>  555
agcattatgt gaattcttca catgttattt ycaatggtcc tccttattca atagttttaa      60

t                                                                    61

<210>  556
<211>  61
<212>  DNA
<213>  Homo Sapiens

<400>  556
attatgtaat taagtcttct atatccattc rgagcaacag caaatatctc taagtcattt      60

a                                                                    61

<210>  557
<211>  61
<212>  DNA
<213>  Homo Sapiens

<400>  557
gaaccttttc aggacaggta tgatgtgtta rgccaaggag ttgaggcagg atgctgaaag      60

c                                                                    61

<210>  558
<211>  61
<212>  DNA
<213>  Homo Sapiens

<400>  558
atttaaatat ctaaaattgc tcaataaagc rcgaaatgtc ttgataacaa tgatctttca      60

t                                                                    61

<210>  559
<211>  61
<212>  DNA
<213>  Homo Sapiens

<400>  559
cttatgttaa aataatctca tcctatctgc yttttctaaa aaattttatt tatttatttg      60

t                                                                    61

<210>  560
<211>  61
<212>  DNA
<213>  Homo Sapiens

<400>  560
cagccttggc tcttgcctag tagcgggcct ktgtagaaaa tcaaaaggga aaatgggaca      60

g                                                                      61


<210>  561
<211>  61
<212>  DNA
<213>  Homo Sapiens

<400>  561
gacaaactta aaaatcaagc gagatgcatt yccctagtaa atccctgttt tcctagtcct      60

t                                                                      61


<210>  562
<211>  61
<212>  DNA
<213>  Homo Sapiens

<400>  562
tgaaagtaaa atatatatcc gttgctcatc raagatgtca actgctttga cttttccgct      60

t                                                                      61


<210>  563
<211>  61
<212>  DNA
<213>  Homo Sapiens

<400>  563
cacagattat gcaggtggtc ctggagactg scacataatg gtgactgaga tatgaaaagc      60

a                                                                      61


<210>  564
<211>  61
<212>  DNA
<213>  Homo Sapiens

<400>  564
atatgttagt gttcttccag agctaagcat ygaagatgtg cacctttctg aaagattgca      60

g                                                                      61


<210>  565
<211>  61
<212>  DNA
<213>  Homo Sapiens

<400>  565
ctcatatatt cctaaaatga ctgaaactcc rtcgtcgttt ttcttcattc cttcattcaa      60

g                                                                      61


<210>  566
<211>  61

<212>  DNA
<213>  Homo Sapiens

<400>  566
aagaactcat tgcatttctt gtttttacca ytcatttgac atatctaata gttatatttt      60

t                                                                     61


<210>  567
<211>  61
<212>  DNA
<213>  Homo Sapiens

<400>  567
aaggacttat ttacagatgc agtgaaggaa sacgtttgaa atgctccaat tcttcaagtt      60

g                                                                     61


<210>  568
<211>  61
<212>  DNA
<213>  Homo Sapiens

<400>  568
tgctgaccta acgtaaagag attagaatcc raggaaattg tggtacagct gacttctact      60

g                                                                     61


<210>  569
<211>  61
<212>  DNA
<213>  Homo Sapiens

<400>  569
ttaaaattgt ggtaagtttt tatcgctcac ktaacatgtc accagtttct cccgcctcca      60

c                                                                     61


<210>  570
<211>  61
<212>  DNA
<213>  Homo Sapiens

<400>  570
tggggattta gcctacagaa tattttgtca mccagttaag aaacatagta cccgatgggt      60

a                                                                     61


<210>  571
<211>  61
<212>  DNA
<213>  Homo Sapiens

<400>  571
tcatggtatt tattccattc ttggcttact matcttctct cttattcttt atgaagtcct      60

t                                                                     61

<210> 572
<211> 61
<212> DNA
<213> Homo Sapiens

<400> 572
ccctgggctg agtgccttcc ctttgaccaa ygctgaagcc ttggaaattt atagaaatga 60

t 61


<210> 573
<211> 61
<212> DNA
<213> Homo Sapiens

<400> 573
actagataat tatcaccaac aaagcattcc rgaaaaacca gaacaatcaa aaggattaca 60

t 61


<210> 574
<211> 61
<212> DNA
<213> Homo Sapiens

<400> 574
cgaagtgata ggcattcaat acagaggaaa ygcagaggac agtgcaatca atcactgcca 60

a 61


<210> 575
<211> 61
<212> DNA
<213> Homo Sapiens

<400> 575
gcggaaacct gccacaatcc ctagtcaaca ytgcctgaga agcaccattt ggcctccctg 60

g 61


<210> 576
<211> 61
<212> DNA
<213> Homo Sapiens

<400> 576
cagctgagga tggactcacg agtcccatat rtagagcttc acaagcacta gggaaaggct 60

g 61


<210> 577
<211> 61
<212> DNA
<213> Homo Sapiens

<400> 577
aaaaagacaa aaaaaacaac aacacagcat sgattcatag aagttgtggg atacgtcacg 60

t                                                                   61

<210>  578
<211>  61
<212>  DNA
<213>  Homo Sapiens

<400>  578
ctaggcacag tacactttga tcgttttccc raaaccgcgt tggacaccat ttttccatga      60

t                                                                   61

<210>  579
<211>  61
<212>  DNA
<213>  Homo Sapiens

<400>  579
gaaattgcta atgggatcta ttgcggattc stcacctaag ggaaagggaa atatagctag      60

g                                                                   61

<210>  580
<211>  61
<212>  DNA
<213>  Homo Sapiens

<400>  580
tctcagactt agggatgtct ttctttggat raactattta cttccttcat tttctttact      60

t                                                                   61

<210>  581
<211>  61
<212>  DNA
<213>  Homo Sapiens

<400>  581
attatttatt aaaaactgga ttccagcaaa matgcattct aaacaatgat ggataatttt      60

t                                                                   61

<210>  582
<211>  61
<212>  DNA
<213>  Homo Sapiens

<400>  582
tcataatagg gtgattatga ggattgcatc mggtaacttt gtaaaggttc tggaacatat      60

a                                                                   61

<210>  583
<211>  61
<212>  DNA
<213>  Homo Sapiens

<400>  583
catggcagcc tcagtttcct ggctcagtca rtaatcccac ctcagcctcc taagtagctg      60

a                                                                      61


<210>  584
<211>  61
<212>  DNA
<213>  Homo Sapiens

<400>  584
aaatcatccc tgccattttt cctattcaaa yatgttctgg tatctactaa cacaactgta      60

t                                                                      61


<210>  585
<211>  61
<212>  DNA
<213>  Homo Sapiens

<400>  585
ttcagtggtt tgaattttct ttgaacccag sagaagtgaa cagatgtgga ttttacaatt      60

t                                                                      61


<210>  586
<211>  61
<212>  DNA
<213>  Homo Sapiens

<400>  586
aaggggatgt ttcagtttga tttcagggct staaaaaatg gtgaagccat taaaagagac      60

a                                                                      61


<210>  587
<211>  61
<212>  DNA
<213>  Homo Sapiens

<400>  587
acaactcttt cttcaggaaa cctttgactc rtggtttata catacttttg tattaagttg      60

t                                                                      61


<210>  588
<211>  61
<212>  DNA
<213>  Homo Sapiens

<400>  588
aagtcattct gcctctctgt gactgaatca ratgaagcct aatgatgctt ataaagttgt      60

t                                                                      61


<210>  589

<211>  61
<212>  DNA
<213>  Homo Sapiens

<400>  589
ccagcggcag cactgcgatt gctctgagca rgtgccgcat caacccctca tgtccctgcc      60

g                                                                      61


<210>  590
<211>  61
<212>  DNA
<213>  Homo Sapiens

<400>  590
ttttcttcat tcagtctacg gtcaatgggc mtttaggtct ctgctgctgt gaatagttct      60

g                                                                      61


<210>  591
<211>  61
<212>  DNA
<213>  Homo Sapiens

<400>  591
aatagaaacc aacttaattg ccctttagct yatgtgactt gagtaattct ttaaaaataa      60

a                                                                      61


<210>  592
<211>  61
<212>  DNA
<213>  Homo Sapiens

<400>  592
ctgaacacaa agcctcagga gtgataaagc ygtcacggag taaaacgcac catcctctcc      60

t                                                                      61


<210>  593
<211>  61
<212>  DNA
<213>  Homo Sapiens

<400>  593
gctctccaag ctggcaccaa ttatagtaca kggaacccaa tttccaacca aaagacctcc      60

a                                                                      61


<210>  594
<211>  61
<212>  DNA
<213>  Homo Sapiens

<400>  594
ccaccctcca ccatgagggg tgatttcaac maaccaggaa acacagtcca tgatcaggaa      60

a                                                                      61

<210>  595
<211>  61
<212>  DNA
<213>  Homo Sapiens

<400>  595
caacctcttg ggggccctgg ctggagacct sttcctgctt caacccatgg gaagcgctgc        60

a                                                                         61


<210>  596
<211>  61
<212>  DNA
<213>  Homo Sapiens

<400>  596
acgcatgcca catgcacacc cctaacttaa rgcctagctc caagtttctg tcacaaccaa        60

g                                                                         61


<210>  597
<211>  61
<212>  DNA
<213>  Homo Sapiens

<400>  597
acataaaaat actcattgaa gcaattcttt yagcatagat gggcaacatg cggaggttat        60

t                                                                         61


<210>  598
<211>  61
<212>  DNA
<213>  Homo Sapiens

<400>  598
gtagtgtggt attaaaatct aggacttcgg ratgcaatag cagtgctcag tttatttgtt        60

t                                                                         61


<210>  599
<211>  61
<212>  DNA
<213>  Homo Sapiens

<400>  599
agttaaaggt ctaattttct tctgtgatgg ygacctcatc gctttgtaaa atacacgatg        60

c                                                                         61


<210>  600
<211>  61
<212>  DNA
<213>  Homo Sapiens

<400>  600

caacacctgt gagattttcc attctaaata yggagcttaa tctatggggc atggccaacc 60

a 61

<210> 601
<211> 61
<212> DNA
<213> Homo Sapiens

<400> 601
atatggatgg accccacaga cttaaggttg saggaaagaa gctggacaaa caggaccttc 60

c 61

<210> 602
<211> 61
<212> DNA
<213> Homo Sapiens

<400> 602
tcctctgtgg gaaaatgcca gggctgggta ygcatagtcc atgttgcaga ttcacatatg 60

a 61

<210> 603
<211> 61
<212> DNA
<213> Homo Sapiens

<400> 603
tcctctcagt ctacagtccg tgtctttgtt yccgtggact gaatgtctag cagagccttc 60

t 61

<210> 604
<211> 61
<212> DNA
<213> Homo Sapiens

<400> 604
acctaccacc gtgtatgtac tagatagaac ractgtcctt caactttatc cccgaatgaa 60

c 61

<210> 605
<211> 61
<212> DNA
<213> Homo Sapiens

<400> 605
gggtatataa ttactgctca gtgaaagtga ytatgcatac ctgtatcatc taaaggaatt 60

t 61

<210> 606
<211> 61
<212> DNA

<213>  Homo Sapiens

<400>  606
cgctgtgaag gtgcagtccc tgccaccccc ygctcttctc ctttgaaatc acacatctat        60

c                                                                         61


<210>  607
<211>  61
<212>  DNA
<213>  Homo Sapiens

<400>  607
agaagaagaa gctcaaggac atactcacac rtacaagacc gatctggtgt ctcccacctt        60

c                                                                         61


<210>  608
<211>  61
<212>  DNA
<213>  Homo Sapiens

<400>  608
taccaaagga aagccccttc aagttaccgt waagacagaa gaaaaggaag aaaaatataa        60

a                                                                         61


<210>  609
<211>  61
<212>  DNA
<213>  Homo Sapiens

<400>  609
gagtgtctta cttctgcaag cctgctttat rgtgagcaaa gcatcaccag caagtgatca        60

c                                                                         61


<210>  610
<211>  61
<212>  DNA
<213>  Homo Sapiens

<400>  610
agaagaacag tctggaaggt actggtaaga rtgactcaag gagaggagag ttctgtccat        60

t                                                                         61


<210>  611
<211>  61
<212>  DNA
<213>  Homo Sapiens

<400>  611
tacagtcaga atgatgtttc tcaaattcta ygtctggctt aaagttctca gtgatttcct        60

a                                                                         61

<210>  612
<211>  61
<212>  DNA
<213>  Homo Sapiens

<400>  612
gctgtagaat acgtaatgat taagttttta ygtgctttta gatgatagct aagtcctgta      60

a                                                                      61


<210>  613
<211>  61
<212>  DNA
<213>  Homo Sapiens

<400>  613
gagatcagca tttgcatttt aggacaataa mtagctgcat ggtctctttg ccttgatgag      60

a                                                                      61


<210>  614
<211>  61
<212>  DNA
<213>  Homo Sapiens

<400>  614
tgcagatcac aataattctt agaaggaaat rctctcgcaa aactaagacc ctgccaatca      60

t                                                                      61


<210>  615
<211>  61
<212>  DNA
<213>  Homo Sapiens

<400>  615
tcaaaacatt ttaggaaaac gtaactttaa yttcaggact aaccagcagg tattagtttt      60

a                                                                      61


<210>  616
<211>  61
<212>  DNA
<213>  Homo Sapiens

<400>  616
ctgcctggtt ggaatagtgt gcgcccgctg ygaggggaag aaaaagaaca gtagagggac      60

a                                                                      61


<210>  617
<211>  61
<212>  DNA
<213>  Homo Sapiens

<400>  617
gatgattttc agggtaatca tgtatctagg ratccgcagt gcaacttaga agtcaaattg      60

c 61

<210> 618
<211> 61
<212> DNA
<213> Homo Sapiens

<400> 618
ttcacagagg ccctcacgac aaagaattat ycacaccaaa atgttaatgc tgctgcgctc 60

g 61

<210> 619
<211> 61
<212> DNA
<213> Homo Sapiens

<400> 619
ggtagatccc tcatgaatgg cttgatgcca ygcttatgat aatgaggtag ttcttgcttt 60

a 61

<210> 620
<211> 61
<212> DNA
<213> Homo Sapiens

<400> 620
acccctcctg cccccaacac ctgacgaaat sccgccttca agcctaaaac agcatgaggg 60

a 61

<210> 621
<211> 61
<212> DNA
<213> Homo Sapiens

<400> 621
tgtgtgtggt tgtggcagat tggataaaaa rcttctttag acccttggta acaataatgg 60

t 61

<210> 622
<211> 61
<212> DNA
<213> Homo Sapiens

<400> 622
gaattgactt ctctagccct gacacaagga ygcagtgatt agaatgctca gtacaatgta 60

t 61

<210> 623
<211> 61
<212> DNA
<213> Homo Sapiens

<400>  623
gcaagatagg atgtgggctg cagaaacaat yggggagaca tgagcctggt taaatattca      60

g                                                                      61


<210>  624
<211>  61
<212>  DNA
<213>  Homo Sapiens

<400>  624
ctctaaaaga aggcattcac tgtttaacca ygttggtgta cagttacatc agacagtctt      60

c                                                                      61


<210>  625
<211>  61
<212>  DNA
<213>  Homo Sapiens

<400>  625
tcagcctttc tcttacagct aaaagtgatc rtggcacgac acaggggcaa tgagctctaa      60

c                                                                      61


<210>  626
<211>  61
<212>  DNA
<213>  Homo Sapiens

<400>  626
tctcttagct caggcgtgaa gaaattcaga scacaggtag attaattaac tccaggaagg      60

a                                                                      61


<210>  627
<211>  61
<212>  DNA
<213>  Homo Sapiens

<400>  627
cactacatgt ggtgaagaca aacggttatc yttcagtggc ttaatgttag ggcaacaaag      60

a                                                                      61


<210>  628
<211>  61
<212>  DNA
<213>  Homo Sapiens

<400>  628
gcagaagatc accttagaga aaatccgaac ygactctcct atcttagaag tcatccaggc      60

t                                                                      61


<210>  629
<211>  61

<212>  DNA
<213>  Homo Sapiens

<400>  629
ttctatatca cctaggactt caactgtgac yaaggaattc attatgatgg gtaaaaaggc      60

a                                                                     61


<210>  630
<211>  61
<212>  DNA
<213>  Homo Sapiens

<400>  630
ataatttaac agtttctcac ataacaaaac rtactgttac catccaaatc ttattccatg      60

g                                                                     61


<210>  631
<211>  61
<212>  DNA
<213>  Homo Sapiens

<400>  631
aaagaaaaga aaaagaaaaa tgcagtacaa rataagtgtt agaacatatg gagaagatag      60

t                                                                     61


<210>  632
<211>  61
<212>  DNA
<213>  Homo Sapiens

<400>  632
cggacaggac ttcttctgag ctttttcaat wcagagctag attctcaggc cctctcccca      60

a                                                                     61


<210>  633
<211>  61
<212>  DNA
<213>  Homo Sapiens

<400>  633
tttggtgcct ggaatgcatt ccccaggttt ygctgaggtt cccatctcag gcctccaccc      60

a                                                                     61


<210>  634
<211>  61
<212>  DNA
<213>  Homo Sapiens

<400>  634
atcagcacaa tagggtgact aagtattacc rcagtacaag aatttggaca aagttgattt      60

g                                                                     61

<210>  635
<211>  61
<212>  DNA
<213>  Homo Sapiens

<400>  635
tttatctcca ctttagagat ggggtgtaac maacaaaatg cagtaacaac aaatgtcacc      60

t                                                                      61


<210>  636
<211>  61
<212>  DNA
<213>  Homo Sapiens

<400>  636
taagtttatt aatattatgg aagtgacaat yttaggcccc tttcggaggg tacatctgga      60

g                                                                      61


<210>  637
<211>  61
<212>  DNA
<213>  Homo Sapiens

<400>  637
gaaatgaagg caagtttggc aagaggttgc rtggaactga atcccatctt gcagagccat      60

t                                                                      61


<210>  638
<211>  61
<212>  DNA
<213>  Homo Sapiens

<400>  638
aaagatggag caaatacgtg agacactagc ratctttagg aacagcgctg agttgtcttt      60

t                                                                      61


<210>  639
<211>  61
<212>  DNA
<213>  Homo Sapiens

<400>  639
tacaaaataa attaacgtgt cagaaaatca raaacctgtg aagatagtgt ttttaggaca      60

c                                                                      61


<210>  640
<211>  61
<212>  DNA
<213>  Homo Sapiens

<400>  640
atgcatcttg ttctcattct tgtttgtcca ygaattggct gtataaccat ttaactttcc      60

a                                                                   61

<210>  641
<211>  61
<212>  DNA
<213>  Homo Sapiens

<400>  641
aaaccatggt acttcctggc aggcattctt ygctagattt tggagttagt ttttcttcaa      60

g                                                                   61

<210>  642
<211>  61
<212>  DNA
<213>  Homo Sapiens

<400>  642
accgttctcc atagtggttg taatgattta ygtttccacc aacagtgtac agggtttccc      60

t                                                                   61

<210>  643
<211>  61
<212>  DNA
<213>  Homo Sapiens

<400>  643
ttttcaccaa agtaatagac aaactttatc magctgagtg atcttattta taactcccat      60

g                                                                   61

<210>  644
<211>  61
<212>  DNA
<213>  Homo Sapiens

<400>  644
cactcaaggg gctgttgaaa agattaatga sattgtgtat acaaaattcc agccaccata      60

g                                                                   61

<210>  645
<211>  61
<212>  DNA
<213>  Homo Sapiens

<400>  645
cactttagtt caataaggat actggccatt rttcaagaac tggtcttagt cacacacaca      60

a                                                                   61

<210>  646
<211>  61
<212>  DNA
<213>  Homo Sapiens

<400>  646
ggccttgaac cagatgtttg acaacagcaa rgaaaatgag atggaaactt gggaagccag      60

c                                                                      61


<210>  647
<211>  61
<212>  DNA
<213>  Homo Sapiens

<400>  647
tgtatctctt ctctgtggct gctaaaagct ragaaagcct ccatctgtta ctaattcttg      60

t                                                                      61


<210>  648
<211>  61
<212>  DNA
<213>  Homo Sapiens

<400>  648
atttgactag gcaacatctg gagactagaa mgagataatc aaagacatgt tagtaacgga      60

t                                                                      61


<210>  649
<211>  61
<212>  DNA
<213>  Homo Sapiens

<400>  649
ttagcactta ttacattgta atatcagtat ytgcttacat atctatcttt ttcatgagac      60

t                                                                      61


<210>  650
<211>  61
<212>  DNA
<213>  Homo Sapiens

<400>  650
tgtagatatc agaatggcta agtattttag yagcaatagt cagcacagaa gtaactggat      60

c                                                                      61


<210>  651
<211>  61
<212>  DNA
<213>  Homo Sapiens

<400>  651
ttattgttct cccgtcctca ttattctctg kctaggctca acctaggtca ccctgctata      60

c                                                                      61


<210>  652

<211>  61
<212>  DNA
<213>  Homo Sapiens

<400>  652
atgacatttt cacattggtt gctggttata yatgtgcctc caaaatcagt aatctccaat        60

t                                                                         61


<210>  653
<211>  61
<212>  DNA
<213>  Homo Sapiens

<400>  653
tacttcctta ttattctggc actagtcaag wtagaccttc aaacaatgtg aactaatttg        60

t                                                                         61


<210>  654
<211>  61
<212>  DNA
<213>  Homo Sapiens

<400>  654
aactggcttg aaggtaacat ggtaaatgca rtttggatgt attgagtata aagtgactca        60

a                                                                         61


<210>  655
<211>  61
<212>  DNA
<213>  Homo Sapiens

<400>  655
aacaccaggt taccatccca gtttgtctta yaagattaac acaaaagaga accctatata        60

c                                                                         61


<210>  656
<211>  61
<212>  DNA
<213>  Homo Sapiens

<400>  656
cgggcactgg agttgaaatg cttggtctta ratctgaatt ctgtcactgc tgagcttgtg        60

g                                                                         61


<210>  657
<211>  61
<212>  DNA
<213>  Homo Sapiens

<400>  657
ataagccact ctcttcataa gaccatacca rttttctaat aaagctcaca cagatttcgt        60

g                                                                         61

<210>  658
<211>  61
<212>  DNA
<213>  Homo Sapiens

<400>  658
gttttacaat aggaaaagaa aatggtatgc yaagtggcct cttgtttctg aaatttttgg      60

c                                                                      61


<210>  659
<211>  61
<212>  DNA
<213>  Homo Sapiens

<400>  659
tctgacttcc aaaagatgca tcataacagc ktaggtggtt tttatgacat taaactactt      60

t                                                                      61


<210>  660
<211>  61
<212>  DNA
<213>  Homo Sapiens

<400>  660
tacagggtac aataaaacaa ataaccagag mtttttatat acatgtaaaa gggcactgca      60

a                                                                      61


<210>  661
<211>  61
<212>  DNA
<213>  Homo Sapiens

<400>  661
ctttggtaaa catgcagata tttttctggc wtaaggtacc tcagaatcaa ttgattaatc      60

t                                                                      61


<210>  662
<211>  61
<212>  DNA
<213>  Homo Sapiens

<400>  662
gtcaaccaaa gaggcaatat acttttgccc ycttggattt tacattctat caggtgaaga      60

c                                                                      61


<210>  663
<211>  61
<212>  DNA
<213>  Homo Sapiens

<400>  663

tgggagaaag aaagtcagtt tacttctgga wacatgtgtc tagtgtatat agctgaataa 60

t 61

<210> 664
<211> 61
<212> DNA
<213> Homo Sapiens

<400> 664
acaacggcat aatcgacagt gtcaaaagag wtaaggagat tggagacaac tgaggtatta 60

g 61

<210> 665
<211> 61
<212> DNA
<213> Homo Sapiens

<400> 665
tacaaagaag ctagaatgta cataagaaac wagcacacac cttggaaaac agttagaaat 60

c 61

<210> 666
<211> 61
<212> DNA
<213> Homo Sapiens

<400> 666
catacagggt ggtaagtatt tgctaatgta ytggactcct ccactagcct gtgaaatttt 60

t 61

<210> 667
<211> 61
<212> DNA
<213> Homo Sapiens

<400> 667
tattcgctta aagaatacca gttaccatcc rtatgctttc ggtactaaat ctccatttat 60

c 61

<210> 668
<211> 61
<212> DNA
<213> Homo Sapiens

<400> 668
aagatggacc aaaattgcac agtctcaaaa ygatcttaac ttctgtgaaa tcagtgcctt 60

t 61

<210> 669
<211> 61
<212> DNA

<213>  Homo Sapiens

<400>  669
tttattgaat atttctggaa ttacactaac raagttccat ctacaatctt taccataaaa      60

g                                                                      61


<210>  670
<211>  61
<212>  DNA
<213>  Homo Sapiens

<400>  670
tgcgttttcg gcactgaaaa gtagcatcac ygtagtaaaa tggaaaagat gtgttttatg      60

a                                                                      61


<210>  671
<211>  61
<212>  DNA
<213>  Homo Sapiens

<400>  671
tggttttcaa actttctcaa attttgtagt wtcatcaaca gctatcattt ttgattgctc      60

t                                                                      61


<210>  672
<211>  61
<212>  DNA
<213>  Homo Sapiens

<400>  672
gatcacactt tcttaatccg ttcatacatc wttggacact tagagtcgat tacttgacta      60

t                                                                      61


<210>  673
<211>  61
<212>  DNA
<213>  Homo Sapiens

<400>  673
taaagagaat attatatata gctgctatca raatttaaca aagactgcat ctgaggttgc      60

a                                                                      61


<210>  674
<211>  61
<212>  DNA
<213>  Homo Sapiens

<400>  674
cttccacatt cacctccttt tattatgcat yccttcattt aatcagccat ccttgcatgt      60

a                                                                      61

<210>  675
<211>  61
<212>  DNA
<213>  Homo Sapiens

<400>  675
tttattgtca aatcacatag ataagaaatg rtaagtccat gatttctatc tgaaaccttc      60

a                                                                      61


<210>  676
<211>  61
<212>  DNA
<213>  Homo Sapiens

<400>  676
tgtcaagaag gtagagagta tgacatttga saacccttga aagtagttca gtgggccttc      60

a                                                                      61


<210>  677
<211>  61
<212>  DNA
<213>  Homo Sapiens

<400>  677
attcccttgt ggagggactt ccctgactaa ygctcttggc ctctctcctg ccctccattt      60

g                                                                      61


<210>  678
<211>  61
<212>  DNA
<213>  Homo Sapiens

<400>  678
gggctggtaa atgcacactg gctgaagtcc ygaaagctat tttctggcca cttggcactt      60

g                                                                      61


<210>  679
<211>  61
<212>  DNA
<213>  Homo Sapiens

<400>  679
ggcccccaaa cacatgctgc tgggcatcag sgtgtctaca ataaaaactc atagagggaa      60

t                                                                      61


<210>  680
<211>  61
<212>  DNA
<213>  Homo Sapiens

<400>  680
gatactgtac atttgtgagt cagtcacgca ytacatactc tgggcttcat atctcgtaag      60

a                                                                   61

<210>  681
<211>  61
<212>  DNA
<213>  Homo Sapiens

<400>  681
ggtgtgcaca tcttgcagca gagatgcaac rtgtccaggt ttccactgcc agaatacaga      60

t                                                                   61

<210>  682
<211>  61
<212>  DNA
<213>  Homo Sapiens

<400>  682
acctgtcaca ggccatttac cctggaggta mtggccacct gagcgtgtgt gtgtgtgttt      60

g                                                                   61

<210>  683
<211>  61
<212>  DNA
<213>  Homo Sapiens

<400>  683
ttcaccttgg ttctctgaca cccatggaaa rtttaggtta gggagagaga gtggaaagag      60

a                                                                   61

<210>  684
<211>  61
<212>  DNA
<213>  Homo Sapiens

<400>  684
cattgttctc cagggcaaag cccatgacct sgatgataga gtagactgca ttttgattta      60

t                                                                   61

<210>  685
<211>  61
<212>  DNA
<213>  Homo Sapiens

<400>  685
acaacactgt ggagttccag ggccttagtg magtacagga tttggactaa atccttggtc      60

t                                                                   61

<210>  686
<211>  61
<212>  DNA
<213>  Homo Sapiens

<400>  686
agtgaccctg cagacctgag cccaagggat rctccacaag gagaagacat gctgaacgcc      60

a                                                                      61


<210>  687
<211>  61
<212>  DNA
<213>  Homo Sapiens

<400>  687
gcacatcctc agtgtcattt tacactaaca yaacagtgtc atggctggaa gatctaagga      60

g                                                                      61


<210>  688
<211>  61
<212>  DNA
<213>  Homo Sapiens

<400>  688
aggtttttaa acactgtaag atgattccag wtttttagat agctcagagg gccgtgggga      60

c                                                                      61


<210>  689
<211>  61
<212>  DNA
<213>  Homo Sapiens

<400>  689
ataaagttct taagagagct aaatgagcct raatccattc ataggaggaa aacaagcaca      60

c                                                                      61


<210>  690
<211>  61
<212>  DNA
<213>  Homo Sapiens

<400>  690
caaataggtc ctgctgggcc agctgacctt ygtaatgaac tcaggggcga tttacttagc      60

a                                                                      61


<210>  691
<211>  61
<212>  DNA
<213>  Homo Sapiens

<400>  691
aattcagcct gaggtatata tacactagga yacctgttta ttggatgtat ttaagacaag      60

g                                                                      61


<210>  692
<211>  61

<212>  DNA
<213>  Homo Sapiens

<400>  692
ttgctacttt tgcctcccag cagtctggta ycaacacagc atccaatgca acactgttat      60

a                                                                     61


<210>  693
<211>  61
<212>  DNA
<213>  Homo Sapiens

<400>  693
attgcctgtc caaagcacag tgcctggaac wagcaaataa tagttagatg gatgaaagta      60

t                                                                     61


<210>  694
<211>  61
<212>  DNA
<213>  Homo Sapiens

<400>  694
   aggttcttgt aatgaacatt cacatggccc rcatcttaac catgcactca ttttttacag      60
   c                                                                     61


<210>  695
<211>  61
<212>  DNA
<213>  Homo Sapiens

<400>  695
tttatataat tatttattaa caaataagag rtaaaagagc agcaagaaac gagagtcaaa      60

c                                                                     61


<210>  696
<211>  61
<212>  DNA
<213>  Homo Sapiens

<400>  696
ttcctctctc caacatagtg ccagactctc rtattaggaa atgatgaaac catcaaccaa      60

a                                                                     61


<210>  697
<211>  61
<212>  DNA
<213>  Homo Sapiens

<400>  697
aaaaaaaaac aaaaaaaaag aatatttcct sccaaccaat agcaacgatc cccaccctca      60

g                                                                     61

<210>  698
<211>  61
<212>  DNA
<213>  Homo Sapiens

<400>  698
gtgtgtgatg ttcttgcttt gacaattcac ractcaccag gaaacactgt cgtcaggtgt      60

a                                                                     61


<210>  699
<211>  61
<212>  DNA
<213>  Homo Sapiens

<400>  699
gcattattca cagaacccac tgcttctaca rggtttgttt cctgatgttt ctagctgctc      60

c                                                                     61


<210>  700
<211>  61
<212>  DNA
<213>  Homo Sapiens

<400>  700
gcgattaaag ctttggcaat gggcgttgac rtcgccagat tgaaactagt ggtggggaaa      60

a                                                                     61


<210>  701
<211>  61
<212>  DNA
<213>  Homo Sapiens

<400>  701
tccacagtgg ctcccgggtc ttgctctgcc yatgttacag attaagagca atccccatgt      60

t                                                                     61


<210>  702
<211>  61
<212>  DNA
<213>  Homo Sapiens

<400>  702
cagtctttgg ttattccatt gtgtgttgaa matttacata tttttgtcgc ctaaggatta      60

a                                                                     61


<210>  703
<211>  61
<212>  DNA
<213>  Homo Sapiens

<400>  703
acctcaagga ggggcttgcc aagatggtca ygtcaattaa taaaagagct ggattcgaat      60

c                                                                   61

<210>  704
<211>  61
<212>  DNA
<213>  Homo Sapiens

<400>  704
aacagcacag gtttgcagag ttctccagga yggcagataa caaccagagc acgtgtaccc      60

a                                                                   61

<210>  705
<211>  61
<212>  DNA
<213>  Homo Sapiens

<400>  705
gggatgatac aaccacctac tgagcagtta ygaatgggtt ctaaagctgg ggcacagcac      60

c                                                                   61

<210>  706
<211>  61
<212>  DNA
<213>  Homo Sapiens

<400>  706
cagccggccg ggatgcccac cttgtgctgc ygcgtctccg gatgatcttg gtgcggctct      60

t                                                                   61

<210>  707
<211>  61
<212>  DNA
<213>  Homo Sapiens

<400>  707
ttaagaagct gatttgacac tttggattat stgtagttct tcttccctcc attgcagtga      60

a                                                                   61

<210>  708
<211>  61
<212>  DNA
<213>  Homo Sapiens

<400>  708
caaatcagat attaggatct tgaacctaaa ytgtgaatct ctttgtgcct ttaaccacat      60

c                                                                   61

<210>  709
<211>  61
<212>  DNA
<213>  Homo Sapiens

<400>  709
tcagataaac taaaaacact ttctgagagt rtccatgtcc taaaggcttt aagtacttct      60

a                                                                      61


<210>  710
<211>  61
<212>  DNA
<213>  Homo Sapiens

<400>  710
ggagcagatg ttcaggtctg aatccagttt yttctagaca cctctttccg gatcaccccc      60

t                                                                      61


<210>  711
<211>  61
<212>  DNA
<213>  Homo Sapiens

<400>  711
agatataaat atatctacat ctatgactat rtctacatat cttccaaaaa agtcatcaaa      60

a                                                                      61


<210>  712
<211>  61
<212>  DNA
<213>  Homo Sapiens

<400>  712
tttctctttg tctctcttct gttgtagccc ytccctcacc gctaagcctt caccctgact      60

c                                                                      61


<210>  713
<211>  61
<212>  DNA
<213>  Homo Sapiens

<400>  713
ttttatcata tttttattta aggtggaatg rcatttttcc aaaaaagcaa agatgacaaa      60

c                                                                      61


<210>  714
<211>  61
<212>  DNA
<213>  Homo Sapiens

<400>  714
tcaccactta cttgagcttt cctcagtctc rtcttctttt tcctccactt tgatttgtac      60

t                                                                      61


<210>  715

<211>  61
<212>  DNA
<213>  Homo Sapiens

<400>  715
aaaaaaacct atgtctagat tagagtcagg kgcggtctcc ttattgtcag tgtctgcttt      60

c                                                                     61


<210>  716
<211>  61
<212>  DNA
<213>  Homo Sapiens

<400>  716
tacaaatata tgtggtttta ggcaagtctc sattttgaat tacgtgcaaa attatatttt      60

a                                                                     61


<210>  717
<211>  61
<212>  DNA
<213>  Homo Sapiens

<400>  717
gaaaagcttt ttattttctt ttttatgtca rtcttctatg accatgtctt acttttataa      60

a                                                                     61


<210>  718
<211>  56
<212>  DNA
<213>  Homo Sapiens

<400>  718
agatcagtag atactcaaat ctttatccta yagggagtgc tgacattggc atgaag          56


<210>  719
<211>  61
<212>  DNA
<213>  Homo Sapiens

<400>  719
tataatatac aaaatatact aaaggcacct raaattatca tgaaaagata taaaatataa      60

a                                                                     61


<210>  720
<211>  61
<212>  DNA
<213>  Homo Sapiens

<400>  720
aaagagtaaa ctttccacgt ggagtctttt ytgttgactt atgacctaag tagctgggta      60

g                                                                     61

<210>  721
<211>  61
<212>  DNA
<213>  Homo Sapiens

<400>  721
gcagagccac tgaatttagt gattggtaac raaatagtta aattgagagt gctagaatgt      60

c                                                                     61


<210>  722
<211>  61
<212>  DNA
<213>  Homo Sapiens

<400>  722
ccgtaaaacc ctgcgaatgt actaaaaacc rctgaaatgt acactttaat gcgggaatta      60

t                                                                     61


<210>  723
<211>  61
<212>  DNA
<213>  Homo Sapiens

<400>  723
aaagccaatt caagtgattc ctgcaaaagg yatattgtgc ttccaggcat catgcactca      60

a                                                                     61


<210>  724
<211>  61
<212>  DNA
<213>  Homo Sapiens

<400>  724
accagttgac atatttttcc atacatactc ygaaggtgcc atcactaatt aatgcttttg      60

t                                                                     61


<210>  725
<211>  61
<212>  DNA
<213>  Homo Sapiens

<400>  725
taagtttctt aagttcaaga ttgccaatca sgtcctaaaa cttgtattta ttcatacagc      60

t                                                                     61


<210>  726
<211>  61
<212>  DNA
<213>  Homo Sapiens

<400>  726
atttaataac ttagtaaagt tcaaaattgt sagttactta ttaaacataa acatgtgaca      60

a                                                                   61

<210>  727
<211>  61
<212>  DNA
<213>  Homo Sapiens

<400>  727
tttagcattt caggatgcag gacttccgct maaacacaaa tttgggaagg tcagtaacaa      60

a                                                                   61

<210>  728
<211>  61
<212>  DNA
<213>  Homo Sapiens

<400>  728
tatttctcag ctatgagatt ttccgtggcc rccttttcac agtagccagc tgggattgtc      60

c                                                                   61

<210>  729
<211>  61
<212>  DNA
<213>  Homo Sapiens

<400>  729
accttagcct tggacttcta gtttcctgaa ytgtgagaaa atagatttct attgttgaaa      60

c                                                                   61

<210>  730
<211>  61
<212>  DNA
<213>  Homo Sapiens

<400>  730
attaaaccag tctgcatcag ctacagtgta mgtctcagaa acagatctga aaccccagct      60

g                                                                   61

<210>  731
<211>  61
<212>  DNA
<213>  Homo Sapiens

<400>  731
ttggttgcat ttgaggtgac atcctctaat yttcctaagc acagtggggc ctcctggaaa      60

c                                                                   61

<210>  732
<211>  61
<212>  DNA
<213>  Homo Sapiens

<400>  732
tccttcattg tagggagaaa aagttttaga rggaggatac tgcagaatgg agtttctgat        60

g                                                                         61

<210>  733
<211>  61
<212>  DNA
<213>  Homo Sapiens

<400>  733
gtaatataat gtaaaggaaa aagaggaaaa raagttctgc taccatgtta aaaaaatcat        60

g                                                                         61

<210>  734
<211>  61
<212>  DNA
<213>  Homo Sapiens

<400>  734
aaaggaatga taagaaccac ttcattttga raggattact ctgattgcga tgcaacagat        60

g                                                                         61

<210>  735
<211>  61
<212>  DNA
<213>  Homo Sapiens

<400>  735
tttggaatat tacagaggtt aaatgatgag ragtactagc tggtcaagaa gaggagaggc        60

a                                                                         61

<210>  736
<211>  61
<212>  DNA
<213>  Homo Sapiens

<400>  736
ttgttactgt actctgcaca ttgcttcctc rcttactgta cattaaaagt tttcccttga        60

t                                                                         61

<210>  737
<211>  61
<212>  DNA
<213>  Homo Sapiens

<400>  737
tcctttcctc ttctgttttc cagggtctta ygaatgcgga atctgtggca agaagtacaa        60

g                                                                         61

<210>  738
<211>  61

<212>  DNA
<213>  Homo Sapiens

<400>  738
ctttctccat ggtgatagac agaaaaatgc yatttcccac ttgactatta acaattagag      60

a                                                                     61

<210>  739
<211>  61
<212>  DNA
<213>  Homo Sapiens

<400>  739
agcctgggct atataggtca ttcagtaccc rccatagagc cagatcttgt gtggagagag      60

a                                                                     61

<210>  740
<211>  61
<212>  DNA
<213>  Homo Sapiens

<400>  740
ggccttcttt ccactgactg ccagatgagc ytgcgtttca tgttatctct gtagaacatt      60

t                                                                     61

<210>  741
<211>  61
<212>  DNA
<213>  Homo Sapiens

<400>  741
gaactctatt gccaggacaa atgaagcatt yccacataag cttctggcta ggatcctcgg      60

t                                                                     61

<210>  742
<211>  61
<212>  DNA
<213>  Homo Sapiens

<400>  742
cctaaagttc tccttggctc ctcctcgctc scatctagaa cccgcctagg aggtaatcac      60

t                                                                     61

<210>  743
<211>  61
<212>  DNA
<213>  Homo Sapiens

<400>  743
aggaatgata ttgcaataac ctacaaaaac rtagaggtgt gaagaagtgt taagtggtct      60

g                                                                     61

<210>  744
<211>  61
<212>  DNA
<213>  Homo Sapiens

<400>  744
gtcattgctc tgccatcatt ggcttcttcc rtgtttactt tgtgccaggc catactgagc      60

c                                                                      61


<210>  745
<211>  61
<212>  DNA
<213>  Homo Sapiens

<400>  745
gtaggggctg tcgggggaat tacacgtgcc rtagaagtac atagaaggag catccagctt      60

t                                                                      61


<210>  746
<211>  61
<212>  DNA
<213>  Homo Sapiens

<400>  746
acgtgtgatt ctttgacatg caggaatcta ytaaattgta catgtaaact atcacacttg      60

a                                                                      61


<210>  747
<211>  61
<212>  DNA
<213>  Homo Sapiens

<400>  747
gagactgtgg tgctgtcctg caggcacatc rtggaagaac ttatcagacc catgactaca      60

a                                                                      61


<210>  748
<211>  61
<212>  DNA
<213>  Homo Sapiens

<400>  748
agtagaagga gtaattactc atgatttgca ktttgtccaa agattatctc atggtcagaa      60

g                                                                      61


<210>  749
<211>  61
<212>  DNA
<213>  Homo Sapiens

<400>  749
aagtatcaac atcttgtaac accctcttta ygagttaaca acaagcgttt tagtgcatta      60

a                                                                   61

<210>  750
<211>  61
<212>  DNA
<213>  Homo Sapiens

<400>  750
atcaaggcct caggtgtcaa atccatccct rgaccagaaa cagagtataa aaagacaaac      60

t                                                                   61

<210>  751
<211>  61
<212>  DNA
<213>  Homo Sapiens

<400>  751
cagctcagtc tgtaaatttc ctttatctcc ytcgctcagt cttaccctca gttccactct      60

c                                                                   61

<210>  752
<211>  61
<212>  DNA
<213>  Homo Sapiens

<400>  752
ctgtttctac actgtctgga agaagaatgg rcattgttac ctcaatatgg gaccaccagg      60

g                                                                   61

<210>  753
<211>  61
<212>  DNA
<213>  Homo Sapiens

<400>  753
ggcatgatcc ctacattcag actcaactgt kggagatact gggatacaga atgagcaaga      60

t                                                                   61

<210>  754
<211>  61
<212>  DNA
<213>  Homo Sapiens

<400>  754
ctcattatca gggccagcca gcaaggcacg sggttccagt gctctcccgg ccactgcagg      60

a                                                                   61

<210>  755
<211>  61
<212>  DNA
<213>  Homo Sapiens

<400>  755
ccccgcacat gggaagacca aaaggtgggt ycttgaagcc ataaggaaat gcgaggtcac      60

t                                                                      61


<210>  756
<211>  61
<212>  DNA
<213>  Homo Sapiens

<400>  756
tggcggaggg actggtgggg cctcagaggc rttagttatc ctctagaaag tactcagggt      60

g                                                                      61


<210>  757
<211>  61
<212>  DNA
<213>  Homo Sapiens

<400>  757
tttggaagca atataactac ttgccctcaa yctatttgca cttctagcac aaaattagcc      60

t                                                                      61


<210>  758
<211>  61
<212>  DNA
<213>  Homo Sapiens

<400>  758
cagttcttgc aatgttggga aaacttacaa raatatcttt gaaaatcctc ttaatacaca      60

t                                                                      61


<210>  759
<211>  61
<212>  DNA
<213>  Homo Sapiens

<400>  759
gaaaacttct acaattactt ctgcatttac rtggtaagac agagacaata agggcagaat      60

t                                                                      61


<210>  760
<211>  61
<212>  DNA
<213>  Homo Sapiens

<400>  760
acccacatct attgcgcatt gaacctacca ygcactatgc tagctgcttt acaaacattg      60

t                                                                      61


<210>  761

<211>  61
<212>  DNA
<213>  Homo Sapiens

<400>  761
ttgaagtcaa aagtattatc agaatttgtc rtcgattctt aaggttgtat ctactaatac      60

a                                                                     61


<210>  762
<211>  61
<212>  DNA
<213>  Homo Sapiens

<400>  762
agtggtccca gatcatgagg tgctaattgt rtaggaaaag acagagatag ataaacagga      60

g                                                                     61


<210>  763
<211>  61
<212>  DNA
<213>  Homo Sapiens

<400>  763
gaataggaga ttgagttatc taatgtgcta ktcttagcgt ctggcactta atagggattc      60

a                                                                     61


<210>  764
<211>  61
<212>  DNA
<213>  Homo Sapiens

<400>  764
gatgaaaaga cagagagaca aggaggttta ygctttagca gaagtgttat tcagataata      60

g                                                                     61


<210>  765
<211>  61
<212>  DNA
<213>  Homo Sapiens

<400>  765
cacctagcac acttaggggc tcaatgtgcg wcccttctct cttcccttct tagcctttgg      60

t                                                                     61


<210>  766
<211>  61
<212>  DNA
<213>  Homo Sapiens

<400>  766
gaataaaata ttttaactgc attcgtctga waagccattg ggaatatttt atgttaaaaa      60

g                                                                     61

<210>  767
<211>  61
<212>  DNA
<213>  Homo Sapiens

<400>  767
aatattcctt tggggttgcc agaaaagaaa rttggccttg atgacttatg aggcccatct      60

a                                                                      61


<210>  768
<211>  61
<212>  DNA
<213>  Homo Sapiens

<400>  768
gagcataagg catcagggga agcttaggga rgaaagtgag gcttgatctg aatttggact      60

t                                                                      61


<210>  769
<211>  61
<212>  DNA
<213>  Homo Sapiens

<400>  769
tcaagaacat caaacatctt gaaatcaaat yatgttggca agtcagagat tgcagcacct      60

t                                                                      61


<210>  770
<211>  61
<212>  DNA
<213>  Homo Sapiens

<400>  770
tgaaaaatat taaaaaatgt gtcttctcaa sgtaaagaaa cttattacca ctttgcttaa      60

t                                                                      61


<210>  771
<211>  61
<212>  DNA
<213>  Homo Sapiens

<400>  771
ctcacacaca ggatttcagt ttggggctta wgtaagaaac tagcaagtta tgtgaactat      60

a                                                                      61


<210>  772
<211>  61
<212>  DNA
<213>  Homo Sapiens

<400>  772

aatacataca agaagaagcc ctgtactcaa stcatctccc acaaatgaat aaatgcatgt 60

a 61

<210> 773
<211> 61
<212> DNA
<213> Homo Sapiens

<400> 773
gtcttgacat gacttttaga ttaatttttg katcctatgg tctcatcaat acagagcctc 60

a 61

<210> 774
<211> 61
<212> DNA
<213> Homo Sapiens

<400> 774
catacactta agaaatatgg aaagcaaagt mcaaaattat agagcagaaa aaatttaaat 60

c 61

<210> 775
<211> 61
<212> DNA
<213> Homo Sapiens

<400> 775
aagtattgat tatgtatgtg gtcagtgttg yaggctaaca tagcatttaa gaagtaaacc 60

c 61

<210> 776
<211> 61
<212> DNA
<213> Homo Sapiens

<400> 776
tggctagaca tctccaggtc tcaagaacat ygcagcatgc cctttgtgcc agctgtttgg 60

g 61

<210> 777
<211> 61
<212> DNA
<213> Homo Sapiens

<400> 777
agaaatagaa agaaaaggga ttagttagga sgataaggta tgaagtatga tgtgctgaga 60

a 61

<210> 778
<211> 61
<212> DNA

<213>  Homo Sapiens

<400>  778
agtaaatgat aggtataaag agccactagc yatgaaaatt ccctttcaaa aaaagcctag      60

t                                                                     61


<210>  779
<211>  61
<212>  DNA
<213>  Homo Sapiens

<400>  779
gtcaacagat gtcttgcaag caggaagccc ytgacttata tacatctttg cattcctaac      60

a                                                                     61


<210>  780
<211>  61
<212>  DNA
<213>  Homo Sapiens

<400>  780
taggcaagct aactacctcc tcctcactct rcctagacct gtcattggtg gtatcttagc      60

t                                                                     61


<210>  781
<211>  61
<212>  DNA
<213>  Homo Sapiens

<400>  781
tatataaaca gggttacagg tctgtgtaaa saagttacat tgattacaag agctaatata      60

a                                                                     61


<210>  782
<211>  61
<212>  DNA
<213>  Homo Sapiens

<400>  782
aagtgtgttt gccaaaacag ggagtgaagt rtcttgcaag gccacttgtt ccagaagaaa      60

g                                                                     61


<210>  783
<211>  61
<212>  DNA
<213>  Homo Sapiens

<400>  783
gctggaacaa ggttccccat gtgtttctca sttgtaccta gcaatttccc taggaagttt      60

t                                                                     61

<210>  784
<211>  61
<212>  DNA
<213>  Homo Sapiens

<400>  784
cttagcccaa gccagctgtg atcctcaggc ygccccgctc attaaaatcc ccccctttcg      60

a                                                                      61


<210>  785
<211>  61
<212>  DNA
<213>  Homo Sapiens

<400>  785
acatctagat cctcactggt caccaaagca satgtagaga gagtacctgg agagttgaca      60

t                                                                      61


<210>  786
<211>  61
<212>  DNA
<213>  Homo Sapiens

<400>  786
catcgcacat gaagcagacc caatgagtga stcagcaata aatcaacagt cttaatggag      60

a                                                                      61


<210>  787
<211>  61
<212>  DNA
<213>  Homo Sapiens

<400>  787
gaaaataact cccagtgagg aagcaggtga yggcctgacc actgtacttc cggtatctct      60

g                                                                      61


<210>  788
<211>  61
<212>  DNA
<213>  Homo Sapiens

<400>  788
caaacagaat attgagtaaa gggatcctga ygtgaaagac agcctgctgt atgttccatt      60

t                                                                      61


<210>  789
<211>  61
<212>  DNA
<213>  Homo Sapiens

<400>  789
agccacggac acaacagaat tcacttttaa ygaggcttca gaaaacaacg tttaactata      60

t                                                                    61

<210>  790
<211>  61
<212>  DNA
<213>  Homo Sapiens

<400>  790
tactactatt agtaataaat gtcttgtcac rgaccttgac acagactaca atacacaagt      60

g                                                                    61

<210>  791
<211>  61
<212>  DNA
<213>  Homo Sapiens

<400>  791
aaatgttcct agggatatag ataattcaca raatgaaaga gtctaatcac atacataaga      60

g                                                                    61

<210>  792
<211>  61
<212>  DNA
<213>  Homo Sapiens

<400>  792
tgttttctct aggaacctga aagtagtggt wtataaggtc agtcatccac tctaggtcaa      60

c                                                                    61

<210>  793
<211>  61
<212>  DNA
<213>  Homo Sapiens

<400>  793
ggatttaaaa tgctcacatg tatgagtcat rtgagtgttc acagaagttc agtaatacac      60

a                                                                    61

<210>  794
<211>  61
<212>  DNA
<213>  Homo Sapiens

<400>  794
ttcggtctct gcacccgtct gcctcgtcta rggtttcttt tcatgacaaa ttcgagttcg      60

a                                                                    61

<210>  795
<211>  61
<212>  DNA
<213>  Homo Sapiens

<400>  795
gtgcaggggg taacctgttc agatcaagcc rgtcagaagg ctccacaagg gacttttctc      60

a                                                                      61


<210>  796
<211>  61
<212>  DNA
<213>  Homo Sapiens

<400>  796
tgggggcaaa tacatgcttc aaaatgttaa ygcacttttc acattttcta tcaaaaaaaa      60

a                                                                      61


<210>  797
<211>  61
<212>  DNA
<213>  Homo Sapiens

<400>  797
actcagagaa atagaaataa atttttgagg wgagttttga cattcttgac cccaagggcc      60

t                                                                      61


<210>  798
<211>  61
<212>  DNA
<213>  Homo Sapiens

<400>  798
gactgacagg aatattaaag aagagagtgg wtacaaagcg cccatcatac tttatagcaa      60

g                                                                      61


<210>  799
<211>  61
<212>  DNA
<213>  Homo Sapiens

<400>  799
acttctggac ctttccctga tctgtgccaa raatggcctg gtagtccagg atacctagtt      60

c                                                                      61


<210>  800
<211>  61
<212>  DNA
<213>  Homo Sapiens

<400>  800
atctagtggg gttcactcac ctttacaggg ractcaacgc tggtagccag ctccctattt      60

c                                                                      61


<210>  801
<211>  61

<212>  DNA
<213>  Homo Sapiens

<400>  801
gttttgggtc cctggtgaag caggtcaggg rcgtgagaaa agaggaaata atgggtttcc      60

a                                                                     61


<210>  802
<211>  61
<212>  DNA
<213>  Homo Sapiens

<400>  802
tcaacactag agggtagata actttattct yaagcatgac accccaggta cttcctcacc      60

t                                                                     61


<210>  803
<211>  61
<212>  DNA
<213>  Homo Sapiens

<400>  803
ggaagatttc agagctgttt cggaggcatc rctgccataa tatactgagg gcaagaaggg      60

g                                                                     61


<210>  804
<211>  61
<212>  DNA
<213>  Homo Sapiens

<400>  804
aagcagccct catccattag ttcaactgac ytttgaataa cccagaccag aaaagaatag      60

g                                                                     61


<210>  805
<211>  61
<212>  DNA
<213>  Homo Sapiens

<400>  805
   attttaaaat cttcctacac acatctagac kttcaagttt gcaaatcagt ttttagcaag      60
   a                                                                     61


<210>  806
<211>  61
<212>  DNA
<213>  Homo Sapiens

<400>  806
ggcccactgt gacttccatg gtctgaggac ktttctagtc tctgaggctt cgggaccata      60

g                                                                     61

<210>  807
<211>  61
<212>  DNA
<213>  Homo Sapiens

<400>  807
ctgcttcctc ctcccctccc tttttttccc rttaaatcag gagccatttc ccatattcaa      60

g                                                                       61


<210>  808
<211>  61
<212>  DNA
<213>  Homo Sapiens

<400>  808
ggaggtatca ttgtttctgc ttctctttaa ygcatccatg aatcagaatg ccaatttgat      60

t                                                                       61


<210>  809
<211>  61
<212>  DNA
<213>  Homo Sapiens

<400>  809
cagctggtcc tctgggagca catggagaac raccacattg tgtcccaggg ttgcttgcct      60

g                                                                       61


<210>  810
<211>  61
<212>  DNA
<213>  Homo Sapiens

<400>  810
agtagatgtg gcattgttta tatgacaaag rttagaaata gtgtcgacag cacagcacac      60

g                                                                       61


<210>  811
<211>  61
<212>  DNA
<213>  Homo Sapiens

<400>  811
gagacagccc acaccctggg caatttctgg sgcaaggagg aaaccccctg ttcactgagc      60

t                                                                       61


<210>  812
<211>  61
<212>  DNA
<213>  Homo Sapiens

<400>  812
cagagaggcc ttcgtgactg cctaccttgt yacttactat ctctctctcc ccatcttagt      60

t 61

<210> 813
<211> 61
<212> DNA
<213> Homo Sapiens

<400> 813
cagggagagt gggtggcttg gtcaaggtct ycctgacagt gacagggcta agatttggtc 60

c 61

<210> 814
<211> 61
<212> DNA
<213> Homo Sapiens

<400> 814
cccctctcct agcccctctt tcctttcatc yctttccagg cctggttcat cattgtcttt 60

g 61

<210> 815
<211> 61
<212> DNA
<213> Homo Sapiens

<400> 815
attgagaatc agtgacttaa actttcctac rgtgtgaccc ccaatcccat cattctcaat 60

c 61

<210> 816
<211> 61
<212> DNA
<213> Homo Sapiens

<400> 816
tgtgcactgc accctgcttc acaatctgca ygcctggcaa gtccgtgatc tgggggtcag 60

g 61

<210> 817
<211> 61
<212> DNA
<213> Homo Sapiens

<400> 817
ttaggctctt gcccaaccac tcacggtcaa yagtgagctg ggccttttgt accaggttcc 60

c 61

<210> 818
<211> 61
<212> DNA
<213> Homo Sapiens

<400>  818
ctattgaaat ccgtattgtt caagggtcaa ycgtatttca ctttgaagga ctggtcctca      60

g                                                                      61


<210>  819
<211>  61
<212>  DNA
<213>  Homo Sapiens

<400>  819
gtgtcattat atcacaatgt aaataaatag yaagtggaag ttgctgctat gggagtgctg      60

t                                                                      61


<210>  820
<211>  61
<212>  DNA
<213>  Homo Sapiens

<400>  820
actgaggacc cacctcacag gaagaaaacc rtctgactca taaagggatc cgaccaaggc      60

t                                                                      61


<210>  821
<211>  61
<212>  DNA
<213>  Homo Sapiens

<400>  821
gatatggtca aatctttgga gctccagggt rtataagaca atgatacctc ctccccagtt      60

t                                                                      61


<210>  822
<211>  61
<212>  DNA
<213>  Homo Sapiens

<400>  822
acacctcatt gtctcaccct gcccaacatt yggtgtggaa gattgagaat aatgtgaatt      60

t                                                                      61


<210>  823
<211>  61
<212>  DNA
<213>  Homo Sapiens

<400>  823
gtataaactg ccattgattt ctgataacac rtagcaatta cttaatgctg cagacttgat      60

t                                                                      61


<210>  824

<211>  61
<212>  DNA
<213>  Homo Sapiens

<400>  824
cagcttaaaa ttgagaaata ttattttaac rtcattcttg tgccccagga gtgcttacag        60

g                                                                        61


<210>  825
<211>  61
<212>  DNA
<213>  Homo Sapiens

<400>  825
gggaagaaat tggtgtagga tccaaactat ytctacttta ttcagagaga ccatggatag        60

g                                                                        61


<210>  826
<211>  61
<212>  DNA
<213>  Homo Sapiens

<400>  826
gcacctgatg tgataatcac taggttggag wttgcagaga tgagataatc tttgctggaa        60

g                                                                        61


<210>  827
<211>  61
<212>  DNA
<213>  Homo Sapiens

<400>  827
aaaattgaat atgtttttcc tcctgcctaa racaaaacta cttaagggca aaaatttaaa        60

t                                                                        61


<210>  828
<211>  61
<212>  DNA
<213>  Homo Sapiens

<400>  828
ttctagtcaa tattttgacg cacctaacaa ygatagttaa catttacata gtgctcattt        60

t                                                                        61


<210>  829
<211>  61
<212>  DNA
<213>  Homo Sapiens

<400>  829
tgagctccct atttctctgt tccgccttcc rtccactcat gtaaacactg gcttccccca        60

a                                                                        61

<210>  830
<211>  61
<212>  DNA
<213>  Homo Sapiens

<400>  830
ccatcttctt aatatgattt ttccagtgca rtgttttcaa ttttgattaa atccagttta        60

t                                                                         61


<210>  831
<211>  61
<212>  DNA
<213>  Homo Sapiens

<400>  831
tattggcaga attttaatca gataacccat ycttccctat tgaaacactt ccttcatttg        60

a                                                                         61


<210>  832
<211>  61
<212>  DNA
<213>  Homo Sapiens

<400>  832
tctaggttct tcttgctgct ataaactgga ygtgacattt gccttcttgg accataaata        60

c                                                                         61


<210>  833
<211>  61
<212>  DNA
<213>  Homo Sapiens

<400>  833
acgaggttgc tgccacttct cacatgcacc wcaacactgc tgttttacaa aaacttcaga        60

t                                                                         61


<210>  834
<211>  61
<212>  DNA
<213>  Homo Sapiens

<400>  834
aatgatactc agagagaata agtatcttat ytaaggtcac aacactcatt aagtgaaaga        60

a                                                                         61


<210>  835
<211>  61
<212>  DNA
<213>  Homo Sapiens

<400>  835

gatttggttt aaactctctt cttcgtgccc rtgcctgagg cagtgaggag taagcctcca 60

t 61

<210> 836
<211> 61
<212> DNA
<213> Homo Sapiens

<400> 836
gtgctgggga tatggctgtg agtgagacag ytgaccgcat ggcatttgca ctcttatctg 60

t 61

<210> 837
<211> 61
<212> DNA
<213> Homo Sapiens

<400> 837
cctccagcaa tctgatgact caaaaccctg stgagctgca ggctccctcc catggctgca 60

g 61

<210> 838
<211> 61
<212> DNA
<213> Homo Sapiens

<400> 838
ccccaaaata gtgtgtacct gtatgcaaat rtgcaagcgt gtttatttaa ggggtggatg 60

a 61

<210> 839
<211> 61
<212> DNA
<213> Homo Sapiens

<400> 839
tgaacagggg cttggtaaaa caaggttgag rtctactagg ttgcattccc aggaggttag 60

g 61

<210> 840
<211> 61
<212> DNA
<213> Homo Sapiens

<400> 840
ccattaatta gactgtccct aagtatgaaa yatgggtgag gaaaagagac agagagtgaa 60

a 61

<210> 841
<211> 61
<212> DNA

<213>  Homo Sapiens

<400>  841
gatttgctct gaccatgtca attgttaaga mttagctgtc agctgacctc cgtggctgac      60

t                                                                      61


<210>  842
<211>  61
<212>  DNA
<213>  Homo Sapiens

<400>  842
cattttcctc ttacatatga tggggctaca raaatctacc tccaaagaat tgttgtgaag      60

a                                                                      61


<210>  843
<211>  61
<212>  DNA
<213>  Homo Sapiens

<400>  843
tgaatccctg ctgggttgcc tgggagaaaa yaggagaagt cagaaatgcc tggagggagc      60

c                                                                      61


<210>  844
<211>  61
<212>  DNA
<213>  Homo Sapiens

<400>  844
agttttgggg gcaagagtgg acataaaaag yggaggatat ggcctgagga caccactcac      60

t                                                                      61


<210>  845
<211>  61
<212>  DNA
<213>  Homo Sapiens

<400>  845
ttctaggatg gtagagagta gtcttcagcc ygagagccat ccctagggaa gtagctgaat      60

t                                                                      61


<210>  846
<211>  61
<212>  DNA
<213>  Homo Sapiens

<400>  846
tttatctgtt tataagtacc aatacatcca rttccagtaa tgtgccacat atttgccaac      60

t                                                                      61

<210>  847
<211>  61
<212>  DNA
<213>  Homo Sapiens

<400>  847
aaagggcaac aacaaaaagc agttagatct sattccacta tttgtattat atctcatatc      60

c                                                                     61


<210>  848
<211>  61
<212>  DNA
<213>  Homo Sapiens

<400>  848
attactgttc cattgatgaa tcatattatg magtgtttga ccacaaagac tgcgtctgat      60

g                                                                     61


<210>  849
<211>  61
<212>  DNA
<213>  Homo Sapiens

<400>  849
aagcaaattg ctatttgggg accatagaca wgaggtaagg ctgtaagcag agatagccca      60

t                                                                     61


<210>  850
<211>  61
<212>  DNA
<213>  Homo Sapiens

<400>  850
gtgaatgtat agatagtgtc taacatagtg yttgatgcat agtagatcca gctcttcttg      60

a                                                                     61


<210>  851
<211>  61
<212>  DNA
<213>  Homo Sapiens

<400>  851
taaacactcc tttgcctcag tctgctcatc ktgaaagtgg gaaagttgat aacgcctgtg      60

t                                                                     61


<210>  852
<211>  61
<212>  DNA
<213>  Homo Sapiens

<400>  852
aaacaaatct ccaggtgatt cagtgcatat yaaaacctga gaagcacttt tgagaggcct      60

g                                                                     61

<210>  853
<211>  61
<212>  DNA
<213>  Homo Sapiens

<400>  853
gggcgggact aatagagctt acattttcat ycagtttata aagcaatgca gactgagtat      60

c                                                                     61

<210>  854
<211>  61
<212>  DNA
<213>  Homo Sapiens

<400>  854
ataattaaca catacatcat gatgacattt mccatagcac aataaaactt aactttcaaa      60

t                                                                     61

<210>  855
<211>  61
<212>  DNA
<213>  Homo Sapiens

<400>  855
aatctgcaaa caagtaaaga gttctttcct rtaccagcca ctcagtgact tacttactta      60

a                                                                     61

<210>  856
<211>  61
<212>  DNA
<213>  Homo Sapiens

<400>  856
gaattcaggc tgaagcttcc tgtgtacgtg rgcttggcca tgcctggctg cctggagttc      60

a                                                                     61

<210>  857
<211>  61
<212>  DNA
<213>  Homo Sapiens

<400>  857
ttatcccctt atccaggaaa gttaacagaa ragacacgtt aagagaagaa aatattctct      60

a                                                                     61

<210>  858
<211>  61
<212>  DNA
<213>  Homo Sapiens

<400>  858
ttgtgtatca acagatgaag tgacaaaacc rtccttagta aattaatgta actacaggca      60

g                                                                      61


<210>  859
<211>  61
<212>  DNA
<213>  Homo Sapiens

<400>  859
gtggtggtgg caggcaagag gagccgtgga rgagaggaga aaggagaaga gaaggaaatc      60

c                                                                      61


<210>  860
<211>  61
<212>  DNA
<213>  Homo Sapiens

<400>  860
aaaggggatt tatttcttaa aagctgccat ytggtagctg agataagatg ttttatataa      60

t                                                                      61


<210>  861
<211>  61
<212>  DNA
<213>  Homo Sapiens

<400>  861
   tgcctctcag gcaaatctcc ctctgctctc rtcccacgaa aagggggtgc ccaattccag      60
   t                                                                      61


<210>  862
<211>  61
<212>  DNA
<213>  Homo Sapiens

<400>  862
tagagaaatc atgtttattt gcacagtttt rgtattcaat ggagaaaaat atgatataga      60

a                                                                      61


<210>  863
<211>  61
<212>  DNA
<213>  Homo Sapiens

<400>  863
agaaatcatg aaaggactga gaaaggagaa ygggggaagt atgaaggatg aaggggagat      60

t                                                                      61


<210>  864
<211>  61

<212>  DNA
<213>  Homo Sapiens

<400>  864
ttccatcttt acagcctcat cttctaattg ytccccttgt ctgcaccaac ctggtatata        60

c                                                                        61


<210>  865
<211>  61
<212>  DNA
<213>  Homo Sapiens

<400>  865
agccattatt tatttgctgt caacgtttca rtttatcaac caattgggat tatgagataa        60

c                                                                        61


<210>  866
<211>  61
<212>  DNA
<213>  Homo Sapiens

<400>  866
tttgctaatg gaggaaattt atccttctac rctcatctta ctgtgactca aaactctcaa        60

a                                                                        61


<210>  867
<211>  61
<212>  DNA
<213>  Homo Sapiens

<400>  867
aatgacaaga agaggaagag cagactgcag stactttggc taattttaag aaaccttggg        60

t                                                                        61


<210>  868
<211>  61
<212>  DNA
<213>  Homo Sapiens

<400>  868
actctctcat tgtttacagg ctctataaaa ygccgacacc tctgagagaa ctttctggtt        60

t                                                                        61


<210>  869
<211>  61
<212>  DNA
<213>  Homo Sapiens

<400>  869
gccaaaaatc aattaaaaag aaatagatga yttgaatagc caatattggt caagcaaagt        60

g                                                                        61

<210>  870
<211>  61
<212>  DNA
<213>  Homo Sapiens

<400>  870
gactctatga ctgccgatat ggccgtttcc rtaaattcaa gagcatcaag gcccgcgacg      60

t                                                                      61


<210>  871
<211>  61
<212>  DNA
<213>  Homo Sapiens

<400>  871
gggctgtaaa gggcacggca aggagagaac sagtgatgat gtgtagggtt ggggagagtg      60

c                                                                      61


<210>  872
<211>  61
<212>  DNA
<213>  Homo Sapiens

<400>  872
ccctcaaaga ggtgaaaact aaaagttata ygtgaaatgg ttatttagaa taccaattta      60

g                                                                      61


<210>  873
<211>  61
<212>  DNA
<213>  Homo Sapiens

<400>  873
gaaaagggga actggtgctt ttagatggag ygcataaaat tacctcacac catctgtgtc      60

a                                                                      61


<210>  874
<211>  61
<212>  DNA
<213>  Homo Sapiens

<400>  874
atgggtgctt gctgaattgt tgggctttta rttgtttatt tacataactg tttaggggat      60

a                                                                      61


<210>  875
<211>  61
<212>  DNA
<213>  Homo Sapiens

<400>  875
tgaggaaagc aaaacatttt caggttgata ygtcatgctt cttcatgctt ataaattata      60

t 61

<210> 876
<211> 61
<212> DNA
<213> Homo Sapiens

<400> 876
cgtattggca cggttgtctt ccaaacccat ygatgccgga acatgggtca ggaagaacac 60

a 61

<210> 877
<211> 61
<212> DNA
<213> Homo Sapiens

<400> 877
tgagggctgg cgagtttgaa atttgtgcaa yaggctggaa actcagccag gatttgatgt 60

t 61

<210> 878
<211> 61
<212> DNA
<213> Homo Sapiens

<400> 878
tcagccagga tttgatgttt cagccttgag rtagaacttc tttcagaaac gtcagttctt 60

g 61

<210> 879
<211> 61
<212> DNA
<213> Homo Sapiens

<400> 879
aatcaggcac aggtatagat tatttccacc rtaacacctg agtagccact tgcccaagac 60

c 61

<210> 880
<211> 61
<212> DNA
<213> Homo Sapiens

<400> 880
gaagggtaaa gttaactttc taagacatac ragaggagtt gaaattagat gtgaatctag 60

a 61

<210> 881
<211> 61
<212> DNA
<213> Homo Sapiens

<400>  881
ttgtatggtt tctgaaatgg atgctagaag mgataacttg aatgagtact tttcccctgg      60

t                                                                     61


<210>  882
<211>  61
<212>  DNA
<213>  Homo Sapiens

<400>  882
cagtagtacg agtgttcagt gtaactgaag yataagctca cacatacaga aaaccaccat      60

g                                                                     61


<210>  883
<211>  61
<212>  DNA
<213>  Homo Sapiens

<400>  883
aaagtttatt ttttaagggg cagttccata yagtaggtga cacaggttag gttcctccag      60

a                                                                     61


<210>  884
<211>  61
<212>  DNA
<213>  Homo Sapiens

<400>  884
tcaacttatt caagacatat tcagcaatat yctataaggc agtgctcatt tactgaggct      60

c                                                                     61


<210>  885
<211>  61
<212>  DNA
<213>  Homo Sapiens

<400>  885
ataatgagcc tgctttaatg tgatccaaat rttcatggga aactctaaag cagttttgaa      60

g                                                                     61


<210>  886
<211>  61
<212>  DNA
<213>  Homo Sapiens

<400>  886
cggatgcatc ttttgtgtcc tctatgtgct kaactgctga gccccagctc gggttaagcg      60

g                                                                     61


<210>  887

<211>  61
<212>  DNA
<213>  Homo Sapiens

<400>  887
tacaaatacc ttcactaaca ttcttttaca rtgtcaacaa gcatctaccc gtcaagagaa        60

a                                                                         61


<210>  888
<211>  61
<212>  DNA
<213>  Homo Sapiens

<400>  888
atatggggca atacaaaagt ggtgagaaaa rttgatttgt ccactgttaa caagtcctat        60

g                                                                         61


<210>  889
<211>  61
<212>  DNA
<213>  Homo Sapiens

<400>  889
gcctggcata tagtaggggt tcagtaattg rtgtacttgt tatttaagac tgagaaacat        60

g                                                                         61


<210>  890
<211>  61
<212>  DNA
<213>  Homo Sapiens

<400>  890
ggtaaacgat ttttgagtgt gtaaaatgac rtagctttca tagcctgatt ttatggaata        60

c                                                                         61


<210>  891
<211>  61
<212>  DNA
<213>  Homo Sapiens

<400>  891
aattgctcct ttcatttatt tctgaaggta ycaaagcaga gagagtttct tttaaatgaa        60

g                                                                         61


<210>  892
<211>  61
<212>  DNA
<213>  Homo Sapiens

<400>  892
tgtaactatg gttaaggact tgaaaaactt yatataggcc ttcctatttg atctgtaatt        60

a                                                                         61

<210>  893
<211>  61
<212>  DNA
<213>  Homo Sapiens

<400>  893
caccttaaaa tcctctgtat cttggaaatg yatgtcagat tggcaaaaat ttaaggtatt        60

t                                                                         61


<210>  894
<211>  61
<212>  DNA
<213>  Homo Sapiens

<400>  894
actatctggt ttcctttggc ctatcactaa ycttgatcag ttttctgagc tagaaacttt        60

t                                                                         61


<210>  895
<211>  61
<212>  DNA
<213>  Homo Sapiens


<220>
<221>  misc_feature
<222>  (31)..(31)
<223>  n is a, c, g, or t

<400>  895
ttccacttct tccaagttta tcagtcagca ntgcaccaca gagaagagac ctcccttatt        60

c                                                                         61


<210>  896
<211>  61
<212>  DNA
<213>  Homo Sapiens

<400>  896
tgtctgtgca aggacaagga aaagaatttc yctactggct cctgccccct ctagttcagg        60

g                                                                         61


<210>  897
<211>  61
<212>  DNA
<213>  Homo Sapiens

<400>  897
cactgaattt gtcaacttgg gtaccactga ygacaatatc cagagcagtt ttggcaaggc        60

a                                                                         61

<210>  898
<211>  61
<212>  DNA
<213>  Homo Sapiens

<400>  898
gaaaagtgga gtcctagtaa gagtgcttta sactttagga aaatcttcaa caaatatgag      60

t                                                                      61

<210>  899
<211>  61
<212>  DNA
<213>  Homo Sapiens

<400>  899
cagggatggc tagagacagg aaatgctcca yaggtaatgg cctagctggc cggctggcat      60

t                                                                      61

<210>  900
<211>  61
<212>  DNA
<213>  Homo Sapiens

<400>  900
agcctggggt ggcccaaccg acctttgagg mcattccaac agtctggcag catctgcagt      60

g                                                                      61

<210>  901
<211>  61
<212>  DNA
<213>  Homo Sapiens

<400>  901
ggaggcggca gcagcaaagt cccaagaaca magtgctggt gtaggagtca ggcccgcatc      60

t                                                                      61

<210>  902
<211>  61
<212>  DNA
<213>  Homo Sapiens

<400>  902
ctgaaaggtt cttcaaagtc aggtccctaa saaagctgtt ccatctccag ctcagcgtgt      60

t                                                                      61

<210>  903
<211>  61
<212>  DNA
<213>  Homo Sapiens

<400>  903
aagcagcctc cctctgttac tactataaac rcccattctg caggcagtgt gagggcacag      60

c                                                                    61

<210>  904
<211>  61
<212>  DNA
<213>  Homo Sapiens

<400>  904
attccttgca gaagaaacaa acaaaagaga yagaataagc actgcagaaa atagaaaggt    60

t                                                                    61

<210>  905
<211>  61
<212>  DNA
<213>  Homo Sapiens

<400>  905
ttcacctccc tggagaagtc ctgagcgcca ygatcccttc cttgagaatc atacaatact    60

g                                                                    61

<210>  906
<211>  61
<212>  DNA
<213>  Homo Sapiens

<400>  906
ctcggcaacc actggggggg ccatgctatc raagatcagc ctgagtgttt attactggac    60

g                                                                    61

<210>  907
<211>  61
<212>  DNA
<213>  Homo Sapiens

<400>  907
aatgaattcc tgggctcacc cataacaaag sctttgagag gcaaactctg caacagactg    60

c                                                                    61

<210>  908
<211>  61
<212>  DNA
<213>  Homo Sapiens

<400>  908
acatttcaga agtgaaaggc tctttcagag rttattgctc acttctcgcc tctccgtgct    60

g                                                                    61

<210>  909
<211>  61
<212>  DNA
<213>  Homo Sapiens

<400>  909
atgtggttct tgagtatgtc cagaattata wggctgttaa tgtgtcttga gccctgacct      60

a                                                                      61


<210>  910
<211>  61
<212>  DNA
<213>  Homo Sapiens

<400>  910
tattttggcc tcagagagaa aaactataat yctgtgactt cttgggaaat gctaattgaa      60

t                                                                      61


<210>  911
<211>  61
<212>  DNA
<213>  Homo Sapiens

<400>  911
taaagatctt cagaacagct tggtccttta ytctgaaact cttgtagctt aagcatcttt      60

c                                                                      61


<210>  912
<211>  61
<212>  DNA
<213>  Homo Sapiens

<400>  912
gggtcaggag ctgagaaaca gttttctaaa yacccagttg gcacacatca tggcatttga      60

c                                                                      61


<210>  913
<211>  61
<212>  DNA
<213>  Homo Sapiens

<400>  913
agtacgtgtt cgccatctgg tggtaaagtc rtggagacac aggctgtact gaccctagag      60

a                                                                      61


<210>  914
<211>  61
<212>  DNA
<213>  Homo Sapiens

<400>  914
ctcccactaa tcatcatgtg agattcagac yctttaagga tgtagatgct aaagggaagg      60

c                                                                      61


<210>  915
<211>  61

<212>  DNA
<213>  Homo Sapiens

<400>  915
cagaatttat ttcctgatgt ctcccagaat racatccttg tggcacctga tctaatccat      60

a                                                                     61


<210>  916
<211>  61
<212>  DNA
<213>  Homo Sapiens

<400>  916
ctgcgtcaca ctgaggagtc aagacgctat yctgtcagcc aggggctttc ctagccaggg      60

g                                                                     61


<210>  917
<211>  61
<212>  DNA
<213>  Homo Sapiens

<400>  917
aggctggtgt gtgaggttcc taatctccca rtcacagaag aaattatgtt caagacagct      60

c                                                                     61


<210>  918
<211>  61
<212>  DNA
<213>  Homo Sapiens

<400>  918
ggactagcaa gtccagcctc accgtgtatc rccaaattgc tctccaaacg ataccaatct      60

c                                                                     61


<210>  919
<211>  61
<212>  DNA
<213>  Homo Sapiens

<400>  919
aggctgtgac taagaaggtt ccagacttcc ygtctatcaa aggtgaggac tgaattagac      60

t                                                                     61


<210>  920
<211>  61
<212>  DNA
<213>  Homo Sapiens

<400>  920
gttatgtccc ctaccttggt gcatgaagga raactacaca aagatgtgtg gtatcaactc      60

a                                                                     61

<210>  921
<211>  61
<212>  DNA
<213>  Homo Sapiens

<400>  921
ttttgctgtg agtccacaga gcgagtatta yaaacccatt ttacagatga gggaactgag      60

g                                                                      61


<210>  922
<211>  47
<212>  DNA
<213>  Homo Sapiens

<400>  922
atttcgtcac tttccaktta ttgaatacat accaggaact tggccaa                    47


<210>  923
<211>  61
<212>  DNA
<213>  Homo Sapiens

<400>  923
gtgtcacagt gcctggccca cagtaggtgc ytgaaaaata ttaactgagt taataacaac      60

a                                                                      61


<210>  924
<211>  61
<212>  DNA
<213>  Homo Sapiens

<400>  924
tgacagaaaa ggatatatgt tagggacagt rgtagagatg aagtgaggaa catgtttccc      60

a                                                                      61


<210>  925
<211>  61
<212>  DNA
<213>  Homo Sapiens

<400>  925
gacacgagct tgcctccatg taaaaagcgc rtcagagaac tacagaccaa gctcttggga      60

t                                                                      61


<210>  926
<211>  61
<212>  DNA
<213>  Homo Sapiens

<400>  926
aggtttttct acatacaaaa tcatgttgtc rgcaaagagg tacaatctga cttcctcttt      60

t                                                                      61

<210>  927
<211>  61
<212>  DNA
<213>  Homo Sapiens

<400>  927
atttacattt aaattaaaaa ttcacttcct ygtgtcacta gccacatatc aagtgctcaa      60

t                                                                      61


<210>  928
<211>  61
<212>  DNA
<213>  Homo Sapiens

<400>  928
tagctagaga ttcagaagga tttaattctc ygaagcaatg tgatagttcc aatattagtc      60

a                                                                      61


<210>  929
<211>  61
<212>  DNA
<213>  Homo Sapiens

<400>  929
aggattataa tcagcttcct acaaaaatga sggtgtagga atctcacacc cacagtaaat      60

c                                                                      61


<210>  930
<211>  61
<212>  DNA
<213>  Homo Sapiens

<400>  930
cttatctgag ttcctttctt agaaaactga ygctcgggcc tcccagagag tataaaggaa      60

c                                                                      61


<210>  931
<211>  61
<212>  DNA
<213>  Homo Sapiens

<400>  931
tttatccagg ttctcattgt cgttttatta yctcctgctc ataatatgta gagacaaatg      60

c                                                                      61


<210>  932
<211>  61
<212>  DNA
<213>  Homo Sapiens

<400>  932

agaggtcaac tggctctatc tggtggttca rggaagactt tctagaaggg gtgatgcctg 60

a 61

<210> 933
<211> 61
<212> DNA
<213> Homo Sapiens

<400> 933
cctgtaccag ctgtgaaaac aatgagacac rttcagtaat agaatatagt gtgcttacaa 60

a 61

<210> 934
<211> 61
<212> DNA
<213> Homo Sapiens

<400> 934
gctcctgtac tccccacaaa cctagttgat yccagaccag taatgtcttg gggacagtgt 60

t 61

<210> 935
<211> 61
<212> DNA
<213> Homo Sapiens

<400> 935
attgcatcct tgagttgcat atgatgggat mtcttgaatc atttacattt tgaaaaaaga 60

a 61

<210> 936
<211> 61
<212> DNA
<213> Homo Sapiens

<400> 936
gtttgaaaag cagtgacctc tctgtgcaaa ygattttttt ttccaaacag agctgaacaa 60

g 61

<210> 937
<211> 61
<212> DNA
<213> Homo Sapiens

<400> 937
ggactacagg catgagccac tgcgaatggc statctttca aaacattatg aatgcttcct 60

t 61

<210> 938
<211> 61
<212> DNA

<213>  Homo Sapiens

<400>  938
gtggatatgt ccttggcatt gctgttgtgg stagtatagt gactatctct gaggaacagc      60

a                                                                      61


<210>  939
<211>  61
<212>  DNA
<213>  Homo Sapiens

<400>  939
gtctcttgtg ttttcacttg acagttccca statttcaat atttgtaaat atttaaactt      60

a                                                                      61


<210>  940
<211>  61
<212>  DNA
<213>  Homo Sapiens

<400>  940
cagtgttttg taatatatag gtacacagca raactggatc ttcaaagcct tcaaataggc      60

t                                                                      61


<210>  941
<211>  61
<212>  DNA
<213>  Homo Sapiens

<400>  941
ttgacactaa acgacatggc tcgcctttta yattaattgc aaacacctct caactgtctc      60

a                                                                      61


<210>  942
<211>  61
<212>  DNA
<213>  Homo Sapiens

<400>  942
agaaaagaaa taaacagaac agtaaactta sctgcaaaag aagtcatcca gaatattgga      60

g                                                                      61


<210>  943
<211>  61
<212>  DNA
<213>  Homo Sapiens

<400>  943
aggcaggaga gaaataataa ccttttcata ytctttgccc agacttgagg aagcccaggg      60

t                                                                      61

<210>  944
<211>  61
<212>  DNA
<213>  Homo Sapiens

<400>  944
acagcccaaa ttaagagctt ggcacccaat ycccacttgg actacaaatc acttcctctt      60

g                                                                      61


<210>  945
<211>  61
<212>  DNA
<213>  Homo Sapiens

<400>  945
agagaggaga ataaatcttt gcaggctgct stgtccttcc attgggggtt acagttcttc      60

t                                                                      61


<210>  946
<211>  61
<212>  DNA
<213>  Homo Sapiens

<400>  946
agtactggga agatagaagg tggtcaaagc rtatgtatgg aatgaacact ggaaatcagt      60

a                                                                      61


<210>  947
<211>  61
<212>  DNA
<213>  Homo Sapiens

<400>  947
aaaaaatcaa ttcagttgac attaaagttg raagtgtact cctcttatcc aacaacaaca      60

a                                                                      61


<210>  948
<211>  61
<212>  DNA
<213>  Homo Sapiens

<400>  948
gtatccacaa aatctcactc gggctagacc kcactgtctt tttttgttgt tgttgttttg      60

a                                                                      61


<210>  949
<211>  61
<212>  DNA
<213>  Homo Sapiens

<400>  949
cccgccaggc cccacctcca gcatttgaca rttcgacctg aaatttctca gggatgtata      60

t 61

<210> 950
<211> 61
<212> DNA
<213> Homo Sapiens

<400> 950
agtcctgaga aaaatgccag acaaatccca wccgagaggc attctacaaa atgcctgatt 60

a 61

<210> 951
<211> 61
<212> DNA
<213> Homo Sapiens

<400> 951
gtctatgcag atagcctttt ctcttacacc matttgaggt attcataccc atatatcttc 60

t 61

<210> 952
<211> 61
<212> DNA
<213> Homo Sapiens

<400> 952
ggctgtcttc cagaaatgat tttactctac kttctgcttt catcctcacc tcttctcctc 60

t 61

<210> 953
<211> 61
<212> DNA
<213> Homo Sapiens

<400> 953
ggcacatgcg tgtacaagaa gagcccattc rcaaatcgga tcaagaaaat gcagttgcgc 60

c 61

<210> 954
<211> 61
<212> DNA
<213> Homo Sapiens

<400> 954
catcccggtg ggtttgcttg gcctttagga ygtgtgacta cagcttttgg gtaggtacag 60

g 61

<210> 955
<211> 61
<212> DNA
<213> Homo Sapiens

<400>  955
tctatcccta caataagggt gaaagaattt yagctttttt gtgagtttta actcttaccg        60

g                                                                         61


<210>  956
<211>  61
<212>  DNA
<213>  Homo Sapiens

<400>  956
agaagcaccc aggcctgtaa tcatccagag rctaggcagc ccagcaaaaa ccttacactg        60

t                                                                         61


<210>  957
<211>  61
<212>  DNA
<213>  Homo Sapiens

<400>  957
ttcaaggagt ggtctaatcc ctggactgct ktctgagtat cagtaattag taagcaggcc        60

a                                                                         61


<210>  958
<211>  61
<212>  DNA
<213>  Homo Sapiens

<400>  958
agggtggtcc ccttgccagt ttgctgtctg yagctaaaag ccacatcttg atcccctagg        60

t                                                                         61


<210>  959
<211>  61
<212>  DNA
<213>  Homo Sapiens

<400>  959
agaaacattg tacccattta tatccacacc yctgaaagat gcacctgaat catttgaatc        60

t                                                                         61


<210>  960
<211>  61
<212>  DNA
<213>  Homo Sapiens

<400>  960
aaactttaaa aagtaaactt ctcaaacttg rcaggttcca acactgactt aaagctaatt        60

g                                                                         61


<210>  961
<211>  61

<212>  DNA
<213>  Homo Sapiens

<400>  961
atcaaatatt acataacttc agaaaacatt rttataggaa aggcatgaat atcctagaaa      60

g                                                                     61


<210>  962
<211>  61
<212>  DNA
<213>  Homo Sapiens

<400>  962
gtctccaaag ttgcagcctc atctctcaaa mctgagtgct ctgcttggtg tcactgctcc      60

a                                                                     61


<210>  963
<211>  61
<212>  DNA
<213>  Homo Sapiens

<400>  963
aatacttaat atgttttagt ataacttatc wgcccaaata actgcataag atattagata      60

a                                                                     61


<210>  964
<211>  61
<212>  DNA
<213>  Homo Sapiens

<400>  964
gtttctcagt tgaatattgg tattaggtca rgaagggctg gagtgggagg agtaagacag      60

t                                                                     61


<210>  965
<211>  61
<212>  DNA
<213>  Homo Sapiens

<400>  965
aagaactggt tggctctgtt ctcccctttt ycatgacatt gtctctttct gtactatcac      60

c                                                                     61


<210>  966
<211>  61
<212>  DNA
<213>  Homo Sapiens

<400>  966
aatggaagcc aagatactgg atcacgtcta rgaaaattta aatatgctag ctacactcaa      60

t                                                                     61

<210>  967
<211>  61
<212>  DNA
<213>  Homo Sapiens

<400>  967
tcttatctgg ggattttcag ttaaataatc rgacaatgta tatgattgat ttctatactg      60

c                                                                     61


<210>  968
<211>  61
<212>  DNA
<213>  Homo Sapiens

<400>  968
tcaaaatgca aatatgaaaa ctatcacctt scaaccacgg ataacactta aatgaattac      60

c                                                                     61


<210>  969
<211>  61
<212>  DNA
<213>  Homo Sapiens

<400>  969
tatatacaat ggcctaaaaa tatgtaacgt rgaccctgac tgggaagaaa tctaagaaaa      60

a                                                                     61


<210>  970
<211>  61
<212>  DNA
<213>  Homo Sapiens

<400>  970
ccagagggga gggatggggt cgaaatcgtt mtttagagag atccttttgg cagagctgta      60

g                                                                     61


<210>  971
<211>  61
<212>  DNA
<213>  Homo Sapiens

<400>  971
tgaatacata gactagaaat aaggtacctc rgttcttacc tggccacatt aattggcact      60

c                                                                     61


<210>  972
<211>  61
<212>  DNA
<213>  Homo Sapiens

<400>  972
aacatggaca agttggaaga ggagcgactc yaaagtttgg caatggagcc cggagttttg      60

a                                                                   61

<210>  973
<211>  61
<212>  DNA
<213>  Homo Sapiens

<400>  973
tttcaaacaa ataattttcc ccacgtttta wcatctctat aatctgcata cgggttttca      60

c                                                                   61

<210>  974
<211>  61
<212>  DNA
<213>  Homo Sapiens

<400>  974
tcacttgttt gtcgttggtc cagtgctaac scagcttctt ggaggaggaa accctggaaa      60

g                                                                   61

<210>  975
<211>  61
<212>  DNA
<213>  Homo Sapiens

<400>  975
agatgggttg gggcagatcc agcagattac rttactgtga gggggtggtg gggaacacag      60

c                                                                   61

<210>  976
<211>  61
<212>  DNA
<213>  Homo Sapiens

<400>  976
ggatcctcct ctgcctgttg atcagtcttt mactttactt ctccctcagc tcacctcttc      60

a                                                                   61

<210>  977
<211>  61
<212>  DNA
<213>  Homo Sapiens

<400>  977
gacaagtatg gtctaaggaa aaaacaagga wgaactcaag caagagttat cacattggcc      60

a                                                                   61

<210>  978
<211>  61
<212>  DNA
<213>  Homo Sapiens

<400>  978
gggtgattca tttcttaaat ttatctttga sagttgctga aaatctcaca gcttgtattc      60

t                                                                      61


<210>  979
<211>  61
<212>  DNA
<213>  Homo Sapiens

<400>  979
tttgtaggcg ttcaaagacc ttctgaatcc racctctgac acttctttgc aatccgaaat      60

c                                                                      61


<210>  980
<211>  61
<212>  DNA
<213>  Homo Sapiens

<400>  980
actgatctta ttccttcttc attaaatgcg ytcaagtcag gaaaaggggt gatgacgatg      60

a                                                                      61


<210>  981
<211>  61
<212>  DNA
<213>  Homo Sapiens

<400>  981
cagattctct agggactgat cagaatctgc rttaaaccaa gctctccagg caactgatgc      60

t                                                                      61


<210>  982
<211>  61
<212>  DNA
<213>  Homo Sapiens

<400>  982
agatggaaag aatgctgagg gacccaaaga rcatcaaagc cgatcgctct aaaactcggc      60

g                                                                      61


<210>  983
<211>  61
<212>  DNA
<213>  Homo Sapiens

<400>  983
ttggttatgg ggccagcttg agaatccata ygtcctgact acctgcacca cttctccttt      60

t                                                                      61


<210>  984

<211>  61
<212>  DNA
<213>  Homo Sapiens

<400>  984
gaactctcca ggtttagaat atcttcaatt rtgggttcct tattattttt gtcttttttc        60

t                                                                         61


<210>  985
<211>  61
<212>  DNA
<213>  Homo Sapiens

<400>  985
cttccatggg tggaatcatt ggaggtccca rattaagagg tcatgtattc aatattcaat        60

a                                                                         61


<210>  986
<211>  61
<212>  DNA
<213>  Homo Sapiens

<400>  986
gtaatcaagc ccttctacca tttagttgaa ratgagagat tgggggattt gacctcataa        60

a                                                                         61


<210>  987
<211>  61
<212>  DNA
<213>  Homo Sapiens

<400>  987
ccatggcagt ggctctgata accaagacag stcctgaatg actaacaggg ctggtagcgt        60

c                                                                         61


<210>  988
<211>  61
<212>  DNA
<213>  Homo Sapiens

<400>  988
ataaattctg caagtgagtt taatgttcac raaaatcatc aacagcaaat gtagatgcat        60

g                                                                         61


<210>  989
<211>  61
<212>  DNA
<213>  Homo Sapiens

<400>  989
ttttaacttg aatatcaagc tcgctatgca rtttgactta tggagtgctg cgggaaacct        60

g                                                                         61

<210> 990
<211> 61
<212> DNA
<213> Homo Sapiens

<400> 990
cccaatttcc tgatttttat tcagcctgaa wcactgctca ctttgccagg gcagccctgt 60

g 61


<210> 991
<211> 61
<212> DNA
<213> Homo Sapiens

<400> 991
ccaatggcca ggccagtaac ccagcccagt kaaatatgaa tctaaggggg tgtgaaccta 60

g 61


<210> 992
<211> 61
<212> DNA
<213> Homo Sapiens

<400> 992
gggtttgaag tttcctccta aatctccgca ytgaatagga ctcaaggtta agtggcttct 60

t 61


<210> 993
<211> 61
<212> DNA
<213> Homo Sapiens

<400> 993
agttatacca gagctgaact ccttgaagga rgtgtgtagg tctctcaggg actgctcttg 60

g 61


<210> 994
<211> 61
<212> DNA
<213> Homo Sapiens

<400> 994
atccagcttt ttaagattta ccctgacaga ytgactctca cccaacaaaa gcttccgaca 60

t 61


<210> 995
<211> 61
<212> DNA
<213> Homo Sapiens

<400> 995

tggtgcaaaa ggaagttaca ggactaggga sttctgaatt atttatcttt ctccttccat 60

t 61

<210> 996
<211> 61
<212> DNA
<213> Homo Sapiens

<400> 996
gtcaaatgcc tccatggtac tcagtaatta rccacagatc tactctagca ctctacattc 60

a 61

<210> 997
<211> 61
<212> DNA
<213> Homo Sapiens

<400> 997
ctgtagactg aaatggcaca tctcccaaaa sttgtaggac taggcgaaat ccctgcaagt 60

g 61

<210> 998
<211> 61
<212> DNA
<213> Homo Sapiens

<400> 998
tgtcaccagc aaggtgtcaa gcctctttcg rgaacagcag tcgctgctgg actgcatcct 60

c 61

<210> 999
<211> 61
<212> DNA
<213> Homo Sapiens

<400> 999
gcctggccag gcgtgaacat tttaacagtc rtgaaagatt ccattgatgc gcctgtttta 60

c 61

<210> 1000
<211> 61
<212> DNA
<213> Homo Sapiens

<400> 1000
cagccctggg aagggaaggg aggtgctgtc staactctgg tcttacaaat ggactccagc 60

c 61

<210> 1001
<211> 61
<212> DNA

<213>  Homo Sapiens

<400>  1001
agtaaatcaa ggaatgactc caggactgat sgtaaggaca ccagtcacgt cctcccttga      60

c                                                                      61


<210>  1002
<211>  61
<212>  DNA
<213>  Homo Sapiens

<400>  1002
catggccacc ctgtcaatct tgagggccat rgagtgacat ttgggtgccc tgtggtgccc      60

a                                                                      61


<210>  1003
<211>  61
<212>  DNA
<213>  Homo Sapiens

<400>  1003
gtctttggag agacctcggg aagatgacct ytgaaccaca gctgctagaa tcctgggcca      60

t                                                                      61


<210>  1004
<211>  61
<212>  DNA
<213>  Homo Sapiens

<400>  1004
agatcagaaa tattcggagg aagaattgtg sctgtactga acatggacag attttttctt      60

g                                                                      61


<210>  1005
<211>  61
<212>  DNA
<213>  Homo Sapiens

<400>  1005
accacctcga atttggaaat actttcttca yttaacagag aggttgagta aattgcctca      60

g                                                                      61


<210>  1006
<211>  61
<212>  DNA
<213>  Homo Sapiens

<400>  1006
gaccgaaagc agtctgaaaa tgtctgaaaa ragtcggaaa atgtgtgtgt gtgtgtgtgt      60

g                                                                      61

<210>  1007
<211>  61
<212>  DNA
<213>  Homo Sapiens

<400>  1007
gtggggaggt ttacaaggag aaaaactgaa rtctctgcca acaaccctgt gtgtgagctt      60

a                                                                      61


<210>  1008
<211>  61
<212>  DNA
<213>  Homo Sapiens

<400>  1008
gattacagtc actctatgca ttaatgaaaa ytcggctttt gtatttttac agattatttt      60

g                                                                      61


<210>  1009
<211>  61
<212>  DNA
<213>  Homo Sapiens

<400>  1009
tacatgagtt aacaagacag cctgaggatc wttcaaagct cacaatatag atatatttta      60

c                                                                      61


<210>  1010
<211>  61
<212>  DNA
<213>  Homo Sapiens

<400>  1010
aactcccaca ttaaattttg caattgcccc ytaaaattac atagccagta cagttcctga      60

c                                                                      61


<210>  1011
<211>  61
<212>  DNA
<213>  Homo Sapiens

<400>  1011
cagaaaggct ggaaggaggg gacccaaaca rgaccttgaa ggttcattct ccattgagaa      60

g                                                                      61


<210>  1012
<211>  61
<212>  DNA
<213>  Homo Sapiens

<400>  1012
aaccaaggcc acacccctag caagaggacc kgctttcacc agtaactgaa gctgtgcaga      60

g                                                                     61

<210>  1013
<211>  61
<212>  DNA
<213>  Homo Sapiens

<400>  1013
agaactgctt gaacccaggg gttcaaggcc rcagtaagct aggatcacac cattgcattc      60

c                                                                     61

<210>  1014
<211>  61
<212>  DNA
<213>  Homo Sapiens

<400>  1014
tattgggtga caactctgtt tcaggtacta ygctagtctc ttcccacaag gacctcacag      60

c                                                                     61

<210>  1015
<211>  61
<212>  DNA
<213>  Homo Sapiens

<400>  1015
cctttgttgc cactcaagtc tagcagtctt maatatttgt cgaatgagtg aatgaagtca      60

a                                                                     61

<210>  1016
<211>  61
<212>  DNA
<213>  Homo Sapiens

<400>  1016
tgcaatgtgt ttggcattgt gataagctcc rtaaagatga tctcatttca tcttccctgc      60

a                                                                     61

<210>  1017
<211>  61
<212>  DNA
<213>  Homo Sapiens

<400>  1017
aggaggagaa cactgtgtga gcctttgatt ytttaatcgc aactgagata gtcctagagg      60

g                                                                     61

<210>  1018
<211>  61
<212>  DNA
<213>  Homo Sapiens

<400>  1018
atctcctcca atggagtagg cagtggtttt yttcaggcaa gacacagaaa gcaataacca      60

t                                                                      61


<210>  1019
<211>  61
<212>  DNA
<213>  Homo Sapiens

<400>  1019
gctccttaac tccactggct cgtcaaagta mctgtaggag caagagtaca acagtgactt      60

t                                                                      61


<210>  1020
<211>  61
<212>  DNA
<213>  Homo Sapiens

<400>  1020
aatacacagt gctaactagc agagtcacgc rtcggaagtg tgctgggccc ataaaataca      60

c                                                                      61


<210>  1021
<211>  61
<212>  DNA
<213>  Homo Sapiens

<400>  1021
cagtgagctg gacaacatgt tcgttcatct yaccaacgtc gccatccaga aacacggggt      60

g                                                                      61


<210>  1022
<211>  61
<212>  DNA
<213>  Homo Sapiens

<400>  1022
ccagtgagac gcagaaacgg atcctttgtc ygacttcctc cagattgcct ggggttggac      60

c                                                                      61


<210>  1023
<211>  61
<212>  DNA
<213>  Homo Sapiens

<400>  1023
tcttcagtga cgcagagtga tcttcagtgt rtctaaataa ctttgtgatg atgttttcct      60

t                                                                      61


<210>  1024
<211>  61

<212>  DNA
<213>  Homo Sapiens

<400>  1024
taccatctcc agctcttgaa aaactgctct mtaactcaac aaaaggagac accaaatcag      60

a                                                                      61


<210>  1025
<211>  61
<212>  DNA
<213>  Homo Sapiens

<400>  1025
cccagctcaa attcgtgacc agggggccta ygggacctgg tcgtcttttc agatggacag      60

t                                                                      61